# EUROPEAN PATENT APPLICATION

(11) **EP 2 546 268 A1**
(43) Date of publication of application: **16.01.2013**
(21) Application number: 11173784.7
(22) Date of filing: 13.07.2011
(51) Int. Cl.: C07K 16/28, C07K 16/22, C07K 16/46, C07K 19/00

(54) **Internalising immunoglobulin**

(71) Applicant: F-Star Biotechnologische Forschungs - und Entwicklungsges. M.B.H., 1230 Wien (AT)
(72) Inventor: The designation of the inventor has not yet been filed
(74) Representative: Maschio, Antonio

(57) **Abstract**

The disclosure relates to internalising modular antibody based on one or more immunoglobulin domains, which comprises at least one binding structure located within the N-terminal portion of a domain, and comprises at least one binding structure acting as a binding site located within the non-CDR region of a domain, wherein
a) a first binding structure has a specificity to bind an extra-cellular portion of a receptor on a tumor cell, and
b) a second binding structure has a specificity to bind a soluble tumor associated antigen (sTAG), and an Fc fragment of an immunoglobulin, which binds to an EGFR epitope.

SEQUENCE LISTING

<110>  f-star Biotechnologische Forschungs- und
       Entwicklungsges.m.b.H.

<120>  Internalising Immunoglobulin

<130>  FS015EP

<160>  748

<170>  PatentIn version 3.3

<210>  1
<211>  5
<212>  PRT
<213>  Artificial Sequence

<220>
<223>  mutant loop

<400>  1

Leu Thr Lys Asn Gln
1               5


<210>  2
<211>  5
<212>  PRT
<213>  Artificial Sequence

<220>
<223>  mutant loop

<400>  2

Asn Gly Gln Pro Glu
1               5


<210>  3
<211>  10
<212>  PRT
<213>  Artificial Sequence

<220>
<223>  mutant loop

<400>  3

Asp Lys Ser Arg Trp Gln Gln Gly Asn Val
1               5                   10


<210>  4
<211>  5
<212>  PRT
<213>  Artificial Sequence

<220>
<223>  mutant loop

<400>  4

Asn Asp Glu Gly Pro
1               5

<210>  5
<211>  5
<212>  PRT
<213>  Artificial Sequence

<220>
<223>  mutant loop

<400>  5

Asn Phe Gly Pro Glu
1               5


<210>  6
<211>  7
<212>  PRT
<213>  Artificial Sequence

<220>
<223>  mutant loop

<400>  6

Ser His Leu Arg Trp Thr Asn
1               5


<210>  7
<211>  7
<212>  PRT
<213>  Artificial Sequence

<220>
<223>  mutant loop

<400>  7

Ser His Leu Arg Trp Thr Ser
1               5


<210>  8

<400>  8
000

<210>  9
<211>  5
<212>  PRT
<213>  Artificial Sequence

<220>
<223>  mutant loop

<400>  9

Gly Asp Asp Gly Pro
1               5


<210>  10
<211>  5
<212>  PRT
<213>  Artificial Sequence

<220>

<223>  mutant loop

<400>  10

Gly Ala Glu Gly Pro
1               5


<210>  11
<211>  5
<212>  PRT
<213>  Artificial Sequence

<220>
<223>  mutant loop

<400>  11

Gly Pro Asn Gly Pro
1               5


<210>  12
<211>  5
<212>  PRT
<213>  Artificial Sequence

<220>
<223>  mutant loop

<400>  12

Gly Pro Glu Gly Pro
1               5


<210>  13
<211>  5
<212>  PRT
<213>  Artificial Sequence

<220>
<223>  mutant loop

<400>  13

Gly Glu Ala Gly Pro
1               5


<210>  14
<211>  5
<212>  PRT
<213>  Artificial Sequence

<220>
<223>  mutant loop

<400>  14

Gly Met Leu Gly Pro
1               5


<210>  15
<211>  5
<212>  PRT
<213>  Artificial Sequence

<220>
<223>  mutant loop

<400>  15

Gly Gln Ser Gly Pro
1               5


<210>  16
<211>  5
<212>  PRT
<213>  Artificial Sequence

<220>
<223>  mutant loop

<400>  16

Thr Gln Asp Gly Pro
1               5


<210>  17
<211>  5
<212>  PRT
<213>  Artificial Sequence

<220>
<223>  mutant loop

<400>  17

Thr Gln Leu Gly Pro
1               5


<210>  18
<211>  4
<212>  PRT
<213>  Artificial Sequence

<220>
<223>  mutant loop

<400>  18

Thr Asp Gly Pro
1


<210>  19
<211>  6
<212>  PRT
<213>  Artificial Sequence

<220>
<223>  mutant loop

<400>  19

Thr Asp Trp Asp Gly Pro
1               5


<210>  20
<211>  5

<212>  PRT
<213>  Artificial Sequence

<220>
<223>  mutant loop

<400>  20

Thr Gly Asp Gly Pro
1               5


<210>  21
<211>  5
<212>  PRT
<213>  Artificial Sequence

<220>
<223>  mutant loop

<400>  21

Thr Thr Glu Gly Pro
1               5


<210>  22
<211>  0
<212>  PRT
<213>  Artifical

<400>  22
<210>  23
<211>  5
<212>  PRT
<213>  Artificial Sequence

<220>
<223>  mutant loop

<400>  23

Thr Val Glu Gly Pro
1               5


<210>  24
<211>  6
<212>  PRT
<213>  Artificial Sequence

<220>
<223>  mutant loop

<400>  24

Thr Val Asp Gly Gly Pro
1               5


<210>  25
<211>  5
<212>  PRT
<213>  Artificial Sequence

<220>
<223>  mutant loop

<400>  25

Thr Glu Glu Gly Pro
1               5


<210>  26
<211>  5
<212>  PRT
<213>  Artificial Sequence

<220>
<223>  mutant loop

<400>  26

Thr Glu Ser Gly Pro
1               5


<210>  27
<211>  5
<212>  PRT
<213>  Artificial Sequence

<220>
<223>  mutant loop

<400>  27

Glu Asp Leu Asn His
1               5


<210>  28
<211>  5
<212>  PRT
<213>  Artificial Sequence

<220>
<223>  mutant loop

<400>  28

Thr Asn Ser Ile Gly
1               5


<210>  29
<211>  5
<212>  PRT
<213>  Artificial Sequence

<220>
<223>  mutant loop

<400>  29

Thr Asn Glu Gly Pro
1               5


<210>  30
<211>  5
<212>  PRT
<213>  Artificial Sequence

<220>

<223>  mutant loop

<400>  30

Ala Asn Glu Gly Pro
1               5


<210>  31
<211>  5
<212>  PRT
<213>  Artificial Sequence

<220>
<223>  mutant loop

<400>  31

Ile Ser Thr Gly Pro
1               5


<210>  32
<211>  5
<212>  PRT
<213>  Artificial Sequence

<220>
<223>  mutant loop

<400>  32

Tyr Gly Thr Gly Pro
1               5


<210>  33
<211>  5
<212>  PRT
<213>  Artificial Sequence

<220>
<223>  mutant loop

<400>  33

Ile Gly Glu Gly Pro
1               5


<210>  34
<211>  5
<212>  PRT
<213>  Artificial Sequence

<220>
<223>  mutant loop

<400>  34

Ile Lys Glu Gly Pro
1               5


<210>  35
<211>  5
<212>  PRT
<213>  Artificial Sequence

<220>
<223>  mutant loop

<400>  35

Ile Thr Glu Gly Pro
1               5


<210>  36
<211>  5
<212>  PRT
<213>  Artificial Sequence

<220>
<223>  mutant loop

<400>  36

Ile Glu Glu Gly Pro
1               5


<210>  37
<211>  5
<212>  PRT
<213>  Artificial Sequence

<220>
<223>  mutant loop

<400>  37

Gly Gly Glu Gly Pro
1               5


<210>  38
<211>  5
<212>  PRT
<213>  Artificial Sequence

<220>
<223>  mutant loop

<400>  38

Phe Gly Glu Gly Pro
1               5


<210>  39
<211>  5
<212>  PRT
<213>  Artificial Sequence

<220>
<223>  mutant loop

<400>  39

Gln Gly Glu Gly Pro
1               5


<210>  40
<211>  5

<212>  PRT
<213>  Artificial Sequence

<220>
<223>  mutant loop

<400>  40

Glu Gly Glu Gly Pro
1               5


<210>  41
<211>  5
<212>  PRT
<213>  Artificial Sequence

<220>
<223>  mutant loop

<400>  41

Met Gly Glu Gly Pro
1               5


<210>  42
<211>  5
<212>  PRT
<213>  Artificial Sequence

<220>
<223>  mutant loop

<400>  42

Val Val Glu Gly Pro
1               5


<210>  43
<211>  5
<212>  PRT
<213>  Artificial Sequence

<220>
<223>  mutant loop

<400>  43

Val Gly Glu Gly Pro
1               5


<210>  44
<211>  5
<212>  PRT
<213>  Artificial Sequence

<220>
<223>  mutant loop

<400>  44

Val Gly Glu Gly Pro
1               5

<210>  45
<211>  5
<212>  PRT
<213>  Artificial Sequence

<220>
<223>  mutant loop

<400>  45

Ser Glu Gln Gly Pro
1               5


<210>  46
<211>  5
<212>  PRT
<213>  Artificial Sequence

<220>
<223>  mutant loop

<400>  46

Ser Glu Glu Gly Pro
1               5


<210>  47
<211>  5
<212>  PRT
<213>  Artificial Sequence

<220>
<223>  mutant loop

<400>  47

Ser Glu Asn Gly Pro
1               5


<210>  48
<211>  5
<212>  PRT
<213>  Artificial Sequence

<220>
<223>  mutant loop

<400>  48

Ser Ile Asn Gly Pro
1               5


<210>  49
<211>  5
<212>  PRT
<213>  Artificial Sequence

<220>
<223>  mutant loop

<400>  49

Ser Gln Asp Gly Pro
1               5

<210>  50
<211>  5
<212>  PRT
<213>  Artificial Sequence

<220>
<223>  mutant loop

<400>  50

Ser Val Asp Gly Pro
1               5


<210>  51
<211>  7
<212>  PRT
<213>  Artificial

<220>
<223>  loop sequence

<400>  51

Thr Glu Ser Gly Pro Val Ser
1               5


<210>  52
<211>  5
<212>  PRT
<213>  Artificial Sequence

<220>
<223>  mutant loop

<400>  52

Tyr Glu Asp Gly Pro
1               5


<210>  53
<211>  5
<212>  PRT
<213>  Artificial Sequence

<220>
<223>  mutant loop

<400>  53

Ser Glu Asp Gly Pro
1               5


<210>  54
<211>  5
<212>  PRT
<213>  Artificial Sequence

<220>
<223>  mutant loop

<400>  54

Ser Lys Asp Gly Pro
1               5

<210>  55
<211>  5
<212>  PRT
<213>  Artificial Sequence

<220>
<223>  mutant loop

<400>  55

Ser Lys Ser Gly Pro
1               5

<210>  56
<211>  5
<212>  PRT
<213>  Artificial Sequence

<220>
<223>  mutant loop

<400>  56

Ser Ser Glu Gly Pro
1               5

<210>  57
<211>  5
<212>  PRT
<213>  Artificial Sequence

<220>
<223>  mutant loop

<400>  57

Asp Glu Glu Gly Pro
1               5

<210>  58
<211>  5
<212>  PRT
<213>  Artificial Sequence

<220>
<223>  mutant loop

<400>  58

Ile Glu Asp Gly Pro
1               5

<210>  59
<211>  5
<212>  PRT
<213>  Artificial Sequence

<220>
<223>  mutant loop

<400>  59

Glu Glu Asp Ala Pro
1               5


<210>  60
<211>  5
<212>  PRT
<213>  Artificial Sequence

<220>
<223>  mutant loop

<400>  60

Lys Glu Asp Gly Pro
1               5


<210>  61
<211>  5
<212>  PRT
<213>  Artificial Sequence

<220>
<223>  mutant loop

<400>  61

Asn Glu Asp Gly Pro
1               5


<210>  62
<211>  5
<212>  PRT
<213>  Artificial Sequence

<220>
<223>  mutant loop

<400>  62

Asp Glu Asp Gly Pro
1               5


<210>  63
<211>  5
<212>  PRT
<213>  Artificial Sequence

<220>
<223>  mutant loop

<400>  63

Asp Ser Asp Gly Pro
1               5


<210>  64
<211>  5
<212>  PRT
<213>  Artificial Sequence

<220>

<223>  mutant loop

<400>  64

Met Gln Asp Gly Pro
1               5


<210>  65
<211>  5
<212>  PRT
<213>  Artificial Sequence

<220>
<223>  mutant loop

<400>  65

Met Pro Asp Gly Pro
1               5


<210>  66
<211>  5
<212>  PRT
<213>  Artificial Sequence

<220>
<223>  mutant loop

<400>  66

Met Gln Glu Gly Pro
1               5


<210>  67
<211>  5
<212>  PRT
<213>  Artificial Sequence

<220>
<223>  mutant loop

<400>  67

Met Ser Gly Gly Pro
1               5


<210>  68
<211>  5
<212>  PRT
<213>  Artificial Sequence

<220>
<223>  mutant loop

<400>  68

Met Ser Asn Gly Pro
1               5


<210>  69
<211>  5
<212>  PRT
<213>  Artificial Sequence

<220>
<223>  mutant loop

<400>  69

Met Asn Gln Gly Pro
1               5


<210>  70
<211>  5
<212>  PRT
<213>  Artificial Sequence

<220>
<223>  mutant loop

<400>  70

Met Asn Gly Gly Pro
1               5


<210>  71
<211>  5
<212>  PRT
<213>  Artificial Sequence

<220>
<223>  mutant loop

<400>  71

Met Arg His Gly Thr
1               5


<210>  72
<211>  5
<212>  PRT
<213>  Artificial Sequence

<220>
<223>  mutant loop

<400>  72

Met Ala Ser Gly Pro
1               5


<210>  73
<211>  5
<212>  PRT
<213>  Artificial Sequence

<220>
<223>  mutant loop

<400>  73

Asp Ala Asn Gly Pro
1               5


<210>  74
<211>  5

<212>  PRT
<213>  Artificial Sequence

<220>
<223>  mutant loop

<400>  74

Gln Ala Asn Gly Pro
1               5


<210>  75
<211>  5
<212>  PRT
<213>  Artificial Sequence

<220>
<223>  mutant loop

<400>  75

Ile Ala Asn Gly Pro
1               5


<210>  76
<211>  5
<212>  PRT
<213>  Artificial Sequence

<220>
<223>  mutant loop

<400>  76

Tyr Asp Asn Gly Pro
1               5


<210>  77
<211>  5
<212>  PRT
<213>  Artificial Sequence

<220>
<223>  mutant loop

<400>  77

Phe Asp Asn Gly Pro
1               5


<210>  78
<211>  5
<212>  PRT
<213>  Artificial Sequence

<220>
<223>  mutant loop

<400>  78

Met Asp Asn Gly Pro
1               5

<210>  79
<211>  6
<212>  PRT
<213>  Artificial Sequence

<220>
<223>  mutant loop

<400>  79

Asn Thr Glu Gly Gly Pro
1               5


<210>  80
<211>  4
<212>  PRT
<213>  Artificial Sequence

<220>
<223>  mutant loop

<400>  80

Asn Gly Gly Pro
1


<210>  81
<211>  5
<212>  PRT
<213>  Artificial Sequence

<220>
<223>  mutant loop

<400>  81

Phe Glu Gly Gly Pro
1               5


<210>  82
<211>  4
<212>  PRT
<213>  Artificial Sequence

<220>
<223>  mutant loop

<400>  82

Phe Glu Gly Pro
1


<210>  83
<211>  5
<212>  PRT
<213>  Artificial Sequence

<220>
<223>  mutant loop

<400>  83

Ile Asp Gly Gly Pro
1               5

<210>  84
<211>  5
<212>  PRT
<213>  Artificial Sequence

<220>
<223>  mutant loop

<400>  84

Ile Asp Gly Gly Pro
1               5


<210>  85
<211>  5
<212>  PRT
<213>  Artificial Sequence

<220>
<223>  mutant loop

<400>  85

Leu Ser Gly Gly Pro
1               5


<210>  86
<211>  4
<212>  PRT
<213>  Artificial Sequence

<220>
<223>  mutant loop

<400>  86

Glu His Gly Pro
1


<210>  87
<211>  5
<212>  PRT
<213>  Artificial Sequence

<220>
<223>  mutant loop

<400>  87

Thr Asp Asn Gly Pro
1               5


<210>  88
<211>  4
<212>  PRT
<213>  Artificial Sequence

<220>
<223>  mutant loop

<400>  88

Thr Asp Gly Pro
1

<210> 89
<211> 5
<212> PRT
<213> Artificial Sequence

<220>
<223> mutant loop

<400> 89

Thr Asp Glu Thr Pro
1               5

<210> 90
<211> 5
<212> PRT
<213> Artificial Sequence

<220>
<223> mutant loop

<400> 90

Met Asp Asn Gly Pro
1               5

<210> 91
<211> 5
<212> PRT
<213> Artificial Sequence

<220>
<223> mutant loop

<400> 91

Gln Lys Asp Gly Pro
1               5

<210> 92
<211> 6
<212> PRT
<213> Artificial Sequence

<220>
<223> mutant loop

<400> 92

Gln Leu Lys Asp Glu Pro
1               5

<210> 93
<211> 5
<212> PRT
<213> Artificial Sequence

<220>
<223> mutant loop

<400>  93

Leu Asn Asp Gly Pro
1               5



<210>  94
<211>  5
<212>  PRT
<213>  Artificial Sequence

<220>
<223>  mutant loop

<400>  94

Glu Val Asp Gly Pro
1               5



<210>  95
<211>  5
<212>  PRT
<213>  Artificial Sequence

<220>
<223>  mutant loop

<400>  95

Asp Glu Asp Gly Pro
1               5



<210>  96
<211>  5
<212>  PRT
<213>  Artificial Sequence

<220>
<223>  mutant loop

<400>  96

Gly Gln Tyr Gly Pro
1               5



<210>  97
<211>  5
<212>  PRT
<213>  Artificial Sequence

<220>
<223>  mutant loop

<400>  97

Gly Ala Gln Gly Pro
1               5



<210>  98
<211>  5
<212>  PRT
<213>  Artificial Sequence

<220>

<223>  mutant loop

<400>  98

Gly Pro Asn Gly Pro
1               5


<210>  99
<211>  5
<212>  PRT
<213>  Artificial Sequence

<220>
<223>  mutant loop

<400>  99

Gly Pro Glu Gly Pro
1               5


<210>  100
<211>  6
<212>  PRT
<213>  Artificial Sequence

<220>
<223>  mutant loop

<400>  100

Val Phe Ala Gly Gln Pro
1               5


<210>  101
<211>  5
<212>  PRT
<213>  Artificial Sequence

<220>
<223>  mutant loop

<400>  101

Leu Ala Val Gly Pro
1               5


<210>  102
<211>  5
<212>  PRT
<213>  Artificial Sequence

<220>
<223>  mutant loop

<400>  102

Ile Ala Val Gly Pro
1               5


<210>  103
<211>  5
<212>  PRT
<213>  Artificial Sequence

<220>
<223>  mutant loop

<400>  103

His Ser Val Gly Pro
1               5


<210>  104
<211>  5
<212>  PRT
<213>  Artificial Sequence

<220>
<223>  mutant loop

<400>  104

Ile Glu Gln Gly Pro
1               5


<210>  105
<211>  5
<212>  PRT
<213>  Artificial Sequence

<220>
<223>  mutant loop

<400>  105

Ile Glu Glu Gly Pro
1               5


<210>  106
<211>  5
<212>  PRT
<213>  Artificial Sequence

<220>
<223>  mutant loop

<400>  106

Thr Glu Ser Gly Pro
1               5


<210>  107
<211>  4
<212>  PRT
<213>  Artificial Sequence

<220>
<223>  mutant loop

<400>  107

His Glu Gly Pro
1


<210>  108
<211>  5

<212>  PRT
<213>  Artificial Sequence

<220>
<223>  mutant loop

<400>  108

Trp Glu Gly Gly Pro
1               5


<210>  109
<211>  5
<212>  PRT
<213>  Artificial Sequence

<220>
<223>  mutant loop

<400>  109

Val Glu Gly Gly Pro
1               5


<210>  110
<211>  5
<212>  PRT
<213>  Artificial Sequence

<220>
<223>  mutant loop

<400>  110

Ile Gly Ser Gly Pro
1               5


<210>  111
<211>  5
<212>  PRT
<213>  Artificial Sequence

<220>
<223>  mutant loop

<400>  111

Ile Gly Asn Gly Pro
1               5


<210>  112
<211>  5
<212>  PRT
<213>  Artificial Sequence

<220>
<223>  mutant loop

<400>  112

Ile Asn His Gly Pro
1               5

<210>  113
<211>  4
<212>  PRT
<213>  Artificial Sequence

<220>
<223>  mutant loop

<400>  113

Asn Gly Gly Pro
1

<210>  114
<211>  5
<212>  PRT
<213>  Artificial Sequence

<220>
<223>  mutant loop

<400>  114

Val Asn Glu Gly Pro
1               5

<210>  115
<211>  5
<212>  PRT
<213>  Artificial Sequence

<220>
<223>  mutant loop

<400>  115

Asp Asn Glu Gly Pro
1               5

<210>  116
<211>  5
<212>  PRT
<213>  Artificial Sequence

<220>
<223>  mutant loop

<400>  116

Phe Gly Glu Gly Pro
1               5

<210>  117
<211>  5
<212>  PRT
<213>  Artificial Sequence

<220>
<223>  mutant loop

<400>  117

Ser Gly Glu Gly Pro
1               5

<210>  118
<211>  5
<212>  PRT
<213>  Artificial Sequence

<220>
<223>  mutant loop

<400>  118

Glu Gly Glu Gly Pro
1               5


<210>  119
<211>  5
<212>  PRT
<213>  Artificial Sequence

<220>
<223>  mutant loop

<400>  119

Met Gln His Gly Pro
1               5


<210>  120
<211>  5
<212>  PRT
<213>  Artificial Sequence

<220>
<223>  mutant loop

<400>  120

Met Gln Asp Gly Pro
1               5


<210>  121
<211>  5
<212>  PRT
<213>  Artificial Sequence

<220>
<223>  mutant loop

<400>  121

Met Gln Glu Gly Pro
1               5


<210>  122
<211>  5
<212>  PRT
<213>  Artificial Sequence

<220>
<223>  mutant loop

<400>  122

Met Lys Glu Gly Pro
1               5


<210>  123
<211>  5
<212>  PRT
<213>  Artificial Sequence

<220>
<223>  mutant loop

<400>  123

Met Lys Glu Gly Pro
1               5


<210>  124
<211>  5
<212>  PRT
<213>  Artificial Sequence

<220>
<223>  mutant loop

<400>  124

Met Thr Glu Gly Pro
1               5


<210>  125
<211>  5
<212>  PRT
<213>  Artificial Sequence

<220>
<223>  mutant loop

<400>  125

Met Asn Gln Gly Pro
1               5


<210>  126
<211>  5
<212>  PRT
<213>  Artificial Sequence

<220>
<223>  mutant loop

<400>  126

Met Asn Asp Gly Pro
1               5


<210>  127
<211>  5
<212>  PRT
<213>  Artificial Sequence

<220>
<223>  mutant loop

<400>  127

Met Leu Gly Gly Pro
1               5


<210>  128
<211>  4
<212>  PRT
<213>  Artificial Sequence

<220>
<223>  mutant loop

<400>  128

Leu Gly Gly Pro
1


<210>  129
<211>  5
<212>  PRT
<213>  Artificial Sequence

<220>
<223>  mutant loop

<400>  129

Asn Ser Asp Phe Gly
1               5


<210>  130
<211>  5
<212>  PRT
<213>  Artificial Sequence

<220>
<223>  mutant loop

<400>  130

Ser Asp Thr Ile Gly
1               5


<210>  131
<211>  7
<212>  PRT
<213>  Artificial

<220>
<223>  loop sequence

<400>  131

Ser Glu Ser Gly Pro Val Ser
1               5


<210>  132
<211>  7
<212>  PRT
<213>  Artificial

<220>

<223>  loop sequence

<400>  132

Thr Ile Ser Gly Pro Val Ser
1               5


<210>  133
<211>  7
<212>  PRT
<213>  Artificial

<220>
<223>  loop sequence

<400>  133

Thr Glu Ser Gly Pro Val Thr
1               5


<210>  134
<211>  7
<212>  PRT
<213>  Artificial

<220>
<223>  loop sequence

<400>  134

Thr Glu Phe Gly Pro Val Thr
1               5


<210>  135
<211>  5
<212>  PRT
<213>  Artificial Sequence

<220>
<223>  mutant loop

<400>  135

Thr Glu Ser Gly Pro
1               5


<210>  136
<211>  7
<212>  PRT
<213>  Artificial Sequence

<220>
<223>  mutant loop

<400>  136

Ala Ser Tyr Arg Trp Thr Ser
1               5


<210>  137
<211>  7
<212>  PRT
<213>  Artificial Sequence

<220>
<223>  mutant loop

<400>  137

Ala Thr Tyr Arg Trp Met Gly
1               5


<210>  138
<211>  7
<212>  PRT
<213>  Artificial Sequence

<220>
<223>  mutant loop

<400>  138

Phe Glu Tyr Arg Trp Ala Leu
1               5


<210>  139
<211>  7
<212>  PRT
<213>  Artificial Sequence

<220>
<223>  mutant loop

<400>  139

Met Pro Tyr Arg Trp Pro Pro
1               5


<210>  140
<211>  7
<212>  PRT
<213>  Artificial Sequence

<220>
<223>  mutant loop

<400>  140

Ser Ala Ala Arg Trp Glu Arg
1               5


<210>  141
<211>  7
<212>  PRT
<213>  Artificial Sequence

<220>
<223>  mutant loop

<400>  141

Ser Ala His Arg Trp Lys Ser
1               5


<210>  142
<211>  7

<212>  PRT
<213>  Artificial Sequence

<220>
<223>  mutant loop

<400>  142

Ser Ala His Arg Trp Ser Ser
1               5


<210>  143
<211>  7
<212>  PRT
<213>  Artificial Sequence

<220>
<223>  mutant loop

<400>  143

Ser Ala Lys Arg Trp Lys Ser
1               5


<210>  144
<211>  7
<212>  PRT
<213>  Artificial Sequence

<220>
<223>  mutant loop

<400>  144

Ser Ala Leu Arg Trp Glu His
1               5


<210>  145
<211>  7
<212>  PRT
<213>  Artificial Sequence

<220>
<223>  mutant loop

<400>  145

Ser Ala Leu Arg Trp Met Gln
1               5


<210>  146
<211>  7
<212>  PRT
<213>  Artificial Sequence

<220>
<223>  mutant loop

<400>  146

Ser Ala Met Arg Trp Glu Lys
1               5

<210>  147
<211>  7
<212>  PRT
<213>  Artificial Sequence

<220>
<223>  mutant loop

<400>  147

Ser Ala Asn Arg Trp Glu Ser
1               5


<210>  148
<211>  7
<212>  PRT
<213>  Artificial Sequence

<220>
<223>  mutant loop

<400>  148

Ser Ala Gln Arg Trp Lys Tyr
1               5


<210>  149
<211>  7
<212>  PRT
<213>  Artificial Sequence

<220>
<223>  mutant loop

<400>  149

Ser Ala Gln Arg Trp Val Thr
1               5


<210>  150
<211>  7
<212>  PRT
<213>  Artificial Sequence

<220>
<223>  mutant loop

<400>  150

Ser Ala Arg Arg Trp Ala Ser
1               5


<210>  151
<211>  7
<212>  PRT
<213>  Artificial Sequence

<220>
<223>  mutant loop

<400>  151

Ser Ala Arg Arg Trp Glu Glu
1               5

<210>  152
<211>  7
<212>  PRT
<213>  Artificial Sequence

<220>
<223>  mutant loop

<400>  152

Ser Ala Ser Arg Trp Arg Phe
1               5


<210>  153
<211>  7
<212>  PRT
<213>  Artificial Sequence

<220>
<223>  mutant loop

<400>  153

Ser Ala Tyr Arg Trp Glu Ala
1               5


<210>  154
<211>  7
<212>  PRT
<213>  Artificial Sequence

<220>
<223>  mutant loop

<400>  154

Ser Ala Tyr Arg Trp Gln Gln
1               5


<210>  155
<211>  7
<212>  PRT
<213>  Artificial Sequence

<220>
<223>  mutant loop

<400>  155

Ser Asp Tyr Arg Trp Gln Ser
1               5


<210>  156
<211>  7
<212>  PRT
<213>  Artificial Sequence

<220>
<223>  mutant loop

<400>  156

Ser Glu Met Arg Trp His Ser
1               5

<210>  157
<211>  7
<212>  PRT
<213>  Artificial Sequence

<220>
<223>  mutant loop

<400>  157

Ser Glu Met Arg Trp Ile Lys
1               5

<210>  158
<211>  7
<212>  PRT
<213>  Artificial Sequence

<220>
<223>  mutant loop

<400>  158

Ser Glu Met Arg Trp Arg Lys
1               5

<210>  159
<211>  7
<212>  PRT
<213>  Artificial Sequence

<220>
<223>  mutant loop

<400>  159

Ser Glu Arg Arg Trp Ser Gln
1               5

<210>  160
<211>  7
<212>  PRT
<213>  Artificial Sequence

<220>
<223>  mutant loop

<400>  160

Ser Glu Tyr Arg Trp Arg Ser
1               5

<210>  161
<211>  7
<212>  PRT
<213>  Artificial Sequence

<220>
<223>  mutant loop

<400>  161

Ser Gly Ala Arg Trp Lys Ala
1               5


<210>  162
<211>  7
<212>  PRT
<213>  Artificial Sequence

<220>
<223>  mutant loop

<400>  162

Ser Gly Met Arg Trp Gln Lys
1               5


<210>  163
<211>  7
<212>  PRT
<213>  Artificial Sequence

<220>
<223>  mutant loop

<400>  163

Ser His Arg Arg Trp Phe Gln
1               5


<210>  164
<211>  7
<212>  PRT
<213>  Artificial Sequence

<220>
<223>  mutant loop

<400>  164

Ser His Arg Arg Trp His Ser
1               5


<210>  165
<211>  7
<212>  PRT
<213>  Artificial Sequence

<220>
<223>  mutant loop

<400>  165

Ser His Arg Arg Trp Met Arg
1               5


<210>  166
<211>  7
<212>  PRT
<213>  Artificial Sequence

<220>

<223>  mutant loop

<400>  166

Ser His Thr Arg Trp His Ser
1               5


<210>  167
<211>  7
<212>  PRT
<213>  Artificial Sequence

<220>
<223>  mutant loop

<400>  167

Ser Lys Ala Arg Trp Ser Gln
1               5


<210>  168
<211>  7
<212>  PRT
<213>  Artificial Sequence

<220>
<223>  mutant loop

<400>  168

Ser Lys Met Arg Trp Leu Gln
1               5


<210>  169
<211>  7
<212>  PRT
<213>  Artificial Sequence

<220>
<223>  mutant loop

<400>  169

Ser Lys Tyr Arg Trp Met Ala
1               5


<210>  170
<211>  7
<212>  PRT
<213>  Artificial Sequence

<220>
<223>  mutant loop

<400>  170

Ser Met Arg Arg Trp Thr Arg
1               5


<210>  171
<211>  7
<212>  PRT
<213>  Artificial Sequence

<220>
<223>  mutant loop

<400>  171

Ser Asn Phe Arg Trp Asn Asn
1               5


<210>  172
<211>  7
<212>  PRT
<213>  Artificial Sequence

<220>
<223>  mutant loop

<400>  172

Ser Asn His Arg Trp Trp Ser
1               5


<210>  173
<211>  7
<212>  PRT
<213>  Artificial Sequence

<220>
<223>  mutant loop

<400>  173

Ser Asn Ile Arg Trp Arg Gln
1               5


<210>  174
<211>  7
<212>  PRT
<213>  Artificial Sequence

<220>
<223>  mutant loop

<400>  174

Ser Asn Met Arg Trp Ser Thr
1               5


<210>  175
<211>  7
<212>  PRT
<213>  Artificial Sequence

<220>
<223>  mutant loop

<400>  175

Ser Asn Arg Arg Trp His Thr
1               5


<210>  176
<211>  7

<212>  PRT
<213>  Artificial Sequence

<220>
<223>  mutant loop

<400>  176

Ser Asn Arg Arg Trp Met Ala
1               5


<210>  177
<211>  7
<212>  PRT
<213>  Artificial Sequence

<220>
<223>  mutant loop

<400>  177

Ser Asn Arg Arg Trp Arg His
1               5


<210>  178
<211>  7
<212>  PRT
<213>  Artificial Sequence

<220>
<223>  mutant loop

<400>  178

Ser Asn Thr Arg Trp Phe Lys
1               5


<210>  179
<211>  7
<212>  PRT
<213>  Artificial Sequence

<220>
<223>  mutant loop

<400>  179

Ser Asn Val Arg Trp Ala Arg
1               5


<210>  180
<211>  7
<212>  PRT
<213>  Artificial Sequence

<220>
<223>  mutant loop

<400>  180

Ser Asn Trp Arg Trp Lys Arg
1               5

<210>  181
<211>  7
<212>  PRT
<213>  Artificial Sequence

<220>
<223>  mutant loop

<400>  181

Ser Pro Arg Arg Trp Ser Asn
1               5


<210>  182
<211>  7
<212>  PRT
<213>  Artificial Sequence

<220>
<223>  mutant loop

<400>  182

Ser Pro Tyr Arg Trp Glu Ser
1               5


<210>  183
<211>  7
<212>  PRT
<213>  Artificial Sequence

<220>
<223>  mutant loop

<400>  183

Ser Gln Ala Arg Trp Gln Arg
1               5


<210>  184
<211>  7
<212>  PRT
<213>  Artificial Sequence

<220>
<223>  mutant loop

<400>  184

Ser Gln Ala Arg Trp Thr Ser
1               5


<210>  185
<211>  7
<212>  PRT
<213>  Artificial Sequence

<220>
<223>  mutant loop

<400>  185

Ser Gln His Arg Trp Thr Ser
1               5

<210>  186
<211>  7
<212>  PRT
<213>  Artificial Sequence

<220>
<223>  mutant loop

<400>  186

Ser Gln Leu Arg Trp Gln Ser
1               5


<210>  187
<211>  7
<212>  PRT
<213>  Artificial Sequence

<220>
<223>  mutant loop

<400>  187

Ser Gln Leu Arg Trp Ser Arg
1               5


<210>  188
<211>  7
<212>  PRT
<213>  Artificial Sequence

<220>
<223>  mutant loop

<400>  188

Ser Gln Met Arg Trp His Gln
1               5


<210>  189
<211>  7
<212>  PRT
<213>  Artificial Sequence

<220>
<223>  mutant loop

<400>  189

Ser Gln Met Arg Trp Thr Arg
1               5


<210>  190
<211>  7
<212>  PRT
<213>  Artificial Sequence

<220>
<223>  mutant loop

<400>  190

Ser Gln Ser Arg Trp Ser His
1               5

<210>  191
<211>  7
<212>  PRT
<213>  Artificial Sequence

<220>
<223>  mutant loop

<400>  191

Ser Arg Phe Arg Trp Gln Glu
1               5

<210>  192
<211>  7
<212>  PRT
<213>  Artificial Sequence

<220>
<223>  mutant loop

<400>  192

Ser Arg His Arg Trp Ser Thr
1               5

<210>  193
<211>  7
<212>  PRT
<213>  Artificial Sequence

<220>
<223>  mutant loop

<400>  193

Ser Arg Met Arg Trp Glu Ser
1               5

<210>  194
<211>  7
<212>  PRT
<213>  Artificial Sequence

<220>
<223>  mutant loop

<400>  194

Ser Arg Gln Arg Trp Gln Ser
1               5

<210>  195
<211>  7
<212>  PRT
<213>  Artificial Sequence

<220>
<223>  mutant loop

<400>  195

Ser Arg Arg Arg Trp His Ser
1               5


<210>  196
<211>  7
<212>  PRT
<213>  Artificial Sequence

<220>
<223>  mutant loop

<400>  196

Ser Ser Phe Arg Trp Glu Ser
1               5


<210>  197
<211>  7
<212>  PRT
<213>  Artificial Sequence

<220>
<223>  mutant loop

<400>  197

Ser Ser Met Arg Trp Ala Thr
1               5


<210>  198
<211>  7
<212>  PRT
<213>  Artificial Sequence

<220>
<223>  mutant loop

<400>  198

Ser Ser Met Arg Trp Leu Gln
1               5


<210>  199
<211>  7
<212>  PRT
<213>  Artificial Sequence

<220>
<223>  mutant loop

<400>  199

Ser Ser Asn Arg Trp Glu Thr
1               5


<210>  200
<211>  7
<212>  PRT
<213>  Artificial Sequence

<220>

<223>  mutant loop

<400>  200

Ser Ser Gln Arg Trp Lys Arg
1               5


<210>  201
<211>  7
<212>  PRT
<213>  Artificial Sequence

<220>
<223>  mutant loop

<400>  201

Ser Ser Gln Arg Trp Leu Ser
1               5


<210>  202
<211>  7
<212>  PRT
<213>  Artificial Sequence

<220>
<223>  mutant loop

<400>  202

Ser Ser Gln Arg Trp Thr Arg
1               5


<210>  203
<211>  7
<212>  PRT
<213>  Artificial Sequence

<220>
<223>  mutant loop

<400>  203

Ser Ser Arg Arg Trp Ser Ala
1               5


<210>  204
<211>  7
<212>  PRT
<213>  Artificial Sequence

<220>
<223>  mutant loop

<400>  204

Ser Ser Tyr Arg Trp Glu Lys
1               5


<210>  205
<211>  7
<212>  PRT
<213>  Artificial Sequence

<220>
<223>  mutant loop

<400>  205

Ser Ser Tyr Arg Trp Met Gln
1               5


<210>  206
<211>  7
<212>  PRT
<213>  Artificial Sequence

<220>
<223>  mutant loop

<400>  206

Ser Thr His Arg Trp Ala Thr
1               5


<210>  207
<211>  7
<212>  PRT
<213>  Artificial Sequence

<220>
<223>  mutant loop

<400>  207

Ser Thr Arg Arg Trp Ala Asn
1               5


<210>  208
<211>  7
<212>  PRT
<213>  Artificial Sequence

<220>
<223>  mutant loop

<400>  208

Ser Thr Arg Arg Trp Lys Thr
1               5


<210>  209
<211>  7
<212>  PRT
<213>  Artificial Sequence

<220>
<223>  mutant loop

<400>  209

Ser Thr Arg Arg Trp Thr Ser
1               5


<210>  210
<211>  7

<212>  PRT
<213>  Artificial Sequence

<220>
<223>  mutant loop

<400>  210

Ser Thr Tyr Arg Trp Glu Ala
1               5


<210>  211
<211>  7
<212>  PRT
<213>  Artificial Sequence

<220>
<223>  mutant loop

<400>  211

Ser Val His Arg Trp Leu Ala
1               5


<210>  212
<211>  7
<212>  PRT
<213>  Artificial Sequence

<220>
<223>  mutant loop

<400>  212

Ser Val Gln Arg Trp Arg Ser
1               5


<210>  213
<211>  7
<212>  PRT
<213>  Artificial Sequence

<220>
<223>  mutant loop

<400>  213

Ser Trp His Arg Trp Asn Ser
1               5


<210>  214
<211>  7
<212>  PRT
<213>  Artificial Sequence

<220>
<223>  mutant loop

<400>  214

Ser Trp Trp Arg Trp Lys Lys
1               5

<210>  215
<211>  7
<212>  PRT
<213>  Artificial Sequence

<220>
<223>  mutant loop

<400>  215

Ser Trp Tyr Arg Trp Lys Ser
1               5


<210>  216
<211>  7
<212>  PRT
<213>  Artificial Sequence

<220>
<223>  mutant loop

<400>  216

Ser Tyr His Arg Trp Met Thr
1               5


<210>  217
<211>  7
<212>  PRT
<213>  Artificial Sequence

<220>
<223>  mutant loop

<400>  217

Ser Tyr Tyr Arg Trp Arg Ala
1               5


<210>  218
<211>  7
<212>  PRT
<213>  Artificial Sequence

<220>
<223>  mutant loop

<400>  218

Ser Gln Ala Arg Trp Lys Lys
1               5


<210>  219
<211>  7
<212>  PRT
<213>  Artificial Sequence

<220>
<223>  mutant loop

<400>  219

Ser Asn Ala Arg Trp Phe Lys
1               5

<210>  220
<211>  7
<212>  PRT
<213>  Artificial Sequence

<220>
<223>  mutant loop

<400>  220

Ser Thr Ser Arg Trp Lys Ser
1               5


<210>  221
<211>  7
<212>  PRT
<213>  Artificial Sequence

<220>
<223>  mutant loop

<400>  221

Ser His Ser Arg Trp Arg Ser
1               5


<210>  222
<211>  7
<212>  PRT
<213>  Artificial Sequence

<220>
<223>  mutant loop

<400>  222

Ser Asn Tyr Arg Trp Gln Ser
1               5


<210>  223
<211>  7
<212>  PRT
<213>  Artificial Sequence

<220>
<223>  mutant loop

<400>  223

Ser Thr Tyr Arg Trp Gln Ser
1               5


<210>  224
<211>  7
<212>  PRT
<213>  Artificial Sequence

<220>
<223>  mutant loop

<400>  224

Ser Asn His Arg Trp His Ser
1               5

<210>  225
<211>  7
<212>  PRT
<213>  Artificial Sequence

<220>
<223>  mutant loop

<400>  225

Ser Asn Arg Arg Trp Glu His
1               5

<210>  226
<211>  7
<212>  PRT
<213>  Artificial Sequence

<220>
<223>  mutant loop

<400>  226

Ser Asn Arg Arg Trp Glu Ser
1               5

<210>  227
<211>  7
<212>  PRT
<213>  Artificial Sequence

<220>
<223>  mutant loop

<400>  227

Ser Asn Arg Arg Trp Ala Ser
1               5

<210>  228
<211>  7
<212>  PRT
<213>  Artificial Sequence

<220>
<223>  mutant loop

<400>  228

Ser Asn Arg Arg Trp Met Ser
1               5

<210>  229
<211>  7
<212>  PRT
<213>  Artificial Sequence

<220>
<223>  mutant loop

<400>  229

Ser Asn Arg Arg Trp Met Ser
1               5


<210>  230
<211>  7
<212>  PRT
<213>  Artificial Sequence

<220>
<223>  mutant loop

<400>  230

Ser Gln Arg Arg Trp Thr Ser
1               5


<210>  231
<211>  7
<212>  PRT
<213>  Artificial Sequence

<220>
<223>  mutant loop

<400>  231

Ser Gln Arg Arg Trp Glu Ser
1               5


<210>  232
<211>  7
<212>  PRT
<213>  Artificial Sequence

<220>
<223>  mutant loop

<400>  232

Ser Arg Arg Arg Trp Glu Glu
1               5


<210>  233
<211>  7
<212>  PRT
<213>  Artificial Sequence

<220>
<223>  mutant loop

<400>  233

Ser Glu Arg Arg Trp Ser Thr
1               5


<210>  234
<211>  7
<212>  PRT
<213>  Artificial Sequence

<220>

<223>  mutant loop

<400>  234

Ser Gln Gln Arg Trp Ala Ser
1               5


<210>  235
<211>  7
<212>  PRT
<213>  Artificial Sequence

<220>
<223>  mutant loop

<400>  235

Ser Ser Gln Arg Trp Ala Lys
1               5


<210>  236
<211>  7
<212>  PRT
<213>  Artificial Sequence

<220>
<223>  mutant loop

<400>  236

Ser Gln Met Arg Trp Glu Gln
1               5


<210>  237
<211>  7
<212>  PRT
<213>  Artificial Sequence

<220>
<223>  mutant loop

<400>  237

Ser Gln Gln Arg Trp His Gln
1               5


<210>  238
<211>  7
<212>  PRT
<213>  Artificial Sequence

<220>
<223>  mutant loop

<400>  238

Ser Gln Arg Arg Trp Ala Gln
1               5


<210>  239
<211>  7
<212>  PRT
<213>  Artificial Sequence

<220>
<223>  mutant loop

<400>  239

Gln Thr Met Arg Trp Glu Trp
1               5


<210>  240
<211>  7
<212>  PRT
<213>  Artificial Sequence

<220>
<223>  mutant loop

<400>  240

Ser Ser Met Arg Trp Glu Ser
1               5


<210>  241
<211>  7
<212>  PRT
<213>  Artificial Sequence

<220>
<223>  mutant loop

<400>  241

Ser Thr His Arg Trp Ser Ala
1               5


<210>  242
<211>  7
<212>  PRT
<213>  Artificial Sequence

<220>
<223>  mutant loop

<400>  242

Ser Arg Asn Arg Trp Asp Ala
1               5


<210>  243
<211>  7
<212>  PRT
<213>  Artificial Sequence

<220>
<223>  mutant loop

<400>  243

Ser His His Arg Trp Val Arg
1               5


<210>  244
<211>  7

<212>  PRT
<213>  Artificial Sequence

<220>
<223>  mutant loop

<400>  244

Ser His Met Arg Trp Gln Thr
1               5


<210>  245
<211>  7
<212>  PRT
<213>  Artificial Sequence

<220>
<223>  mutant loop

<400>  245

Ser Arg Ala Arg Trp Asn Ser
1               5


<210>  246
<211>  7
<212>  PRT
<213>  Artificial Sequence

<220>
<223>  mutant loop

<400>  246

Ser Asn Leu Arg Trp Thr Lys
1               5


<210>  247
<211>  10
<212>  PRT
<213>  Artificial Sequence

<220>
<223>  mutant loop

<400>  247

Ser Thr Tyr Arg Trp Gln Ser Gly Asn Val
1               5                   10


<210>  248
<211>  10
<212>  PRT
<213>  Artificial Sequence

<220>
<223>  mutant loop

<400>  248

Ser Val Tyr Arg Trp Met Ser Gly Asn Val
1               5                   10

<210>  249
<211>  10
<212>  PRT
<213>  Artificial Sequence

<220>
<223>  mutant loop

<400>  249

Ser Asn Phe Arg Trp Asn Ser Gly Asn Val
1               5                   10


<210>  250
<211>  10
<212>  PRT
<213>  Artificial Sequence

<220>
<223>  mutant loop

<400>  250

Ser Asn Leu Arg Trp Thr Lys Gly His Val
1               5                   10


<210>  251
<211>  7
<212>  PRT
<213>  Artificial Sequence

<220>
<223>  mutant loop

<400>  251

Cys Glu Ser Gly Pro Val Thr
1               5


<210>  252
<211>  7
<212>  PRT
<213>  Artificial Sequence

<220>
<223>  mutant loop

<400>  252

Thr Glu Ser Gly Pro Val Thr
1               5


<210>  253
<211>  7
<212>  PRT
<213>  Artificial Sequence

<220>
<223>  mutant loop

<400>  253

Thr Ala Pro Gly Pro Val Thr
1               5

<210>  254
<211>  7
<212>  PRT
<213>  Artificial Sequence

<220>
<223>  mutant loop

<400>  254

Thr Asp Ser Gly Pro Val Thr
1               5


<210>  255
<211>  7
<212>  PRT
<213>  Artificial Sequence

<220>
<223>  mutant loop

<400>  255

Asp Asp Ser Gly Pro Val Thr
1               5


<210>  256
<211>  7
<212>  PRT
<213>  Artificial Sequence

<220>
<223>  mutant loop

<400>  256

Thr Gly Ser Gly Pro Val Thr
1               5


<210>  257
<211>  7
<212>  PRT
<213>  Artificial Sequence

<220>
<223>  mutant loop

<400>  257

Thr Tyr Ser Gly Pro Val Thr
1               5


<210>  258
<211>  7
<212>  PRT
<213>  Artificial Sequence

<220>
<223>  mutant loop

<400>  258

Thr Asp Ser Gly Pro Val Trp
1               5


<210>  259
<211>  7
<212>  PRT
<213>  Artificial Sequence

<220>
<223>  mutant loop

<400>  259

Thr Glu Ser Gly Pro Val Trp
1               5


<210>  260
<211>  7
<212>  PRT
<213>  Artificial Sequence

<220>
<223>  mutant loop

<400>  260

Asn Glu Ser Gly Pro Val Trp
1               5


<210>  261
<211>  7
<212>  PRT
<213>  Artificial Sequence

<220>
<223>  mutant loop

<400>  261

Thr Asp Leu Gly Pro Val Ser
1               5


<210>  262
<211>  7
<212>  PRT
<213>  Artificial Sequence

<220>
<223>  mutant loop

<400>  262

Thr Glu Ser Gly Pro Val Trp
1               5


<210>  263
<211>  7
<212>  PRT
<213>  Artificial Sequence

<220>
<223>  mutant loop

<400>  263

Met Asp Ser Gly Pro Val Ser
1               5


<210>  264
<211>  7
<212>  PRT
<213>  Artificial Sequence

<220>
<223>  mutant loop

<400>  264

Phe Glu Ser Gly Pro Val Ser
1               5


<210>  265
<211>  7
<212>  PRT
<213>  Artificial Sequence

<220>
<223>  mutant loop

<400>  265

Thr Asp Ser Gly Pro Val Ser
1               5


<210>  266
<211>  7
<212>  PRT
<213>  Artificial Sequence

<220>
<223>  mutant loop

<400>  266

Thr Asp Ala Gly Pro Val Ser
1               5


<210>  267
<211>  7
<212>  PRT
<213>  Artificial Sequence

<220>
<223>  mutant loop

<400>  267

Thr Ala Ser Gly Pro Val Ser
1               5


<210>  268
<211>  7
<212>  PRT
<213>  Artificial Sequence

<220>

<223>  mutant loop

<400>  268

Thr Gln Ser Gly Pro Val Ser
1               5


<210>  269
<211>  7
<212>  PRT
<213>  Artificial Sequence

<220>
<223>  mutant loop

<400>  269

Thr Glu Ser Gly Pro Val Ser
1               5


<210>  270
<211>  7
<212>  PRT
<213>  Artificial Sequence

<220>
<223>  mutant loop

<400>  270

Met Gln Ser Gly Pro Val Ser
1               5


<210>  271
<211>  7
<212>  PRT
<213>  Artificial Sequence

<220>
<223>  mutant loop

<400>  271

Met Glu Ser Gly Pro Val Ser
1               5


<210>  272
<211>  7
<212>  PRT
<213>  Artificial Sequence

<220>
<223>  mutant loop

<400>  272

Ile Glu Ser Gly Pro Val Ser
1               5


<210>  273
<211>  7
<212>  PRT
<213>  Artificial Sequence

<220>
<223>  mutant loop

<400>  273

Leu Glu Ser Gly Pro Val Ser
1               5


<210>  274
<211>  7
<212>  PRT
<213>  Artificial Sequence

<220>
<223>  mutant loop

<400>  274

Ser Glu Ser Gly Pro Val Ser
1               5


<210>  275
<211>  7
<212>  PRT
<213>  Artificial Sequence

<220>
<223>  mutant loop

<400>  275

Thr Asp Ser Gly Pro Val Thr
1               5


<210>  276
<211>  7
<212>  PRT
<213>  Artificial Sequence

<220>
<223>  mutant loop

<400>  276

Leu Asp Ser Gly Pro Val Thr
1               5


<210>  277
<211>  7
<212>  PRT
<213>  Artificial Sequence

<220>
<223>  mutant loop

<400>  277

Thr Glu Ser Gly Pro Val Thr
1               5


<210>  278
<211>  7

<212>  PRT
<213>  Artificial Sequence

<220>
<223>  mutant loop

<400>  278

Thr Glu Ser Gly Pro Leu Cys
1               5


<210>  279
<211>  5
<212>  PRT
<213>  Artificial Sequence

<220>
<223>  mutant loop

<400>  279

Thr Glu Ser Gly Pro
1               5


<210>  280
<211>  6
<212>  PRT
<213>  Artificial Sequence

<220>
<223>  mutant loop

<400>  280

Ile Arg Ala Gly Glu Pro
1               5


<210>  281
<211>  6
<212>  PRT
<213>  Artificial Sequence

<220>
<223>  mutant loop

<400>  281

Ile Glu Ala Gly Asp Pro
1               5


<210>  282
<211>  5
<212>  PRT
<213>  Artificial Sequence

<220>
<223>  mutant loop

<400>  282

Ile Gly Glu Gly Pro
1               5

<210>  283
<211>  5
<212>  PRT
<213>  Artificial Sequence

<220>
<223>  mutant loop

<400>  283

Glu Gly Glu Gly Pro
1               5


<210>  284
<211>  4
<212>  PRT
<213>  Artificial Sequence

<220>
<223>  mutant loop

<400>  284

Asn Gly Gly Pro
1


<210>  285
<211>  4
<212>  PRT
<213>  Artificial Sequence

<220>
<223>  mutant loop

<400>  285

Thr Asp Gly Pro
1


<210>  286
<211>  6
<212>  PRT
<213>  Artificial Sequence

<220>
<223>  mutant loop

<400>  286

Thr Val Asp Gly Gly Pro
1               5


<210>  287
<211>  5
<212>  PRT
<213>  Artificial Sequence

<220>
<223>  mutant loop

<400>  287

Asp Glu Asp Gly Pro
1               5

<210>  288
<211>  5
<212>  PRT
<213>  Artificial Sequence

<220>
<223>  mutant loop

<400>  288

Asn Glu Asp Gly Pro
1               5


<210>  289
<211>  5
<212>  PRT
<213>  Artificial Sequence

<220>
<223>  mutant loop

<400>  289

Thr Glu Asn Gly Pro
1               5


<210>  290
<211>  5
<212>  PRT
<213>  Artificial Sequence

<220>
<223>  mutant loop

<400>  290

Leu Asn Asp Gly Pro
1               5


<210>  291
<211>  5
<212>  PRT
<213>  Artificial Sequence

<220>
<223>  mutant loop

<400>  291

Leu Lys Asp Gly Pro
1               5


<210>  292
<211>  5
<212>  PRT
<213>  Artificial Sequence

<220>
<223>  mutant loop

<400>  292

Leu Leu Glu Gly Pro
1               5

<210>  293
<211>  5
<212>  PRT
<213>  Artificial Sequence

<220>
<223>  mutant loop

<400>  293

Leu Asp Ser Gly Pro
1               5

<210>  294
<211>  5
<212>  PRT
<213>  Artificial Sequence

<220>
<223>  mutant loop

<400>  294

Tyr Gln Asn Gly Pro
1               5

<210>  295
<211>  5
<212>  PRT
<213>  Artificial Sequence

<220>
<223>  mutant loop

<400>  295

Thr Glu Ser Gly Pro
1               5

<210>  296
<211>  6
<212>  PRT
<213>  Artificial Sequence

<220>
<223>  mutant loop

<400>  296

Thr His Glu Gly Gly Pro
1               5

<210>  297
<211>  5
<212>  PRT
<213>  Artificial Sequence

<220>
<223>  mutant loop

<400>  297

Thr His Asp Gly Pro
1               5


<210>  298
<211>  4
<212>  PRT
<213>  Artificial Sequence

<220>
<223>  mutant loop

<400>  298

Val Asn Gly Pro
1


<210>  299
<211>  5
<212>  PRT
<213>  Artificial Sequence

<220>
<223>  mutant loop

<400>  299

Gly Val Leu Gly Pro
1               5


<210>  300
<211>  5
<212>  PRT
<213>  Artificial Sequence

<220>
<223>  mutant loop

<400>  300

Gly Glu Phe Gly Pro
1               5


<210>  301
<211>  5
<212>  PRT
<213>  Artificial Sequence

<220>
<223>  mutant loop

<400>  301

Met Glu Ser Gly Pro
1               5


<210>  302
<211>  5
<212>  PRT
<213>  Artificial Sequence

<220>

<223>  mutant loop

<400>  302

Met Glu Asn Gly Pro
1               5


<210>  303
<211>  5
<212>  PRT
<213>  Artificial Sequence

<220>
<223>  mutant loop

<400>  303

Met Gly Asn Gly Pro
1               5


<210>  304
<211>  5
<212>  PRT
<213>  Artificial Sequence

<220>
<223>  mutant loop

<400>  304

Asp Gly Asn Gly Pro
1               5


<210>  305
<211>  5
<212>  PRT
<213>  Artificial Sequence

<220>
<223>  mutant loop

<400>  305

Ile Lys Asn Gly Pro
1               5


<210>  306
<211>  4
<212>  PRT
<213>  Artificial Sequence

<220>
<223>  mutant loop

<400>  306

Lys Asn Gly Pro
1


<210>  307
<211>  5
<212>  PRT
<213>  Artificial Sequence

<220>
<223>  mutant loop

<400>  307

Met Asn Asp Gly Pro
1               5


<210>  308
<211>  5
<212>  PRT
<213>  Artificial Sequence

<220>
<223>  mutant loop

<400>  308

Met Thr Asp Gly Pro
1               5


<210>  309
<211>  6
<212>  PRT
<213>  Artificial Sequence

<220>
<223>  mutant loop

<400>  309

Leu Asn Ala Gly Ser Pro
1               5


<210>  310
<211>  5
<212>  PRT
<213>  Artificial Sequence

<220>
<223>  mutant loop

<400>  310

Leu Asn Glu Gly Pro
1               5


<210>  311
<211>  5
<212>  PRT
<213>  Artificial Sequence

<220>
<223>  mutant loop

<400>  311

Leu Asn Asp Gly Pro
1               5


<210>  312
<211>  5

<212>  PRT
<213>  Artificial Sequence

<220>
<223>  mutant loop

<400>  312

Tyr Asn Asp Gly Pro
1               5


<210>  313
<211>  5
<212>  PRT
<213>  Artificial Sequence

<220>
<223>  mutant loop

<400>  313

Thr Ser Asp Gly Pro
1               5


<210>  314
<211>  6
<212>  PRT
<213>  Artificial Sequence

<220>
<223>  mutant loop

<400>  314

Thr Ser Ala Asp Gly Pro
1               5


<210>  315
<211>  5
<212>  PRT
<213>  Artificial Sequence

<220>
<223>  mutant loop

<400>  315

Met Ser Asp Gly Pro
1               5


<210>  316
<211>  5
<212>  PRT
<213>  Artificial Sequence

<220>
<223>  mutant loop

<400>  316

Lys Ser Asp Gly Pro
1               5

<210>  317
<211>  5
<212>  PRT
<213>  Artificial Sequence

<220>
<223>  mutant loop

<400>  317

Glu Gln Asp Gly Pro
1               5


<210>  318
<211>  4
<212>  PRT
<213>  Artificial Sequence

<220>
<223>  mutant loop

<400>  318

Gln Asp Gly Pro
1


<210>  319
<211>  5
<212>  PRT
<213>  Artificial Sequence

<220>
<223>  mutant loop

<400>  319

Thr Gln Asp Gly Pro
1               5


<210>  320
<211>  5
<212>  PRT
<213>  Artificial Sequence

<220>
<223>  mutant loop

<400>  320

Ser Lys Asp Gly Pro
1               5


<210>  321
<211>  5
<212>  PRT
<213>  Artificial Sequence

<220>
<223>  mutant loop

<400>  321

Ser Val Asp Gly Pro
1               5

<210>  322
<211>  5
<212>  PRT
<213>  Artificial Sequence

<220>
<223>  mutant loop

<400>  322

Ser Gly Asp Gly Pro
1               5


<210>  323
<211>  5
<212>  PRT
<213>  Artificial Sequence

<220>
<223>  mutant loop

<400>  323

Ser Glu Asp Gly Pro
1               5


<210>  324
<211>  5
<212>  PRT
<213>  Artificial Sequence

<220>
<223>  mutant loop

<400>  324

Gly Asp Asp Gly Pro
1               5


<210>  325
<211>  5
<212>  PRT
<213>  Artificial Sequence

<220>
<223>  mutant loop

<400>  325

Val Asp Asp Gly Pro
1               5


<210>  326
<211>  5
<212>  PRT
<213>  Artificial Sequence

<220>
<223>  mutant loop

<400>  326

Val Arg Asp Gly Pro
1               5


<210>  327
<211>  5
<212>  PRT
<213>  Artificial Sequence

<220>
<223>  mutant loop

<400>  327

Gly Pro Asn Gly Pro
1               5


<210>  328
<211>  5
<212>  PRT
<213>  Artificial Sequence

<220>
<223>  mutant loop

<400>  328

Gly Pro Asp Gly Pro
1               5


<210>  329
<211>  5
<212>  PRT
<213>  Artificial Sequence

<220>
<223>  mutant loop

<400>  329

Glu Pro Asp Gly Pro
1               5


<210>  330
<211>  5
<212>  PRT
<213>  Artificial Sequence

<220>
<223>  mutant loop

<400>  330

Asn Glu Glu Gly Pro
1               5


<210>  331
<211>  5
<212>  PRT
<213>  Artificial Sequence

<220>
<223>  mutant loop

<400>  331

Asn Glu Asn Gly Pro
1               5


<210>  332
<211>  5
<212>  PRT
<213>  Artificial Sequence

<220>
<223>  mutant loop

<400>  332

Asn Glu Asp Gly Pro
1               5


<210>  333
<211>  5
<212>  PRT
<213>  Artificial Sequence

<220>
<223>  mutant loop

<400>  333

Asp Glu Asp Gly Pro
1               5


<210>  334
<211>  5
<212>  PRT
<213>  Artificial Sequence

<220>
<223>  mutant loop

<400>  334

His Glu Asp Gly Pro
1               5


<210>  335
<211>  5
<212>  PRT
<213>  Artificial Sequence

<220>
<223>  mutant loop

<400>  335

Lys Glu Asp Gly Pro
1               5


<210>  336
<211>  6
<212>  PRT
<213>  Artificial Sequence

<220>

<223>  mutant loop

<400>  336

Asn Thr Glu Gly Gly Pro
1               5


<210>  337
<211>  4
<212>  PRT
<213>  Artificial Sequence

<220>
<223>  mutant loop

<400>  337

Asn Gly Gly Pro
1


<210>  338
<211>  5
<212>  PRT
<213>  Artificial Sequence

<220>
<223>  mutant loop

<400>  338

Tyr Glu Gly Gly Pro
1               5


<210>  339
<211>  6
<212>  PRT
<213>  Artificial Sequence

<220>
<223>  mutant loop

<400>  339

Ile Glu Ala Gly Asp Pro
1               5


<210>  340
<211>  6
<212>  PRT
<213>  Artificial Sequence

<220>
<223>  mutant loop

<400>  340

Ile Arg Ala Gly Glu Pro
1               5


<210>  341
<211>  5
<212>  PRT
<213>  Artificial Sequence

<220>
<223>  mutant loop

<400>  341

Ile Asp Gly Gly Pro
1               5


<210>  342
<211>  5
<212>  PRT
<213>  Artificial Sequence

<220>
<223>  mutant loop

<400>  342

Thr Asp Asn Gly Pro
1               5


<210>  343
<211>  5
<212>  PRT
<213>  Artificial Sequence

<220>
<223>  mutant loop

<400>  343

Thr Asp Phe Gly Pro
1               5


<210>  344
<211>  5
<212>  PRT
<213>  Artificial Sequence

<220>
<223>  mutant loop

<400>  344

Ser Asp His Gly Pro
1               5


<210>  345
<211>  5
<212>  PRT
<213>  Artificial Sequence

<220>
<223>  mutant loop

<400>  345

Met Ser Glu Gly Pro
1               5


<210>  346
<211>  5

<212>  PRT
<213>  Artificial Sequence

<220>
<223>  mutant loop

<400>  346

Ala Ser Glu Gly Pro
1               5


<210>  347
<211>  5
<212>  PRT
<213>  Artificial Sequence

<220>
<223>  mutant loop

<400>  347

Met Asp Glu Gly Pro
1               5


<210>  348
<211>  5
<212>  PRT
<213>  Artificial Sequence

<220>
<223>  mutant loop

<400>  348

Ile Asn Glu Gly Pro
1               5


<210>  349
<211>  5
<212>  PRT
<213>  Artificial Sequence

<220>
<223>  mutant loop

<400>  349

Ala Asn Glu Gly Pro
1               5


<210>  350
<211>  5
<212>  PRT
<213>  Artificial Sequence

<220>
<223>  mutant loop

<400>  350

Gly Ala Glu Gly Pro
1               5

<210>  351
<211>  5
<212>  PRT
<213>  Artificial Sequence

<220>
<223>  mutant loop

<400>  351

Val Gly Glu Gly Pro
1               5


<210>  352
<211>  5
<212>  PRT
<213>  Artificial Sequence

<220>
<223>  mutant loop

<400>  352

Thr Gly Glu Gly Pro
1               5


<210>  353
<211>  5
<212>  PRT
<213>  Artificial Sequence

<220>
<223>  mutant loop

<400>  353

Asp Glu Glu Gly Pro
1               5


<210>  354
<211>  5
<212>  PRT
<213>  Artificial Sequence

<220>
<223>  mutant loop

<400>  354

Ser Glu Glu Gly Pro
1               5


<210>  355
<211>  5
<212>  PRT
<213>  Artificial Sequence

<220>
<223>  mutant loop

<400>  355

Thr Glu Glu Gly Pro
1               5

<210>  356
<211>  5
<212>  PRT
<213>  Artificial Sequence

<220>
<223>  mutant loop

<400>  356

Leu Glu Glu Gly Pro
1               5


<210>  357
<211>  5
<212>  PRT
<213>  Artificial Sequence

<220>
<223>  mutant loop

<400>  357

Ser Gln Asp Gly Pro
1               5


<210>  358
<211>  5
<212>  PRT
<213>  Artificial Sequence

<220>
<223>  mutant loop

<400>  358

Thr Gln Asp Gly Pro
1               5


<210>  359
<211>  5
<212>  PRT
<213>  Artificial Sequence

<220>
<223>  mutant loop

<400>  359

Asn Ala Asp Gly Pro
1               5


<210>  360
<211>  6
<212>  PRT
<213>  Artificial Sequence

<220>
<223>  mutant loop

<400>  360

Met Tyr Ala Asp Gly Pro
1               5

<210>  361
<211>  5
<212>  PRT
<213>  Artificial Sequence

<220>
<223>  mutant loop

<400>  361

Trp Glu Asp Gly Pro
1               5

<210>  362
<211>  5
<212>  PRT
<213>  Artificial Sequence

<220>
<223>  mutant loop

<400>  362

Asp Glu Asp Gly Pro
1               5

<210>  363
<211>  5
<212>  PRT
<213>  Artificial Sequence

<220>
<223>  mutant loop

<400>  363

Asn Glu Asp Gly Pro
1               5

<210>  364
<211>  5
<212>  PRT
<213>  Artificial Sequence

<220>
<223>  mutant loop

<400>  364

Phe Ser Asp Gly Pro
1               5

<210>  365
<211>  5
<212>  PRT
<213>  Artificial Sequence

<220>
<223>  mutant loop

<400>  365

Tyr Asp Asn Gly Pro
1               5


<210>  366
<211>  5
<212>  PRT
<213>  Artificial Sequence

<220>
<223>  mutant loop

<400>  366

Met Asp Asn Gly Pro
1               5


<210>  367
<211>  5
<212>  PRT
<213>  Artificial Sequence

<220>
<223>  mutant loop

<400>  367

Ser Asp Asn Gly Pro
1               5


<210>  368
<211>  5
<212>  PRT
<213>  Artificial Sequence

<220>
<223>  mutant loop

<400>  368

Ser Glu Asn Gly Pro
1               5


<210>  369
<211>  5
<212>  PRT
<213>  Artificial Sequence

<220>
<223>  mutant loop

<400>  369

Ile Gly Ser Gly Pro
1               5


<210>  370
<211>  4
<212>  PRT
<213>  Artificial Sequence

<220>

<223>  mutant loop

<400>  370

Leu Gly Gly Pro
1

<210>  371
<211>  5
<212>  PRT
<213>  Artificial Sequence

<220>
<223>  mutant loop

<400>  371

Met Gly Glu Gly Pro
1               5

<210>  372
<211>  5
<212>  PRT
<213>  Artificial Sequence

<220>
<223>  mutant loop

<400>  372

Thr His Glu Gly Pro
1               5

<210>  373
<211>  5
<212>  PRT
<213>  Artificial Sequence

<220>
<223>  mutant loop

<400>  373

Asn Asp Glu Gly Pro
1               5

<210>  374
<211>  6
<212>  PRT
<213>  Artificial Sequence

<220>
<223>  mutant loop

<400>  374

Val Lys Met Gly Ala Pro
1               5

<210>  375
<211>  5
<212>  PRT
<213>  Artificial Sequence

<220>
<223>  mutant loop

<400>  375

Val Thr Asp Gly Pro
1               5


<210>  376
<211>  5
<212>  PRT
<213>  Artificial Sequence

<220>
<223>  mutant loop

<400>  376

Val Asp Asp Gly Pro
1               5


<210>  377
<211>  7
<212>  PRT
<213>  Artificial Sequence

<220>
<223>  mutant loop

<400>  377

Lys Asp Asp Gly Pro Val Thr
1               5


<210>  378
<211>  7
<212>  PRT
<213>  Artificial Sequence

<220>
<223>  mutant loop

<400>  378

Thr Asp Glu Gly Pro Val Thr
1               5


<210>  379
<211>  7
<212>  PRT
<213>  Artificial Sequence

<220>
<223>  mutant loop

<400>  379

Thr Asp Ser Gly Pro Val Thr
1               5


<210>  380
<211>  7

<212>  PRT
<213>  Artificial Sequence

<220>
<223>  mutant loop

<400>  380

Ile Glu Ser Gly Pro Val Thr
1               5


<210>  381
<211>  7
<212>  PRT
<213>  Artificial Sequence

<220>
<223>  mutant loop

<400>  381

Thr Glu Ala Gly Pro Val Ser
1               5


<210>  382
<211>  7
<212>  PRT
<213>  Artificial Sequence

<220>
<223>  mutant loop

<400>  382

Ile Glu Ser Gly Pro Val Thr
1               5


<210>  383
<211>  7
<212>  PRT
<213>  Artificial Sequence

<220>
<223>  mutant loop

<400>  383

Thr Glu Ser Gly Pro Val Ser
1               5


<210>  384
<211>  8
<212>  PRT
<213>  Artificial Sequence

<220>
<223>  mutant loop

<400>  384

Trp Phe Leu Thr Ala Asp Asp Ser
1               5

<210>  385
<211>  7
<212>  PRT
<213>  Artificial Sequence

<220>
<223>  mutant loop

<400>  385

Arg Asp Ser Gly Pro Val Ser
1               5


<210>  386
<211>  7
<212>  PRT
<213>  Artificial Sequence

<220>
<223>  mutant loop

<400>  386

Thr Asp Ser Gly Pro Val Thr
1               5


<210>  387
<211>  7
<212>  PRT
<213>  Artificial Sequence

<220>
<223>  mutant loop

<400>  387

Ser Glu Asp Gly Pro Val Thr
1               5


<210>  388
<211>  7
<212>  PRT
<213>  Artificial Sequence

<220>
<223>  mutant loop

<400>  388

Ile Glu Ser Gly Pro Val Ser
1               5


<210>  389
<211>  7
<212>  PRT
<213>  Artificial Sequence

<220>
<223>  mutant loop

<400>  389

Met Glu Ser Gly Pro Val Ser
1               5

<210>  390
<211>  7
<212>  PRT
<213>  Artificial Sequence

<220>
<223>  mutant loop

<400>  390

Thr Asn Ser Gly Pro Val Ser
1               5


<210>  391
<211>  7
<212>  PRT
<213>  Artificial Sequence

<220>
<223>  mutant loop

<400>  391

Thr Glu Ser Gly Pro Val Gly
1               5


<210>  392
<211>  7
<212>  PRT
<213>  Artificial Sequence

<220>
<223>  mutant loop

<400>  392

Ser Asp Ser Gly Pro Ile Trp
1               5


<210>  393
<211>  7
<212>  PRT
<213>  Artificial Sequence

<220>
<223>  mutant loop

<400>  393

Thr Asp Ser Gly Pro Val Gly
1               5


<210>  394
<211>  7
<212>  PRT
<213>  Artificial Sequence

<220>
<223>  mutant loop

<400>  394

Thr Asp Ser Gly Pro Ile Trp
1               5

<210>  395
<211>  7
<212>  PRT
<213>  Artificial Sequence

<220>
<223>  mutant loop

<400>  395

Met Asp Pro Gly Pro Val Ser
1               5

<210>  396
<211>  7
<212>  PRT
<213>  Artificial Sequence

<220>
<223>  mutant loop

<400>  396

Thr Glu Asn Gly Pro Val Ser
1               5

<210>  397
<211>  7
<212>  PRT
<213>  Artificial Sequence

<220>
<223>  mutant loop

<400>  397

Ala Glu Ser Gly Pro Val Thr
1               5

<210>  398
<211>  8
<212>  PRT
<213>  Artificial Sequence

<220>
<223>  mutant loop

<400>  398

Trp Phe Leu Thr Ala Asp Asp Ser
1               5

<210>  399
<211>  8
<212>  PRT
<213>  Artificial Sequence

<220>
<223>  mutant loop

<400>  399

Trp Phe Leu Thr Ala Asp Asp Ser
1               5


<210>  400
<211>  7
<212>  PRT
<213>  Artificial Sequence

<220>
<223>  mutant loop

<400>  400

Thr Asp Asp Gly Pro Val Ser
1               5


<210>  401
<211>  7
<212>  PRT
<213>  Artificial Sequence

<220>
<223>  mutant loop

<400>  401

Ser Asp Ala Gly Pro Val Ser
1               5


<210>  402
<211>  7
<212>  PRT
<213>  Artificial Sequence

<220>
<223>  mutant loop

<400>  402

Thr His Ser Gly Pro Val Thr
1               5


<210>  403
<211>  7
<212>  PRT
<213>  Artificial Sequence

<220>
<223>  mutant loop

<400>  403

Thr Asp Ser Gly Pro Val Asn
1               5


<210>  404
<211>  7
<212>  PRT
<213>  Artificial Sequence

<220>

<223>  mutant loop

<400>  404

Ser Asp Ser Gly Pro Val Ser
1               5


<210>  405
<211>  5
<212>  PRT
<213>  Artificial Sequence

<220>
<223>  mutant loop

<400>  405

Thr Gly Pro Val Ser
1               5


<210>  406
<211>  7
<212>  PRT
<213>  Artificial Sequence

<220>
<223>  mutant loop

<400>  406

Ile Asp Pro Gly Pro Val Ser
1               5


<210>  407
<211>  7
<212>  PRT
<213>  Artificial Sequence

<220>
<223>  mutant loop

<400>  407

Leu Ala Ser Gly Pro Val Ser
1               5


<210>  408
<211>  7
<212>  PRT
<213>  Artificial Sequence

<220>
<223>  mutant loop

<400>  408

Ala Glu Ser Gly Pro Val Ser
1               5


<210>  409
<211>  5
<212>  PRT
<213>  Artificial Sequence

<220>
<223>  mutant loop

<400>  409

Leu Thr Lys Asn Gln
1               5


<210>  410
<211>  5
<212>  PRT
<213>  Artificial Sequence

<220>
<223>  mutant loop

<400>  410

Tyr Glu Gly Glu His
1               5


<210>  411
<211>  5
<212>  PRT
<213>  Artificial Sequence

<220>
<223>  mutant loop

<400>  411

Ser Trp Gly Trp Asn
1               5


<210>  412
<211>  5
<212>  PRT
<213>  Artificial Sequence

<220>
<223>  mutant loop

<400>  412

Leu Ser Cys Asn Asn
1               5


<210>  413
<211>  5
<212>  PRT
<213>  Artificial Sequence

<220>
<223>  mutant loop

<400>  413

Lys Lys Lys Lys Lys
1               5


<210>  414
<211>  5

<212>  PRT
<213>  Artificial Sequence

<220>
<223>  mutant loop

<400>  414

Trp Leu Met Thr Asn
1               5


<210>  415
<211>  5
<212>  PRT
<213>  Artificial Sequence

<220>
<223>  mutant loop

<400>  415

Gln Arg Gly Arg Met
1               5


<210>  416
<211>  5
<212>  PRT
<213>  Artificial Sequence

<220>
<223>  mutant loop

<400>  416

Tyr Asp Ser Asp Tyr
1               5


<210>  417
<211>  5
<212>  PRT
<213>  Artificial Sequence

<220>
<223>  mutant loop

<400>  417

Pro Ser Glu Asn Tyr
1               5


<210>  418
<211>  4
<212>  PRT
<213>  Artificial Sequence

<220>
<223>  mutant loop

<400>  418

Ile Asp Asn Leu
1

<210>  419
<211>  5
<212>  PRT
<213>  Artificial Sequence

<220>
<223>  mutant loop

<400>  419

Ala Ala Glu Trp Met
1               5


<210>  420
<211>  5
<212>  PRT
<213>  Artificial Sequence

<220>
<223>  mutant loop

<400>  420

Glu His Asn Gly Pro
1               5


<210>  421
<211>  5
<212>  PRT
<213>  Artificial Sequence

<220>
<223>  mutant loop

<400>  421

Phe Ser Glu Gly Pro
1               5


<210>  422
<211>  5
<212>  PRT
<213>  Artificial Sequence

<220>
<223>  mutant loop

<400>  422

Val Asn His Lys Ile
1               5


<210>  423
<211>  5
<212>  PRT
<213>  Artificial Sequence

<220>
<223>  mutant loop

<400>  423

Ser Tyr Ala Glu Asp
1               5

<210>  424
<211>  5
<212>  PRT
<213>  Artificial Sequence

<220>
<223>  mutant loop

<400>  424

Glu Ser Asp His Gly
1               5


<210>  425
<211>  5
<212>  PRT
<213>  Artificial Sequence

<220>
<223>  mutant loop

<400>  425

Gly Leu Gly Leu Trp
1               5


<210>  426
<211>  4
<212>  PRT
<213>  Artificial Sequence

<220>
<223>  mutant loop

<400>  426

Glu Arg Thr Glu
1


<210>  427
<211>  5
<212>  PRT
<213>  Artificial Sequence

<220>
<223>  mutant loop

<400>  427

Tyr Asn Arg Gly Ala
1               5


<210>  428
<211>  5
<212>  PRT
<213>  Artificial Sequence

<220>
<223>  mutant loop

<400>  428

Gly Arg Arg Gly Arg
1               5


<210>  429
<211>  5
<212>  PRT
<213>  Artificial Sequence

<220>
<223>  mutant loop

<400>  429

Glu Val His Asp Val
1               5


<210>  430
<211>  5
<212>  PRT
<213>  Artificial Sequence

<220>
<223>  mutant loop

<400>  430

Glu Glu Glu Tyr Val
1               5


<210>  431
<211>  5
<212>  PRT
<213>  Artificial Sequence

<220>
<223>  mutant loop

<400>  431

Trp Leu Lys Gly Val
1               5


<210>  432
<211>  5
<212>  PRT
<213>  Artificial Sequence

<220>
<223>  mutant loop

<400>  432

Val Gly Gly Met Gly
1               5


<210>  433
<211>  5
<212>  PRT
<213>  Artificial Sequence

<220>
<223>  mutant loop

<400>  433

Ser Phe Val Cys Cys
1               5


<210>  434
<211>  5
<212>  PRT
<213>  Artificial Sequence

<220>
<223>  mutant loop

<400>  434

Cys Phe Ser Leu Ser
1               5


<210>  435
<211>  5
<212>  PRT
<213>  Artificial Sequence

<220>
<223>  mutant loop

<400>  435

Ser Asn Asn Thr Ser
1               5


<210>  436
<211>  5
<212>  PRT
<213>  Artificial Sequence

<220>
<223>  mutant loop

<400>  436

Ser Ser Glu Ser Asp
1               5


<210>  437
<211>  5
<212>  PRT
<213>  Artificial Sequence

<220>
<223>  mutant loop

<400>  437

Thr His Val Gly Ser
1               5


<210>  438
<211>  5
<212>  PRT
<213>  Artificial Sequence

<220>

<223>  mutant loop

<400>  438

His Arg Gly Arg Gly
1               5


<210>  439
<211>  5
<212>  PRT
<213>  Artificial Sequence

<220>
<223>  mutant loop

<400>  439

Val His Ala Ala Thr
1               5


<210>  440
<211>  5
<212>  PRT
<213>  Artificial Sequence

<220>
<223>  mutant loop

<400>  440

Leu Asn His Ser Phe
1               5


<210>  441
<211>  5
<212>  PRT
<213>  Artificial Sequence

<220>
<223>  mutant loop

<400>  441

Tyr Arg Asn Phe Asp
1               5


<210>  442
<211>  5
<212>  PRT
<213>  Artificial Sequence

<220>
<223>  mutant loop

<400>  442

Thr Thr Asn Leu Phe
1               5


<210>  443
<211>  5
<212>  PRT
<213>  Artificial Sequence

<220>
<223>  mutant loop

<400>  443

Gly Arg Arg Gly Glu
1               5


<210>  444
<211>  5
<212>  PRT
<213>  Artificial Sequence

<220>
<223>  mutant loop

<400>  444

Asn Phe Gly Pro Asp
1               5


<210>  445
<211>  5
<212>  PRT
<213>  Artificial Sequence

<220>
<223>  mutant loop

<400>  445

Asn Ala Ser Phe Glu
1               5


<210>  446
<211>  7
<212>  PRT
<213>  Artificial Sequence

<220>
<223>  mutant loop

<400>  446

Thr Glu Asp Gly Pro Val Thr
1               5


<210>  447
<211>  5
<212>  PRT
<213>  Artificial Sequence

<220>
<223>  mutant loop

<400>  447

Thr Tyr Gly Pro Glu
1               5


<210>  448
<211>  5

<212>  PRT
<213>  Artificial Sequence

<220>
<223>  mutant loop

<400>  448

Lys Gly Gly Arg Glu
1               5


<210>  449
<211>  5
<212>  PRT
<213>  Artificial Sequence

<220>
<223>  mutant loop

<400>  449

Pro Val Pro Ala His
1               5


<210>  450
<211>  5
<212>  PRT
<213>  Artificial Sequence

<220>
<223>  mutant loop

<400>  450

Asn Met Gly Pro Glu
1               5


<210>  451
<211>  5
<212>  PRT
<213>  Artificial Sequence

<220>
<223>  mutant loop

<400>  451

Asn Tyr Thr Thr Ile
1               5


<210>  452
<211>  5
<212>  PRT
<213>  Artificial Sequence

<220>
<223>  mutant loop

<400>  452

Lys Asn Trp Gln Ala
1               5

<210>  453
<211>  10
<212>  PRT
<213>  Artificial Sequence

<220>
<223>  mutant loop

<400>  453

Ser His Leu Arg Trp Thr Asn Gly Asn Val
1               5                   10


<210>  454
<211>  7
<212>  PRT
<213>  Artificial Sequence

<220>
<223>  mutant loop

<400>  454

Ser Trp Lys Arg Trp Lys Thr
1               5


<210>  455
<211>  7
<212>  PRT
<213>  Artificial Sequence

<220>
<223>  mutant loop

<400>  455

Ser Ser Lys Arg Trp Ala Thr
1               5


<210>  456
<211>  7
<212>  PRT
<213>  Artificial Sequence

<220>
<223>  mutant loop

<400>  456

Ser Gln Lys Arg Trp Met Thr
1               5


<210>  457
<211>  7
<212>  PRT
<213>  Artificial Sequence

<220>
<223>  mutant loop

<400>  457

Ser Gln Gln Arg Trp Met Thr
1               5

<210>  458
<211>  7
<212>  PRT
<213>  Artificial Sequence

<220>
<223>  mutant loop

<400>  458

Ser Gln Gln Arg Trp Met Lys
1               5


<210>  459
<211>  7
<212>  PRT
<213>  Artificial Sequence

<220>
<223>  mutant loop

<400>  459

Ser Gln Arg Arg Trp Glu Ala
1               5


<210>  460
<211>  7
<212>  PRT
<213>  Artificial Sequence

<220>
<223>  mutant loop

<400>  460

Ser Gln Arg Arg Trp Glu Asn
1               5


<210>  461
<211>  7
<212>  PRT
<213>  Artificial Sequence

<220>
<223>  mutant loop

<400>  461

Ser Gln Arg Arg Trp Met Ala
1               5


<210>  462
<211>  7
<212>  PRT
<213>  Artificial Sequence

<220>
<223>  mutant loop

<400>  462

Ser Glu Arg Arg Trp Met Thr
1               5

<210>  463
<211>  7
<212>  PRT
<213>  Artificial Sequence

<220>
<223>  mutant loop

<400>  463

Ser Glu Arg Arg Trp Met Ser
1               5

<210>  464
<211>  7
<212>  PRT
<213>  Artificial Sequence

<220>
<223>  mutant loop

<400>  464

Ser Thr Arg Arg Trp Met Val
1               5

<210>  465
<211>  7
<212>  PRT
<213>  Artificial Sequence

<220>
<223>  mutant loop

<400>  465

Ser Arg Arg Arg Trp Ser Met
1               5

<210>  466
<211>  7
<212>  PRT
<213>  Artificial Sequence

<220>
<223>  mutant loop

<400>  466

Ser Ala Arg Arg Trp Ser Met
1               5

<210>  467
<211>  7
<212>  PRT
<213>  Artificial Sequence

<220>
<223>  mutant loop

<400>  467

Ser Ala Arg Arg Trp Ser Val
1               5


<210>  468
<211>  7
<212>  PRT
<213>  Artificial Sequence

<220>
<223>  mutant loop

<400>  468

Ser Arg Arg Arg Trp Asp Ser
1               5


<210>  469
<211>  7
<212>  PRT
<213>  Artificial Sequence

<220>
<223>  mutant loop

<400>  469

Ser Ala Arg Arg Trp Asp Ser
1               5


<210>  470
<211>  7
<212>  PRT
<213>  Artificial Sequence

<220>
<223>  mutant loop

<400>  470

Ser Pro Arg Arg Trp Asn Ser
1               5


<210>  471
<211>  7
<212>  PRT
<213>  Artificial Sequence

<220>
<223>  mutant loop

<400>  471

Ser Asn Arg Arg Trp Asn Ser
1               5


<210>  472
<211>  7
<212>  PRT
<213>  Artificial Sequence

<220>

<223>  mutant loop

<400>  472

Ser Gln Arg Arg Trp Asn Ser
1               5


<210>  473
<211>  7
<212>  PRT
<213>  Artificial Sequence

<220>
<223>  mutant loop

<400>  473

Ser Thr Arg Arg Trp Ala Ser
1               5


<210>  474
<211>  7
<212>  PRT
<213>  Artificial Sequence

<220>
<223>  mutant loop

<400>  474

Ser Asn Arg Arg Trp Ala Ser
1               5


<210>  475
<211>  7
<212>  PRT
<213>  Artificial Sequence

<220>
<223>  mutant loop

<400>  475

Ser Asn Arg Arg Trp Ile Asn
1               5


<210>  476
<211>  7
<212>  PRT
<213>  Artificial Sequence

<220>
<223>  mutant loop

<400>  476

Ser Asn Arg Arg Trp Ile Ala
1               5


<210>  477
<211>  7
<212>  PRT
<213>  Artificial Sequence

<220>
<223>  mutant loop

<400>  477

Ser Tyr Arg Arg Trp Ile Ser
1               5


<210>  478
<211>  7
<212>  PRT
<213>  Artificial Sequence

<220>
<223>  mutant loop

<400>  478

Ser Glu Met Arg Trp Asp Ala
1               5


<210>  479
<211>  7
<212>  PRT
<213>  Artificial Sequence

<220>
<223>  mutant loop

<400>  479

Ser Glu Ala Arg Trp Lys Gly
1               5


<210>  480
<211>  7
<212>  PRT
<213>  Artificial Sequence

<220>
<223>  mutant loop

<400>  480

Ser Pro Ala Arg Trp Gly Ala
1               5


<210>  481
<211>  7
<212>  PRT
<213>  Artificial Sequence

<220>
<223>  mutant loop

<400>  481

Ser Arg Ala Arg Trp Gly Ala
1               5


<210>  482
<211>  7

<212>  PRT
<213>  Artificial Sequence

<220>
<223>  mutant loop

<400>  482

Ser Ser Ala Arg Trp Met Ala
1               5


<210>  483
<211>  7
<212>  PRT
<213>  Artificial Sequence

<220>
<223>  mutant loop

<400>  483

Ser Val Asn Arg Trp Thr Ala
1               5


<210>  484
<211>  7
<212>  PRT
<213>  Artificial Sequence

<220>
<223>  mutant loop

<400>  484

Ser Ser Arg Arg Trp Leu His
1               5


<210>  485
<211>  7
<212>  PRT
<213>  Artificial Sequence

<220>
<223>  mutant loop

<400>  485

Ser Ser Arg Arg Trp Leu Gly
1               5


<210>  486
<211>  7
<212>  PRT
<213>  Artificial Sequence

<220>
<223>  mutant loop

<400>  486

Ser Ser Arg Arg Trp Asp Arg
1               5

<210>  487
<211>  7
<212>  PRT
<213>  Artificial Sequence

<220>
<223>  mutant loop

<400>  487

Ser Ser His Arg Trp Ala Ala
1               5


<210>  488
<211>  7
<212>  PRT
<213>  Artificial Sequence

<220>
<223>  mutant loop

<400>  488

Ser Ser Arg Arg Trp Ala Leu
1               5


<210>  489
<211>  7
<212>  PRT
<213>  Artificial Sequence

<220>
<223>  mutant loop

<400>  489

Ser Ser Met Arg Trp Met Asn
1               5


<210>  490
<211>  7
<212>  PRT
<213>  Artificial Sequence

<220>
<223>  mutant loop

<400>  490

Ser Ser Tyr Arg Trp His Asn
1               5


<210>  491
<211>  7
<212>  PRT
<213>  Artificial Sequence

<220>
<223>  mutant loop

<400>  491

Ser His His Arg Trp Phe Ser
1               5

<210>  492
<211>  7
<212>  PRT
<213>  Artificial Sequence

<220>
<223>  mutant loop

<400>  492

Ser Ser His Arg Trp Asn Ser
1               5


<210>  493
<211>  7
<212>  PRT
<213>  Artificial Sequence

<220>
<223>  mutant loop

<400>  493

Ser Ser Trp Arg Trp Thr Ser
1               5


<210>  494
<211>  7
<212>  PRT
<213>  Artificial Sequence

<220>
<223>  mutant loop

<400>  494

Ser Ser Gln Arg Trp Val Ser
1               5


<210>  495
<211>  7
<212>  PRT
<213>  Artificial Sequence

<220>
<223>  mutant loop

<400>  495

Ser Thr Trp Arg Trp Gln Ser
1               5


<210>  496
<211>  7
<212>  PRT
<213>  Artificial Sequence

<220>
<223>  mutant loop

<400>  496

Ser Thr Lys Arg Trp Leu Ser
1               5

<210>  497
<211>  7
<212>  PRT
<213>  Artificial Sequence

<220>
<223>  mutant loop

<400>  497

Ser Arg Met Arg Trp Gln Ser
1               5

<210>  498
<211>  7
<212>  PRT
<213>  Artificial Sequence

<220>
<223>  mutant loop

<400>  498

Ser Gln Tyr Arg Trp Leu Ser
1               5

<210>  499
<211>  7
<212>  PRT
<213>  Artificial Sequence

<220>
<223>  mutant loop

<400>  499

Ser Asn Tyr Arg Trp Leu Ser
1               5

<210>  500
<211>  7
<212>  PRT
<213>  Artificial Sequence

<220>
<223>  mutant loop

<400>  500

Ser His Tyr Arg Trp Ser Ser
1               5

<210>  501
<211>  7
<212>  PRT
<213>  Artificial Sequence

<220>
<223>  mutant loop

<400>  501

Ala His Tyr Arg Trp Arg Ser
1               5


<210>  502
<211>  7
<212>  PRT
<213>  Artificial Sequence

<220>
<223>  mutant loop

<400>  502

Thr Leu Tyr Arg Trp Asn Ser
1               5


<210>  503
<211>  7
<212>  PRT
<213>  Artificial Sequence

<220>
<223>  mutant loop

<400>  503

Ser Trp Ala Arg Trp Gln Ser
1               5


<210>  504
<211>  7
<212>  PRT
<213>  Artificial Sequence

<220>
<223>  mutant loop

<400>  504

Ser Gln Tyr Arg Trp Met Ser
1               5


<210>  505
<211>  7
<212>  PRT
<213>  Artificial Sequence

<220>
<223>  mutant loop

<400>  505

Ser Ala Leu Arg Trp Gln Arg
1               5


<210>  506
<211>  7
<212>  PRT
<213>  Artificial Sequence

<220>

<223>  mutant loop

<400>  506

Ser Gln Tyr Arg Trp Gln Arg
1               5


<210>  507
<211>  7
<212>  PRT
<213>  Artificial Sequence

<220>
<223>  mutant loop

<400>  507

Ser Ser Ala Arg Trp Gln Arg
1               5


<210>  508
<211>  7
<212>  PRT
<213>  Artificial Sequence

<220>
<223>  mutant loop

<400>  508

Ser Gln Ala Arg Trp Arg Asn
1               5


<210>  509
<211>  7
<212>  PRT
<213>  Artificial Sequence

<220>
<223>  mutant loop

<400>  509

Ser Gln Tyr Arg Trp Arg Thr
1               5


<210>  510
<211>  7
<212>  PRT
<213>  Artificial Sequence

<220>
<223>  mutant loop

<400>  510

Ser Asn Ala Arg Trp Arg Ser
1               5


<210>  511
<211>  7
<212>  PRT
<213>  Artificial Sequence

<220>
<223>  mutant loop

<400>  511

Ser Tyr Trp Arg Trp Arg Arg
1               5


<210>  512
<211>  7
<212>  PRT
<213>  Artificial Sequence

<220>
<223>  mutant loop

<400>  512

Ser Arg Leu Arg Trp Arg His
1               5


<210>  513
<211>  7
<212>  PRT
<213>  Artificial Sequence

<220>
<223>  mutant loop

<400>  513

Ser Ala Lys Arg Trp Glu His
1               5


<210>  514
<211>  7
<212>  PRT
<213>  Artificial Sequence

<220>
<223>  mutant loop

<400>  514

Ser Ala Lys Arg Trp Glu Tyr
1               5


<210>  515
<211>  7
<212>  PRT
<213>  Artificial Sequence

<220>
<223>  mutant loop

<400>  515

Ala Ala His Arg Trp Glu Ala
1               5


<210>  516
<211>  7

<212>  PRT
<213>  Artificial Sequence

<220>
<223>  mutant loop

<400>  516

Ser Ala Tyr Arg Trp Glu Ser
1               5


<210>  517
<211>  7
<212>  PRT
<213>  Artificial Sequence

<220>
<223>  mutant loop

<400>  517

Ser His Met Arg Trp Lys Lys
1               5


<210>  518
<211>  7
<212>  PRT
<213>  Artificial Sequence

<220>
<223>  mutant loop

<400>  518

Ser His His Arg Trp Lys Lys
1               5


<210>  519
<211>  7
<212>  PRT
<213>  Artificial Sequence

<220>
<223>  mutant loop

<400>  519

Ser Tyr Tyr Arg Trp Lys Lys
1               5


<210>  520
<211>  7
<212>  PRT
<213>  Artificial Sequence

<220>
<223>  mutant loop

<400>  520

Ser Lys Met Arg Trp Glu Lys
1               5

<210>  521
<211>  7
<212>  PRT
<213>  Artificial Sequence

<220>
<223>  mutant loop

<400>  521

Ser Ala Trp Arg Trp Asn Lys
1               5


<210>  522
<211>  7
<212>  PRT
<213>  Artificial Sequence

<220>
<223>  mutant loop

<400>  522

Ser His Trp Arg Trp His Lys
1               5


<210>  523
<211>  7
<212>  PRT
<213>  Artificial Sequence

<220>
<223>  mutant loop

<400>  523

Ser Ala Gln Arg Trp Leu Lys
1               5


<210>  524
<211>  7
<212>  PRT
<213>  Artificial Sequence

<220>
<223>  mutant loop

<400>  524

Ser Asn Leu Arg Trp Gly Lys
1               5


<210>  525
<211>  7
<212>  PRT
<213>  Artificial Sequence

<220>
<223>  mutant loop

<400>  525

Ser Gln Met Arg Trp Lys Thr
1               5

<210>  526
<211>  7
<212>  PRT
<213>  Artificial Sequence

<220>
<223>  mutant loop

<400>  526

Ser Gln Leu Arg Trp Lys Ser
1               5


<210>  527
<211>  7
<212>  PRT
<213>  Artificial Sequence

<220>
<223>  mutant loop

<400>  527

Ser Ala Phe Arg Trp Lys His
1               5


<210>  528
<211>  7
<212>  PRT
<213>  Artificial Sequence

<220>
<223>  mutant loop

<400>  528

Ser Trp Phe Arg Trp Lys Ser
1               5


<210>  529
<211>  7
<212>  PRT
<213>  Artificial Sequence

<220>
<223>  mutant loop

<400>  529

Ser Ser Trp Arg Trp Lys Ser
1               5


<210>  530
<211>  7
<212>  PRT
<213>  Artificial Sequence

<220>
<223>  mutant loop

<400>  530

Ser Asn Tyr Arg Trp Lys His
1               5

<210>  531
<211>  7
<212>  PRT
<213>  Artificial Sequence

<220>
<223>  mutant loop

<400>  531

Ser Asn Arg Arg Trp Gly Ala
1               5

<210>  532
<211>  7
<212>  PRT
<213>  Artificial Sequence

<220>
<223>  mutant loop

<400>  532

Ser Gln Arg Arg Trp Gly Val
1               5

<210>  533
<211>  7
<212>  PRT
<213>  Artificial Sequence

<220>
<223>  mutant loop

<400>  533

Ser Thr Arg Arg Trp Met Gln
1               5

<210>  534
<211>  7
<212>  PRT
<213>  Artificial Sequence

<220>
<223>  mutant loop

<400>  534

Ser Thr Arg Arg Trp Asn Thr
1               5

<210>  535
<211>  7
<212>  PRT
<213>  Artificial Sequence

<220>
<223>  mutant loop

<400>  535

Ser Trp Ala Arg Trp His Ser
1               5


<210>  536
<211>  7
<212>  PRT
<213>  Artificial Sequence

<220>
<223>  mutant loop

<400>  536

Ser Trp Ala Arg Trp Gln Asn
1               5


<210>  537
<211>  7
<212>  PRT
<213>  Artificial Sequence

<220>
<223>  mutant loop

<400>  537

Ser Ser Ala Arg Trp Phe His
1               5


<210>  538
<211>  7
<212>  PRT
<213>  Artificial

<220>
<223>  loop sequences

<400>  538

Ser Gln Ala Arg Trp Phe His
1               5


<210>  539
<211>  7
<212>  PRT
<213>  Artificial Sequence

<220>
<223>  mutant loop

<400>  539

Ser Thr Phe Arg Trp Gln Arg
1               5


<210>  540
<211>  7
<212>  PRT
<213>  Artificial Sequence

<220>

<223>  mutant loop

<400>  540

Ser Arg Met Arg Trp Gln Arg
1               5


<210>  541
<211>  7
<212>  PRT
<213>  Artificial Sequence

<220>
<223>  mutant loop

<400>  541

Ser Trp Leu Arg Trp Lys Arg
1               5


<210>  542
<211>  7
<212>  PRT
<213>  Artificial Sequence

<220>
<223>  mutant loop

<400>  542

Ser Arg Tyr Arg Trp Leu Asn
1               5


<210>  543
<211>  7
<212>  PRT
<213>  Artificial Sequence

<220>
<223>  mutant loop

<400>  543

Ser Ser Met Arg Trp Met Asn
1               5


<210>  544
<211>  7
<212>  PRT
<213>  Artificial Sequence

<220>
<223>  mutant loop

<400>  544

Ser Ser Met Arg Trp Gly Leu
1               5


<210>  545
<211>  7
<212>  PRT
<213>  Artificial Sequence

<220>
<223>  mutant loop

<400>  545

Ser Ser Met Arg Trp Glu Ser
1               5


<210>  546
<211>  7
<212>  PRT
<213>  Artificial Sequence

<220>
<223>  mutant loop

<400>  546

Ser Ser Trp Arg Trp Met Ser
1               5


<210>  547
<211>  7
<212>  PRT
<213>  Artificial Sequence

<220>
<223>  mutant loop

<400>  547

Ser His Trp Arg Trp Met Ala
1               5


<210>  548
<211>  7
<212>  PRT
<213>  Artificial Sequence

<220>
<223>  mutant loop

<400>  548

Ser Ser Phe Arg Trp Thr Ala
1               5


<210>  549
<211>  7
<212>  PRT
<213>  Artificial Sequence

<220>
<223>  mutant loop

<400>  549

Ser Ser Asn Arg Trp Lys Gln
1               5


<210>  550
<211>  7

<212>  PRT
<213>  Artificial Sequence

<220>
<223>  mutant loop

<400>  550

Ser Ser Ser Arg Trp Ser Asp
1               5


<210>  551
<211>  7
<212>  PRT
<213>  Artificial Sequence

<220>
<223>  mutant loop

<400>  551

Ser Ala Gln Arg Trp Ser Thr
1               5


<210>  552
<211>  7
<212>  PRT
<213>  Artificial Sequence

<220>
<223>  mutant loop

<400>  552

Ser Ala Gln Arg Trp Ala Thr
1               5


<210>  553
<211>  7
<212>  PRT
<213>  Artificial Sequence

<220>
<223>  mutant loop

<400>  553

Ser Ala Gly Arg Trp Glu Lys
1               5


<210>  554
<211>  7
<212>  PRT
<213>  Artificial Sequence

<220>
<223>  mutant loop

<400>  554

Ser Ala His Arg Trp Ala Lys
1               5

<210>  555
<211>  7
<212>  PRT
<213>  Artificial Sequence

<220>
<223>  mutant loop

<400>  555

Ser Asn Arg Arg Trp Ala Ser
1               5


<210>  556
<211>  7
<212>  PRT
<213>  Artificial Sequence

<220>
<223>  mutant loop

<400>  556

Thr Asp Asp Gly Pro Val Thr
1               5


<210>  557
<211>  7
<212>  PRT
<213>  Artificial Sequence

<220>
<223>  mutant loop

<400>  557

Ser Asn Lys Arg Trp Lys Ser
1               5


<210>  558
<211>  7
<212>  PRT
<213>  Artificial Sequence

<220>
<223>  mutant loop

<400>  558

Ser Ala Arg Arg Trp Tyr Ser
1               5


<210>  559
<211>  7
<212>  PRT
<213>  Artificial Sequence

<220>
<223>  mutant loop

<400>  559

Ser Phe Gln Arg Trp Gln Ser
1               5

<210>  560
<211>  7
<212>  PRT
<213>  Artificial Sequence

<220>
<223>  mutant loop

<400>  560

Ala Ser Arg Arg Trp Glu Thr
1               5


<210>  561
<211>  7
<212>  PRT
<213>  Artificial Sequence

<220>
<223>  mutant loop

<400>  561

Ser Lys Tyr Arg Trp Glu Thr
1               5


<210>  562
<211>  10
<212>  PRT
<213>  Artificial Sequence

<220>
<223>  mutant loop

<400>  562

Gln Asp Asp Arg Trp Glu Ser Gly Asn Val
1               5                   10


<210>  563
<211>  10
<212>  PRT
<213>  Artificial Sequence

<220>
<223>  mutant loop

<400>  563

Ser Trp Met Arg Trp His Arg Gly Asn Val
1               5                   10


<210>  564
<211>  10
<212>  PRT
<213>  Artificial Sequence

<220>
<223>  mutant loop

<400>  564

Ser Trp Met Arg Trp His Arg Gly Asn Val
1               5                   10


<210>  565
<211>  10
<212>  PRT
<213>  Artificial Sequence

<220>
<223>  mutant loop

<400>  565

Ser Thr Tyr Arg Trp Gln Ser Gly Asp Val
1               5                   10


<210>  566
<211>  10
<212>  PRT
<213>  Artificial Sequence

<220>
<223>  mutant loop

<400>  566

Ser Asn Arg Arg Trp Asn Ser Gly Asn Val
1               5                   10


<210>  567
<211>  10
<212>  PRT
<213>  Artificial Sequence

<220>
<223>  mutant loop

<400>  567

Ser Thr Met Arg Trp Phe Ser Gly Asn Val
1               5                   10


<210>  568
<211>  10
<212>  PRT
<213>  Artificial Sequence

<220>
<223>  mutant loop

<400>  568

Ser Thr Leu Arg Trp Gln Ser Gly Asn Val
1               5                   10


<210>  569
<211>  10
<212>  PRT
<213>  Artificial Sequence

<220>
<223>  mutant loop

<400>  569

Ser Val Tyr Arg Trp Gln Ser Gly Asn Val
1               5                   10


<210>  570
<211>  10
<212>  PRT
<213>  Artificial Sequence

<220>
<223>  mutant loop

<400>  570

Ser Val Tyr Arg Trp Glu Ser Gly Asn Val
1               5                   10


<210>  571
<211>  10
<212>  PRT
<213>  Artificial Sequence

<220>
<223>  mutant loop

<400>  571

Ser His Tyr Arg Trp Lys Ser Gly Asn Val
1               5                   10


<210>  572
<211>  10
<212>  PRT
<213>  Artificial Sequence

<220>
<223>  mutant loop

<400>  572

Ser Trp Ala Arg Trp Phe Ser Gly Asn Val
1               5                   10


<210>  573
<211>  10
<212>  PRT
<213>  Artificial Sequence

<220>
<223>  mutant loop

<400>  573

Ser Val Leu Arg Trp Arg Thr Gly Asn Val
1               5                   10


<210>  574
<211>  10
<212>  PRT
<213>  Artificial Sequence

<220>

<223>  mutant loop

<400>  574

Ser Val Leu Arg Trp Gln Thr Gly Asn Val
1               5                   10


<210>  575
<211>  10
<212>  PRT
<213>  Artificial Sequence

<220>
<223>  mutant loop

<400>  575

Ser Val Leu Arg Trp Gln Ser Gly Asn Val
1               5                   10


<210>  576
<211>  10
<212>  PRT
<213>  Artificial Sequence

<220>
<223>  mutant loop

<400>  576

Ser Ala Leu Arg Trp Glu Ser Gly Asn Val
1               5                   10


<210>  577
<211>  10
<212>  PRT
<213>  Artificial Sequence

<220>
<223>  mutant loop

<400>  577

Ser Ala Arg Arg Trp Glu Thr Gly Asn Val
1               5                   10


<210>  578
<211>  10
<212>  PRT
<213>  Artificial Sequence

<220>
<223>  mutant loop

<400>  578

Ser Ser Arg Arg Trp Gln Thr Gly Asn Val
1               5                   10


<210>  579
<211>  10
<212>  PRT
<213>  Artificial Sequence

<220>
<223>  mutant loop

<400>  579

Ser Ala Leu Arg Trp Met Thr Gly Asn Val
1               5                   10


<210>  580
<211>  10
<212>  PRT
<213>  Artificial Sequence

<220>
<223>  mutant loop

<400>  580

Ser Thr Tyr Arg Trp Met Ser Gly Asn Val
1               5                   10


<210>  581
<211>  10
<212>  PRT
<213>  Artificial Sequence

<220>
<223>  mutant loop

<400>  581

Ser Thr Met Arg Trp Met Ser Gly Asn Val
1               5                   10


<210>  582
<211>  10
<212>  PRT
<213>  Artificial Sequence

<220>
<223>  mutant loop

<400>  582

Ser Ala Tyr Arg Trp Arg Ser Gly Asn Val
1               5                   10


<210>  583
<211>  10
<212>  PRT
<213>  Artificial Sequence

<220>
<223>  mutant loop

<400>  583

Ser Ala Tyr Arg Trp Gln Ser Gly Asn Val
1               5                   10


<210>  584
<211>  10

<212>  PRT
<213>  Artificial Sequence

<220>
<223>  mutant loop

<400>  584

Ser Tyr Trp Arg Trp His Ser Gly Asn Val
1               5                   10


<210>  585
<211>  10
<212>  PRT
<213>  Artificial Sequence

<220>
<223>  mutant loop

<400>  585

Ser Tyr Tyr Arg Trp His Ser Gly Asn Val
1               5                   10


<210>  586
<211>  10
<212>  PRT
<213>  Artificial Sequence

<220>
<223>  mutant loop

<400>  586

Ser Tyr Tyr Arg Trp Gln Ala Gly Asn Val
1               5                   10


<210>  587
<211>  10
<212>  PRT
<213>  Artificial Sequence

<220>
<223>  mutant loop

<400>  587

Ser Trp Tyr Arg Trp His Ser Gly Asn Val
1               5                   10


<210>  588
<211>  10
<212>  PRT
<213>  Artificial Sequence

<220>
<223>  mutant loop

<400>  588

Ser Trp Tyr Arg Trp His Ala Gly Asn Val
1               5                   10

<210>  589
<211>  10
<212>  PRT
<213>  Artificial Sequence

<220>
<223>  mutant loop

<400>  589

Ser Asn Tyr Arg Trp His Ser Gly Asn Val
1               5                   10


<210>  590
<211>  10
<212>  PRT
<213>  Artificial Sequence

<220>
<223>  mutant loop

<400>  590

Ser Ser Arg Arg Trp Met Ser Gly Asp Val
1               5                   10


<210>  591
<211>  10
<212>  PRT
<213>  Artificial Sequence

<220>
<223>  mutant loop

<400>  591

Ser Asn Arg Arg Trp Met Ser Gly Asn Val
1               5                   10


<210>  592
<211>  10
<212>  PRT
<213>  Artificial Sequence

<220>
<223>  mutant loop

<400>  592

Ser Ser Leu Arg Trp Met Ser Gly Asn Val
1               5                   10


<210>  593
<211>  10
<212>  PRT
<213>  Artificial Sequence

<220>
<223>  mutant loop

<400>  593

Ser His Trp Arg Trp Met Lys Gly Asn Val
1               5                   10

<210>  594
<211>  10
<212>  PRT
<213>  Artificial Sequence

<220>
<223>  mutant loop

<400>  594

Ser Ala Leu Arg Trp Ala Gln Gly Ser Val
1               5                   10


<210>  595
<211>  10
<212>  PRT
<213>  Artificial Sequence

<220>
<223>  mutant loop

<400>  595

Ser Thr Leu Arg Trp Ile Ser Gly Tyr Pro
1               5                   10


<210>  596
<211>  10
<212>  PRT
<213>  Artificial Sequence

<220>
<223>  mutant loop

<400>  596

Ser Ser Leu Arg Trp Thr Ser Gly Asn Val
1               5                   10


<210>  597
<211>  10
<212>  PRT
<213>  Artificial Sequence

<220>
<223>  mutant loop

<400>  597

Ser Thr Leu Arg Trp Thr Ser Gly Asn Val
1               5                   10


<210>  598
<211>  10
<212>  PRT
<213>  Artificial Sequence

<220>
<223>  mutant loop

<400>  598

Ser His Leu Arg Trp Thr Asn Gly Asn Val
1               5                   10


<210>  599
<211>  10
<212>  PRT
<213>  Artificial Sequence

<220>
<223>  mutant loop

<400>  599

Ser Asp Leu Arg Trp Lys Asn Gly Asn Val
1               5                   10


<210>  600
<211>  10
<212>  PRT
<213>  Artificial Sequence

<220>
<223>  mutant loop

<400>  600

Ser Gln Leu Arg Trp Thr Asn Asp Asn Ala
1               5                   10


<210>  601
<211>  10
<212>  PRT
<213>  Artificial Sequence

<220>
<223>  mutant loop

<400>  601

Ser Tyr Leu Arg Trp Thr His Gly Asn Ala
1               5                   10


<210>  602
<211>  10
<212>  PRT
<213>  Artificial Sequence

<220>
<223>  mutant loop

<400>  602

Ser Asn Leu Arg Trp Arg Thr Gly Lys Asp
1               5                   10


<210>  603
<211>  10
<212>  PRT
<213>  Artificial Sequence

<220>
<223>  mutant loop

<400>  603

Ser Gln Leu Arg Trp Ala Thr Asp Lys Val
1               5                   10


<210>  604
<211>  10
<212>  PRT
<213>  Artificial Sequence

<220>
<223>  mutant loop

<400>  604

Ser Gln Leu Arg Trp Ala His Gly Arg Val
1               5                   10


<210>  605
<211>  10
<212>  PRT
<213>  Artificial Sequence

<220>
<223>  mutant loop

<400>  605

Ser Gln Leu Arg Trp Ala Lys Gly Lys Ala
1               5                   10


<210>  606
<211>  10
<212>  PRT
<213>  Artificial Sequence

<220>
<223>  mutant loop

<400>  606

Ser Gln Leu Arg Trp Thr Ser Gly Lys Val
1               5                   10


<210>  607
<211>  10
<212>  PRT
<213>  Artificial Sequence

<220>
<223>  mutant loop

<400>  607

Ser Gln Arg Arg Trp Thr Ser Gly Lys Val
1               5                   10


<210>  608
<211>  10
<212>  PRT
<213>  Artificial Sequence

<220>

<223>  mutant loop

<400>  608

Ser Ser Leu Arg Trp Thr Ser Gly Lys Val
1               5                   10


<210>  609
<211>  10
<212>  PRT
<213>  Artificial Sequence

<220>
<223>  mutant loop

<400>  609

Ser Gln Leu Arg Trp Thr His Gly Lys Val
1               5                   10


<210>  610
<211>  10
<212>  PRT
<213>  Artificial Sequence

<220>
<223>  mutant loop

<400>  610

Ser His Leu Arg Trp Thr Ser Gly Lys Val
1               5                   10


<210>  611
<211>  10
<212>  PRT
<213>  Artificial Sequence

<220>
<223>  mutant loop

<400>  611

Ser Gln Met Arg Trp Thr Lys Gly Lys Val
1               5                   10


<210>  612
<211>  10
<212>  PRT
<213>  Artificial Sequence

<220>
<223>  mutant loop

<400>  612

Ser Gln Leu Arg Trp Thr Lys Gly Lys Val
1               5                   10


<210>  613
<211>  10
<212>  PRT
<213>  Artificial Sequence

<220>
<223>  mutant loop

<400>  613

Ser Gln Leu Arg Trp Thr Lys Gly Lys Met
1               5                   10


<210>  614
<211>  10
<212>  PRT
<213>  Artificial Sequence

<220>
<223>  mutant loop

<400>  614

Ser Asn Leu Arg Trp Ser Lys Gly Lys Val
1               5                   10


<210>  615
<211>  10
<212>  PRT
<213>  Artificial Sequence

<220>
<223>  mutant loop

<400>  615

Ser Asn Leu Arg Trp Thr Lys Gly Asp Val
1               5                   10


<210>  616
<211>  10
<212>  PRT
<213>  Artificial Sequence

<220>
<223>  mutant loop

<400>  616

Ser Asn Met Arg Trp Thr Lys Gly Asp Val
1               5                   10


<210>  617
<211>  10
<212>  PRT
<213>  Artificial Sequence

<220>
<223>  mutant loop

<400>  617

Ser Ser Leu Arg Trp Thr Lys Arg Asp Val
1               5                   10


<210>  618
<211>  10

<212>  PRT
<213>  Artificial Sequence

<220>
<223>  mutant loop

<400>  618

Ser Gln Leu Arg Trp Thr Ser Arg Asp Val
1               5                   10


<210>  619
<211>  10
<212>  PRT
<213>  Artificial Sequence

<220>
<223>  mutant loop

<400>  619

Ser His Phe Arg Trp Thr Ser Gly Arg Val
1               5                   10


<210>  620
<211>  10
<212>  PRT
<213>  Artificial Sequence

<220>
<223>  mutant loop

<400>  620

Ser Asn Leu Arg Trp Thr Lys Gly Arg Val
1               5                   10


<210>  621
<211>  10
<212>  PRT
<213>  Artificial Sequence

<220>
<223>  mutant loop

<400>  621

Ser Ser Leu Arg Trp Thr Lys Gly Arg Val
1               5                   10


<210>  622
<211>  10
<212>  PRT
<213>  Artificial Sequence

<220>
<223>  mutant loop

<400>  622

Ser Asn Leu Arg Trp Thr Ser Arg Lys Val
1               5                   10

<210>  623
<211>  10
<212>  PRT
<213>  Artificial Sequence

<220>
<223>  mutant loop

<400>  623

Ser Asn Leu Arg Trp Thr Lys Arg Lys Val
1               5                   10


<210>  624
<211>  10
<212>  PRT
<213>  Artificial Sequence

<220>
<223>  mutant loop

<400>  624

Ser Asn Leu Arg Trp Thr Arg Gly Lys Val
1               5                   10


<210>  625
<211>  10
<212>  PRT
<213>  Artificial Sequence

<220>
<223>  mutant loop

<400>  625

Ser Asn Leu Arg Trp Lys Ser Gly Lys Val
1               5                   10


<210>  626
<211>  10
<212>  PRT
<213>  Artificial Sequence

<220>
<223>  mutant loop

<400>  626

Ser Asn Leu Arg Trp Thr Lys Gly Lys Val
1               5                   10


<210>  627
<211>  10
<212>  PRT
<213>  Artificial Sequence

<220>
<223>  mutant loop

<400>  627

Ser Arg Tyr Arg Trp Gln Thr Gly Asn Val
1               5                   10

<210>  628
<211>  10
<212>  PRT
<213>  Artificial Sequence

<220>
<223>  mutant loop

<400>  628

Ser Gln Arg Arg Trp Ala Ala Gly Asn Val
1               5                   10


<210>  629
<211>  10
<212>  PRT
<213>  Artificial Sequence

<220>
<223>  mutant loop

<400>  629

Ser Ser Leu Arg Trp Thr Lys Gly Gly Val
1               5                   10


<210>  630
<211>  10
<212>  PRT
<213>  Artificial Sequence

<220>
<223>  mutant loop

<400>  630

Ser Ala Leu Arg Trp Thr Ser Gly Asn Val
1               5                   10


<210>  631
<211>  10
<212>  PRT
<213>  Artificial Sequence

<220>
<223>  mutant loop

<400>  631

Ser Leu Tyr Arg Trp Met Ser Gly Asn Val
1               5                   10


<210>  632
<211>  10
<212>  PRT
<213>  Artificial Sequence

<220>
<223>  mutant loop

<400>  632

Ser Trp Trp Arg Trp Tyr Arg Gly Asn Val
1               5                   10


<210>  633
<211>  10
<212>  PRT
<213>  Artificial Sequence

<220>
<223>  mutant loop

<400>  633

Lys Gln Glu Arg Trp Ser Leu Gly Asn Val
1               5                   10


<210>  634
<211>  10
<212>  PRT
<213>  Artificial Sequence

<220>
<223>  mutant loop

<400>  634

Ser Ala Leu Arg Trp Leu Ser Gly Asn Val
1               5                   10


<210>  635
<211>  10
<212>  PRT
<213>  Artificial Sequence

<220>
<223>  mutant loop

<400>  635

Ser Ala Leu Arg Trp Glu Gln Gly Asn Val
1               5                   10


<210>  636
<211>  10
<212>  PRT
<213>  Artificial Sequence

<220>
<223>  mutant loop

<400>  636

Ser Tyr Tyr Arg Trp Leu Ser Gly Asn Val
1               5                   10


<210>  637
<211>  10
<212>  PRT
<213>  Artificial Sequence

<220>
<223>  mutant loop

<400>  637

Ser Ser Tyr Arg Trp Ala Ala Gly Asn Val
1               5                   10


<210>  638
<211>  10
<212>  PRT
<213>  Artificial Sequence

<220>
<223>  mutant loop

<400>  638

Ser Tyr Tyr Arg Trp His Lys Gly Asn Val
1               5                   10


<210>  639
<211>  10
<212>  PRT
<213>  Artificial Sequence

<220>
<223>  mutant loop

<400>  639

Ser Ser Arg Arg Trp Glu Ser Gly Asn Val
1               5                   10


<210>  640
<211>  10
<212>  PRT
<213>  Artificial Sequence

<220>
<223>  mutant loop

<400>  640

Ser Gln Arg Arg Trp Met Ser Gly Asn Val
1               5                   10


<210>  641
<211>  10
<212>  PRT
<213>  Artificial Sequence

<220>
<223>  mutant loop

<400>  641

Ser Gln Arg Arg Trp Glu Leu Gly Asp Val
1               5                   10


<210>  642
<211>  10
<212>  PRT
<213>  Artificial Sequence

<220>

<223>  mutant loop

<400>  642

Ser Gln Arg Arg Trp Ser Lys Gly Ser Val
1               5                   10


<210>  643
<211>  10
<212>  PRT
<213>  Artificial Sequence

<220>
<223>  mutant loop

<400>  643

Ser Gln Leu Arg Trp Lys Asn Gly Lys Ala
1               5                   10


<210>  644
<211>  10
<212>  PRT
<213>  Artificial Sequence

<220>
<223>  mutant loop

<400>  644

Ser Gln Leu Arg Trp Ser Lys Gly Asn Ala
1               5                   10


<210>  645
<211>  10
<212>  PRT
<213>  Artificial Sequence

<220>
<223>  mutant loop

<400>  645

Ser Gln Leu Arg Trp Ala Thr Arg Lys Val
1               5                   10


<210>  646
<211>  10
<212>  PRT
<213>  Artificial Sequence

<220>
<223>  mutant loop

<400>  646

Ser Gln Leu Arg Trp Met Ser Arg Lys Val
1               5                   10


<210>  647
<211>  10
<212>  PRT
<213>  Artificial Sequence

<220>
<223>  mutant loop

<400>  647

Ser Ala Leu Arg Trp Ser Asn Arg Lys Val
1               5                   10


<210>  648
<211>  10
<212>  PRT
<213>  Artificial Sequence

<220>
<223>  mutant loop

<400>  648

Ser Ser Leu Arg Trp Thr Lys Gly Tyr Val
1               5                   10


<210>  649
<211>  10
<212>  PRT
<213>  Artificial Sequence

<220>
<223>  mutant loop

<400>  649

Ser His Leu Arg Trp Pro Lys Gly Asn Val
1               5                   10


<210>  650
<211>  10
<212>  PRT
<213>  Artificial Sequence

<220>
<223>  mutant loop

<400>  650

Ser Ala Leu Arg Trp Thr Lys Gly Asn Val
1               5                   10


<210>  651
<211>  10
<212>  PRT
<213>  Artificial Sequence

<220>
<223>  mutant loop

<400>  651

Ser Ala Leu Arg Trp Thr Asn Gly Lys Val
1               5                   10


<210>  652
<211>  10

<212>  PRT
<213>  Artificial Sequence

<220>
<223>  mutant loop

<400>  652

Ser Lys Leu Arg Trp Thr Asn Gly Ile Val
1               5                   10


<210>  653
<211>  10
<212>  PRT
<213>  Artificial Sequence

<220>
<223>  mutant loop

<400>  653

Ser Ser Leu Arg Trp Thr Ser Gly Arg Ile
1               5                   10


<210>  654
<211>  10
<212>  PRT
<213>  Artificial Sequence

<220>
<223>  mutant loop

<400>  654

Ser Asn Leu Arg Trp Thr His Gly Asn Gly
1               5                   10


<210>  655
<211>  10
<212>  PRT
<213>  Artificial Sequence

<220>
<223>  mutant loop

<400>  655

Ser Gln Leu Arg Trp Arg Ala Gly Lys Val
1               5                   10


<210>  656
<211>  10
<212>  PRT
<213>  Artificial Sequence

<220>
<223>  mutant loop

<400>  656

Ser Gln Leu Arg Trp Thr Lys Gly Ser Val
1               5                   10

<210>  657
<211>  10
<212>  PRT
<213>  Artificial Sequence

<220>
<223>  mutant loop

<400>  657

Ser Gln Leu Arg Trp Thr Lys Gly Gly Val
1               5                   10


<210>  658
<211>  10
<212>  PRT
<213>  Artificial Sequence

<220>
<223>  mutant loop

<400>  658

Ser Ala Leu Arg Trp Thr Lys Gly Lys Val
1               5                   10


<210>  659
<211>  10
<212>  PRT
<213>  Artificial Sequence

<220>
<223>  mutant loop

<400>  659

Ser Ser Leu Arg Trp Thr Lys Gly Lys Val
1               5                   10


<210>  660
<211>  10
<212>  PRT
<213>  Artificial Sequence

<220>
<223>  mutant loop

<400>  660

Ser His Leu Arg Trp Thr Lys Gly Lys Val
1               5                   10


<210>  661
<211>  10
<212>  PRT
<213>  Artificial Sequence

<220>
<223>  mutant loop

<400>  661

Ser Gln Leu Arg Trp Thr Lys Gly Lys Gly
1               5                   10

<210>  662
<211>  10
<212>  PRT
<213>  Artificial Sequence

<220>
<223>  mutant loop

<400>  662

Ser Trp Leu Arg Trp His Ser Gly Asn Val
1               5                   10


<210>  663
<211>  10
<212>  PRT
<213>  Artificial Sequence

<220>
<223>  mutant loop

<400>  663

Ser His Tyr Arg Trp Ile Ser Gly Asn Val
1               5                   10


<210>  664
<211>  10
<212>  PRT
<213>  Artificial Sequence

<220>
<223>  mutant loop

<400>  664

Ser Asn Leu Arg Trp Arg Ala Gly Arg Val
1               5                   10


<210>  665
<211>  10
<212>  PRT
<213>  Artificial Sequence

<220>
<223>  mutant loop

<400>  665

Ser Ser Leu Arg Trp Arg Ser Gly Arg Val
1               5                   10


<210>  666
<211>  10
<212>  PRT
<213>  Artificial Sequence

<220>
<223>  mutant loop

<400>  666

Ser Ala Leu Arg Trp Thr Ser Gly Arg Val
1               5                   10


<210>  667
<211>  10
<212>  PRT
<213>  Artificial Sequence

<220>
<223>  mutant loop

<400>  667

Ser Ala Leu Arg Trp Met Ser Arg Lys Lys
1               5                   10


<210>  668
<211>  10
<212>  PRT
<213>  Artificial Sequence

<220>
<223>  mutant loop

<400>  668

Ser Ala Leu Arg Trp Ser Ser Gly Lys Val
1               5                   10


<210>  669
<211>  10
<212>  PRT
<213>  Artificial Sequence

<220>
<223>  mutant loop

<400>  669

Ser Ser Leu Arg Trp Ala Ser Gly Lys Val
1               5                   10


<210>  670
<211>  10
<212>  PRT
<213>  Artificial Sequence

<220>
<223>  mutant loop

<400>  670

Ser Thr Leu Arg Trp Ser Ala Gly Lys Val
1               5                   10


<210>  671
<211>  10
<212>  PRT
<213>  Artificial Sequence

<220>
<223>  mutant loop

<400>  671

Ser Gln Leu Arg Trp Thr Lys Gly Asp Val
1               5                   10


<210>  672
<211>  10
<212>  PRT
<213>  Artificial Sequence

<220>
<223>  mutant loop

<400>  672

Ser Trp Trp Arg Trp Tyr Lys Gly Asn Val
1               5                   10


<210>  673
<211>  10
<212>  PRT
<213>  Artificial Sequence

<220>
<223>  mutant loop

<400>  673

Ser Trp His Arg Trp Phe Lys Gly Asn Val
1               5                   10


<210>  674
<211>  10
<212>  PRT
<213>  Artificial Sequence

<220>
<223>  mutant loop

<400>  674

Ser His Trp Arg Trp Met Arg Gly Asn Val
1               5                   10


<210>  675
<211>  10
<212>  PRT
<213>  Artificial Sequence

<220>
<223>  mutant loop

<400>  675

Ser Tyr Tyr Arg Trp Phe Ser Gly Asn Val
1               5                   10


<210>  676
<211>  10
<212>  PRT
<213>  Artificial Sequence

<220>

<223>  mutant loop

<400>  676

Ser Ser Leu Arg Trp Ala Ser Gly Asn Val
1               5                   10


<210>  677
<211>  10
<212>  PRT
<213>  Artificial Sequence

<220>
<223>  mutant loop

<400>  677

Ser Tyr Leu Arg Trp Thr Ser Gly Arg Ile
1               5                   10


<210>  678
<211>  10
<212>  PRT
<213>  Artificial Sequence

<220>
<223>  mutant loop

<400>  678

Ser Gln Leu Arg Trp Thr Ala Gly Arg Val
1               5                   10


<210>  679
<211>  10
<212>  PRT
<213>  Artificial Sequence

<220>
<223>  mutant loop

<400>  679

Ser Thr Leu Arg Trp Thr Ser Gly Asp Val
1               5                   10


<210>  680
<211>  10
<212>  PRT
<213>  Artificial Sequence

<220>
<223>  mutant loop

<400>  680

Ser Asn Leu Arg Trp Thr Lys Gly Tyr Val
1               5                   10


<210>  681
<211>  10
<212>  PRT
<213>  Artificial Sequence

<220>
<223>  mutant loop

<400>  681

Ser Asn Leu Arg Trp Thr Lys Gly Ser Val
1               5                   10


<210>  682
<211>  10
<212>  PRT
<213>  Artificial Sequence

<220>
<223>  mutant loop

<400>  682

Ser Ser Leu Arg Trp Thr Lys Gly Ser Val
1               5                   10


<210>  683
<211>  10
<212>  PRT
<213>  Artificial Sequence

<220>
<223>  mutant loop

<400>  683

Ser Ser Leu Arg Trp Ser Lys Gly Gly Val
1               5                   10


<210>  684
<211>  10
<212>  PRT
<213>  Artificial Sequence

<220>
<223>  mutant loop

<400>  684

Ser Ser Leu Arg Trp Pro Lys Gly Gly Val
1               5                   10


<210>  685
<211>  10
<212>  PRT
<213>  Artificial Sequence

<220>
<223>  mutant loop

<400>  685

Ser Gln Leu Arg Trp Asp Ser Gly Ala Trp
1               5                   10


<210>  686
<211>  10

<212>  PRT
<213>  Artificial Sequence

<220>
<223>  mutant loop

<400>  686

Ser Gln Leu Arg Trp Ala Ser Gly Lys Val
1               5                   10


<210>  687
<211>  10
<212>  PRT
<213>  Artificial Sequence

<220>
<223>  mutant loop

<400>  687

Ser Gln Leu Arg Trp Ser Ser Gly Lys Val
1               5                   10


<210>  688
<211>  7
<212>  PRT
<213>  Artificial Sequence

<220>
<223>  mutant loop

<400>  688

Asp Lys Ser Arg Trp Gln Gln
1               5


<210>  689
<211>  7
<212>  PRT
<213>  Artificial Sequence

<220>
<223>  mutant loop

<400>  689

Pro Ser Lys Gln Gly Ser Phe
1               5


<210>  690
<211>  7
<212>  PRT
<213>  Artificial Sequence

<220>
<223>  mutant loop

<400>  690

Pro Ser Asn Ala Gln Glu Phe
1               5

<210>  691
<211>  7
<212>  PRT
<213>  Artificial Sequence

<220>
<223>  mutant loop

<400>  691

Pro Ala Tyr Asn Tyr Ile His
1               5


<210>  692
<211>  7
<212>  PRT
<213>  Artificial Sequence

<220>
<223>  mutant loop

<400>  692

Pro Pro Ala Ala Thr Ser His
1               5


<210>  693
<211>  7
<212>  PRT
<213>  Artificial Sequence

<220>
<223>  mutant loop

<400>  693

Pro Leu Ala Leu Pro Asp Met
1               5


<210>  694
<211>  7
<212>  PRT
<213>  Artificial Sequence

<220>
<223>  mutant loop

<400>  694

Pro Asn Asp Phe His Ser Val
1               5


<210>  695
<211>  7
<212>  PRT
<213>  Artificial Sequence

<220>
<223>  mutant loop

<400>  695

Tyr Leu Asp His Gly His Pro
1               5

<210>  696
<211>  7
<212>  PRT
<213>  Artificial Sequence

<220>
<223>  mutant loop

<400>  696

Ser Thr Asp Lys Val Trp Ala
1               5


<210>  697
<211>  7
<212>  PRT
<213>  Artificial Sequence

<220>
<223>  mutant loop

<400>  697

Gly Tyr Gly Lys Val Gly Gln
1               5


<210>  698
<211>  7
<212>  PRT
<213>  Artificial Sequence

<220>
<223>  mutant loop

<400>  698

Asp Asn Val Lys Glu Tyr Lys
1               5


<210>  699
<211>  7
<212>  PRT
<213>  Artificial Sequence

<220>
<223>  mutant loop

<400>  699

Ser Asn Asn Gln Asp Thr Thr
1               5


<210>  700
<211>  7
<212>  PRT
<213>  Artificial Sequence

<220>
<223>  mutant loop

<400>  700

Ser Ser Pro Tyr Met Asn Gly
1               5


<210>  701
<211>  7
<212>  PRT
<213>  Artificial Sequence

<220>
<223>  mutant loop

<400>  701

Val Gly Tyr Ser Leu Arg Gln
1               5


<210>  702
<211>  7
<212>  PRT
<213>  Artificial Sequence

<220>
<223>  mutant loop

<400>  702

Pro Lys Met Tyr Ser Met Ala
1               5


<210>  703
<211>  7
<212>  PRT
<213>  Artificial Sequence

<220>
<223>  mutant loop

<400>  703

Asp Ser Gly Arg Trp Leu Gly
1               5


<210>  704
<211>  7
<212>  PRT
<213>  Artificial Sequence

<220>
<223>  mutant loop

<400>  704

Asp Arg Glu Arg Trp Arg Arg
1               5


<210>  705
<211>  7
<212>  PRT
<213>  Artificial Sequence

<220>
<223>  mutant loop

<400>  705

Ile Gln Asp Arg Trp Gln Gln
1               5


<210>  706
<211>  7
<212>  PRT
<213>  Artificial Sequence

<220>
<223>  mutant loop

<400>  706

Ser Trp Met Glu Trp Ser Gln
1               5


<210>  707
<211>  7
<212>  PRT
<213>  Artificial Sequence

<220>
<223>  mutant loop

<400>  707

Met Gln Asp Gln Trp Trp Gln
1               5


<210>  708
<211>  7
<212>  PRT
<213>  Artificial Sequence

<220>
<223>  mutant loop

<400>  708

Ser Glu Met Arg Trp Lys Arg
1               5


<210>  709
<211>  7
<212>  PRT
<213>  Artificial Sequence

<220>
<223>  mutant loop

<400>  709

Ser His Leu Arg Trp Thr Asn
1               5


<210>  710
<211>  7
<212>  PRT
<213>  Artificial Sequence

<220>

<223>  mutant loop

<400>  710

Asp Arg Ile Arg Trp Pro Ala
1               5


<210>  711
<211>  7
<212>  PRT
<213>  Artificial Sequence

<220>
<223>  mutant loop

<400>  711

Ser Arg Ala Arg Trp Arg His
1               5


<210>  712
<211>  7
<212>  PRT
<213>  Artificial Sequence

<220>
<223>  mutant loop

<400>  712

Ala Ser Gln Arg Trp Arg Asn
1               5


<210>  713
<211>  7
<212>  PRT
<213>  Artificial Sequence

<220>
<223>  mutant loop

<400>  713

Asn Glu His Arg Trp Thr Met
1               5


<210>  714
<211>  7
<212>  PRT
<213>  Artificial Sequence

<220>
<223>  mutant loop

<400>  714

Val Glu Asn Arg Trp Thr Met
1               5


<210>  715
<211>  7
<212>  PRT
<213>  Artificial Sequence

<220>
<223>  mutant loop

<400>  715

Gln His Asn Arg Trp Glu Ser
1               5


<210>  716
<211>  7
<212>  PRT
<213>  Artificial Sequence

<220>
<223>  mutant loop

<400>  716

Ser Asn Arg Arg Trp Met Ser
1               5


<210>  717
<211>  7
<212>  PRT
<213>  Artificial Sequence

<220>
<223>  mutant loop

<400>  717

Ser Asn Met Arg Trp Thr Ser
1               5


<210>  718
<211>  7
<212>  PRT
<213>  Artificial Sequence

<220>
<223>  mutant loop

<400>  718

Lys Tyr Asp Arg Trp Thr Ala
1               5


<210>  719
<211>  7
<212>  PRT
<213>  Artificial Sequence

<220>
<223>  mutant loop

<400>  719

Ala Glu Asp Arg Trp Met Asn
1               5


<210>  720
<211>  7

<212>  PRT
<213>  Artificial Sequence

<220>
<223>  mutant loop

<400>  720

Val Lys Ser Arg Trp Gln Ala
1               5


<210>  721
<211>  7
<212>  PRT
<213>  Artificial Sequence

<220>
<223>  mutant loop

<400>  721

Glu Lys Ser Arg Trp Leu Thr
1               5


<210>  722
<211>  7
<212>  PRT
<213>  Artificial Sequence

<220>
<223>  mutant loop

<400>  722

Met Lys Asp Arg Trp His Gly
1               5


<210>  723
<211>  7
<212>  PRT
<213>  Artificial Sequence

<220>
<223>  mutant loop

<400>  723

Pro Lys Asn Met Trp Trp Asn
1               5


<210>  724
<211>  7
<212>  PRT
<213>  Artificial Sequence

<220>
<223>  mutant loop

<400>  724

Asp Ser Gln Arg Trp Val Gln
1               5

<210>  725
<211>  7
<212>  PRT
<213>  Artificial Sequence

<220>
<223>  mutant loop

<400>  725

Pro Trp Lys Arg Lys Ser Lys
1               5


<210>  726
<211>  7
<212>  PRT
<213>  Artificial Sequence

<220>
<223>  mutant loop

<400>  726

Pro His Ala Arg Trp Val Pro
1               5


<210>  727
<211>  7
<212>  PRT
<213>  Artificial Sequence

<220>
<223>  mutant loop

<400>  727

Pro Ser Trp Arg Trp Tyr Tyr
1               5


<210>  728
<211>  7
<212>  PRT
<213>  Artificial Sequence

<220>
<223>  mutant loop

<400>  728

Pro Leu Pro Leu Pro Pro Pro
1               5


<210>  729
<211>  7
<212>  PRT
<213>  Artificial Sequence

<220>
<223>  mutant loop

<400>  729

Ser Ser Gln Arg Trp Leu Glu
1               5

<210>  730
<211>  7
<212>  PRT
<213>  Artificial Sequence

<220>
<223>  mutant loop

<400>  730

Ser Glu Met Arg Trp Asn Gly
1               5


<210>  731
<211>  7
<212>  PRT
<213>  Artificial Sequence

<220>
<223>  mutant loop

<400>  731

Asp Asp Tyr Arg Trp His Ile
1               5


<210>  732
<211>  7
<212>  PRT
<213>  Artificial Sequence

<220>
<223>  mutant loop

<400>  732

Pro Glu Asn Arg Trp His Arg
1               5


<210>  733
<211>  7
<212>  PRT
<213>  Artificial Sequence

<220>
<223>  mutant loop

<400>  733

Ser Tyr Asn Arg Trp Asn Arg
1               5


<210>  734
<211>  7
<212>  PRT
<213>  Artificial Sequence

<220>
<223>  mutant loop

<400>  734

Leu Gly Asn Arg Trp Lys Ser
1               5

<210>  735
<211>  7
<212>  PRT
<213>  Artificial Sequence

<220>
<223>  mutant loop

<400>  735

Pro His Asp Asp Trp Trp Ser
1               5

<210>  736
<211>  7
<212>  PRT
<213>  Artificial Sequence

<220>
<223>  mutant loop

<400>  736

Pro Ser Val Arg Trp Gly Gly
1               5

<210>  737
<211>  7
<212>  PRT
<213>  Artificial Sequence

<220>
<223>  mutant loop

<400>  737

Pro Trp Lys Arg Lys Ser Lys
1               5

<210>  738
<211>  7
<212>  PRT
<213>  Artificial Sequence

<220>
<223>  mutant loop

<400>  738

Pro Ser Trp Arg Trp Tyr Tyr
1               5

<210>  739
<211>  7
<212>  PRT
<213>  Artificial Sequence

<220>
<223>  mutant loop

<400>  739

Pro His Ser Trp Trp Leu Val
1               5


<210>  740
<211>  7
<212>  PRT
<213>  Artificial Sequence

<220>
<223>  mutant loop

<400>  740

Pro Phe Glu Ala Phe Gln Gln
1               5


<210>  741
<211>  7
<212>  PRT
<213>  Artificial Sequence

<220>
<223>  mutant loop

<400>  741

Pro Trp Ile Met Asp Lys His
1               5


<210>  742
<211>  7
<212>  PRT
<213>  Artificial Sequence

<220>
<223>  mutant loop

<400>  742

Pro Pro Pro Ser Pro Pro Leu
1               5


<210>  743
<211>  7
<212>  PRT
<213>  Artificial Sequence

<220>
<223>  mutant loop

<400>  743

Pro Gly Tyr Met Val Pro Leu
1               5


<210>  744
<211>  7
<212>  PRT
<213>  Artificial Sequence

<220>

<223>  mutant loop

<400>  744

Asp Arg Gln His His Asn Asp
1               5


<210>  745
<211>  7
<212>  PRT
<213>  Artificial Sequence

<220>
<223>  mutant loop

<400>  745

Asp Ala Pro Gln Pro Val Glu
1               5


<210>  746
<211>  7
<212>  PRT
<213>  Artificial Sequence

<220>
<223>  mutant loop

<400>  746

Tyr Glu Val Thr Glu Glu Pro
1               5


<210>  747
<211>  242
<212>  PRT
<213>  artificial

<220>
<223>  EAM151-5 Fcab

<400>  747

Met Glu Leu Gly Leu Ser Trp Ile Phe Leu Leu Ala Ile Leu Lys Gly
1               5                   10                  15


Val Gln Cys Thr Cys Pro Pro Cys Pro Ala Pro Glu Leu Leu Gly Gly
            20                  25                  30


Pro Ser Val Phe Leu Phe Pro Pro Lys Pro Lys Asp Thr Leu Met Ile
        35                  40                  45


Ser Arg Thr Pro Glu Val Thr Cys Val Val Val Asp Val Ser His Glu
    50                  55                  60


Asp Pro Glu Val Lys Phe Asn Trp Tyr Val Asp Gly Val Glu Val His
65                  70                  75                  80


Asn Ala Lys Thr Lys Pro Arg Glu Glu Gln Tyr Asn Ser Thr Tyr Arg
                85                  90                  95

Val Val Ser Val Leu Thr Val Leu His Gln Asp Trp Leu Asn Gly Lys
            100                 105                 110

Glu Tyr Lys Cys Lys Val Ser Asn Lys Ala Leu Pro Ala Pro Ile Glu
        115                 120                 125

Lys Thr Ile Ser Lys Ala Lys Gly Gln Pro Arg Glu Pro Gln Val Tyr
    130                 135                 140

Thr Leu Pro Pro Ser Arg Asp Glu Thr Glu Ser Gly Pro Val Ser Leu
145                 150                 155                 160

Thr Cys Leu Val Lys Gly Phe Tyr Pro Ser Asp Ile Val Val Glu Trp
                165                 170                 175

Glu Ser Lys Phe Gly Pro Glu Asn Asn Tyr Lys Thr Thr Pro Pro Val
            180                 185                 190

Leu Asp Ser Asp Gly Ser Phe Phe Leu Tyr Ser Lys Leu Thr Val Ser
        195                 200                 205

Asn Leu Arg Trp Thr Lys Gly His Val Phe Ser Cys Ser Val Met His
    210                 215                 220

Glu Ala Leu His Asn His Tyr Thr Gln Lys Ser Leu Ser Leu Ser Pro
225                 230                 235                 240

Gly Lys

<210>  748
<211>  242
<212>  PRT
<213>  human

<400>  748

Met Glu Leu Gly Leu Ser Trp Ile Phe Leu Leu Ala Ile Leu Lys Gly
1               5                   10                  15

Val Gln Cys Thr Cys Pro Pro Cys Pro Ala Pro Glu Leu Leu Gly Gly
            20                  25                  30

Pro Ser Val Phe Leu Phe Pro Pro Lys Pro Lys Asp Thr Leu Met Ile
        35                  40                  45

Ser Arg Thr Pro Glu Val Thr Cys Val Val Val Asp Val Ser His Glu
    50                  55                  60

Asp Pro Glu Val Lys Phe Asn Trp Tyr Val Asp Gly Val Glu Val His
65                  70                  75                  80

Asn Ala Lys Thr Lys Pro Arg Glu Glu Gln Tyr Asn Ser Thr Tyr Arg
                85                  90                  95

Val Val Ser Val Leu Thr Val Leu His Gln Asp Trp Leu Asn Gly Lys
            100                 105                 110

Glu Tyr Lys Cys Lys Val Ser Asn Lys Ala Leu Pro Ala Pro Ile Glu
        115                 120                 125

Lys Thr Ile Ser Lys Ala Lys Gly Gln Pro Arg Glu Pro Gln Val Tyr
    130                 135                 140

Thr Leu Pro Pro Ser Arg Asp Glu Leu Thr Lys Asn Gln Val Ser Leu
145                 150                 155                 160

Thr Cys Leu Val Lys Gly Phe Tyr Pro Ser Asp Ile Ala Val Glu Trp
                165                 170                 175

Glu Ser Asn Gly Gln Pro Glu Asn Asn Tyr Lys Thr Thr Pro Pro Val
            180                 185                 190

Leu Asp Ser Asp Gly Ser Phe Phe Leu Tyr Ser Lys Leu Thr Val Asp
        195                 200                 205

Lys Ser Arg Trp Gln Gln Gly Asn Val Phe Ser Cys Ser Val Met His
    210                 215                 220

Glu Ala Leu His Asn His Tyr Thr Gln Lys Ser Leu Ser Leu Ser Pro
225                 230                 235                 240

Gly Lys

## Description

The invention relates to an internalising modular antibody based on one or more immunoglobulin domains having at least two specificities and an Fc fragment specifically binding an EGFR epitope.

### Background

Monoclonal antibodies have been widely used as therapeutic binding agents. The basic antibody structure will be explained here using as example an intact IgG1 immunoglobulin.

Two identical heavy (H) and two identical light (L) chains combine to form the Y-shaped antibody molecule. The heavy chains each have four domains. The amino terminal variable domains (VH) are at the tips of the Y. These are followed by three constant domains: CH1, CH2, and the carboxy-terminal CH3, at the base of the Y's stem. A short stretch, the switch, connects the heavy chain variable and constant regions. The hinge connects CH2 and CH3 (the Fc fragment) to the remainder of the antibody (the Fab fragments). One Fc and two identical Fab fragments can be produced by proteolytic cleavage of the hinge in an intact antibody molecule. The light chains are constructed of two domains, variable (VL) and constant (CL), separated by a switch.

Disulfide bonds in the hinge region connect the two heavy chains. The light chains are coupled to the heavy chains by additional disulfide bonds. Asn-linked carbohydrate moieties are attached at different positions in constant domains depending on the class of immunoglobulin. For IgG1 two disulfide bonds in the hinge region, between Cys235 and Cys238 pairs, unite the two heavy chains. The light chains are coupled to the heavy chains by two additional disulfide bonds, between Cys229s in the CH1 domains and Cys214s in the CL domains. Carbohydrate moieties are attached to Asn306 of each CH2, generating a pronounced bulge in the stem of the Y.

These features have profound functional consequences. The variable regions of both the heavy and light chains (VH) and (VL) lay at the N-terminal region, i.e. the "tips" of the Y, where they are positioned to react with antigen. This tip of the molecule is the side on which the N-terminus of the amino acid sequence is located. The stem of the Y projects in a way to efficiently mediate effector functions such as the activation of complement and interaction with Fc receptors, or ADCC and ADCP. Its CH2 and CH3 domains bulge to facilitate interaction with effector proteins. The C-terminus of the amino acid sequence is located on the opposite side of the tip, which can be termed "bottom" of the Y.

Two types of light chain, termed lambda (λ) and kappa (κ), are found in antibodies. A given immunoglobulin either has κ chains or λ chains, never one of each. No functional difference has been found between antibodies having λ or κ light chains.

Each domain in an antibody molecule has a similar structure of two beta sheets packed tightly against each other in a compressed antiparallel beta barrel. This conserved structure is termed the immunoglobulin fold. The immunoglobulin fold of constant domains contains a 3-stranded sheet packed against a 4-stranded sheet. The fold is stabilized by hydrogen bonding between the beta strands of each sheet, by hydrophobic bonding between residues of opposite sheets in the interior, and by a disulfide bond between the sheets. The 3-stranded sheet comprises strands C, F, and G, and the 4-stranded sheet has strands A, B, E, and D. The letters A through G denote the sequential positions of the beta strands along the amino acid sequence of the immunoglobulin fold.

The fold of variable domains has 9 beta strands arranged in two sheets of 4 and 5 strands. The 5-stranded sheet is structurally homologous to the 3-stranded sheet of constant domains, but contains the extra strands C' and C". The remainder of the strands (A, B, C, D, E, F, G) have the same topology and similar structure as their counterparts in constant domain immunoglobulin folds. A disulfide bond links strands B and F in opposite sheets, as in constant domains.

The variable domains of both light and heavy immunoglobulin chains contain three hypervariable loops, or complementarity-determining regions (CDRs). The three CDRs of a V domain (CDR1, CDR2, CDR3) cluster at one end of the beta barrel. The CDRs are loops that connect beta strands B-C, C'-C", and F-G of the immunoglobulin fold. The residues in the CDRs vary from one immunoglobulin molecule to the next, imparting antigen specificity to each antibody.

The VL and VH domains at the tips of antibody molecules are closely packed such that the 6 CDRs (3 on each domain) cooperate in constructing a surface (or cavity) for antigen-specific binding. The natural antigen binding site of an antibody thus is composed of the loops which connect strands B-C, C'-C", and F-G of the light chain variable domain and strands B-C, C'-C", and F-G of the heavy chain variable domain.

The loops which are not CDR-loops in a native immunoglobulin, or not part of the antigen-binding pocket as determined by the CDR loops and optionally adjacent loops within the CDR loop region, do not have antigen binding or epitope binding specificity, but contribute to the correct folding of the entire immunoglobulin molecule and/or its effector or other functions and are therefore called structural loops for the purpose of this invention.

Prior art documents show that the immunoglobulin-like scaffold has been employed so far for the purpose of manipulating the existing antigen binding site, thereby introducing novel binding properties. In most cases the CDR regions have been engineered for antigen binding, in other words, in the case of the immunoglobulin fold, only the natural antigen binding site has been modified in order to change its binding affinity or specificity. A vast body of literature exists which describes different formats of such manipulated immunoglobulins, frequently expressed in the form of single-chain Fv fragments (scFv) or Fab fragments, either displayed on the surface of phage particles or solubly expressed in various prokaryotic or eukaryotic expression systems.

WO06/072620A1 describes a method of engineering an immunoglobulin which comprises a modification in a structural loop region to obtain new antigen binding sites. This method is broadly applicable to immunoglobulins and may be used to produce a library of immunoglobulins targeting a variety of antigens. A CH3 library has been shown to be useful for selecting specific binders to an antigen.

WO08/003103A2 describes the panning of a CH3, CH1 or CL library on a synthetic peptide, representing a mimotope of the CD20 antigen.

Immunoglobulins targeting a receptor of the erbB class have been widely used for treating cancer patients. Among those receptors are EGFR (Her1), Her2, Her2neu, Her3 and Her4.

ErbB receptors are made up of an extra-cellular region or ectodomain that contains approximately 620 amino acids, a single transmembrane-spanning region, and a cytoplasmic tyrosine kinase domain. The extra-cellular region of each family member is made up of four subdomains, L1, CR1, L2, and CR2, where "L" signifies a leucine-rich repeat domain and "CR" a cysteine-rich region. These subdomains are also referred to as domains I-IV, respectively.

Herceptin (trastuzumab, humAb4D5) is a product based on a monoclonal antibody for use in breast cancer therapy. Herceptin antibody is specific for the 4D5 epitope of the HER2 extracellular domain of her2neu (also called c-erbB-2 or MAC117).

The extra-cellular portion of the HER2 receptor, "HER2 extracellular domain" or "HER2 ECD" refers to a domain of HER2 that is outside of a cell, either anchored to a cell membrane, or in circulation, including fragments thereof. The extracellular domain of HER2 may comprise four domains: "Domain I" (amino acid residues from about 1-195, "Domain II" (amino acid residues from about 196-319), "Domain III" (amino acid residues from about 320-488), and "Domain IV" (amino acid residues from about 489-630) (residue numbering without signal peptide).

The "epitope 4D5" is the region in the extracellular domain of HER2 to which the antibody 4D5 (ATCC CRL 10463) and trastuzumab bind. This epitope is close to the transmembrane domain of HER2, and within Domain IV of HER2. The 4D5 epitope of HER2 encompasses any one or more residues in the region from about residue 529 to about residue 625, inclusive of the HER2 ECD, residue numbering including signal peptide.

The EGFR is a large (1,186 residues), monomeric glycoprotein with a single transmembrane region and a cytoplasmic tyrosine kinase domain flanked by noncatalytic regulatory regions. Sequence analyses have shown that the ectodomain (residues 1-621) contains four sub-domains, here termed L1, CR1, L2 and CR2, where L and CR are acronyms for large and Cys-rich respectively. The L1 and L2 domains have also been referred to as domains I and III, respectively. The CR domains have been previously referred to as domains II and IV, or as S1.1-S1.3 and S2.1-S2.3 where S is an abbreviation for small.

MAbs to the external domain of the EGFR, the extra-cellular portion of the receptor, have been developed that disrupt ligand binding to the receptor and subsequent signal transduction. Three EGFR-specific blocking antibodies have been characterized in greater detail in vitro and are presently used in clinical studies; these are mAbC225 (ERBITUX/cetuximab), mAb425 (EMD72000) and the human mAb ABX-EGF. C225 (ERBITUX/cetuximab) is FDA approved for metastatic colorectal cancer and mAb425 (EMD59000) whose humanized version (EMD72000) is currently in phase II clinical trials for various solid tumors expressing EGFr. C225 binds to distinct epitopes on the extracellular domain of EGFr. Independent binding of both antibodies to the wild type receptor and to the mutant receptor (EGFrVIII) which is prominently expressed in tumor cells, has been shown. Cetuximab interacts exclusively with domain III of the extracellular region of EGFR (sEGFR), particularly occluding the ligand binding region on this domain and sterically preventing the receptor from dimerization.

The spontaneously occurring mutant EGF receptor was first shown in glioblastoma. Known as EGFRvIII, this molecule represents a deletion of exons 2 through 7 in the extracellular domain of the EGF receptor. This removes 273 amino acids and creates a novel glycine at the fusion junction. The EGFRvIII (variously called de2-7 EGFR or deltaEGFR) has an in-frame deletion of the extracellular domain and is found in numerous types of human tumors.

WO97/20858A1 relates to anti-Her2 antibodies which induce apoptosis in Her2 expressing cells. Therefore the monoclonal antibodies (mAbs), which bind to Her2, are generated by immunizing mice with purified soluble Her2.

WO06/087637A2 relates to antibodies that recognise Her2/neu and exert an antiproliferative effect on Her2/neu expressing cells. This document describes an isolated antibody or a fragment, variant or derivative thereof, in particular the human Fab fragment, and the scFv fragment, capable of specifically binding to Her2neu.

Some prior art disclosures relate to antibody formats with a potential to inhibit tumor growth, in the absence of cytotoxic activities, such as ADCC.

Rovers et al (Cancer Immunol. Immunother. (2007) 56:303-317) describe anti-EGFR nanobodies with a potential to inhibit tumour cell growth through a process called acinosis.

WO06/036834A2 describes a biologically active peptide incorporated as an internal sequence into a loop region of an Fc domain; the specification concerns a molecule of which the internal peptide sequence may be added by insertion or replacement of amino acids in the previously existing Fc domain. An exemplary peptide is targeting p185HER2/neu.

WO09/132876A1 describes a cytotoxic modular antibody with a molecular weight up to 60kD, specifically binding to a cell surface target with a high binding affinity, and eventual ADCC, ADCP, CDC or apoptotic activity. Specific examples are directed to an Fc fragment of an antibody binding to the Her2neu epitope.

Other targeted cancer therapies employ antibodies directed against soluble tumor-associated antigens (sTAG). Several of these soluble factors act along the same type of pathways that lead from the plasma membrane, from growth factors like vascular endothelial growth factor (VEGF), epidermal growth factor (EGF), and fibroblast growth factor, down to effectors like KRAS and BRAF, and phosphoinositide 3-kinase (PI3K), as well as transcription factors, such as Myc and p53. Exemplary sTAGs employed for targeted tumor therapies are tumor associated growth factors which control phases of tumor development and metastasis, e.g. the initial expansion of a clone of cells, angiogenesis and metastasis, by enhancing motility, penetration, and invasion into blood vessels and lymph vessels, and colonization of distant organs.

In particular, VEGF is an endothelial cell-specific mitogen binding to VEGF receptor of a tumor cell. Bevacizumab (Avastin), a humanized anti-VEGF monoclonal antibody, is widely used for cancer therapy.

W02005123104A2 describes a VEGF fusion protein trap antagonist which is a dimer comprising two fusion proteins each composed of immunoglobulin (Ig)-like domains from two different VEGF receptors (VEGFR1 and VEGFR2) fused to a multimerizing component.

Aflibercept, also known as VEGF Trap, is a fusion protein that incorporates portions of the human receptors VEGFR1 and VEGFR2, fused to the constant region of human IgG1. The VEGF family of angiogenic factors includes VEGF-A and placental growth factor (PIGF). PIGF is an important regulator of angiogenesis in cancer. Aflibercept binds circulating VEGF in the bloodstream and in the extravascular space, as well as PIGF, thereby blocking tumor angiogenesis and growth.

A further example of an sTAG is RANKL (also designated TRANCE or ODF). RANKL is a type II transmembrane protein expressed in lymphoid cell lines and tissues but can also be secreted as soluble protein. It is highly expressed in T cells and osteoblasts but not in B cells. RANKL appears to be an important regulator of immune reactions by acting on dendritic cells and T cells. In addition, it regulates vascular homeostasis and bone metabolism. RANKL, by binding to its receptor RANK on osteoclasts, mediates bone resorption.

WO2011/017294A1 describes human anti-RANKL antibodies useful for treating or preventing bone disorders and immune/inflammatory conditions in a subject. Denosumab, a fully human anti-RANKL monoclonal antibody, inhibits osteoclastic-medicated bone resorption by binding to osteoblast-produced RANKL. Denosumab has received US Food and Drug Administration approval to prevent skeletal-related events in cancer patients with solid tumors and bone metastases.

Inhibition of RANKL may also be effected by osteoprotegerin (OPG), an endogenous soluble decoy receptor for RANKL that blocks its binding to RANK, thereby limiting osteoclastogenesis. Such inhibition was shown to increase the anti-tumor effect of panitumomab, a targeted anti-EGFR antibody (Canon et al. Bone (2010), 46(6):1613-9).

Some bispecific antibodies are currently under development to improve anti-tumor activity through increased binding of tumor cells.

Dong et al. (mABs 3:3 (2011), DOI:10.4161/mabs.3.3.15188) describe a bispecific dual-targeting epidermal growth factor receptor (EGFR) and type I insulin-like growth factor receptor (IGF-1 R) antibody developed for cancer therapy. EGFR and IGF-1R signal pathways were concurrently blocked by such construct. A synergistic effect was found to be associated with the bispecific binding to the tumor cell increasing the avidity.

Antibodies typically mediate Antibody-Dependent Cell-Mediated Cytotoxicity (ADCC), which is a mechanism of cell-mediated immunity whereby an effector cell (for instance a natural killer cell, NK cell) of the immune system actively lyses a target cell that has been bound by specific antibodies. An NK cell's Fc receptor recognizes the Fc portion of an antibody, such as IgG, which has bound to the surface of a target cell. The most common Fc receptor on the surface of an NK Cell is called CD16a or FcγRIII. Once the Fc receptor binds to the Fc region of IgG, the NK cell releases cytokines such as IFN-γ, and cytotoxic granules containing perforin and granzymes that enter the target cell and promote cell death.

Phagocytic effector cells may be activated through another route employing activation of complement. Antibodies that bind to surface antigens on microorganisms attract the first component of the complement cascade with their Fc region and initiate activation of the "classical" complement system. This results in the stimulation of phagocytic effector cells, which ultimately kill the target by complement dependent cytotoxicity (CDC).

NK cell dysfunction or deficiency has been shown to lead to the development of autoimmune diseases (such as diabetes or atherosclerosis) and cancers.

Lymphocytopenia is a frequent, temporary result from many types of chemotherapy, such as with cytotoxic agents or immunosuppressive drugs. Some malignancies in the bone marrow, such as leukemia, also cause lymphocytopenia. Large doses of radiation, such as used for treating tumors, may cause lymphocytopenia.

Antibody internalisation is required for the success of some targeted therapeutics, such as immunotoxins, immunoliposomes, antibody-drug conjugates and for the targeted delivery of genes or viral DNA into cells.

Goenaga et al. (Mol Immunol. (2007) 44(15): 3777-3788) describe identification and characterization of tumor antigens by using antibody phage display and intrabody strategies. A human single chain Fv phage display library was selected for rapid internalisation into SK-BR-3 breast cancer cell line with the potential for the targeted delivery of cytotoxic agents to breast cancers.

US2010009390A1 describes antibodies that have improved affinity for the epidermal growth factor receptor (EGFR) and microparticles and nanoparticles having attached thereto a plurality of EGFR affinity moieties that are internalised by cells expressing an EGFR.

It is the object of present invention to provide improved antibodies that are capable of tumor cell lysis independent of available effector cells.

### Summary of the Invention

The object is solved by the subject matter as claimed.

According to the invention there is provided an internalising modular antibody based on one or more immunoglobulin domains, which comprises at least one binding structure located within the N-terminal portion of a domain, and comprises at least one binding structure acting as a binding site located within the non-CDR region, e.g. the structural loop region at the C-terminal portion of a domain, of a domain, wherein
a) a first binding structure has a specificity to bind an extra-cellular portion of a receptor on a tumor cell, and
b) a second binding structure has a specificity to bind a soluble tumor associated antigen (sTAG).

Engineering a binding site within the N-terminal portion of a domain typically is by fusion through recombinant engineering techniques, or else through covalent coupling. Such binding structure within the N-terminal portion preferably is located at the N-terminal side, e.g. at the end of the amino acid sequence, such as within the first sequence part of the N-terminus, preferably within the first 20 amino acids. Another preferred embodiment refers to the binding structure located at N-terminally exposed amino acid positions of the domain, e.g. at N-terminal loop positions.

The modular antibody may be a single domain or larger constructs comprising more antibody domains, such as antibody fragments or a full-length antibody. It is preferred that the modular antibody contains at least the immunoglobulin Fc fragment.

One of the preferred embodiments is a full length antibody with a binding structure within the CDR loops and another binding structure within the non-CDR region.

Specifically, the modular antibody according to the invention comprises a binding structure derived from CDR loops of an antibody. Preferably such binding structure is within the N-terminal portion of a domain and comprises antibody CDR loops, such as formed by or derived from VH/VL domains or single variable domains.

Alternatively or in addition thereto, the modular antibody according to the invention comprises a binding structure derived from a receptor polypeptide specifically binding to the target antigen, or comprises said receptor polypeptide as a binding moiety. Such binding structure of a receptor polypeptide is preferably attached to the N-terminal side of the domain or modular antibody, e.g. attached to the N-terminal portion of an antibody Fc molecule.

A preferred embodiment is based on an immunoglobulin Fc fragment comprising a binding structure, such as a receptor moiety or an antibody variable domain, at the N-terminal portion of the Fc, e.g. bound to the N-terminal portion of a CH2 domain or to the hinge region.

Preferably the first binding structure a) is located in the structural loop region or non-CDR region of a domain.

Preferably, the second binding structure b) is located within the N-terminal side of a domain.

In one embodiment the first binding structure a) and the second binding structure b) are located within a single domain. In one embodiment the first binding structure a) and the second binding structure b) are located within different domains.

Thus, in one preferred embodiment the binding structure within the N-terminal portion of a domain is the second binding structure b) which has a specificity to bind a soluble tumor associated antigen (sTAG) and the binding structure within the non-CDR region of a domain is the first binding structure a) which has a specificity to bind an extra-cellular portion of a receptor on a tumor cell.

In an alternative embodiment the binding structure within the N-terminal portion of a domain is the first binding structure a) which has a specificity to bind an extra-cellular portion of a receptor of the erbB class on a tumor cell.and the binding structure within the non-CDR region of a domain is the second binding structure b) which has a specificity to bind a soluble tumor associated antigen (sTAG).

According to a specific embodiment the first binding structure a) is located within the Fc part of an immunoglobulin, preferably within the CH3 and/or CH2 domain.

According to a specific embodiment the first binding structure a) is located within the Fc, preferably within the CH3 and/or CH2 domain. The Fc is specifically understood as the Fc part of an immunoglobulin molecule or a modular antibody, in particular the Fc fragment with or without the hinge-region, or variants or derivatives therefrom, bearing a specific binding site.

Specifically, the extra-cellular portion of a receptor on a tumor cell is a tumor associated receptor, like a receptor that is overexpressed on the surface of a tumor cell. Such receptor preferably is selected from the group consisting of EGFR, HER2, HER3, HER4, Insulin growth factor receptor I (IGF-RI), c-met, a receptor having a Lewis Y or Thomsen-Friedenreich glycosylation binding structure, DR4 (TNFRSF10A), DR5 (TNFRSF10B), DcRI (TNFRSF10C), DcR2 (TNFRSF10D), IL-17 receptor, IL-23 receptor, IL-12 receptor and Interferon-alpha receptor.

The receptor preferably is an erbB receptor, also referred to as receptor of the erbB class, specifically including EGFR, HER2, HER3, HER4. Most suitably the first binding structure a) has specificity to bind EGFR.

According to further preferred embodiments, the receptor is a receptor of the tumor necrosis factor family, such as including DR4 (TNFRSF10A), DR5 (TNFRSF10B), DcRI (TNFRSF10C), DcR2 (TNFRSF10D), or a receptor of interleukins or interferons.

It is further preferred that the binding structure specifically binds to a glycoepitope, such as formed by aberrant glycosylation typically found on tumor cells. This includes for example, the Lewis Y or Thomsen-Friedenreich antigen.

It is preferred that the receptor is a tumor associated receptor that would naturally bind to a ligand, which forms oligomers on the surface of the tumor cell, thus provides for cross-linking of the receptors through such binding to their natural ligands. Such ligands are thus capable of cross-linking said receptors. Such receptors are e.g. receptors of the erbB class.

It is preferred that the modular antibody according to the invention has a binding affinity to bind the tumor cell and/or the sTAG with a KD of less than 100 nM. The preferred modular antibody binds to said tumor cell with a Kd<10⁻⁸M, which is determined in an affinity and/or avidity assay, in particular an assay of binding the antigen under saturating conditions.

The modular antibody according to the invention is preferably provided which induces internalisation of the sTAG.

According to a specific embodiment, the modular antibody according to the invention has direct cytotoxic activity, i.e. independent of immune-effector cells, such as NK cells. Specifically the modular antibody according to the invention has apoptotic activity, as measured in a standard apoptosis assay. The direct cytotoxic activity is preferably measured in a standard DNA fragmentation assay.

According to a specific embodiment, the modular antibody according to the invention has a cytotoxic activity in the presence of immune-effector cells as measured in a standard ADCC or CDC assay, e.g.employing cells expressing the receptor target on the cell surface.

According to a specific embodiment, the modular antibody according to the invention has direct cytotoxic activity, and in addition mediates cell killing via ADCC mechanisms.

Specifically, the sTAG is selected from the group consisting of VEGF, RANKL, (TNFSF11), EGF, APRIL (TNFSF13), TWEAK (TNFSF12), TNFSF3, CD27L (TNFSF7), 4-1BBL (TNFSF9), LIGHT (TNFSF14), VEGI (TNFSF15), CD30L (TNFSF8), AITRL (TNFSF18), TNF-alpha (TNFSF2), OX40L (TNFSF4), FasL (TNFSF6), EDA, CD40L (TNFSF5), Blys (TNFSF13B), IFN-alpha, IL-1-beta, IL-6, IL-13, IL-17A, IL-17E, GM-CSF, PIGF, lymphotoxin (TNFSF1), TRAIL (TNFSF10), or any other soluble factor associated with tumor disease or auto-immune disease.

A preferred sTAG is selected from the group of ligands of cell surface receptors capable of cross-linking cell surface receptors due to its properties to oligomerise.

Preferably the sTAG as targeted by the modular antibody according to the invention is not a natural ligand to the receptor of the tumor cell, which is targeted by the same modular antibody according to the invention, so to avoid triggering the tumor mode of action through binding of the sTAG to its tumor cell receptor.

Preferably the second binding structure b) has specificity to bind VEGF or RANKL.

Preferably the first binding structure a) has specificity to bind EGFR and the second binding structure b) has specificity to bind VEGF or RANKL.

It is preferred that the modular antibody according to the invention binds to an EGFR epitope specifically recognised by the EAM151-5 Fcab (SEQ ID NO:747) According to a preferred embodiment, the first binding structure a) binds to such epitope. According to a preferred embodiment, this binding structure is within the non-CDR region of a domain. Specifically, the modular antibody according to the invention consists of or comprises such Fc fragment of an immunoglobulin or modular antibody with the binding site in the structural loop region.

More specifically, the modular antibody according to the invention comprises a binding structure which comprises any of the EF loop sequences, and optionally a further binding loop structure comprising any of the AB and/or CD loop sequences, as listed in Table 1. More specifically any of the AB and/or CD and/or EF loop sequence combinations as indicated in Table 1 are preferred.

Specifically, the binding structure is located within a CH3 domain, specifically at the C-terminal loop region. Preferably, the modular antibody according to the invention comprises the second binding structure b) which comprises any of the EF loop sequences, and optionally a further binding loop structure comprising any of the AB and/or CD loop sequences, as listed in Table 1, wherein a binding loop sequence or structural loop region is of a CH3 domain.

In addition to mutations in the structural loop region to create a new antigen binding site, it may be preferred to provide for further framework mutations, such as point mutations to improve antigen binding.

According to the invention, there is further provided an Fc fragment of an immunoglobulin or modular antibody, which specifically binds to an EGFR epitope, also called EGFR Fcab, and comprises a binding loop structure comprising any of the EF loop sequences, and optionally a further binding loop structure comprising any of the AB and/or CD loop sequences, as listed in Table 1.

It is preferred that said EGFR epitope is specifically recognised by the EAM151-5 Fcab. Thus, the EGFR Fcab preferably binds to an epitope specifically recognised by the EAM151-5 Fcab (also called EAM252-D4, SEQ ID NO: 747) and its functional derivatives or functionally active variants, such as included in Table 1. The EAM151-5 is based on a human IgG1 wild type Fc sequence (SEQ ID NO: 748) and has a binding loop structure as listed in Table 1 (combinations of SEQ ID NO: 135, 438 and 250). Functionally active variants thereof specifically comprise the EF loop sequence, such as from those clones named EAM151, EAM252, EAM253, EAM142 and EAM243, optionally in combination with the AB and/or CD loop sequences, optionally with one or more framework mutations, as listed in Table 1.

Functionally active variants are, for instance, those with one or more point mutations within the loop sequences, specifically within the EF loop sequences, which may or may not change the way of binding to the same epitope, e.g. affinity, avidity, fine specificity or the like, which epitope is still the same. Preferably such Fcab functionally active variants are obtained through mutagenesis, such as site-directed mutagenesis or randomisation, such that they comprise changes in the amino acids sequence at positions of up to 6 amino acids, preferably up to 5, 4, 3, 2 or 1 amino acids, which changes are through deletions, insertions and/or substitutions. Preferably the functionally active variants of EAM151, EAM252, EAM253, EAM142 and EAM243 Fcab have changes of up to 6 amino acids in the EF loop sequence, preferably up to 5, 4, 3, 2 or 1 amino acids changed through deletions, insertions and/or substitutions. Specifically preferred functionally active variants of EGFR Fcab molecules comprise only up 3, more preferred up to 2, 1 or no amino acid changes in the EF loop positions and optionally further changes in the other C-terminal loop sequences, which are changes of up to 6 amino acids, preferably up to 5, 4, 3, 2 or 1 amino acids.

Preferred embodiments of the EGFR Fcab are based on the human IgG1 Fc wild-type sequence, such as listed as SEQ ID NO: 748. Still, it may be preferred to use immunoglobulin Fc sequences of different species, immunoglobulin types or subtypes.

Preferably the EGFR Fcab is based on a native immunoglobulin Fc sequence and functionally active variants thereof, which is then genetically engineered to comprise the specific antigen binding site through the non-CDR loop region, e.g. the EF and optionally further loop sequences. Such functionally active variants of wild-type Fc specifically includes Fc mutants engineered to improve the Fc properties, such as stability, half-life or Fc receptor binding, e.g. through appropriate recombinant engineering to provide for mutations in the framework region, including point mutations. Further preferred functionally active variants may be produced by chemical engineering.

The preferred functionally active variants of EGFR Fcab molecules and particularly the EAM151 or EAM252 Fcab, are specifically binding to the same epitope. Such binding may be tested in an appropriate immunoassay for competitive binding, preferably an assay employing cell-bound antigens or else an ELISA employing soluble antigens.

Specifically, the Fc fragment according to the invention is selected from the group of clones containing EAM151, EAM252, EAM253, EAM142 and EAM243 in their names, as listed in Table 1, and functional variants thereof. Thus, a specific embodiment refers to an Fc, which is derived from EAM151, EAM252, EAM253, EAM142 and EAM243 clones, and functional variants thereof.

According to the invention there is also provided a modular antibody or an immunoglobulin comprising such an Fc fragment or EGFR Fcab.

According to the invention, the Fc fragment may be used for engineering a multivalent and/or multispecific antibody.

Specifically there is provided according to the invention a modular antibody or an immunoglobulin, in particular the Fc fragment according to the invention or modular antibody comprising the Fc fragment, for use in treating a malignant neoplasm, specifically breast cancer, colorectal cancer, head and neck cancer or gastric cancer. Depending on the specificity of binding a target antigen on the surface of diseased cells, the modular antibody according to the invention may also be indicated in treating other disorders or diseases, where internalisation of an antibody into such diseased cell is a desirable mode of action. This includes autoimmune disorders, such as rheumatoid arthritis, Crohn's disease, psoriasis, systemic lupus erythematosus, multiple sclerosis, diabetes, Ankylosing Spondylitis, Autoimmune thrombocytopenic purpura, Celiac disease, Graves' disease, Myasthenia gravis, Pemphigus vulgaris, Psoriatic arthritis, Sjögren's syndrome, Vasculitis, Wegener's granulomatosis and Primary biliary cirrhosis.

The modular antibody according to the invention is preferably provided for manufacturing a pharmaceutical preparation for the treatment of cancer, in particular breast cancer, colorectal cancer, head and neck cancer or gastric cancer.

Preferably the modular antibody according to the invention is used for the treatment of immunocompromised patients, preferably in combination with chemotherapy or radiotherapy.

It is preferred that the modular antibody according to the invention contains an antigen binding site within a structural loop region, specifically an antigen binding site having a randomized antibody sequence. Thereby the binding site may be randomly generated, e.g. generated *in situ* within the modular antibody sequence so to create a library of modular antbody variants, or else derived from a library of binding structures, which libraries are selected according to the suitable binding properties and incorporated within the modular antibody. Accordingly the binding site may be produced through mutagenesis of a nucleotide or amino acid sequence. The site of the randomized sequence may be within the CDR region or the structural loop region, which is always understood to potentially include a terminal domain sequence that could be contributing to antigen binding.

Though the modular antibody according to the invention may be based on a single immunoglobulin domain, the preferred format of the modular antibody according to the invention is an oligomer of immunoglobulin domains, e.g. including modular antibody domains other than immunoglobulin domains. This includes constructs of immunoglobulin oligomers with receptor binding moieties, such as binding sites derived from a receptor molecule.

Specifically said oligomer comprises immunoglobulin domains selected from the group consisting of full length antibodies or fragments thereof, or any kind of combinations of immunoglobulin domains, such as VH/VL, CH1/CL, CH2/CH2, CH3/CH3, Fc, Fab and scFv.

According to a preferred embodiment the modular antibody is a bispecific full-length antibody (mAb²) or an antigen binding Fc molecule (Fcab), specifically those based on human wild-type (wt) Fc of the IgG type, preferably of the IgG1 type, such as having the sequence as provided in WO06/072620A1, which wild-type sequence is modified to include the binding structures in one or at least two of the structural loops of one or both CH3 domains to add one or two antigen binding sites in the structural loop region.

The modular antibody according to the invention preferably is provided as a modular antibody that is capable of simultaneously binding to both the extra-cellular portion of a receptor on a tumor cell, and the sTAG. Simultaneous binding is preferably determined in a cell-based assay with two-dimensional differentiation, e.g. in a FACS system. An alternative method may employ an ELISA format, e.g. which provides for coating with a target antigen, adding a bispecific mAb² specifically binding to said target antigen and another target antigen, and detecting with said another target antigen. It is further preferred that the modular antibody binds to the receptor and the sTAG by at least three binding sites, preferably at least 4, 5 or even 6 antigen binding sites. According to a preferred embodiment, the receptor is bound on the surface of a tumor cell by at least two binding sites and the sTAG is bound by at least one binding site, or *vice versa.* Specifically preferred are modular antibodies capable of binding an sTAG to form multimers, e.g. multimers of at least 2, 3 or 4 sTAGs, which may eventually be formed on the surface of a tumor cell.

### Figures

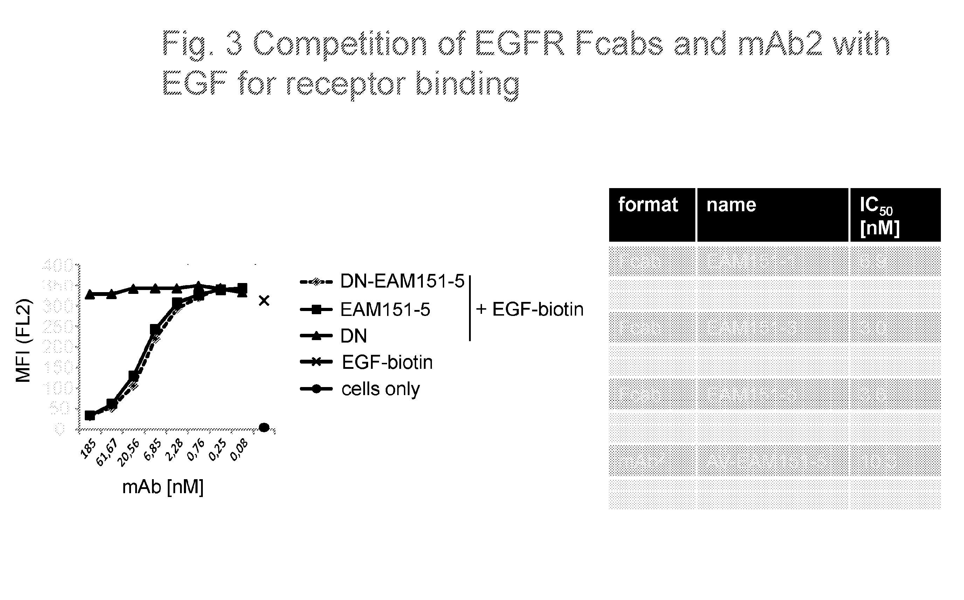

Figure 1: Results of binding affinity measurements of various human EGFR specific Fcabs determined by surface plasmon resonance (SPR) assays in a Biacore instrument. These experiments indicate that these Fcabs have binding affinities for recombinant EGFR ranging between 0.6-15nM (Fig. 1).
Figure 2: Results of binding affinity measurements of mAb² AV-EAM151-5 and DN-EAM151-5 determined by surface plasmon resonance (SPR) assays in a Biacore instrument. These experiments indicate that these mAb² have binding affinities for recombinant EGFR around 1nM, similar to Fcab EAM151-5 (Fig. 2).
Figure 3: Competition of EGFR Fcabs and mAb² with EGF for receptor binding
The data demonstrate that EGFR specific Fcab EAM151-5 and mAb² DN-EAM151-5 compete with EGF for binding to EGFR positive MDA-MB468 cells with similar potencies in the single digit nM range. Denosumab (DN) has no effect. The table depicts the potencies of different EGFR specific Fcabs in this assay (Fig. 3).

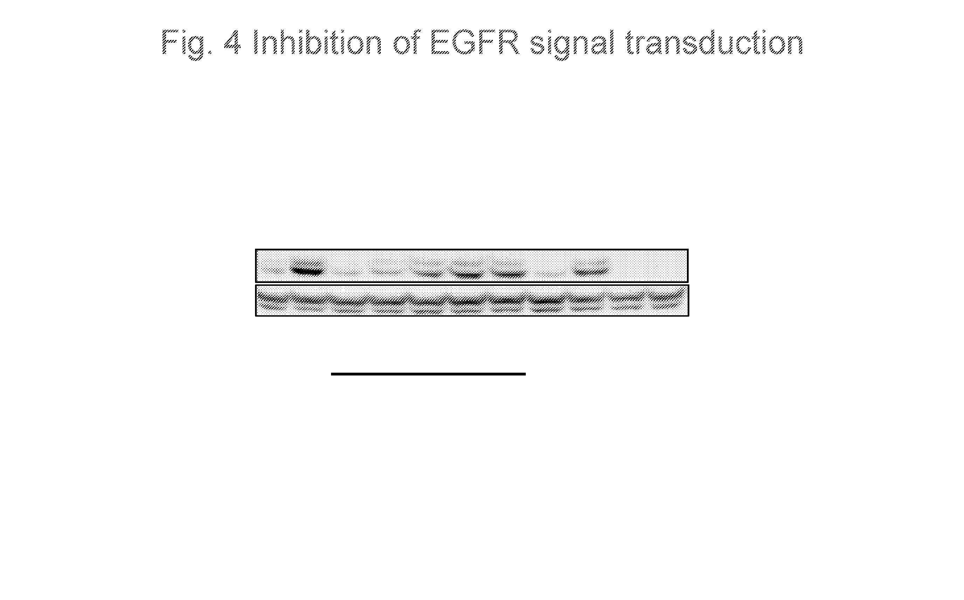

Figure 4: EGFR specific Fcabs inhibit EGFR signal transduction. Phosphorylation of erk1/2 protein in response to EGF stimulation of SKBR3 cells. EGFR specific Fcab EAM151-5 inhibits EGF stimulated erk1/2 phosphorylation in a dose dependent fashion similar to the positive control cetuximab (CX). Equally importantly, EAM151-5 does not induce erk1/2 phosphorylation by itself and therefore is not a signalling agonist. Thus, Fcab EAM151-5 has functional properties similar to cetuximab (Fig. 4).

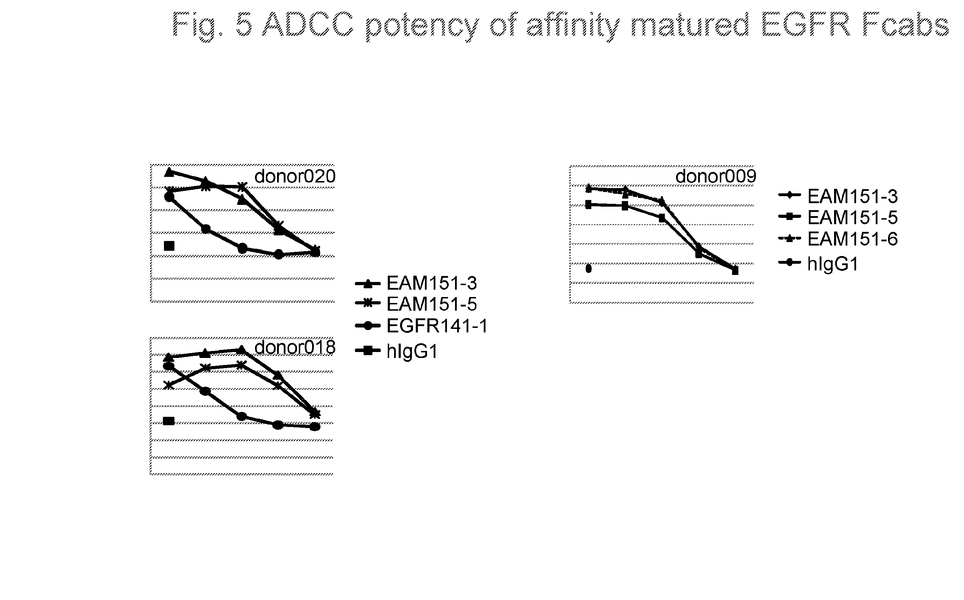

Figure 5: ADCC effector functionality of EGFR Fcabs EGFR specific Fcabs kill EGFR-positive SKBR3 cells by antibody dependent cellular cytotoxicity (ADCC). Co-incubation of opsonised SKBR3 cells with human natural killer (NK) cells at a ratio of 1:5 for 4 hours at 37°C. NK cells purified from three different donors are shown. EGFR specific Fcabs EAM151-3, EAM151-5, EAM151-6 and mediate NK-cell dependent death of SKBR3 target cells with similar potencies in the single digit nM range with different NK cell donors. EGFR141-1 is also active but with lower potency in line with its lower affinity for EGFR (Fig. 5)

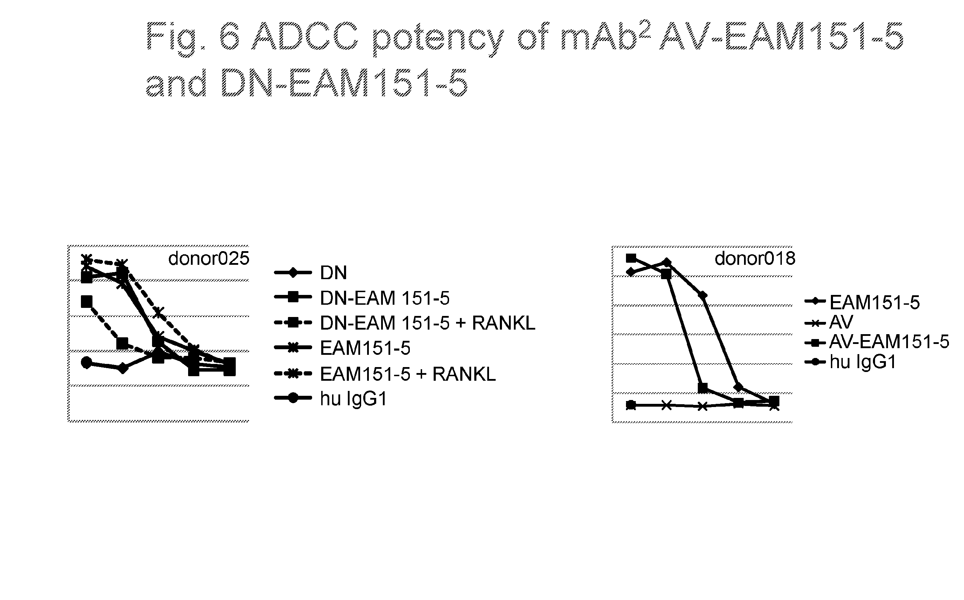

Figure 6: ADCC effector functionality of mAb² AV-EAM151-5 and DN-EAM151-5. mAb² containing the EAM51-5 binding sequence for EGFR kill EGFR-positive MDA-MB468 cells by antibody dependent cellular cytotoxicity (ADCC). Co-incubation of opsonised MDA-MB468 cells with human natural killer (NK) cells at a ratio of 1:5 for 4 hours at 37°C. NK cells purified from two different donors are shown. mAb² AV-EAM151-5 elicits similar ADCC potency as Fcab EAM151-5 while the parental antibody bevacizumab (AV) is inactive. Similarly, mAb² DN-EAM151-5 is as potent as Fcab EAM151-5 while denosumab (DN) does not promote any cell killing. The ADCC activity of DN-EAM151-5 is significantly reduced in the presence of excess RANKL protein (Fig. 6). Similar effects are observed with AV-EAM151-5 (not shown). This phenomenon is most likely due to internalization of the mAb² into cells.

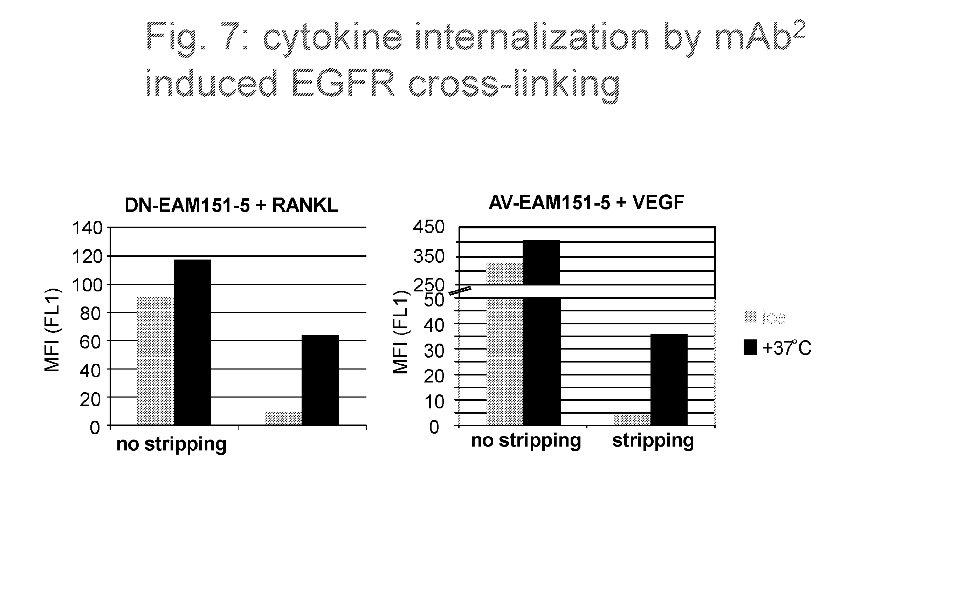

Figure 7: Internalization of VEGF or RANKL upon binding to cell bound mAb² AV-EAM151-5 and DN-EAM151-5 EGFR-positive A431 cells are loaded with 40nM DN-EAM151-5 or AV-EAM151-5 on ice for 40 minutes. Excess mAb² is washed away and the cells are incubated further in the absence or presence of 100nM RANKL or VEGF labelled with the fluorescent dye AlexaFluor488. Incubation is carried out for 90 minutes on ice (non-internalizing conditions) or at 37°C (internalizing conditions). Cell surface bound cytokine/ mAb² complexes are removed by a treatment with 0.2M acetic acid for 5 minutes and cells are analyzed by flow cytometry. The results show that a significant percentage of the fluorescent RANKL or VEGF signal is resistant to acid treatment at 37°C compared to cells incubated on ice indicating that the cytokines are internalized as consequence of EGFR cross-linking by the cytokine bound mAb² (Fig. 7).

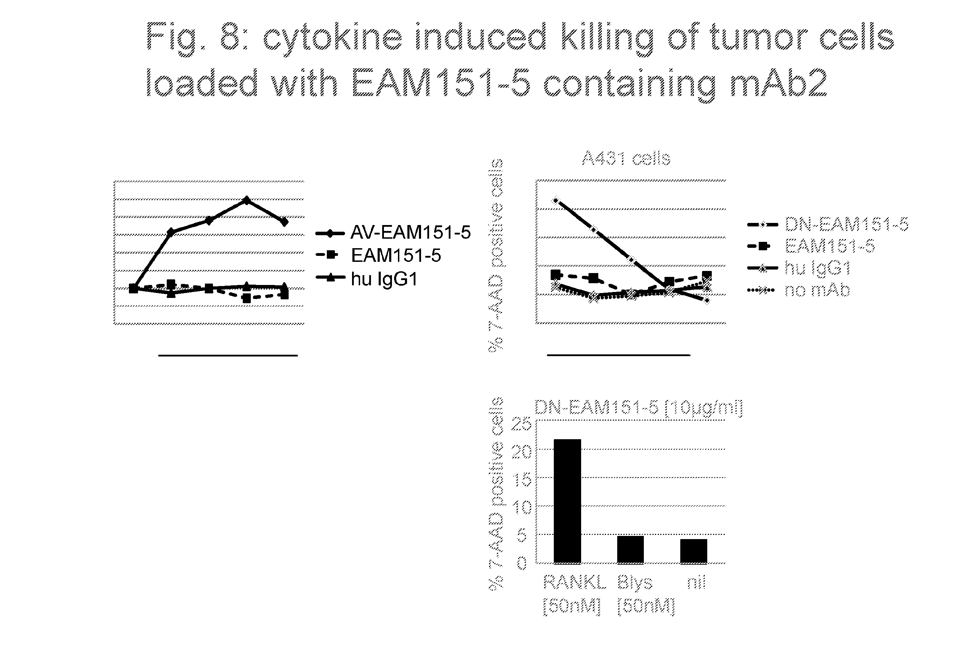

Figure 8: Tumor cell killing induced by cross-linking of cell bound mAb² with sTAG EGFR-positive MDA-MB468 tumor cells are incubated for 4 hours with 10µg/ml Fcab EAM151-5 or mAb² AV-EAM151-5 in the presence of increasing concentrations of human VEGF. Cell death is monitored by flow cytometry after addition of 7-amino-actinomycin D (7-AAD) and presented as percentage of 7-AAD positive cells. Binding of the mAb2 to cells alone does not induce cell death but additional VEGF causes cell killing. This effect is specific for the bi-specific antibody since Fcab EAM151-5 alone or in combination with the cytokine does not kill cells. Similar data are seen when another EGFR-positive cell line, A431, is cultured with mAb² DN-EAM151-5 or Fcab EAM151-5 in the presence of RANKL. Again, specific cell killing is only observed in cultures treated with DN-EAM151-5 and RANKL. Cell death induction is specific for the cognate soluble antigen since addition of the irrelevant cytokine B-lymphocyte stimulator (Blys) is ineffective. It is speculated that cross-linking of cell bound mAb² with sTAGs RANKL or VEGF leads to internalization of the sTAG/ mAb² complex eventually initiating a cell death promoting signal.

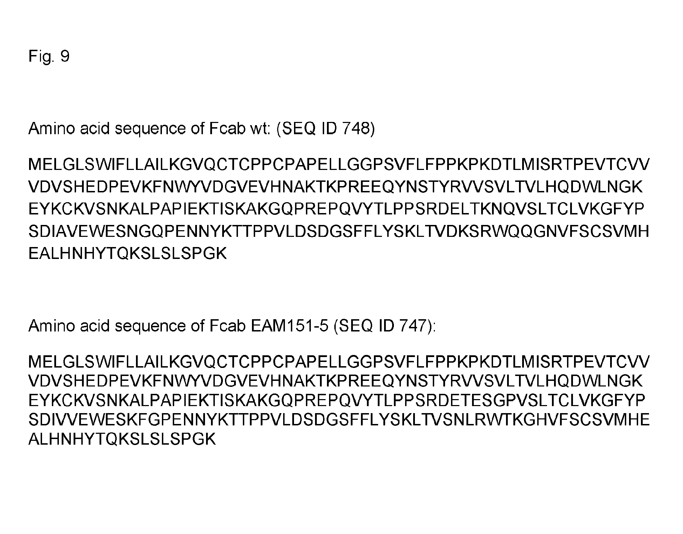

Figure 9: Amino acid sequences of Fcab wt (SEQ ID 748) and Fcab EAM151-5 (SEQ ID 747)

### Detailed Description of the Invention

Specific terms as used throughout the specification have the following meaning.

The term "immunoglobulin" as used according to the present invention is defined as polypeptides or proteins that may exhibit mono- or bi- or multi-specific, or mono-, bi- or multivalent binding properties, at least two, more preferred at least three specific binding sites for epitopes of e.g. antigens, effector molecules or structures either of pathogen origin or of human structure, like self-antigens including cell-associated or serum proteins. The term immunoglobulin as used according to the invention includes full-length antibodies or functional fragments of an antibody, such as Fc, Fab, scFv, single chain dimers of CH1/CL domains, Fv, dimers like VH/VL, CH1/CL, CH2/CH2, CH3/CH3, or other derivatives or combinations of the immunoglobulins, like single chains of pairs of immunoglobulin domains. Immunoglobulins are also understood to consist of or comprise immunoglobulin domains, which are understood as constant and/or variable domains of the heavy and/or light chains. The definition further includes domains of the heavy and light chains of the variable region (such as dAb, Fd, VI, Vk, Vh, VHH) and the constant region or individual domains of an intact antibody such as CH1, CH2, CH3, CH4, Cl and Ck, as well as mini-domains consisting of at least two beta-strands of an immunoglobulin domain connected by a structural loop. The term is understood to include functionally active variants or functional homologues.

"Modular antibodies" as used according to the invention are defined as antigen-binding molecules, like human antibodies, composed of at least one polypeptide module or protein domain, preferably in the natural form. The term "modular antibodies" specifically includes antigen-binding molecules that are either immunoglobulins, immunoglobulin-like proteins, or other proteins exhibiting modular formats and antigen-binding properties similar to immunoglobulins or antibodies, including receptor binding moeties, which can be used for antigen binding or as antigen-binding scaffolds, preferably based on human polypeptides or proteins. The term is understood to include functionally active variants or functional homologues.

A "receptor binding moiety" or binding structure of a "receptor molecule" or "receptor polypeptide" is understood herein as the binding site of a receptor that specifically binds to a target antigen. Particular examples are natural receptors of target antigens, such as sTAG receptor, and their binding sites, respectively. For instance, a binding structure may be derived from the receptor polypeptide, which is a natural receptor to bind an sTAG, including signal transducers. Specific examples of receptor polypeptides are derived, e.g. from the VEGF receptor specifically binding to VEGF, EGF receptor specifically binding to EGF, and OPG (acting as a soluble decoy receptor) binding to RANKL.

The term "multivalent" with respect to an immunoglobulin or modular antibody according to the invention shall refer to a molecule having at least two binding sites to bind the same target antigen, specifically binding the same or different epitopes of such target antigen. The term shall include bivalent modular antibodies or molecules with 2 or more valencies to bind the target antigen, e.g. through at least 2, 3, 4 or even more binding sites. Typically an immunoglobulin having CDR binding sites, typically binds a target antigen bivalently through the CDR loops of VH/VL domains.

The term "multispecific" with respect to an immunoglobulin or modular antibody according to the invention shall refer to a molecule having at least two binding sites specifically binding at least two different target antigens. The term shall include bispecific modular antibodies or molecules with 2 or more specificities to bind more than one target antigen, e.g. through at least 2, 3, 4 or even more binding sites. A preferred multispecific immunoglobulin based on a full-length IgG antibody binds at least one target antigen through the CDR loop region and at least another target antigen through a binding site in the non-CDR region or structural loop region.

The term "immunoglobulin-like molecule" as used according to the invention refers to any antigen-binding protein, in particular to a human protein, which has a domain structure that can be built in a modular way. Immunoglobulin-like molecules as preferably used for the present invention are T-cell receptors (TCR) or soluble parts thereof, fibronectin, transferrin, CTLA-4, single-chain antigen receptors, e.g. those related to T-cell receptors and antibodies, antibody mimetics, adnectins, anticalins, phylomers, repeat proteins such as ankyrin repeats, avimers, Versabodies™, scorpio toxin based molecules, and other non-antibody protein scaffolds with antigen binding properties.

Ankyrin repeat (AR), armadillo repeat (ARM), leucine-rich repeat (LRR) and tetratricopeptide repeat (TPR) proteins are the most prominent members of the protein class of repeat proteins. Repeat proteins are composed of homologous structural units (repeats) that stack to form elongated domains. The binding interaction is usually mediated by several adjacent repeats, leading to large target interaction surfaces.

Avimers contain A-domains as strings of multiple domains in several cell-surface receptors. Domains of this family bind naturally over 100 different known targets, including small molecules, proteins and viruses. Truncation analysis has shown that a target is typically contacted by multiple A-domains with each domain binding independently to a unique epitope. The avidity generated by combining multiple binding domains is a powerful approach to increase affinity and specificity, which these receptors have exploited during evolution.

Anticalins are engineered human proteins derived from the lipocalin scaffold with prescribed binding properties typical for humanized antibodies. Lipocalins comprise 160-180 amino acids and form conical beta-barrel proteins with a ligandbinding pocket surrounded by four loops. Small hydrophobic compounds are the natural ligands of lipocalins, and different lipocalin variants with new compound specificities, also termed 'anticalins', could be isolated after randomizing residues in this binding pocket.

Phylomers are peptides derived from biodiverse natural protein fragments.

It is understood that the term "modular antibody", "immunoglobulin", "immunoglobulin-like proteins" includes a derivative thereof as well. A derivative is any combination of one or more modular antibodies of the invention and or a fusion protein in which any domain or minidomain of the modular antibody of the invention may be fused at any position of one or more other proteins, such as other modular antibodies or immunoglobulins, e.g. a binding structure comprising CDR loops, a receptor polypeptide, but also ligands, scaffold proteins, enzymes, toxins and the like. A derivative of the modular antibody of the invention may also be obtained by association or binding to other substances by various chemical techniques such as covalent coupling, electrostatic interaction, di-sulphide bonding etc. The other substances bound to the immunoglobulins may be lipids, carbohydrates, nucleic acids, organic and inorganic molecules or any combination thereof (e.g. PEG, prodrugs or drugs). A derivative would also comprise an antibody with the same amino acid sequence but made completely or partly from non-natural or chemically modified amino acids. The term derivative also includes fragments, variants or homologues, which are functional and may serve as functional equivalents. The preferred derivatives still are functional with regard to both, the receptor binding on the target cell and the sTAG binding.

A "structural loop" or "non-CDR-loop" according to the present invention is to be understood in the following manner: modular antibodies, immunoglobulins or immunoglobulin-like substances are made of domains with a so called immunoglobulin fold. In essence, antiparallel beta sheets are connected by loops to form a compressed antiparallel beta barrel. In the variable region, some of the loops of the domains contribute essentially to the specificity of the antibody, i.e. the binding to an antigen by the natural binding site of an antibody. These loops are called CDR-loops. The CDR loops are located within the CDR loop region, which may in some cases also include part of the variable framework region (called "VFR"), which is adjacent to the CDR loops. It is known that some loops of the VFR may contribute to the antigen binding pocket of an antibody, which generally is mainly determined by the CDR loops. Thus, those VFR loops are considered as part of the CDR loop region, and would not be appropriately used for engineering new antigen binding sites in the non-CDR region. Loops aside from the antigen-binding pocket or CDR loop region are usually called structural loops or non-CDR-loops. Contrary to the VFR within the CDR loop region or located proximal to the CDR loops, other loops of the VFR of variable domains would be considered structural loops and particularly suitable for use according to the invention. Those are preferably the structural loops of the VFR located opposite to the CDR loop region, or at the C-terminal side of a variable immunoglobulin domain. Constant domains have structural loops within a structural loop region, e.g. either at the C-terminal side of an antibody domain or at an N-terminal side, even within a side chain of an antibody domain. Constant domains are also called part of the framework region. C-terminal amino acid sequences may also contribute to the non-CDR antigen binding, thus, are considered part of a structural loop region, which may be engineered to create a new antigen-binding site.

The "N-terminal portion" or "N-terminal side" of a modular antibody, antibody or immunoglobulin domain is understood herein as the N-terminus of an amino acid sequence or at the upper loop region of the immunoglobulin fold, e.g. comprising the B-C, C'-C", and F-G loop region of an immunoglobulin variable domain or the D-E, B-C, and F-G loop region, in particular the upper loop region. An fusion or attachment of a binding structure or moiety to the N-terminal portion is typically by a linker sequence or directly to the sequence of the modular antibody, either by recombinant techniques or through chemical linkeage, to produce hybrid molecules or fusion molecules.

The term "antigen" or "target" as used according to the present invention shall in particular include all antigens and target molecules capable of being recognised by a binding site of a modular antibody. Specifically preferred antigens as targeted by the molecule according to the invention are those antigens or molecules, which have already been proven to be or are capable of being immunologically or therapeutically relevant, especially those, for which a clinical efficacy has been tested. The term "target" or "antigen" as used herein shall in particular comprise molecules selected from the group consisting of tumor associated receptors and soluble tumor associated antigens, which are self antigens, such as receptors located on the surface of tumor cells or cytokines or growth factors that are abundantly present in the circulation of cancer patients and associated with such tumor.

The term "soluble tumor associated antigen" or "sTAG" as used herein shall refer to antigens or epitopes formed by secreted proteins which contribute to the development, maintenance and/or worsening of cancer and auto-immune diseases, including circulating tumor derived soluble factors or inflammatory proteins, in particular those that are abundantly secreted by a large percentage of solid tumor or autoimmune diseased cells, closely associated with a poor prognosis. Typical examples are extracellular signalling molecules, among them hormones, neurotransmitters, cytokines, growth factors or cell recognition molecules, capable of attaching to the corresponding receptor and triggering changes in the function of the cell. This process is called signal transduction and such sTAGs are called signal transducers: The binding initiates a chemical change on the intracellular side of the membrane. In this way the sTAGs play a unique and important role in cellular communications and signal transduction.

An example of such an sTAG signal transducer is VEGF, a factor not only important for tumor vascularization, but also a key factor produced by solid tumors to inhibit recognition and destruction of tumor cells by the immune system.

Further examples are growth factors which are natural ligands binding to erbB receptors, among them EGF, heregulin, TGF-alpha, amphiregulin, betacellulin, epiregulin and the group of neuregulins.

Further examples are carcinoembryonic antigens, such as CA19-9, CA125, and CA15-3 that are tumor associated and play a role in rheumatoid arthritis.

Further examples are antigens or epitopes derived from molecules of the tumor necrosis factor family, including RANKL, TNF-alpha or Blys.

RANKL, also called osteoclast differentiation and activation factor, is a cytokine that binds to TNFRSF11 B/OPG and to TNFRSF11A/RANK. It augments the ability of dendritic cells to stimulate naive T-cell proliferation. It reportedly may play an important role in enhanced bone-resorption in humoral hypercalcemia of malignancy. The soluble form of isoform 1 derives from the membrane form by proteolytic processing.

sTAGs are specifically localized in the cytoplasm or on the cell membrane and may be secreted by or shedded from the cell. They are typically found in the circulation of tumor patients.

Preferred target antigens are sTAG molecules that form oligo- or multimers on the surface of tumor cells, which may optionally trigger cell death upon internalization. For instance, members of the tumor necrosis factor family, such as RANKL, TNF-alpha, Blys, which form such multimers.

The "extra-cellular portion" or "extracellular domain" of a receptor is understood as the part of a receptor expressed on the surface of a cell that sticks out of the membrane on the outside of the cell still being anchored.

The term "erbB receptor" as used herein shall refer to the extra-cellular portion of the receptor of the erbB class. The erbB receptor particularly targeted by the modular antibody according to the invention may be selected from the group consisting of EGFR (Her1), Her2, Her2neu, Her3 and Her4.

The extra-cellular portion of the tumor associated receptor targeted by the modular antibody according to the invention is specifically understood as a cell surface antigen, which is typically a structure on the surface of a cell capable of being recognised by an antibody. Preferred cell surface antigens are those target antigens, which have already been proven to be or which are capable of being immunologically or therapeutically relevant, especially those, for which a preclinical or clinical efficacy has been tested. Those cell surface antigens are specifically relevant for the purpose of the present invention, which mediate cell killing activity. Upon binding of the modular antibody according to the invention to the receptor and the sTAG, and the eventual oligomerization on the cell surface, a potent means for attacking human cells may be provided.

The target antigen is either recognized as a whole target molecule or as a fragment of such molecule, especially substructures, e.g. a polypeptide or carbohydrate structure of targets, generally referred to as "epitopes", e.g. B-cell epitopes, T-cell epitope), which are immunologically relevant, i.e. are also recognisable by natural or monoclonal antibodies. The term "epitope" as used herein according to the present invention shall in particular refer to a molecular structure which may completely make up a specific binding partner or be part of a specific binding partner to a binding site of modular antibody of the present invention. The term epitope may also refer to haptens. Chemically, an epitope may either be composed of a carbohydrate, a peptide, a fatty acid, an organic, biochemical or inorganic substance or derivatives thereof and any combinations thereof. If an epitope is a polypeptide, it will usually include at least 3 amino acids, preferably 8 to 50 amino acids, and more preferably between about 10-20 amino acids in the peptide. There is no critical upper limit to the length of the peptide, which could comprise nearly the full length of a polypeptide sequence of a protein. Epitopes can be either linear or conformational epitopes. A linear epitope is comprised of a single segment of a primary sequence of a polypeptide or carbohydrate chain. Linear epitopes can be contiguous or overlapping. Conformational epitopes are comprised of amino acids or carbohydrates brought together by folding of the polypeptide to form a tertiary structure and the amino acids are not necessarily adjacent to one another in the linear sequence. Specifically, epitopes are at least part of diagnostically relevant molecules, i.e. the absence or presence of an epitope in a sample is qualitatively or quantitatively correlated to either a disease or to the health status of a patient or to a process status in manufacturing or to environmental and food status. Epitopes may also be at least part of therapeutically relevant molecules, i.e. molecules which can be targeted by the specific binding domain which changes the course of the disease.

As used herein, the term "specificity" or "specific binding" refers to a binding reaction which is determinative of the cognate ligand of interest in a heterogeneous population of molecules. Thus, under designated conditions (e.g. immunoassay conditions), the modular antibody binds to its particular target and does not bind in a significant amount to other molecules present in a sample. The specific binding means that binding is selective in terms of target identity, high, medium or low binding affinity or avidity, as selected. Selective binding is usually achieved if the binding constant or binding dynamics is at least 10 fold different, preferably the difference is at least 100 fold, and more preferred a least 1000 fold.

The term "cytotoxic" or "cytotoxic activity" as used for the purpose of the invention shall refer to any specific molecule directed against cellular antigens that, when bound to the antigen, activates programmed cell death and triggers apoptosis. Besides the apoptotic activity the modular antibody according to the invention may as well mediate ADCC or CDC, which is of particular importance when the target cells are heterogeneous and would express the target antigen to a different extent or even express only one of the relevant targets on the cell surface. The preferred modular antibody according to the invention is effective by its activity on effector cells resulting in activation of cytotoxic T-cells or cells which mediate antibody-dependent cell cytotoxicity (ADCC), complement dependent cytotoxicity (CDC) and/or cellular phagocytosis (ADCP). Preferred modular antibodies according to the invention kill antibody-coated target cells by apoptosis inducing programmed cell death and/or by binding to Fc receptors of effector cells mediating ADCC and/or CDC activity.

Antibody-Dependent Cell-Mediated Cytotoxicity (ADCC) is a mechanism of cell-mediated immunity whereby an effector cell of the immune system actively lyses a target cell that has been bound by specific antibodies. It is one of the mechanisms through which antibodies, as part of the humoral immune response, can act to limit and contain infection. Classical ADCC is mediated by NK cells; monocytes and eosinophils can also mediate ADCC. ADCC is part of the adaptive immune response due to its dependence on a prior antibody response.

The term "foreign" in the context of amino acids shall mean the newly introduced amino acids being naturally occurring, but foreign to the site of modification, or substitutes of naturally occurring amino acids. "Foreign" with reference to an antigen binding sites means that the antigen binding site is not naturally formed by the specific binding region of the agent, and a foreign binding partner, but not the natural binding partner of the agent, is bound by a newly engineered binding site.

The modular antibody according to the invention preferably comprises a foreign antigen-binding site to bind such foreign binding partner.

The term "variable binding region" also called "CDR region" as used herein refers to molecules with varying structures capable of binding interactions with antigens. Those molecules can be used as such or integrated within a larger protein, thus forming a specific region of such protein with binding function. The varying structures can be derived from natural repertoires of binding proteins such as immunoglobulins or phylomers or synthetic diversity, including repeat-proteins, avimers and anticalins. The varying structures can as well be produced by randomization techniques, in particular those described herein. These include mutagenized CDR or non-CDR regions, loop regions of immunoglobulin variable domains or constant domains, in particular CDR loops of immunoglobulins. Typically, binding structures of the modular antibody according to the invention are formed by such variable binding regions.

Modified binding agents with different modifications at specific sites are referred to as "variants". Variants of a scaffold are preferably grouped to form libraries of binding agents, which can be used for selecting members of the library with predetermined functions. In accordance therewith, an antibody sequence is preferably randomized, e.g. through mutagenesis methods. According to a preferred embodiment a loop region of a binding agent, such as the parent antibody sequence comprising positions within one or more loops or at a terminal site, potentially contributing to a binding site, is preferably mutated or modified to produce libraries, preferably by random, semi-random or, in particular, by site-directed random mutagenesis methods, thus, resulting in a randomized sequence, in particular to delete, exchange or introduce randomly generated inserts into loops or a loop region, preferably into the CDR loop region or structural loop region, which may include terminal sequences, that are located at one of the termini of an antibody domain or substructure.

Alternatively preferred is the use of combinatorial approaches. Any of the known mutagenesis methods may be employed, among them cassette mutagenesis. These methods may be used to make amino acid modifications at desired positions of the immunoglobulin of the present invention. In some cases positions are chosen randomly, e.g. with either any of the possible amino acids or a selection of preferred amino acids to randomize loop sequences, or amino acid changes are made using simplistic rules. For example all residues may be mutated preferably to specific amino acids, such as alanine, referred to as amino acid or alanine scanning. Such methods may be coupled with more sophisticated engineering approaches that employ selection methods to screen higher levels of sequence diversity.

The term "functionally equivalent variant" or "functionally active variant" of a modular antibody as used herein means a sequence resulting from modification of this sequence by insertion, deletion or substitution of one or more amino acids or nucleotides within the sequence or at either or both of the distal ends of the sequence, and which modification does not affect (in particular impair) the activity of this sequence. In the case of a binding site having specificity to a selected target antigen, the functionally active variant of a modular antibody according to the invention would still have the predetermined binding specificity, though this could be changed, e.g. to change the fine specificity to a specific epitope, the affinity, the avidity, the Kon or Koff rate, etc. In a preferred embodiment the functionally active variant a) is a biologically active fragment of the modular antibody, the functionally active fragment comprising at least 50% of the sequence of the modular antibody, preferably at least 70%, more preferably at least 80%, still more preferably at least 90%, even more preferably at least 95% and most preferably at least 97%, 98% or 99%; b) is derived from the modular antibody by at least one amino acid substitution, addition and/or deletion, wherein the functionally active variant has a sequence identity to the modular antibody or its relevant antibody domain or the antigen binding site of at least 50%, preferably at least 60%, more preferably at least 70%, more preferably at least 80%, still more preferably at least 90%, even more preferably at least 95% and most preferably at least 97%, 98% or 99%; and/or c) consists of the modular antibody or a functionally active variant thereof and additionally at least one amino acid or nucleotide heterologous to the polypeptide or the nucleotide sequence, preferably wherein the functionally active variant is derived from or identical to an antibody or Fc fragment comprising a constant domain having an EF loop sequence, and optionally a further loop structure comprising any of the AB and/or CD loop sequences, as listed in Table 1, which loop sequences form an antigen binding site, which preferably specifically binds the same epitope recognized by the EAM151-5 Fcab. Further functionally active variants may be obtained by engineering the binding structures to bind the sTAG target. For instance, the CDR binding site of the exemplary bispecific antibodies or the binding moiety derived from a natural receptor capable of binding the target sTAG, may be engineered to create variants which maintain the functional property of antigen binding, e.g. with properties modified to improve its effect.

Specifically when the function as an internalising modular antibody triggering cell killing is proven with a variant, the variant is called "functionally active variant".

Functionally active variants may be obtained by changing the sequence as defined above and are characterized by having a biological activity similar to that displayed by the respective sequence, including the ability to bind the target antigens.

The functionally active variant may be obtained by sequence alterations in the polypeptide or the nucleotide sequence, wherein the sequence alterations retains a function of the unaltered polypeptide or the nucleotide sequence, when used in combination of the invention. Such sequence alterations can include, but are not limited to, (conservative) substitutions, additions, deletions, mutations and insertions.

The variant of the polypeptide or the nucleotide sequence is functionally active in the context of the present invention, if the activity of the composition of the invention including the variant (but not the original) amounts to at least 10%, preferably at least 25%, more preferably at least 50%, even more preferably at least 70%, still more preferably at least 80%, especially at least 90%, particularly at least 95%, most preferably at least 99% of the activity of the internalising modular antibody of the invention including the polypeptide or the nucleotide sequence without sequence alteration (i.e. the original polypeptide or the nucleotide sequence).

In one preferred embodiment of the invention, the functionally active variant of the modular antibody of the invention is essentially identical to the polypeptide or the nucleotide sequence described above, but differs from the polypeptide or the nucleotide sequence, respectively, in that it is derived from a homologous sequence of a different species. These are referred to as naturally occurring variants.

The term "functionally active variant" also includes naturally occurring allelic variants, as well as mutants or any other non-naturally occurring variants. As is known in the art, an allelic variant is an alternate form of a (poly)peptide that is characterized as having a substitution, deletion, or addition of one or more amino acids that does essentially not alter the biological function of the polypeptide.

In a preferred embodiment, the functionally active variant derived from the modular antibody as defined above by amino acid exchanges, deletions or insertions may also conserve, or more preferably improve, the activity.

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

In another embodiment of the invention the polypeptide or the nucleotide sequence as defined above may be modified by a variety of chemical techniques to produce derivatives having essentially the same activity (as defined above for fragments and variants) as the modified modular antibody, and optionally having other desirable properties.

As used herein, a "homologue" or "functional homologue" of a polypeptide shall mean that polypeptides have the same or conserved residues at a corresponding position in their primary, secondary or tertiary structure. The term also extends to two or more nucleotide sequences encoding homologous polypeptides. In particular, homologous compounds usually have at least about 50% amino acid sequence identity with regard to a full-length native sequence or any fragment thereof. Preferably, a homologous compound will have at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity to a native compound, or any other specifically defined fragment of a full-length compound. Specifically when the function as an internalising modular antibody triggering cell killing is proven with such a homologue, the homologue is called "functional homologue".

The term "homologous nucleotide sequences" as used herein refers to nucleotide sequences which are related but not identical in their nucleotide sequence with the contemplated nucleotide sequence, and perform essentially the same function. These are also meant to encompass variations in its nucleotide composition including variations due to the degeneracy of the genetic code, whereby the nucleotide sequence performs essentially the same function.

"Percent (%) amino acid sequence identity" with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

It surprisingly turned out that the modular antibody according to the invention was effectively internalized through binding to the tumor cell receptor, thereby internalising the sTAG and sTAG multimers, respectively, which lead to direct cell killing, as determined by the cytolysis independent of NK cells.

The modular antibody according to the invention could show an improved anti-tumor killing activity, optionally in addition to its cytotoxic activity associated with ADCC and/ or CDC activity, and may therefore be provided specifically for the treatment of immunocompromised patients, such as those suffering from NK deficiency, e.g. transient or local leukocytopenia, e.g. resulting from medication. In accordance therewith, it is preferred to treat solid tumor patients in combination with chemotherapy or radiotherapy.

A preferred embodiment employs an Fc fragment according to the invention, which is directed to the EGFR epitope. Prefereably such EGFR Fcab binds to an epitope specifically recognised by the EAM151-5 Fcab, either used as a single molecule or as a building block to engineer larger constructs.

Functionally active variants of such EGFR Fcab molecules have proven to specifically compete with EGF binding for binding to Her-1 on MB468 tumor cells, with potency similar to cetuximab. The Fcab also showed blocking of EGF induced signal transduction in an assay employing SKBR3 cells. Thus, it has proven that the Fcab molecules would not act as signaling agonist. It is preferred that such EGFR Fcab molecules also have a cytotoxic effect by an ADCC activity. Functionally active EGFR Fcab molecules are preferably selected with an activity profile of high affinity binding to EGFR, in competition with EGF binding, ADCC activity and manufacturability. Preferred Fcab molecules are used as building blocks to create the full length antibodies, which bind either VEGF or RANKL by the CDR binding site formed by the VH/VL immunoglobulin domains, e.g. of parent antibodies, such as avastin or denosumab.

It has been shown that in such constructs the affinities and the competition with EGF for binding EGFR were comparable to the EGFR Fcab molecules. There was still an ADCC activity upon engineering the full-length bispecific modular antibodies. Importantly, such bispecific constructs induced internalisation of the sTAG.

It was shown that VEGF was effectively internalised upon cross-linking of the modular antibody directed to VEGF and EGFR, on EGFR positive tumor cells. Such cross-linking of the cell-bound modular antibodies with VEGF lead to direct killing of EGFR positive tumor cells.

Likewise, a modular antibody directed to RANKL and EGFR induced internalisation of RANKL. Cross-linking of the modular antibodies with RANKL on EGFR positive cells lead to efficient internalisation of the cytokine. There was even such a rapid internalisation of the antibody upon RANKL binding that the ADCC potency was partly lost. By such cross-linking and internalisation there was an effective direct cell killing of EGFR positive tumor cells.

Moreover, some experiments have proven that there was a loss of EGFR expression on the surface of the tumor cells upon internalising bispecific constructs.

The modular antibody according to the invention surprisingly exerts a direct cytotoxicity, which is independent of NK cells. Through binding to at least both specificities, the cell surface receptor and the sTAG, direct cell lysis was observed, which has proven to be synergistic when binding to both targets.

The internalising function of the modular antibody of the invention may be determined in a standard assay, such as described in the Examples. A cytotoxic compound is effective in an apoptosis assay by activating a genetic program of controlled cell death. Apoptosis is characterized by well defined cytological and molecular events including a change in the refractive index of the cell, cytoplasmic shrinkage, nuclear condensation and cleavage of DNA into regularly sized fragments. Cells that are undergoing apoptosis shut down metabolism, lose membrane integrity and form membrane blebs.

The apoptotic activity is preferably measured using standard methods of determinating dying and/or dead cells. In order to measure apoptosis, cytotoxicity assays can be employed. These assays are can be radioactive and non-radioactive assays that measure increases in plasma membrane permeability, since dying cells become leaky or colorimetric assays that measure reduction in the metabolic activity of mitochondria; mitochondria in dead cells cannot metabolize dyes, while mitochondria in live cells can.

One can also measure early indicators for apoptosis such as alterations in membrane asymmetry resulting in occurrence of phosphatidylserine on the outside of the cell surface (Annexin V based assays). Alternatively, later stages of apoptosis, such as activation of caspases can be measured in populations of cells or in individual cells. In addition, measurement of release of cytochrome C and AIF into cytoplasm by mitochondria or fragmentation of chromosomal DNA can be determined.

Terminal deoxynucleotidyl transferase dUTP nick end labeling (TUNEL) is a common method for detecting DNA fragmentation that results from apoptotic signaling cascades. The assay relies on the presence of nicks in the DNA which can be identified by terminal deoxynucleotidyl transferase, an enzyme that will catalyze the addition of bromolated dUTPs that are secondarily detected with a specific labelled antibody

The preferred apoptotic activity of the modular antibody according to the invention amounts to at least 20% of cytolysis, preferably at least 30%, more preferred at least 40%, even more preferred at least 50%, as measured in a respective ex *vivo* cell killing assay.

Though there was a long term need for highly effective cancer immunotherapy, prior art methods mainly relied on ADCC or CDC to kill cancer cells. Any apoptotic activity was considered too weak for anti-tumor therapy. A systemic or local deficiency of lymphocytes or NK cells, however, significantly hampers the chances of a successful therapy. Chemotherapy or radiotherapy is commonly used in treatment of solid tumor disease, which evidently rendered the patients immunocompromised. On the other hand, antibody therapy may lead to local NK cell deficiency at the tumor site because of effector cell consummation due to the antibody's effector function mediating ADCC and/ or CDC activity. The subject matter of the present invention avoids the disadvantages of the prior art immunotherapies and provides for an effective immunotherapy in solid tumor disease.

According to a specific embodiment of the present invention the modular antibody is an immunoglobulin of human or murine origin, or a humanized or chimeric immunoglobulin, which may be employed for various purposes, in particular in pharmaceutical compositions.

The human immunoglobulin, is preferably selected or derived from the group consisting of IgA1, IgA2, IgD, IgE, IgG1, IgG2, IgG3, IgG4 and IgM. The murine immunoglobulin binding agent is preferably selected or derived from the group consisting of IgA, IgD, IgE, IgG1, IgG2A, IgG2B, IgG2C, IgG3 and IgM.

A modular antibody according to the invention may comprise a heavy and/or light chain, at least one variable and/or constant domain, or a part thereof including a minidomain.

A constant domain is an immunoglobulin fold unit of the constant part of an immunoglobulin molecule, also referred to as a domain of the constant region (e.g. CH1, CH2, CH3, CH4, Ck, CI).

A variable domain is an immunoglobulin fold unit of the variable part of an immunoglobulin, also referred to as a domain of the variable region (e.g. Vh, Vk, VI, Vd)

The modular antibody according to the invention preferably is derived from the immunoglobulin structure, such as a full length immunoglobulin. The modular antibody according to the present invention may comprise one or more domains (e.g. at least two, three, four, five, six, ten domains). The preferred format is an oligomer, composed of modular antibody domains, preferably 2 to 12 domains, more preferred at least 4 domains, which oligomer preferably comprises a heterodimer, such as Fab, or a homodimer, such as Fc.

It is feasible to provide the preferred modular antibody of the invention as a single domain antibody. However, antibody domains tend to dimerize upon expression, either as a homodimer, like an Fc, or a heterodimer, like an Fab. The dimeric structure is thus considered advantageous to provide a stable molecule. The preferred dimers of immunoglobulin domains are selected from the group consisting of single domain dimers, like VH/VL, CH1/CL (kappa or lambda), CH2/CH2 and CH3/CH3. Dimers or oligomers of modular antibody domains can also be provided as single chain or two chain molecules, in particular those linking the C-terminus of one domain to the N-terminus of another.

If more than one domain is present in the modular antibody these domains may be of the same type or of varying types (e.g. CH1-CH1-CH2, CH3-CH3, (CH2)₂-(CH3)₂, with or without the hinge region). Of course also the order of the single domains may be of any kind (e.g. CH1-CH3-CH2, CH4-CH1-CH3-CH2).

The invention preferably refers to part of antibodies, such as parts of IgG, IgA, IgM, IgD, IgE and the like. The modular antibodies of the invention may also be a functional antibody fragment such as Fab, Fab₂, scFv, Fv, Fc, Fcab, an antigen-binding Fc, or parts thereof, or other derivatives or combinations of the immunoglobulins such as minibodies, domains of the heavy and light chains of the variable region (such as dAb, Fd, VL, including Vlambda and Vkappa, VH, VHH) as well as mini-domains consisting of two beta-strands of an immunoglobulin domain connected by at least two structural loops, as isolated domains or in the context of naturally associated molecules. A particular embodiment of the present invention refers to the Fc fragment of an antibody molecule, either as antigen-binding Fc fragment (Fcab) through modifications of the amino acid sequence or as conjugates or fusions to receptors, peptides or other antigen-binding modules, such as scFv.

An exemplary modular antibody according to the invention comprises a constant domain selected from the group consisting of CH1, CH2, CH3, CH4, Igk-C, Igl-C, combinations, derivatives or a part thereof including a mini-domain, with at least one structural loop region, and is characterised in that said at least one loop region comprises at least one amino acid modification forming at least one modified loop region, wherein said at least one modified loop region binds specifically to at least one epitope of an antigen.

Another modular antibody according to the invention can comprises a variable domain of a heavy or light chain, combinations, derivatives or a part thereof including a minidomain, with at least one variable and/or structural loop region, and is characterised in that said at least one loop region comprises at least one amino acid modification forming at least one modified loop region, wherein said at least one modified loop region binds specifically to at least one epitope of an antigen.

The modular antibodies can be used as isolated polypeptides or as combination molecules, e.g. through recombination, fusion or conjugation techniques, with other peptides or polypeptides. The peptides are preferably homologous to immunoglobulin domain sequences, and are preferably at least 5 amino acids long, more preferably at least 10 or even at least 50 or 100 amino acids long, and constitute at least partially the loop region of the immunoglobulin domain. The preferred binding characteristics relate to predefined epitope binding, affinity and avidity.

The modular antibody according to the invention is possibly further combined with one or more modified modular antibodies or with unmodified modular antibodies, or parts thereof, to obtain a combination modular antibody. Combinations are preferably obtained by recombination techniques, but also by binding through adsorption, electrostatic interactions or the like, or else through conjugation or chemical binding with or without a linker. The preferred linker sequence is either a natural linker sequence or functionally suitable artificial sequence. By such combination it is possible to link the structures responsible for the individual binding specificities, thereby providing for the crosslinking of the target structures on the cell surface.

In general, the modular antibody according to the invention may be used as a building block to molecularly combine other modular antibodies or biologically active substances or molecules. It is preferred to molecularly combine at least one antibody binding to the specific partner via the variable or non-variable sequences, like structural loops, with at least one other binding molecule which can be an antibody, antibody fragment, a receptor, a ligand or another antibody domain, or a binding moiety thereof. Other combinations refer to proteinaceous molecules, nucleic acids, lipids, organic molecules and carbohydrates.

The engineered molecules according to the present invention will be useful as stand-alone molecules, as well as fusion proteins or derivatives, most typically fused before or after modification in such a way as to be part of larger structures, e.g. of complete antibody molecules, or parts thereof. Immunoglobulins or fusion proteins as produced according to the invention thus also comprise Fc fragments, Fab fragments, Fv fragments, single chain antibodies, in particular single-chain Fv fragments, bi- or multispecific scFv, diabodies, unibodies, multibodies, multivalent or multimers of immunoglobulin domains and others. It will be possible to use the engineered proteins to produce molecules which are monospecific, bispecific, trispecific, and may even carry more specificities. By the invention it is be possible to control and preselect the valency of binding at the same time according to the requirements of the planned use of such molecules.

According to the present invention, the modular antibody optionally exerts further binding regions to antigens, including the binding site binding specifically to a cell surface target and binding sites mediating effector function. Antigen binding sites to one or more antigens may be presented by the CDR-region or any other natural receptor binding structure, or be introduced into a structural loop region of an antibody domain, either of a variable or constant domain structure. The antigens as used for testing the binding properties of the binding sites may be naturally occurring molecules or chemically synthesized molecules or recombinant molecules, either in solution or in suspension, e.g. located on or in particles such as solid phases, on or in cells or on viral surfaces. It is preferred that the binding of an immunoglobulin to an antigen is determined when the antigen is still adhered or bound to molecules and structures in the natural context. Thereby it is possible to identify and obtain those modified immunoglobulins that are best suitable for the purpose of diagnostic or therapeutic use.

It is particularly preferred that the modular antibody according to the invention is capable of binding to said receptor through at least a structural loop region.

It is further preferred that the modular antibody according to the invention is capable of binding to said sTAG through at least a CDR region. The preferred modular antibody according to the invention has a CDR binding site and/or specificity of the avastin antibody or denosumab antibody, including chimeric, humanized or human variants, e.g. glycoengineered variants.

The modular antibody according to the invention may specifically bind to any kind of antigens, in particular to epitope structures derived from proteinaceous molecules, proteins, peptides, polypeptides, but also nucleic acids, glycans and carbohydrates. The preferred modular antibody according to the invention may comprise at least two loops or loop regions whereby each of the loops or loop regions may specifically bind to different molecules or epitopes.

Preferably the target erbB receptor antigen is selected from the group consisting of erbB receptor tyrosine kinases, such as EGFR, HER2 including Her2neu, HER3 and HER4, in particular those epitopes of the extracellular domains of such receptors, e.g. the 4D5 epitope. In addition further antigens may be targeted, e.g. molecules of the TNF-receptor superfamily, such as Apo-1 receptor, TNFR1, TNFR2, nerve growth factor receptor NGFR, CD40, CD40-Ligand, OX40, TACI, BCMA, BAFF-receptor, T-cell surface molecules, T-cell receptors, T-cell antigen, Apo-3, DR4, DR5, DR6, decoy receptors ,such as DcR1, DcR2, CAR1, HVEM, GITR, ZTNFR-5, NTR-1, TNFL1, IGFR-1, c-Met, but not limited to these molecules, B-cell surface antigens, such as CD10, CD19, CD20, CD21, CD22, , DC-SIGN, antigens or markers of solid tumors or hematologic cancer cells, cells of lymphoma or leukaemia, other blood cells including blood platelets, but not limited to these molecules.

According to a preferred embodiment a further target antigen is selected from those antigens presented by cells, like epithelial cells or cells of solid tumors. Those target antigens expressed or overexpressed by cells are preferably targeted, which are selected from the group consisting of tumor associated antigens, in particular EpCAM, tumor-associated glycoprotein-72 (TAG-72), tumor-associated antigen CA 125, Prostate specific membrane antigen (PSMA), High molecular weight melanoma-associated antigen (HMW-MAA), tumor-associated antigen expressing Lewis y related carbohydrate, Carcinoembryonic antigen (CEA), CEACAM5, HMFG PEM, mucin MUC1, MUC18 and cytokeratin tumor-associated antigen, CD44 and its splice variants, bacterial antigens, viral antigens, allergens, allergy related molecules IgE, cKIT and Fc-epsilon-receptorl, IRp60, IL-5 receptor, CCR3, red blood cell receptor (CR1), human serum albumin, mouse serum albumin, rat serum albumin, Fc receptors, like neonatal Fc-gamma-receptor FcRn, Fc-gamma-receptors Fc-gamma RI, Fc-gamma-RII, Fc-gamma RIII, Fc-alpha-receptors, Fc-epsilon-receptors, fluorescein, lysozyme, toll-like receptor 9, erythropoietin, CD2, CD3, CD3E, CD4, CD11, CD11a, CD14, CD16, CD18, CD19, CD20, CD22, CD23, CD25, CD28, CD29, CD30, CD32, CD33 (p67 protein), CD38, CD40, CD40L, CD52, CD54, CD56, CD64, CD80, CD147, GD3, IL-1, IL-1R, IL-2, IL-2R, IL-4, IL-5, IL-6, IL-6R, IL-8, IL-12, IL-15, IL-17, IL-18, IL-23, LIF, OSM, interferon alpha, interferon beta, interferon gamma; TNF-alpha, TNFbeta2, TNFalpha, TNFalphabeta, TNF-R1, TNF-RII, FasL, CD27L, CD30L, 4-1 BBL, TRAIL, RANKL, TWEAK, APRIL, BAFF, LIGHT, VEG1, OX40L, TRAIL Receptor-1, A1 Adenosine Receptor, Lymphotoxin Beta Receptor, TACI, BAFF-R, EPO; LFA-3, ICAM-1, ICAM-3, integrin beta1, integrin beta2, integrin alpha4/beta7, integrin alpha2, integrin alpha3, integrin alpha4, integrin alpha5, integrin alpha6, integrin alphav, alphaVbeta3 integrin, FGFR-3, Keratinocyte Growth Factor, GM-CSF, M-CSF, RANKL, VLA-1, VLA-4, L-selectin, anti-Id, E-selectin, HLA, HLA-DR, CTLA-4, T cell receptor, B7-1, B7-2, VNRintegrin, TGFbeta1, TGFbeta2, eotaxin1, BLyS (B-lymphocyte Stimulator), complement C5, IgE, IgA, IgD, IgM, IgG, factor VII, CBL, NCA 90, EGFR (ErbB-1), Her2/neu (ErbB-2), Her3 (ErbB-3), Her4 (ErbB4), Tissue Factor, VEGF, VEGFR, endothelin receptor, VLA-4, carbohydrates such as blood group antigens and related carbohydrates, Galili-Glycosylation, Gastrin, Gastrin receptors, tumor associated carbohydrates, Hapten NP-cap or NIP-cap, T cell receptor alpha/beta, E-selectin, P-glycoprotein, MRP3, MRP5, glutathione-S-transferase pi (multi drug resistance proteins), alpha-granule membrane protein(GMP) 140, digoxin, placental alkaline phosphatase (PLAP) and testicular PLAP-like alkaline phosphatase, transferrin receptor, Heparanase I, human cardiac myosin, Glycoprotein IIb/IIIa (GPIIb/IIIa), human cytomegalovirus (HCMV) gH envelope glycoprotein, HIV gp120, HCMV, respiratory syncytial virus RSV F, RSVF Fgp, VNRintegrin, Hep B gp120, CMV, gpllbllla, HIV IIIB gp120 V3 loop, respiratory syncytial virus (RSV) Fgp, Herpes simplex virus (HSV) gD glycoprotein, HSV gB glycoprotein, HCMV gB envelope glycoprotein, Clostridium perfringens toxin and fragments thereof.

Preferred multispecific modular antibodies are capable of simultaneously binding specifically to both targets, the sTAG and the tumor cell receptor.

A modular antibody or immunoglobulin domains may be modified to change existing antigen binding sites or to add new antigen binding sites, which modifications are preferably effected in immunoglobulin domains or parts thereof that are either terminal sequences, preferably a C-terminal sequence, and/or part of a loop region, which contains a loop, either a CDR-loop or a non-CDR loop, structural loops being the preferred sites of modifications or mutagenesis. According to a specific embodiment the structural loop region also includes a terminal sequence, which contributes to antigen binding. In some cases it is preferable to use a defined modified structural loop or a structural loop region, or parts thereof, as isolated molecules for binding or combination purposes.

Specific modifications of the nucleic acid or amino acid sequences in a predetermined region, which result from random insertion or exchange or deletion of amino acids, either a selection of amino acids or the whole range of natural or synthetic amino acids, will result in a "randomized" sequence of a modular antibody according to the invention.

In a domain structure of a modular antibody it is preferred to modify or randomize the modular antibody within at least one loop region or terminal region, resulting in a substitution, deletion and/or insertion of one or more nucleotides or amino acids, preferably a point mutation, or even the exchange of whole loops, more preferred the change of at least 2, 3, 4, 5, 6, 7, 8, 9, 10, 11, 12, 13, 14 or 15, up to 30 amino acids. Thereby the modified sequence comprises amino acids not included in the conserved regions of the loops, the newly introduced amino acids being naturally occurring, but foreign to the site of modification, or substitutes of naturally occurring amino acids.

However, the maximum number of amino acids inserted into a loop region of a binding agent preferably may not exceed the number of 30, preferably 25, more preferably 20 amino acids at a maximum. The substitution and the insertion of the amino acids occurs preferably randomly or semi-randomly using all possible amino acids or a selection of preferred amino acids for randomization purposes, by methods known in the art and as disclosed herein.

The site of modification may be at a specific single loop or a loop region, in particular a structural loop or a structural loop region. A loop region usually is composed of at least two, preferably at least 3 or at least 4 loops that are adjacent to each other, and which may contribute to the binding of an antigen through forming an antigen binding site or antigen binding pocket. It is preferred that the one or more sites of modification are located within the area of 10 amino acids, more preferably within 20, 30, 40, 50, 60, 70, 80, 90 up to 100 amino acids, in particular within a structural region to form a surface or pocket where the antigen can sterically access the loop regions.

In this regard the preferred modifications are engineered in the loop regions of CH1, CH2, CH3 and CH4, in particular in the range of amino acids 7 to 21, amino acids 25 to 39, amino acids 41 to 81, amino acids 83 to 85, amino acids 89 to 103 and amino acids 106 to 117, or within the terminal sequences, preferably within 6 amino acids from the C- or N-terminus of the antibody domain.

In another preferred embodiment a modification in the structural loop region comprising amino acids 92 to 98 is combined with a modification in the structural loop region comprising amino acids 8 to 20.

The above identified amino acid regions of the respective immunoglobulins comprise loop regions preferably modified. Preferably, a modification in the structural loop region comprising amino acids 92 to 98 is combined with a modification in one or more of the other structural loops.

In a preferred embodiment a modification in the structural loop region comprising amino acids 92 to 98 is combined with a modification in the structural loop region comprising amino acids 41 to 45.2.

Most preferably each of the structural loops comprising amino acids 92 to 98, amino acids 41 to 45.2 and amino acids 8 to 20 contain at least one amino acid modification.

In another preferred embodiment each of the structural loops comprising amino acids 92 to 98, amino acids 41 to 45.2, and amino acids 8 to 20 contain at least one amino acid modification.

According to another preferred embodiment the amino acid residues in the area of positions 15 to 17, 29 to 34, 41 to 45.2, 84 to 85, 92 to 100, and/or 108 to 115 of CH3 are modified.

The preferred modifications of Igk-C and Igl-C of human origin are engineered in the loop regions in the area of amino acids 8 to 20, amino acids 26 to 36, amino acids 41 to 82, amino acids 83 to 88, amino acids 92 to 100, amino acids 107 to 124 and amino acids 123 to 126, or within the terminal sequences, preferably within 6 amino acids from the C- or N-terminus of the antibody domain.

The preferred modifications of loop regions of Igk-C and Igl-C of murine origin are engineered at sites in the area of amino acids 8 to 20, amino acids 26 to 36, amino acids 43 to 79, amino acids 83 to 85, amino acids 90 to 101, amino acids 108 to 116 and amino acids 122 to 126.

Another preferred modular antibody that is preferably employed consists of a variable domain of a heavy or light chain, or a part thereof including a minidomain, with at least one loop region, preferably a structural loop region, and is characterised in that said at least one loop region comprises at least one amino acid modification forming at least one modified loop region, wherein said at least one modified loop region forms a relevant binding site as described above.

According to a specific embodiment the modular antibody preferably used according to the invention may contain a modification within the variable domain, which is selected from the group of VH, Vkappa, Vlambda, VHH and combinations thereof. More specifically, they comprise at least one modification within amino acids 7 to 22, amino acids 39 to 55, amino acids 66 to 79, amino acids 77 to 89 or amino acids 89 to 104, where the numbering of the amino acid position of the domains is that of the IMGT, or within the terminal sequences, preferably within 6 amino acids from the C- or N-terminus of the antibody domain.

In a specific embodiment, the modular antibody preferably used according to the invention is characterised in that the loop regions of VH or Vkappa or Vlambda of human origin comprise at least one modification within amino acids 7 to 22, amino acids 43 to 51, amino acids 67 to 77, amino acids 77 to 88, and amino acids 89 to 104, most preferably amino acid positions 12 to 17, amino acid positions 45 to 50, amino acid positions 68 to 77, amino acids 79 to 88, and amino acid positions 92 to 99, where the numbering of the amino acid position of the domains is that of the IMGT.

The structural loop regions of the immunoglobulin variable domain of human origin, as possibly selected for modification purposes are preferably located in the area of amino acids 8 to 20, amino acids 44 to 50, amino acids 67 to 76, amino acids 78 to 87, and amino acids 89 to 101, or within the terminal sequences, preferably within 6 amino acids from the C- or N-terminus of the antibody domain.

According to a preferred embodiment the structural loop regions of the immunoglobulin variable domain of murine origin as possibly selected for modification purposes are preferably located in the area of amino acids 6 to 20, amino acids 43 to 52, amino acids 67 to 79, amino acids 79 to 87, and amino acids 91 to 100, or within the terminal sequences, preferably within 6 amino acids from the C- or N-terminus of the antibody domain.

The immunoglobulin preferably used according to the invention may also be of camelid origin. Camel or camelid antibodies comprise only one heavy chain and have the same antigen affinity as normal antibodies consisting of light and heavy chains. Consequently those antibodies are much smaller than, e.g., human antibodies, which allows them to penetrate dense tissues to reach the antigen, where larger proteins cannot. Moreover, the comparative simplicity, high affinity and specificity and the potential to reach and interact with active sites, camel's heavy chain antibodies present advantages over common antibodies in the design, production and application of clinically valuable compounds.

According to another preferred embodiment of the present invention the structural loop regions of a modular antibody or an immunoglobulins of camelid origin are modified, e.g. within a VHH, in the region of amino acids 7 to 19, amino acids 43 to 55, amino acids 68 to 76, amino acids 80 to 87 and amino acids 91 to 101, or within the terminal sequences, preferably within 6 amino acids from the C- or N-terminus of the antibody domain.

All numbering of the amino acid sequences of the immunoglobulins is according to the IMGT numbering scheme (IMGT, the international ImMunoGeneTics, Lefranc et al., 1999, Nucleic Acids Res. 27: 209-212).

The preferred method of producing the modular antibody according to the invention refers to engineering a modular antibody that is binding specifically to at least one first epitope, which comprises modifications in each of at least two sites or loops within a structural loop region, and determining the specific binding of said structural loop region to at least one second epitope in a screening process, wherein the unmodified structural loop region (non-CDR region) does not specifically bind to said at least one second epitope. Thus, an antibody or antigen-binding structure specific for a first antigen may be improved by adding another valency or specificity against a second antigen, which specificity may be identical, either targeting different epitopes or the same epitope, to increase valency or to obtain bi-, oligo- or multispecific molecules.

On the other hand it is preferred to make use of those modular antibodies that contain native structures interacting with effector molecules or immune cells, preferably to bind an effector ligand. Those native structures either remain unchanged or are modulated for an increased effector function. Binding sites for e.g. Fc receptors are described to be located in a CH2 and/or CH3 domain region, and may be mutagenized by well known techniques.

Preferred modular antibodies according to the invention are binding said individual antigens with a high affinity, in particular with a high on and/or a low off rate, or a high avidity of binding. The binding affinity of an antibody is usually characterized in terms of the concentration of the antibody, at which half of the antigen binding sites are occupied, known as the dissociation constant (Kd, or K_{D}). Usually a binder is considered a high affinity binder with a Kd<10⁻⁸ M, preferably a Kd<10⁻⁹ M, even more preferred is a Kd<10⁻¹⁰ M.

Yet, in a particularly preferred embodiment the individual antigen binding affinities are of medium affinity, e.g. with a Kd of less than 10⁻⁶ and up to 10⁻⁸ M, e.g. when binding to at least two antigens.

Medium affinity binders may be provided according to the invention, preferably in conjunction with an affinity maturation process, if necessary.

Affinity maturation is the process by which antibodies with increased affinity for a target antigen are produced. With structural changes of an antibody, including amino acid mutagenesis or as a consequence of somatic mutation in immunoglobulin gene segments, variants of a binding site to an antigen are produced and selected for greater affinities. Affinity matured modular antibodies may exhibit a several logfold greater affinity than a parent antibody. Single parent antibodies may be subject to affinity maturation. Alternatively pools of modular antibodies with similar binding affinity to the target antigen may be considered as parent structures that are varied to obtain affinity matured single antibodies or affinity matured pools of such antibodies.

The preferred affinity maturated variant of a modular antibody according to the invention exhibits at least a 10 fold increase in affinity of binding, preferably at least a 100 fold increase. The affinity maturation may be employed in the course of the selection campaigns employing respective libraries of parent molecules, either with modular antibodies having medium binding affinity to obtain the modular antibody of the invention having the specific target binding property of a binding affinity Kd<10⁻⁸ M. Alternatively, the affinity may be even more increased by affinity maturation of the modular antibody according to the invention to obtain the high values corresponding to a Kd of less than 10⁻⁹ M, preferably less than 10⁻¹⁰ M or even less than 10⁻¹¹ M, most preferred in the picomolar range.

The apoptotic effect of the modular antibody according to the invention has the advantage of a biological cytotoxic activity, which usually differs from any synthetic cytotoxic activity, e.g. as provided through a toxin that may be conjugated to an immunoglobulin structure. Toxins usually do not activate programmed cell death and the biological defence mechanism. Thus, the preferred apoptotic activity of the modular antibodies according to the invention is a biological apoptotic activity, leading to effective cytolysis.

Apoptosis is differentiated from the simple cell inhibition effect, where a substance is inhibiting cell growth, e.g. by binding to the receptor of a growth factor, thus blocking the growth factor function, or by inhibiting angiogenesis. Cytotoxicity through apoptosis is essentially considered as a programmed cell death, and thus considered as a highly efficient way to immediately reduce the number of malignant cells. Cell growth inhibitors do not immediately kill cells, but only reduce the cell growth and proliferation, thus are considered to be less active for therapeutic purposes.

The modular antibody of the present invention may find use in a wide range of indications for antibody products. In one embodiment the modular antibody of the present invention is used for therapy or prophylaxis, e.g. as passive immunotherapy, for preparative, industrial or analytic use, as a diagnostic, an industrial compound or a research reagent, preferably a therapeutic. The modular antibody may find use in an antibody composition that is monoclonal or polyclonal. In a preferred embodiment, the modular antibodies of the present invention are used to capture or kill target cells that bear the target antigen, for example cancer cells that express an tumor associated cell surface receptor.

For particular applications the modular antibody according to the invention is conjugated to a label or reporter molecule, selected from the group consisting of organic molecules, enzyme labels, radioactive labels, colored labels, fluorescent labels, chromogenic labels, luminescent labels, haptens, digoxigenin, biotin, metal complexes, metals, colloidal gold and mixtures thereof. Modified immunoglobulins conjugated to labels or reporter molecules may be used, for instance, in assay systems or diagnostic methods.

The modular antibody according to the invention may be conjugated to other molecules which allow the simple detection of said conjugate in, for instance, binding assays (e.g. ELISA) and binding studies.

In a preferred embodiment, a modular antibody is administered to a patient to treat a specific disorder. A "patient" for the purposes of the present invention includes humans and other animals, preferably mammals and most preferably humans. By "specific disorder" herein is meant a disorder that may be ameliorated by the administration of a pharmaceutical composition comprising a modular antibody of the present invention.

The modular antibody according to the invention is typically used to reduce the likelihood of metastasis developing, shrink tumor size, or slow tumor growth. It may be applied after surgery (adjuvant), before surgery (neo-adjuvant), or as the primary therapy (palliative). In one embodiment, a modular antibody according to the present invention is the only therapeutically active agent administered to a patient.

Alternatively, the modular antibody according the present invention is administered in combination with one or more other therapeutic agents, including but not limited to cytotoxic agents, chemotherapeutic agents, cytokines, growth inhibitory agents, antihormonal agents, kinase inhibitors, anti-angiogenic agents, cardioprotectants, or other therapeutic agents. The modular antibody may be administered concomitantly with one or more other therapeutic regimens. For example, a modular antibody of the present invention may be administered to the patient along with chemotherapy, radiation therapy, or both chemotherapy and radiation therapy. Specifically the modular antibody according to the invention is used for neoadjuvant or adjuvant treatment to treat solid tumor disease conditions, which is either before, simultaneously or after concomitant therapy. Combination with standard treatment is particularly preferred, e.g. as second line treatment. Yet, the modular antibody according to the invention or the combination with standard therapy may as well be indicated as a first line treatment.

A combination therapy is particularly employing a standard regimen, e.g. as used for treating breast cancer, colorectal cancer, head and neck cancer or gastric cancer. This may include cyclophosphamide, doxorubicin, docetaxel, taxane, methotrexate and fluorouracil. Standard treatment of colorectal cancer typically involves the use of 5-fluorouracil (5-FU) or capecitabine (Xeloda), leucovorin (LV, Folinic Acid), oxaliplatin (Eloxatin), oxaliplatin or irinotecan.

In one embodiment, the modular antibody of the present invention may be administered in conjunction with one or more antibodies, which may or may not comprise a modular antibody of the present invention. In accordance with another embodiment of the invention, the modular antibody of the present invention and one or more other anti-cancer therapies is employed to treat cancer cells ex *vivo.* It is contemplated that such ex *vivo* treatment may be useful in bone marrow transplantation and particularly, autologous bone marrow transplantation. It is of course contemplated that the antibodies of the invention can be employed in combination with still other therapeutic techniques such as surgery.

A variety of other therapeutic agents may find use for administration with the modular antibody of the present invention. In one embodiment, the modular antibody is administered with an anti-angiogenic agent, which is a compound that blocks, or interferes to some degree, the development of blood vessels. The anti-angiogenic factor may, for instance, be a small molecule or a protein, for example an antibody, Fc fusion molecule, or cytokine, that binds to a growth factor or growth factor receptor involved in promoting angiogenesis. The preferred anti-angiogenic factor herein is an antibody that binds to VEGF. In an alternate embodiment, the modular antibody is administered with a therapeutic agent that induces or enhances adaptive immune response, for example an antibody that targets CTLA-4. In an alternate embodiment, the modified immunoglobulin is administered with a tyrosine kinase inhibitor, which is a molecule that inhibits to some extent tyrosine kinase activity of a tyrosine kinase. In an alternate embodiment, the modular antibody of the present invention is administered with a cytokine. By "cytokine" as used herein is meant a generic term for proteins released by one cell population that act on another cell as intercellular mediators including chemokines.

Pharmaceutical compositions are contemplated wherein modular antibodies of the present invention and one or more therapeutically active agents are formulated. Stable formulations of the modular antibodies of the present invention are prepared for storage by mixing said immunoglobulin having the desired degree of purity with optional pharmaceutically acceptable carriers, excipients or stabilizers, in the form of lyophilized formulations or aqueous solutions. The formulations to be used for in vivo administration are preferably sterile. This is readily accomplished by filtration through sterile filtration membranes or other methods. The modular antibody and other therapeutically active agents disclosed herein may also be formulated as immunoliposomes, and/or entrapped in microcapsules.

Administration of the pharmaceutical composition comprising a modular antibody of the present invention, preferably in the form of a sterile aqueous solution, may be done in a variety of ways, including, but not limited to, orally, subcutaneously, intravenously, intranasally, intraotically, transdermally, mucosal, topically (e.g., gels, salves, lotions, creams, etc.), intraperitoneally, intramuscularly, intrapulmonary (e.g., AERx™ inhalable technology commercially available from Aradigm, or Inhance™ pulmonary delivery system commercially available from Inhale Therapeutics), vaginally, parenterally, rectally, or intraocularly.

A preferred method of preparing a modular antibody according to the invention comprises the steps of
a. fusing or recombining the following components
   (i) an immunoglobulin domain with a specificity to bind at least a receptor on a tumor cell, and
   (ii) an immunoglobulin domain with a specificity to bind at least an sTAG, to obtain an immunoglobulin with at least both specificities, and
b. determining the cytolysis of said tumor cell.

The preferred method employs engineering steps to build the modular antibody in a modular way. Exemplary modular antibodies are based on an Fc molecule having binding structures in the structural loop region, such as a non-CDR binding site in the C-terminal structural loop region of one or both CH3 domains, a so called "Fcab" molecule (Fc antigen binding). Such Fcab moleculares are preferably used as single molecules, e.g. as a small-sized binder to a target, e.g. composed of four immunoglobulin constant domains, such as a dimer of a CH2-CH3 chain linked to each other with or without a hinge region or a linker, or else used as a building block to create larger constructs, such as full-length antibodies or constructs comprising a binding moiety of a target receptor linked to the Fcab, by recombinant or chemical techniques. Preferably the full-length antibodies are at least bispecific, binding at least two different targets by one or more valencies. Specifically the full-length antibodies would have binding sites of the CDR region and the binding site(s) of an Fcab molecule.

Specifically, there is provided a method of producing a modular antibody according to the invention, which antibody is composed of antibody modules, wherein at least two modules bear an antigen-binding site. The modules may be engineered and selected separately for the desired binding properties and then combined, preferably by fusion or by recombinant techniques. Alternatively, a modular antibody having a binding site for one of the glycoepitope or the receptor may be engineered to introduce a further antigen binding site without any further combination needs.

Engineering new antigen binding sites is preferably employing diversification of nucleic acid or amino acid sequences to provide libraries of variants, which may be selected for specific properties.

According to a preferred aspect modular antibodies are modified by a mutagenesis method to obtain a new (additional) binding site that is foreign to the modular antibody. The preferred mutagenesis refers to randomization techniques, where the amino acid sequence of a peptide or polypeptide is mutated in at least one position, thus a randomized sequence is obtained, which mediates antigen binding. For instance, specific antibody sequences are randomly modified to obtain a nucleic acid molecule coding for an immunoglobulin, immunoglobulin domain or a part thereof which comprises at least one nucleotide repeating unit, preferably within a structural loop coding region or within a terminal region, having the sequence 5'-NNS-3', 5'-NNN-3', 5'- NNB-3' or 5'- NNK-3'. In some embodiments the modified nucleic acid comprises nucleotide codons selected from the group of TMT, WMT, BMT, RMC, RMG, MRT, SRC, KMT, RST, YMT, MKC, RSA, RRC, NNK, NNN, NNS or any combination thereof (the coding is according to IUPAC).

The modification of the nucleic acid molecule may be performed by introducing synthetic oligonuleotides into a larger segment of nucleic acid or by de novo synthesis of a complete nucleic acid molecule. Synthesis of nucleic acid may be performed with tri-nucleotide building blocks which would reduce the number of nonsense sequence combinations if a subset of amino acids is to be encoded (e.g. Yanez et al. Nucleic Acids Res. (2004) 32:e158; Virnekas et al. Nucleic Acids Res. (1994) 22:5600-5607).

Another important aspect of the invention is that each potential binding domain remains physically associated with the particular DNA or RNA molecule which encodes it, and in addition, the fusion proteins oligomerize at the surface of a genetic package to present the binding polypeptide in the native and functional oligomeric structure.

Once successful binding domains are identified, one may readily obtain the gene for expression, recombination or further engineering purposes. The form that this association takes is a "replicable genetic package", such as a virus, cell or spore which replicates and expresses the binding domain-encoding gene, and transports the binding domain to its outer surface. Another form is an in-vitro replicable genetic package such as ribosomes that link coding RNA with the translated protein. In ribosome display the genetic material is replicated by enzymatic amplification with polymerases.

Those cells or viruses or nucleic acid bearing the binding agents which recognize the target molecule are isolated and, if necessary, amplified. The genetic package preferably is M13 phage, and the protein includes the outer surface transport signal of the M13 gene III protein.

The preferred expression system for the fusion proteins is a non-suppressor host cell, which would be sensitive to a stop codon, such as an amber stop codon, and would thus stop translation thereafter. In the absence of such a stop codon such non-suppressor host cells, preferably E.coli, are preferably used. In the presence of such a stop codon supressor host cells would be used.

Preferably in the method of this invention the vector or plasmid of the genetic package is under tight control of the transcription regulatory element, and the culturing conditions are adjusted so that the amount or number of vector or phagemid particles displaying less than two copies of the fusion protein on the surface of the particle is less than about 20%. More preferably, the amount of vector or phagemid particles displaying less than two copies of the fusion protein is less than 10% the amount of particles displaying one or more copies of the fusion protein. Most preferably the amount is less than 1%.

The expression vector preferably used according to the invention is capable of expressing a binding polypeptide, and may be produced as follows: First a binding polypeptide gene library is synthesized by introducing a plurality of polynucleotides encoding different binding sequences. The plurality of polynucleotides may be synthesized in an appropriate amount to be joined in operable combination into a vector that can be propagated to express a fusion protein of said binding polypeptide. Alternatively the plurality of oligonucleotides can also be amplified by polymerase chain reaction to obtain enough material for expression. However, this would only be advantageous if the binding polypeptide would be encoded by a large polynucleotide sequence, e.g. longer than 200 base pairs or sometimes longer than 300 base pairs. Thus, a diverse synthetic library is preferably formed, ready for selecting from said diverse library at least one expression vector capable of producing binding polypeptides having the desired preselected function and binding property, such as specificity.

The randomly modified nucleic acid molecule may comprise the above identified repeating units, which code for all known naturally occurring amino acids or a subset thereof. Those libraries that contain modified sequences wherein a specific subset of amino acids are used for modification purposes are called "focused" libraries. The member of such libraries have an increased probability of an amino acid of such a subset at the modified position, which is at least two times higher than usual, preferably at least 3 times or even at least 4 times higher. Such libraries have also a limited or lower number of library members, so that the number of actual library members reaches the number of theoretical library members. In some cases the number of library members of a focused library is not less than 10³ times the theoretical number, preferably not less than 10² times, most preferably not less than 10 times.

Various alternatives are available for the manufacture of a randomized library. It is possible to produce the DNA by a completely synthetic approach, in which the sequence is divided into overlapping fragments which are subsequently prepared as synthetic oligonucleotides. These oligonucleotides are mixed together, and annealed to each other by first heating to ca. 100°C and then slowly cooling down to ambient temperature. After this annealing step, the synthetically assembled gene can be either cloned directly, or it can be amplified by PCR prior to cloning.

Alternatively, other methods for site directed mutagenesis can be employed for generation of the library insert, such as the Kunkel method (Kunkel TA. Rapid and efficient site-specific mutagenesis without phenotypic selection. Proc Natl Acad Sci U S A. 1985 Jan;82(2):488-92) or the Dpnl method (Weiner MP, Costa GL, Schoettlin W, Cline J, Mathur E, Bauer JC. Site-directed mutagenesis of double-stranded DNA by the polymerase chain reaction. Gene. 1994 Dec 30;151(1-2):119-23.).

For various purposes, it may be advantageous to introduce silent mutations into the sequence encoding the library insert. For example, restriction sites can be introduced which facilitate cloning or modular exchange of parts of the sequence. Another example for the introduction of silent mutations is the ability to "mark" libraries, that means to give them a specific codon at a selected position, allowing them (or selected clones derived from them) e.g. to be recognized during subsequent steps, in which for example different libraries with different characteristics can be mixed together and used as a mixture in the panning procedure.

The method according to the invention can provide a library containing at least 10² independent clones expressing functional modular antibody domains. According to the invention it is also provided a pool of preselected independent clones, which is e.g. affinity maturated, which pool comprises preferably at least 10, more preferably at least 100, more preferably at least 1000, more preferably at least 10000, even more than 100000 independent clones. Those libraries, which contain the preselected pools, are preferred sources to select the high affinity modular antibodies according to the invention.

Usually the libraries as used according to the invention contain variants of the modular antibody, resulting from mutagenesis or randomization techniques. These variants include inactive or non-functional antibodies. Thus, it is preferred that any such libraries be screened with the appropriate assay for determining the functional effect. Preferred libraries, according to the invention, comprise at least 10² variants of modular antibodies, more preferred at least 103, more preferred at least 10⁴, more preferred at least 10⁵, more preferred at least 10⁶, more preferred at least 10⁷, more preferred at least 10⁸, more preferred at least 10⁹, more preferred at least 10¹⁰, more preferred at least 10¹¹, up to 10¹² variants or higher to provide a highly diverse repertoire of modular antibodies for selecting the best suitable binders. Any such synthetic libraries may be generated using mutagenesis methods as disclosed herein.

Libraries as used according to the invention preferably comprise at least 10² library members, more preferred at least 10³, more preferred at least 10⁴, more preferred at least 10⁵, more preferred at least 10⁶ library members, more preferred at least 10⁷, more preferred at least 10⁸, more preferred at least 10⁹, more preferred at least 10¹⁰, more preferred at least 10¹¹, up to 10¹² members of a library, preferably derived from a parent molecule, which is a functional modular antibody as a scaffold containing at least one specific function or binding moiety, and derivatives thereof to engineer a new binding site apart from the original, functional binding region of said parent moiety.

A library as used according to the invention may be designed as a dedicated library based on specific modular antibody formats, preferably selected from the group consisting of a VH library, VHH library, Vkappa library, Vlambda library, Fab library, a CH1/CL library, an Fc library and a CH3 library. Libraries characterized by the content of composite molecules containing more than one antibody domains, such as an IgG library or Fc library are specially preferred. Other preferred libraries are those containing T-cell receptors, forming T-cell receptor libraries. Further preferred libraries are epitope libraries, wherein the fusion protein comprises a molecule with a variant of an epitope, also enabling the selection of competitive molecules having similar binding function, but different functionality.

Preferably the library is a yeast library and the yeast host cell exhibits at the surface of the cell the oligomers with the biological activity. The yeast host cell is preferably selected from the genera Saccharomyces, Pichia, Hansenula, Schizisaccharomyces, Kluyveromyces, Yarrowia and Candida. Most preferred, the host cell is *Saccharomyces cerevisiae.*

As is well-known in the art, there is a variety of display and selection technologies that may be used for the identification and isolation of proteins with certain binding characteristics and affinities, including, for example, display technologies such as cellular and non-cellular, in particular mobilized display systems. Among the cellular systems the phage display, virus display, yeast or other eukaryotic cell display, such as mammalian or insect cell display, may be used. Mobilized systems are relating to display systems in the soluble form, such as in vitro display systems, among them ribosome display, mRNA display or nucleic acid display.

Methods for production and screening of antibody variants are well-known in the art. General methods for antibody molecular biology, expression, purification, and screening are described in Antibody Engineering, edited by Duebel & Kontermann, Springer-Verlag, Heidelberg, 2001; and Hayhurst & Georgiou, 2001, Curr Opin Chem Biol 5:683-689; Maynard & Georgiou, 2000, Annu Rev Biomed Eng 2:339-76.

Specifically modular antibodies may be designed by producing respective variants and screening for specific properties. Typically variants are bound to receptor positive cells and the respective sTAG, coupled to a fluorophore is added. After incubation at 37°C for 1 hour, cell surface bound modular antibody/ sTAG immune complexes can be removed by a short treatment with acetic acid. The remaining fluorescence indicates internalisation of the sTAG. The fluorescent signal may then be compared to parallel cultures performed on ice at which temperature internalization does not occur.

The ADCC of a modular antibody according to the invention may be determined using typical assays employ target cells, like Ramos cells, incubated with serially diluted antibody prior to the addition of freshly isolated effector cells. The ADCC assay is then further incubated for several hours and % cytotoxicity detected. Usually the target: effector ratio is about 1:16, but may be 1:1 up to 1:50.

The CDC of a modular antibody according to the invention may be determined employing the mechanism of killing cells, in which antibody bound to the target cell surface fixes complement, which results in assembly of the membrane attack complex that punches holes in the target cell membrane resulting in subsequent cell lysis. The commonly used CDC assay follows the same procedure as for ADCC determination, however, with complement containing serum instead of effector cells.

A cytotoxic activity as determined by either of an ADCC or CDC assay may be shown for a modular antibody according to the invention, if there is a significant increase in the percentage of cytolysis as compared to a control. The cytotoxic activity related to ADCC or CDC is preferably measured as the absolute percentage increase, which is preferably higher than 5%, more preferably higher than 10%, even more preferred higher than 20%.

The antibody-dependent cellular phagocytosis, ADCP sometimes called ADPC, is usually investigated side by side with cytolysis of cultured human cells. Phagocytosis by phagocytes, usually human monocytes or monocyte-derived macrophages, as mediated by an antibody can be determined as follows. Purified monocytes may be cultured with cytokines to enhance expression of FcyRs or to induce differentiation into macrophages. ADCP and ADCC assays are then performed with target cells. Phagocytosis is determined as the percentage of positive cells measured by flow cytometry. The positive ADCP activity is proven with a significant uptake of the antibody-antigen complex by the phagocytes. The cytotoxic activity related to ADCP is preferably measured as the absolute percentage uptake of the antibody-antigen complex by the phagocytes, which is preferably higher than 5%, more preferably higher than 10%, even more preferred higher than 20%.

In a typical assay PBMC or monoycytes or monocyte derived macrophages are resuspended in RF2 medium (RPMI 1640 supplemented with 2% FCS) in 96-well plates at a concentration of 1 x 10⁵ viable cells in 100 ml/well. Appropriate target cells, expressing the target antigen, e.g. Her2/neu antigen and SKBR3 cells, are stained with PKH2 green fluorescence dye. Subsequently 1x10⁴ PKH2-labeled target cells and an Her2 specific (IgG1) antibody (or modular antibody) or mouse IgG1 isotype control (or modular antibody control) are added to the well of PBMC's in different concentrations (e.g. 1-100 µg/ml) and incubated in a final volume of 200 ml at 37°C for 24 h. Following the incubation, PBMCs or monoycytes or monocyte derived macrophages and target cells are harvested with EDTA-PBS and transferred to 96-well V-bottomed plates. The plates are centrifuged and the supernatant is aspirated. Cells are counterstained with a 100-ml mixture of RPE-conjugated anti-CD11b, anti-CD14, and human IgG, mixed and incubated for 60 min on ice. The cells are washed and fixed with 2% formaldehyde-PBS. Two-color flow cytometric analysis is performed with e.g. a FACS Calibur under optimal gating. PKH2-labeled target cells (green) are detected in the FL-1 channel (emission wavelength, 530 nm) and RPE-labeled PBMC or monoycytes or monocyte derived macrophages (red) are detected in the FL-2 channel (emission wavelength, 575 nm). Residual target cells are defined as cells that are PKH2⁺/RPE⁻ Dual-labeled cells (PKH2⁺/RPE⁻) are considered to represent phagocytosis of targets by PBMC or monoycytes or monocyte derived macrophages. Phagocytosis of target cells is calculated with the following equation: percent phagocytosis = 100 x [(percent dual positive)/ (percent dual positive + percent residual targets)]. All tests are usually performed in duplicate or triplicate and the results are expressed as mean 6 SD.

In a preferred embodiment, antibody variants are screened using one or more cell-based or *in vivo* assays. For such assays, purified or unpurified modified immunoglobulins are typically added exogenously such that cells are exposed to individual immunoglobulins or pools of immunoglobulins belonging to a library. These assays are typically, but not always, based on the function of the immunoglobulin; that is, the ability of the antibody to bind to its target and mediate some biochemical event, for example effector function, ligand/receptor binding inhibition, apoptosis, and the like. Such assays often involve monitoring the response of cells to the antibody, for example cell survival, cell death, change in cellular morphology, or transcriptional activation such as cellular expression of a natural gene or reporter gene. For example, such assays may measure the ability of antibody variants to elicit ADCC, ADCP, CDC, apoptotic activity or internalising properties. For some assays additional cells or components, that is in addition to the target cells, may need to be added, for example example serum complement, or effector cells such as peripheral blood monocytes (PBMCs), NK cells, macrophages, and the like. Such additional cells may be from any organism, preferably humans, mice, rat, rabbit, and monkey. Modular antibodies may cause apoptosis of certain cell lines expressing the target, or they may mediate attack on target cells by immune cells which have been added to the assay. Methods for monitoring cell death or viability are known in the art, and include the use of dyes, immunochemical, cytochemical, and radioactive reagents. For example, caspase staining assays may enable apoptosis to be measured, and uptake or release of radioactive substrates or fluorescent dyes such as alamar blue may enable cell growth or activation to be monitored.

In a preferred embodiment, the DELFIART EuTDA-based cytotoxicity assay (Perkin Elmer, MA) may be used. Alternatively, dead or damaged target cells may be monitored by measuring the release of one or more natural intracellular components, for example lactate dehydrogenase.

Transcriptional activation may also serve as a method for assaying function in cell-based assays. In this case, response may be monitored by assaying for natural genes or immunoglobulins which may be upregulated, for example the release of certain interleukins may be measured, or alternatively readout may be via a reporter construct. Cell-based assays may also involve the measure of morphological changes of cells as a response to the presence of modular antibodies. Cell types for such assays may be prokaryotic or eukaryotic, and a variety of cell lines that are known in the art may be employed. Alternatively, cell-based screens are performed using cells that have been transformed or transfected with nucleic acids encoding the variants. That is, antibody variants are not added exogenously to the cells. For example, in one embodiment, the cell-based screen utilizes cell surface display. A fusion partner can be employed that enables display of modified immunoglobulins on the surface of cells (Witrrup, 2001, Curr Opin Biotechnol, 12:395-399).

In a preferred embodiment, the immunogenicity of the modular antibodies may be determined experimentally using one or more cell-based assays. In a preferred embodiment, *ex vivo* T-cell activation assays are used to experimentally quantitate immunogenicity. In this method, antigen presenting cells and naive T cells from matched donors are challenged with a peptide or whole antibody of interest one or more times. Then, T cell activation can be detected using a number of methods, for example by monitoring production of cytokines or measuring uptake of tritiated thymidine. In the most preferred embodiment, interferon gamma production is monitored using Elispot assays.

The biological properties of the modular antibody according to the invention may be characterized *ex vivo* in cell, tissue, and whole organism experiments. As is known in the art, drugs are often tested *in vivo* in animals, including but not limited to mice, rats, rabbits, dogs, cats, pigs, and monkeys, in order to measure a drug's efficacy for treatment against a disease or disease model, or to measure a drug's pharmacokinetics, pharmacodynamics, toxicity, and other properties. The animals may be referred to as disease models. Therapeutics are often tested in mice, including but not limited to nude mice, SCID mice, xenograft mice, and transgenic mice (including knockins and knockouts). Such experimentation may provide meaningful data for determination of the potential of the antibody to be used as a therapeutic with the appropriate half-life, effector function, apoptotic activity, cytotoxic or cytolytic activity. Any organism, preferably mammals, may be used for testing. For example because of their genetic similarity to humans, primates, monkeys can be suitable therapeutic models, and thus may be used to test the efficacy, toxicity, pharmacokinetics, pharmacodynamics, half-life, or other property of the modular antibody according to the invention. Tests of the substances in humans are ultimately required for approval as drugs, and thus of course these experiments are contemplated. Thus the modular antibodies of the present invention may be tested in humans to determine their therapeutic efficacy, toxicity, immunogenicity, pharmacokinetics, and/or other clinical properties. Especially those modular antibodies according to the invention that bind to single cell or a cellular complex through at least two binding motifs, preferably binding of at least three structures cross-linking target cells, would be considered effective in effector activity or preapoptotic or apoptotic activity upon cell targeting and cross-linking. Multivalent binding provides a relatively large association of binding partners, also called cross-linking, which is a prerequisite for apoptosis and cell death.

The foregoing description will be more fully understood with reference to the following examples. Such examples are, however, merely representative of methods of practicing one or more embodiments of the present invention and should not be read as limiting the scope of invention.

### Examples

### Example 1: Construction of antigen binding Fc (Fcab) binding to EGFR

The identification of antigen specific Fcabs is described previously (WO2009132876A1). Briefly, large libraries expressing Fcabs on the surface of yeast cells are incubated with recombinant biotinylated EGFR comprised of the extracellular domain of the receptor. After addition of streptavidin coupled to a fluorescent dye, populations of antigen binding yeast cells are isolated based on their fluorescent phenotype using a high speed cell sorter. This selection procedure is repeated several times to enrich for a sufficiently pure antigen binding yeast cell population. Individual clones from enriched populations are screened for antigen binding and the best clones are expressed as soluble proteins in mammalian cells for further characterization. Functional binders are identified by their virtue to elicit antibody dependent cellular cytotoxicity (ADCC) against human EGFR positive tumor cell lines SKBR3 or MDA-MB468 (see below). Fcabs with ADCC functionality are chosen for affinity maturation to improve antigen binding. The amino acid sequences of these primary Fcab hits are re-randomized using different strategies varying in their extent of sequence re-randomization and the resulting Fcab libraries are screened as before for improved binders. Selection and screening strategies employed to enrich for high affinity binders include i) lowering antigen concentrations, ii) off-rate selections, or iii) competition with excess of unlabeled antigen or combinations of i)-iii). Individual affinity matured EGFR binding Fcabs are expressed in mammalian cells and characterized in more detail.

Table 1 describes the amino acid sequences of EGFR specific Fcabs. The sequences of the AB-loop, the CD-loop and the EF-loop in the CH3 domain are given and point mutations in the framework region compared to Fcab_wt (loop sequences of wild type human IgG1).

In this table some of the listed loop sequences are longer or shorter than the wild-type sequence, meaning that there are insertions or deletions relative to wildtype. The Fcab_wt is herein provided as a reference for comparison purposes, other Fcab_wt sequences (e.g. from different species, type or subtype) may be possibly used to engineer the specific EGFR Fcab binding structures formed by the specific C-terminal loop region in a similar way as readily understood by a skilled person.

**Table 1: Amino acid sequences of EGFR specific Fcabs. The sequences of the AB-loop, the CD-loop and the EF-loop in the CH3 domain are given and point mutations in the framework region compared to Fcab_wt (loop sequences of wild type human IgG1).**

| **clone** | **AB loop** | **CD loop** | **EF loop** | **framework mutation** |
|---|---|---|---|---|
| Fcab_wt (SEQ ID NO: 748) | LTKNQ SEQ ID NO:1 | NGQPE SEQ ID NO:2 | DKSRWQQGNV SEQ ID NO:3 | |
| EAMC2P31A1 | NDEGP SEQ ID NO:4 | NFGPE SEQ ID NO:5 | SHLRWTN SEQ ID NO:6 | T350A |
| EAMC2P31A2 | GDEGP SEQ ID NO:8 | NFGPE SEQ ID NO:5 | SHLRWTN SEQ ID NO:6 | |
| EAMC2P31B12 | GDDGP SEQ ID NO:9 | NFGPE SEQ ID NO:5 | SHLRWTN SEQ ID NO:6 | |
| EAMC2P31A10 | GAEGP SEQ ID NO:10 | NFGPE SEQ ID NO:5 | SHLRWTN SEQ ID NO:6 | |
| EAMC2P31A9 | GPNGP SEQ ID NO:11 | NFGPE SEQ ID NO:5 | SHLRWTN SEQ ID NO:6 | |
| EAMC2P31G8 | GPEGP SEQ ID NO:12 | NFGPE SEQ ID NO:5 | SHLRWTN SEQ ID NO:6 | V422A |
| EAMC2P31E12 | GEAGP SEQ ID NO:13 | NFGPE SEQ ID NO:5 | SHLRWTN SEQ ID NO:6 | |
| EAMC2P31E9 | GMLGP SEQ ID NO:14 | NFGPE SEQ ID NO:5 | SHLRWTN SEQ ID NO:6 | T437R |
| EAMC2P31G9 | GQSGP SEQ ID NO:15 | NFGPE SEQ ID NO:5 | SHLRWTN SEQ ID NO:6 | |
| EAMC2P31A11 | TQDGP SEQ ID NO:16 | NFGPE SEQ ID NO:5 | SHLRWTN SEQ ID NO:6 | |
| EAMC2P31C12 | TQLGP SEQ ID NO:17 | NFGPE SEQ ID NO:5 | SHLRWTN SEQ ID NO:6 | |
| EAMC2P31C5 | TDGP SEQ ID NO:18 | NFGPE SEQ ID NO:5 | SHLRWTN SEQ ID NO:6 | |
| EAMC2P31G6 | TDWDGP SEQ ID NO:19 | NFGPE SEQ ID NO:5 | SHLRWTN SEQ ID NO:6 | |
| EAMC2P31F3 | TGDGP SEQ ID NO:20 | NFGPE SEQ ID NO:5 | SHLRWTN SEQ ID NO:6 | |
| EAMC2P31A12 | TTEGP SEQ ID NO:21 | NFGPE SEQ ID NO:5 | SHLRWTN SEQ ID NO:6 | |
| EAMC2P31B1 | TTEGP SEQ ID NO:21 | NFGPE SEQ ID NO:5 | SHLRWTN SEQ ID NO:6 | N389S |
| EAMC2P31F1 | TVEGP SEQ ID NO:23 | NFGPE SEQ ID NO:5 | SHLRWTN SEQ ID NO:6 | |
| EAMC2P31G1 | TVDGGP SEQ ID NO:24 | NFGPE SEQ ID NO:5 | SHLRWTN SEQ ID NO:6 | |
| EAMC2P31A8 | TEEGP SEQ ID NO:25 | NFGPE SEQ ID NO:5 | SHLRWTN SEQ ID NO:6 | N384F |
| EAMC2P31D4 | TESGP SEQ ID NO:26 | NFGPE SEQ ID NO:5 | SHLRWTN SEQ ID NO:6 | |
| EAMC2P31G11 | EDLNH SEQ ID NO:27 | NFGPE SEQ ID NO:5 | SHLRWTN SEQ ID NO:6 | |
| EAMC2P31F11 | TNSIG SEQ ID NO:28 | NFGPE SEQ ID NO:5 | SHLRWTN SEQ ID NO:6 | |
| EAMC2P31C7 | TNEGP SEQ ID NO:29 | NFGPE SEQ ID NO:5 | SHLRWTN SEQ ID NO:6 | |
| EAMC2P31F10 | ANEGP SEQ ID NO:30 | NFGPE SEQ ID NO:5 | SHLRWTN SEQ ID NO:6 | T4181 |
| EAMC2P31A3 | ISTGP SEQ ID NO:31 | NFGPE SEQ ID NO:5 | SHLRWTN SEQ ID NO:6 | |
| EAMC2P31E11 | YGTGP SEQ ID NO:32 | NFGPE SEQ ID NO:5 | SHLRWTN SEQ ID NO:6 | |
| EAMC2P31B8 | IGEGP SEQ ID NO:33 | NFGPE SEQ ID NO:5 | SHLRWTN SEQ ID NO:6 | |
| EAMC2P31F9 | IKEGP SEQ ID NO:34 | NFGPE SEQ ID NO:5 | SHLRWTN SEQ ID NO:6 | |
| EAMC2P31G7 | ITEGP SEQ ID NO:35 | NFGPE SEQ ID NO:5 | SHLRWTN SEQ ID NO:6 | |
| EAMC2P31C2 | IEEGP SEQ ID NO:36 | NFGPE SEQ ID NO:5 | SHLRWTN SEQ ID NO:6 | N389S |
| EAMC2P31D10 | GGEGP SEQ ID NO:37 | NFGPE SEQ ID NO:5 | SHLRWTN SEQ ID NO:6 | |
| EAMC2P31D12 | FGEGP SEQ ID NO:38 | NFGPE SEQ ID NO:5 | SHLRWTN SEQ ID NO:6 | |
| EAMC2P31D3 | QGEGP SEQ ID NO:39 | NFGPE SEQ ID NO:5 | SHLRWTN SEQ ID NO:6 | |
| EAMC2P31E3 | EGEGP SEQ ID NO:40 | NFGPE SEQ ID NO:5 | SHLRWTN SEQ ID NO:6 | |
| EAMC2P31D7 | MGEGP SEQ ID NO:41 | NFGPE SEQ ID NO:5 | SHLRWTN SEQ ID NO:6 | |
| EAMC2P31H1 | VVEGP SEQ ID NO:42 | NFGPE SEQ ID NO:5 | SHLRWTN SEQ ID NO:6 | |
| EAMC2P31H12 | VGEGP SEQ ID NO:43 | NFGPE SEQ ID NO:5 | SHLRWTN SEQ ID NO:6 | |
| EAMC2P31H6 | VGEGP SEQ ID NO:44 | NFGPE SEQ ID NO:5 | SHLRWTN SEQ ID NO:6 | E382S, P445S |
| EAMC2P31A4 | SEQGP SEQ ID NO:45 | NFGPE SEQ ID NO:5 | SHLRWTN SEQ ID NO:6 | T418A |
| EAMC2P31H2 | SEEGP SEQ ID NO:46 | NFGPE SEQ ID NO:5 | SHLRWTN SEQ ID NO:6 | |
| EAMC2P31A5 | SENGP SEQ ID NO:47 | NFGPE SEQ ID NO:5 | SHLRWTN SEQ ID NO:6 | |
| EAMC2P31G4 | SINGP SEQ ID NO:48 | NFGPE SEQ ID NO:5 | SHLRWTN SEQ ID NO:6 | |
| EAMC2P31B6 | SQDGP SEQ ID NO:49 | NFGPE SEQ ID NO:5 | SHLRWTN SEQ ID NO:6 | |
| EAMC2P31E1 | SVDGP SEQ ID NO:50 | NFGPE SEQ ID NO:5 | SHLRWTN SEQ ID NO:6 | |
| EAMC2P31C3 | SVDGP SEQ ID NO:50 | NFGPE SEQ ID NO:5 | SHLRWTN SEQ ID NO:6 | A378T, H433R |
| EAMC2P31E5 | YEDGP SEQ ID NO:52 | NFGPE SEQ ID NO:5 | SHLRWTN SEQ ID NO:6 | H433R |
| EAMC2P31F8 | SEDGP SEQ ID NO:53 | NFGPE SEQ ID NO:5 | SHLRWTN SEQ ID NO:6 | |
| EAMC2P31E2 | SKDGP SEQ ID NO:54 | NFGPE SEQ ID NO:5 | SHLRWTN SEQ ID NO:6 | |
| EAMC2P31H8 | SKSGP SEQ ID NO:55 | NFGPE SEQ ID NO:5 | SHLRWTN SEQ ID NO:6 | |
| EAMC2P31B4 | SSEGP SEQ ID NO:56 | NFGPE SEQ ID NO:5 | SHLRWTN SEQ ID NO:6 | |
| EAMC2P31B7 | DEEGP SEQ ID NO:57 | NFGPE SEQ ID NO:5 | SHLRWTN SEQ ID NO:6 | |
| EAMC2P31C9 | IEDGP SEQ ID NO:58 | NFGPE SEQ ID NO:5 | SHLRWTN SEQ ID NO:6 | |
| EAMC2P31D1 | EEDAP SEQ ID NO:59 | NFGPE SEQ ID NO:5 | SHLRWTN SEQ ID NO:6 | |
| EAMC2P31E4 | KEDGP SEQ ID NO:60 | NFGPE SEQ ID NO:5 | SHLRWTN SEQ ID NO:6 | |
| EAMC2P31G3 | NEDGP SEQ ID NO:61 | NFGPE SEQ ID NO:5 | SHLRWTN SEQ ID NO:6 | |
| EAMC2P31E8 | DEDGP SEQ ID NO:62 | NFGPE SEQ ID NO:5 | SHLRWTN SEQ ID NO:6 | |
| EAMC2P31G10 | DSDGP SEQ ID NO:63 | NFGPE SEQ ID NO:5 | SHLRWTN SEQ ID NO:6 | |
| EAMC2P31A7 | MQDGP SEQ ID NO:64 | NFGPE SEQ ID NO:5 | SHLRWTN SEQ ID NO:6 | |
| EAMC2P31D8 | MPDGP SEQ ID NO:65 | NFGPE SEQ ID NO:5 | SHLRWTN SEQ ID NO:6 | |
| EAMC2P31E7 | MQEGP SEQ ID NO:66 | NFGPE SEQ ID NO:5 | SHLRWTN SEQ ID NO:6 | |
| EAMC2P31B11 | MSGGP SEQ ID NO:67 | NFGPE SEQ ID NO:5 | SHLRWTN SEQ ID NO:6 | N389S |
| EAMC2P31F7 | MSNGP SEQ ID NO:68 | NFGPE SEQ ID NO:5 | SHLRWTN SEQ ID NO:6 | |
| EAMC2P31B9 | MNQGP SEQ ID NO:69 | NFGPE SEQ ID NO:5 | SHLRWTN SEQ ID NO:6 | |
| EAMC2P31C4 | MNGGP SEQ ID NO:70 | NFGPE SEQ ID NO:5 | SHLRWTN SEQ ID NO:6 | |
| EAMC2P31F5 | MRHGT SEQ ID NO:71 | NFGPE SEQ ID NO:5 | SHLRWTN SEQ ID NO:6 | |
| EAMC2P31G12 | MASGP SEQ ID NO:72 | NFGPE SEQ ID NO:5 | SHLRWTN SEQ ID NO:6 | |
| EAMC2P31B10 | DANGP SEQ ID NO:73 | NFGPE SEQ ID NO:5 | SHLRWTN SEQ ID NO:6 | |
| EAMC2P31H4 | QANGP SEQ ID NO:74 | NFGPE SEQ ID NO:5 | SHLRWTN SEQ ID NO:6 | |
| EAMC2P31D2 | IANGP SEQ ID NO:75 | NFGPE SEQ ID NO:5 | SHLRWTN SEQ ID NO:6 | |
| EAMC2P31B5 | YDNGP SEQ ID NO:76 | NFGPE SEQ ID NO:5 | SHLRWTN SEQ ID NO:6 | N344D |
| EAMC2P31C8 | FDNGP SEQ ID NO:77 | NFGPE SEQ ID NO:5 | SHLRWTN SEQ ID NO:6 | |
| EAMC2P31F12 | MDNGP SEQ ID NO:78 | NFGPE SEQ ID NO:5 | SHLRWTN SEQ ID NO:6 | |
| EAMC2P31B2 | NTEGGP SEQ ID NO:79 | NFGPE SEQ ID NO:5 | SHLRWTN SEQ ID NO:6 | |
| EAMC2P31B3 | NGGP SEQ ID NO:80 | NFGPE SEQ ID NO:5 | SHLRWTN SEQ ID NO:6 | |
| EAMC2P31G2 | FEGGP SEQ ID NO:81 | NFGPE SEQ ID NO:5 | SHLRWTN SEQ ID NO:6 | |
| EAMC2P31H7 | FEGP SEQ ID NO:82 | NFGPE SEQ ID NO:5 | SHLRWTN SEQ ID NO:6 | |
| EAMC2P31C11 | IDGGP SEQ ID NO:83 | NFGPE SEQ ID NO:5 | SHLRWTN SEQ ID NO:6 | |
| EAMC2P31D6 | LVGGP SEQ ID NO:84 | NFGPE SEQ ID NO:5 | SHLRWTN SEQ ID NO:6 | |
| EAMC2P31F4 | LSGGP SEQ ID NO:85 | NFGPE SEQ ID NO:5 | SHLRWTN SEQ ID NO:6 | |
| EAMC2P31H11 | EHGP SEQ ID NO:86 | NFGPE SEQ ID NO:5 | SHLRWTN SEQ ID NO:6 | |
| EAM231b-A3 | TDNGP SEQ ID NO:87 | NFGPE SEQ ID NO:5 | SHLRWTN SEQ ID NO:6 | |
| EAM231b-H3 | TDGP SEQ ID NO:88 | NFGPE SEQ ID NO:5 | SHLRWTN SEQ ID NO:6 | |
| EAM231b-H2 | TDETP SEQ ID NO:89 | NFGPE SEQ ID NO:5 | SHLRWTN SEQ ID NO:6 | |
| EAM231b-A6 | MDNGP SEQ ID NO:90 | NFGPE SEQ ID NO:5 | SHLRWTN SEQ ID NO:6 | |
| EAM231b-C5 | QKDGP SEQ ID NO:91 | NFGPE SEQ ID NO:5 | SHLRWTN SEQ ID NO:6 | |
| EAM231b-D3 | QLKDEP SEQ ID NO:92 | NFGPE SEQ ID NO:5 | SHLRWTN SEQ ID NO:6 | |
| EAM231b-H6 | LNDGP SEQ ID NO:93 | NFGPE SEQ ID NO:5 | SHLRWTN SEQ ID NO:6 | |
| EAM231b-C4 | EVDGP SEQ ID NO:94 | NFGPE SEQ ID NO:5 | SHLRWTN SEQ ID NO:6 | |
| EAM231b-E2 | DEDGP SEQ ID NO:95 | NFGPE SEQ ID NO:5 | SHLRWTN SEQ ID NO:6 | |
| EAM231b-B6 | GQYGP SEQ ID NO:96 | NFGPE SEQ ID NO:5 | SHLRWTN SEQ ID NO:6 | |
| EAM231b-F2 | GAQGP SEQ ID NO:97 | NFGPE SEQ ID NO:5 | SHLRWTN SEQ ID NO:6 | |
| EAM231b-F5 | GPNGP SEQ ID NO:98 | NFGPE SEQ ID NO:5 | SHLRWTN SEQ ID NO:6 | |
| EAM231b-G5 | GPEGP SEQ ID NO:99 | NFGPE SEQ ID NO:5 | SHLRWTN SEQ ID NO:6 | V422A |
| EAM231b-G6 | VFAGQP SEQ ID NO:100 | NFGPE SEQ ID NO:5 | SHLRWTN SEQ ID NO:6 | |
| EAM231b-F4 | LAVGP SEQ ID NO:101 | NFGPE SEQ ID NO:5 | SHLRWTN SEQ ID NO:6 | |
| EAM231b-G2 | IAVGP SEQ ID NO:102 | NFGPE SEQ ID NO:5 | SHLRWTN SEQ ID NO:6 | |
| EAM231b-H5 | HSVGP SEQ ID NO:103 | NFGPE SEQ ID NO:5 | SHLRWTN SEQ ID NO:6 | |
| EAM231b-A1 | IEQGP SEQ ID NO:104 | NFGPE SEQ ID NO:5 | SHLRWTN SEQ ID NO:6 | |
| EAM231b-A2 | IEEGP SEQ ID NO:105 | NFGPE SEQ ID NO:5 | SHLRWTN SEQ ID NO:6 | |
| EAM231b-B2 | TESGP SEQ ID NO:106 | NFGPE SEQ ID NO:5 | SHLRWTN SEQ ID NO:6 | |
| EAM231b-C6 | TESGP SEQ ID NO:106 | NFGPE SEQ ID NO:5 | SHLRWTS SEQ ID NO:7 | |
| EGFR141-A3 | TESGP SEQ ID NO:106 | NFGPE SEQ ID NO:5 | SHLRWTN SEQ ID NO:6 | |
| EAM231b-E6 | HEGP SEQ ID NO:107 | NFGPE SEQ ID NO:5 | SHLRWTN SEQ ID NO:6 | |
| EAM231b-D6 | WEGGP SEQ ID NO:108 | NFGPE SEQ ID NO:5 | SHLRWTN SEQ ID NO:6 | |
| EAM231b-E3 | VEGGP SEQ ID NO:109 | NFGPE SEQ ID NO:5 | SHLRWTN SEQ ID NO:6 | |
| EAM231b-A4 | IGSGP SEQ ID NO:110 | NFGPE SEQ ID NO:5 | SHLRWTN SEQ ID NO:6 | |
| EAM231b-B1 | IGNGP SEQ ID NO:111 | NFGPE SEQ ID NO:5 | SHLRWTN SEQ ID NO:6 | |
| EAM231b-C2 | INHGP SEQ ID NO:112 | NFGPE SEQ ID NO:5 | SHLRWTN SEQ ID NO:6 | |
| EAM231b-D4 | NGGP SEQ ID NO:113 | NFGPE SEQ ID NO:5 | SHLRWTN SEQ ID NO:6 | |
| EAM231b-C3 | VNEGP SEQ ID NO:114 | NFGPE SEQ ID NO:5 | SHLRWTN SEQ ID NO:6 | F404Y |
| EAM231b-F3 | DNEGP SEQ ID NO:115 | NFGPE SEQ ID NO:5 | SHLRWTN SEQ ID NO:6 | |
| EAM231b-D2 | FGEGP SEQ ID NO:116 | NFGPE SEQ ID NO:5 | SHLRWTN SEQ ID NO:6 | |
| EAM231b-F1 | SGEGP SEQ ID NO:117 | NFGPE SEQ ID NO:5 | SHLRWTN SEQ ID NO:6 | |
| EAM231b-E1 | EGEGP SEQ ID NO:118 | NFGPE SEQ ID NO:5 | SHLRWTN SEQ ID NO:6 | |
| EAM231b-A5 | MQHGP SEQ ID NO:119 | NFGPE SEQ ID NO:5 | SHLRWTN SEQ ID NO:6 | |
| EAM231b-C1 | MQDGP SEQ ID NO:120 | NFGPE SEQ ID NO:5 | SHLRWTN SEQ ID NO:6 | |
| EAM231b-E4 | MQEGP SEQ ID NO:121 | NFGPE SEQ ID NO:5 | SHLRWTN SEQ ID NO:6 | |
| EAM231b-E5 | MKEGP SEQ ID NO:122 | NFGPE SEQ ID NO:5 | SHLRWTN SEQ ID NO:6 | R343L |
| EAM231b-G4 | MKEGP SEQ ID NO:123 | NFGPE SEQ ID NO:5 | SHLRWTN SEQ ID NO:6 | |
| EAM231b-B3 | MTEGP SEQ ID NO:124 | NFGPE SEQ ID NO:5 | SHLRWTN SEQ ID NO:6 | |
| EAM231b-D1 | MNQGP SEQ ID NO:125 | NFGPE SEQ ID NO:5 | SHLRWTN SEQ ID NO:6 | |
| EAM231b-D5 | MNDGP SEQ ID NO:126 | NFGPE SEQ ID NO:5 | SHLRWTN SEQ ID NO:6 | |
| EAM231b-G3 | MLGGP SEQ ID NO:127 | NFGPE SEQ ID NO:5 | SHLRWTN SEQ ID NO:6 | |
| EAM231b-H4 | LGGP SEQ ID NO:128 | NFGPE SEQ ID NO:5 | SHLRWTN SEQ ID NO:6 | E380G |
| EAM231b-F6 | NSDFG SEQ ID NO:129 | NFGPE SEQ ID NO:5 | SHLRWTN SEQ ID NO:6 | |
| EAM231b-G1 | SDTIG SEQ ID NO:130 | NFGPE SEQ ID NO:5 | SHLRWTN SEQ ID NO:6 | N389D |
| EAMC2P32-G11 | TESGP SEQ ID NO:135 | NFGPE SEQ ID NO:5 | ASYRWTS SEQ ID NO:136 | |
| EAMC2P32-F2 | TESGP SEQ ID NO:135 | NFGPE SEQ ID NO:5 | ATYRWMG SEQ ID NO:137 | |
| EAMC2P32-D3 | TESGP SEQ ID NO:135 | NFGPE SEQ ID NO:5 | FEYRWAL SEQ ID NO:138 | |
| EAMC2P32-H2 | TESGP SEQ ID NO:135 | NFGPE SEQ ID NO:5 | MPYRWPP SEQ ID NO:139 | |
| EAMC2P32-A11 | TESGP SEQ ID NO:135 | NFGPE SEQ ID NO:5 | SAARWER SEQ ID NO:140 | |
| EAMC2P32-F9 | TESGP SEQ ID NO:135 | NFGPE SEQ ID NO:5 | SAHRWKS SEQ ID NO:141 | |
| EAMC2P32-C4 | TESGP SEQ ID NO:135 | NFGPE SEQ ID NO:5 | SAHRWSS SEQ ID NO:142 | |
| EAMC2P32-C9 | TESGP SEQ ID NO:135 | NFGPE SEQ ID NO:5 | SAKRWKS SEQ ID NO:143 | |
| EAMC2P32-C7 | TESGP SEQ ID NO:135 | NFGPE SEQ ID NO:5 | SALRWEH SEQ ID NO:144 | |
| EAMC2P32-A10 | TESGP SEQ ID NO:135 | NFGPE SEQ ID NO:5 | SALRWMQ SEQ ID NO:145 | |
| EAMC2P32-H5 | TESGP SEQ ID NO:135 | NFGPE SEQ ID NO:5 | SAMRWEK SEQ ID NO:146 | |
| EAMC2P32-A3 | TESGP SEQ ID NO:135 | NFGPE SEQ ID NO:5 | SANRWES SEQ ID NO:147 | |
| EAMC2P32-E9 | TESGP SEQ ID NO:135 | NFGPE SEQ ID NO:5 | SAQRWKY SEQ ID NO:148 | |
| EAMC2P32-D10 | TESGP SEQ ID NO:135 | NFGPE SEQ ID NO:5 | SAQRWVT SEQ ID NO:149 | |
| EAMC2P32-D12 | TESGP SEQ ID NO:135 | NFGPE SEQ ID NO:5 | SARRWAS SEQ ID NO:150 | |
| EAMC2P32-H4 | TESGP SEQ ID NO:135 | NFGPE SEQ ID NO:5 | SARRWEE SEQ ID NO:151 | |
| EAMC2P32-E12 | TESGP SEQ ID NO:135 | NFGPE SEQ ID NO:5 | SASRWRF SEQ ID NO:152 | |
| EAMC2P32-F3 | TESGP SEQ ID NO:135 | NFGPE SEQ ID NO:5 | SAYRWEA SEQ ID NO:153 | |
| EAMC2P32-B10 | TESGP SEQ ID NO:135 | NFGPE SEQ ID NO:5 | SAYRWQQ SEQ ID NO:154 | |
| EAMC2P32-F12 | TESGP SEQ ID NO:135 | NFGPE SEQ ID NO:5 | SDYRWQS SEQ ID NO:155 | |
| EAMC2P32-D1 | TESGP SEQ ID NO:135 | NFGPE SEQ ID NO:5 | SEMRWHS SEQ ID NO:156 | |
| EAMC2P32-E8 | TESGP SEQ ID NO:135 | NFGPE SEQ ID NO:5 | SEMRWIK SEQ ID NO:157 | |
| EAMC2P32-C1 | TESGP SEQ ID NO:135 | NFGPE SEQ ID NO:5 | SEMRWRK SEQ ID NO:158 | |
| EAMC2P32-G9 | TESGP SEQ ID NO:135 | NFGPE SEQ ID NO:5 | SERRWSQ SEQ ID NO:159 | |
| EAMC2P32-C11 | TESGP SEQ ID NO:135 | NFGPE SEQ ID NO:5 | SEYRWRS SEQ ID NO:160 | |
| EAMC2P32-G3 | TESGP SEQ ID NO:135 | NFGPE SEQ ID NO:5 | SGARWKA SEQ ID NO:161 | |
| EAMC2P32-H3 | TESGP SEQ ID NO:135 | NFGPE SEQ ID NO:5 | SGMRWQK SEQ ID NO:162 | |
| EAMC2P32-F11 | TESGP SEQ ID NO:135 | NFGPE SEQ ID NO:5 | SHRRWFQ SEQ ID NO:163 | |
| EAMC2P32-D5 | TESGP SEQ ID NO:135 | NFGPE SEQ ID NO:5 | SHRRWHS SEQ ID NO:164 | |
| EAMC2P32-B5 | TESGP SEQ ID NO:135 | NFGPE SEQ ID NO:5 | SHRRWMR SEQ ID NO:165 | |
| EAMC2P32-A2 | TESGP SEQ ID NO:135 | NFGPE SEQ ID NO:5 | SHTRWHS SEQ ID NO:166 | V422A |
| EAMC2P32-F4 | TESGP SEQ ID NO:135 | NFGPE SEQ ID NO:5 | SKARWSQ SEQ ID NO:167 | N389Y |
| EAMC2P32-B4 | TESGP SEQ ID NO:135 | NFGPE SEQ ID NO:5 | SKMRWLQ SEQ ID NO:168 | |
| EAMC2P32-G5 | TESGP SEQ ID NO:135 | NFGPE SEQ ID NO:5 | SKYRWMA SEQ ID NO:169 | |
| EAMC2P32-E7 | TESGP SEQ ID NO:135 | NFGPE SEQ ID NO:5 | SMRRWTR SEQ ID NO:170 | |
| EAMC2P32-H1 | TESGP SEQ ID NO:135 | NFGPE SEQ ID NO:5 | SNFRWNN SEQ ID NO:171 | |
| EAMC2P32-A6 | TESGP SEQ ID NO:135 | NFGPE SEQ ID NO:5 | SNHRWWS SEQ ID NO:172 | |
| EAMC2P32-G4 | TESGP SEQ ID NO:135 | NFGPE SEQ ID NO:5 | SNIRWRQ SEQ ID NO:173 | N389D |
| EAMC2P32-E10 | TESGP SEQ ID NO:135 | NFGPE SEQ ID NO:5 | SNMRWST SEQ ID NO:174 | |
| EAMC2P32-D8 | TESGP SEQ ID NO:135 | NFGPE SEQ ID NO:5 | SNRRWHT SEQ ID NO:175 | |
| EAMC2P32-B8 | TESGP SEQ ID NO:135 | NFGPE SEQ ID NO:5 | SNRRWMA SEQ ID NO:176 | P387L, A431T |
| EAMC2P32-G8 | TESGP SEQ ID NO:135 | NFGPE SEQ ID NO:5 | SNRRWRH SEQ ID NO:177 | |
| EAMC2P32-G7 | TESGP SEQ ID NO:135 | NFGPE SEQ ID NO:5 | SNTRWFK SEQ ID NO:178 | |
| EAMC2P32-C12 | TESGP SEQ ID NO:135 | NFGPE SEQ ID NO:5 | SNVRWAR SEQ ID NO:179 | |
| EAMC2P32-D2 | TESGP SEQ ID NO:135 | NFGPE SEQ ID NO:5 | SNWRWKR SEQ ID NO:180 | |
| EAMC2P32-B2 | TESGP SEQ ID NO:135 | NFGPE SEQ ID NO:5 | SPRRWSN SEQ ID NO:181 | |
| EAMC2P32-C8 | TESGP SEQ ID NO:135 | NFGPE SEQ ID NO:5 | SPYRWES SEQ ID NO:182 | |
| EAMC2P32-A12 | TESGP SEQ ID NO:135 | NFGPE SEQ ID NO:5 | SQARWQR SEQ ID NO:183 | |
| EAMC2P32-B9 | TESGP SEQ ID NO:135 | NFGPE SEQ ID NO:5 | SQARWTS SEQ ID NO:184 | |
| EAMC2P32-H6 | TESGP SEQ ID NO:135 | NFGPE SEQ ID NO:5 | SQHRWTS SEQ ID NO:185 | |
| EAMC2P32-A7 | TESGP SEQ ID NO:135 | NFGPE SEQ ID NO:5 | SQLRWQS SEQ ID NO:186 | |
| EAMC2P32-G2 | TESGP SEQ ID NO:135 | NFGPE SEQ ID NO:5 | SQLRWSR SEQ ID NO:187 | |
| EAMC2P32-C3 | TESGP SEQ ID NO:135 | NFGPE SEQ ID NO:5 | SQMRWHQ SEQ ID NO:188 | |
| EAMC2P32-G1 | TESGP SEQ ID NO:135 | NFGPE SEQ ID NO:5 | SQMRWTR SEQ ID NO:189 | |
| EAMC2P32-A8 | TESGP SEQ ID NO:135 | NFGPE SEQ ID NO:5 | SQSRWSH SEQ ID NO:190 | |
| EAMC2P32-C5 | TESGP SEQ ID NO:135 | NFGPE SEQ ID NO:5 | SRFRWQE SEQ ID NO:191 | |
| EAMC2P32-B6 | TESGP SEQ ID NO:135 | NFGPE SEQ ID NO:5 | SRHRWST SEQ ID NO:192 | |
| EAMC2P32-F8 | TESGP SEQ ID NO:135 | NFGPE SEQ ID NO:5 | SRMRWES SEQ ID NO:193 | |
| EAMC2P32-D9 | TESGP SEQ ID NO:135 | NFGPE SEQ ID NO:5 | SRQRWQS SEQ ID NO:194 | |
| EAMC2P32-A5 | TESGP SEQ ID NO:135 | NFGPE SEQ ID NO:5 | SRRRWHS SEQ ID NO:195 | |
| EAMC2P32-E3 | TESGP SEQ ID NO:135 | NFGPE SEQ ID NO:5 | SSFRWES SEQ ID NO:196 | |
| EAMC2P32-G12 | TESGP SEQ ID NO:135 | NFGPE SEQ ID NO:5 | SSMRWAT SEQ ID NO:197 | |
| EAMC2P32-E2 | TESGP SEQ ID NO:135 | NFGPE SEQ ID NO:5 | SSMRWLQ SEQ ID NO:198 | |
| EAMC2P32-G6 | TESGP SEQ ID NO:135 | NFGPE SEQ ID NO:5 | SSNRWET SEQ ID NO:199 | |
| EAMC2P32-B3 | TESGP SEQ ID NO:135 | NFGPE SEQ ID NO:5 | SSQRWKR SEQ ID NO:200 | |
| EAMC2P32-C6 | TESGP SEQ ID NO:135 | NFGPE SEQ ID NO:5 | SSQRWLS SEQ ID NO:201 | |
| EAMC2P32-D11 | TESGP SEQ ID NO:135 | NFGPE SEQ ID NO:5 | SSQRWTR SEQ ID NO:202 | |
| EAMC2P32-E4 | TESGP SEQ ID NO:135 | NFGPE SEQ ID NO:5 | SSRRWSA SEQ ID NO:203 | |
| EAMC2P32-E6 | TESGP SEQ ID NO:135 | NFGPE SEQ ID NO:5 | SSYRWEK SEQ ID NO:204 | |
| EAMC2P32-A4 | TESGP SEQ ID NO:135 | NFGPE SEQ ID NO:5 | SSYRWMQ SEQ ID NO:205 | |
| EAMC2P32-E1 | TESGP SEQ ID NO:135 | NFGPE SEQ ID NO:5 | STHRWAT SEQ ID NO:206 | |
| EAMC2P32-F5 | TESGP SEQ ID NO:135 | NFGPE SEQ ID NO:5 | STRRWAN SEQ ID NO:207 | |
| EAMC2P32-A9 | TESGP SEQ ID NO:135 | NFGPE SEQ ID NO:5 | STRRWKT SEQ ID NO:208 | |
| EAMC2P32-D4 | TESGP SEQ ID NO:135 | NFGPE SEQ ID NO:5 | STRRWTS SEQ ID NO:209 | |
| EAMC2P32-A1 | TESGP SEQ ID NO:135 | NFGPE SEQ ID NO:5 | STYRWEA SEQ ID NO:210 | |
| EAMC2P32-D7 | TESGP SEQ ID NO:135 | NFGPE SEQ ID NO:5 | SVHRWLA SEQ ID NO:211 | |
| EAMC2P32-C2 | TESGP SEQ ID NO:135 | NFGPE SEQ ID NO:5 | SVQRWRS SEQ ID NO:212 | |
| EAMC2P32-B11 | TESGP SEQ ID NO:135 | NFGPE SEQ ID NO:5 | SWHRWNS SEQ ID NO:213 | K439R |
| EAMC2P32-B1 | TESGP SEQ ID NO:135 | NFGPE SEQ ID NO:5 | SWWRWKK SEQ ID NO:214 | |
| EAMC2P32-B12 | TESGP SEQ ID NO:135 | NFGPE SEQ ID NO:5 | SWYRWKS SEQ ID NO:215 | |
| EAMC2P32-G10 | TESGP SEQ ID NO:135 | NFGPE SEQ ID NO:5 | SYHRWMT SEQ ID NO:216 | |
| EAMC2P32-C10 | TESGP SEQ ID NO:135 | NFGPE SEQ ID NO:5 | SYYRWRA SEQ ID NO:217 | |
| EAM232b-B2 | TESGP SEQ ID NO:135 | NFGPE SEQ ID NO:5 | SQARWKK SEQ ID NO:218 | D356G |
| EAM232b-H2 | TESGP SEQ ID NO:135 | NFGPE SEQ ID NO:5 | SNARWFK SEQ ID NO:219 | |
| EAM232b-A3 | TESGP SEQ ID NO:135 | NFGPE SEQ ID NO:5 | STSRWKS SEQ ID NO:220 | |
| EAM232b-G1 | TESGP SEQ ID NO:135 | NFGPE SEQ ID NO:5 | SHSRWRS SEQ ID NO:221 | |
| EAM232b-D4 | TESGP SEQ ID NO:135 | NFGPE SEQ ID NO:5 | SNYRWQS SEQ ID NO:222 | S440G |
| EAM232b-E4 | TESGP SEQ ID NO:135 | NFGPE SEQ ID NO:5 | STYRWQS SEQ ID NO:223 | A378V, N389Y |
| EAM232b-D1 | TESGP SEQ ID NO:135 | NFGPE SEQ ID NO:5 | SNHRWHS SEQ ID NO:224 | |
| EAM232b-H4 | TESGP SEQ ID NO:135 | NFGPE SEQ ID NO:5 | SNRRWEH SEQ ID NO:225 | |
| EAM232b-G3 | TESGP SEQ ID NO:135 | NFGPE SEQ ID NO:5 | SNRRWES SEQ ID NO:226 | |
| EAM232b-A4 | TESGP SEQ ID NO:135 | NFGPE SEQ ID NO:5 | SNRRWAS SEQ ID NO:227 | |
| EAM232b-B3 | TESGP SEQ ID NO:135 | NFGPE SEQ ID NO:5 | SNRRWMS SEQ ID NO:228 | Q438R |
| EAM232b-B4 | TESGP SEQ ID NO:135 | NFGPE SEQ ID NO:5 | SNRRWMS SEQ ID NO:229 | |
| EAM232b-B1 | TESGP SEQ ID NO:135 | NFGPE SEQ ID NO:5 | SQRRWTS SEQ ID NO:230 | |
| EAM232b-F2 | TESGP SEQ ID NO:135 | NFGPE SEQ ID NO:5 | SQRRWES SEQ ID NO:231 | K409R |
| EAM232b-A2 | TESGP SEQ ID NO:135 | NFGPE SEQ ID NO:5 | SRRRWEE SEQ ID NO:232 | |
| EAM232b-D2 | TESGP SEQ ID NO:135 | NFGPE SEQ ID NO:5 | SERRWST SEQ ID NO:233 | |
| EAM232b-A1 | TESGP SEQ ID NO:135 | NFGPE SEQ ID NO:5 | SQQRWAS SEQ ID NO:234 | I377V |
| EAM232b-E3 | TESGP SEQ ID NO:135 | NFGPE SEQ ID NO:5 | SSQRWAK SEQ ID NO:235 | |
| EAM232b-G2 | TESGP SEQ ID NO:135 | NFGPE SEQ ID NO:5 | SQMRWEQ SEQ ID NO:236 | |
| EAM232b-H3 | TESGP SEQ ID NO:135 | NFGPE SEQ ID NO:5 | SQQRWHQ SEQ ID NO:237 | |
| EAM232b-C4 | TESGP SEQ ID NO:135 | NFGPE SEQ ID NO:5 | SQRRWAQ SEQ ID NO:238 | |
| EAM232b-C3 | TESGL SEQ ID NO:438 | NFGPE SEQ ID NO:5 | QTMRWEW SEQ ID NO:239 | |
| EAM232b-F1 | TESGP SEQ ID NO:135 | NFGPE SEQ ID NO:5 | SSMRWES SEQ ID NO:240 | |
| EAM232b-E1 | TESGP SEQ ID NO:135 | NFGPE SEQ ID NO:5 | STHRWSA SEQ ID NO:241 | |
| EAM232b-G4 | TESGP SEQ ID NO:135 | NFGPE SEQ ID NO:5 | SRNRWDA SEQ ID NO:242 | |
| EAM232b-C2 | TESGP SEQ ID NO:135 | NFGPE SEQ ID NO:5 | SHHRWVR SEQ ID NO:243 | |
| EAM232b-F4 | TESGP SEQ ID NO:135 | NFGPE SEQ ID NO:5 | SHMRWQT SEQ ID NO:244 | |
| EAM232b-E2 | TESGP SEQ ID NO:135 | NFGPE SEQ ID NO:5 | SRARWNS SEQ ID NO:245 | N421K |
| EAM232b-H1 | MESGP SEQ ID NO:22 | KFGPE SEQ ID NO:438 | SNLRWTK SEQ ID NO:246 | A378V, N421H |
| EAMC2P33H5 | TESGPVS SEQ ID NO:51 | NFGPE SEQ ID NO:5 | STYRWQSGNV SEQ ID NO:247 | |
| EAMC2P33G9 | SESGPVS SEQ ID NO:131 | NFGPE SEQ ID NO:5 | STYRWQSGNV SEQ ID NO:247 | |
| EAMC2P33E7 | TISGPVS SEQ ID NO:132 | NFGPE SEQ ID NO:5 | STYRWQSGNV SEQ ID NO:247 | |
| EAMC2P33A4 | TESGPVT SEQ ID NO:133 | NFGPE SEQ ID NO:5 | STYRWQSGNV SEQ ID NO:247 | |
| EAMC2P33C1 | TEFGPVT SEQ ID NO:134 | NFGPE SEQ ID NO:5 | STYRWQSGNV SEQ ID NO:247 | |
| EAMC2P33F3 | CESGPVT SEQ ID NO:251 | NFGPE SEQ ID NO:5 | STYRWQSGNV SEQ ID NO:247 | |
| EAMC2P33B8 | TESGPVT SEQ ID NO:252 | KFGPE SEQ ID NO:438 | STYRWQSGNV SEQ ID NO:247 | |
| EAMC2P33H11 | TAPGPVT SEQ ID NO:253 | KFGPE SEQ ID NO:438 | STYRWQSGNV SEQ ID NO:247 | Q438R |
| EAMC2P33D8 | TDSGPVT SEQ ID NO:254 | KFGPE SEQ ID NO:438 | STYRWQSGNV SEQ ID NO:247 | |
| EAMC2P33C8 | DDSGPVT SEQ ID NO:255 | NFGPE SEQ ID NO:5 | STYRWQSGNV SEQ ID NO:247 | |
| EAMC2P33F2 | TGSGPVT SEQ ID NO:256 | NFGPE SEQ ID NO:5 | STYRWQSGNV SEQ ID NO:247 | |
| EAMC2P33D11 | TYSGPVT SEQ ID NO:257 | NFGPE SEQ ID NO:5 | STYRWQSGNV SEQ ID NO:247 | |
| EAMC2P33D1 | TDSGPVW SEQ ID NO:258 | NFGPE SEQ ID NO:5 | STYRWQSGNV SEQ ID NO:247 | |
| EAMC2P33D7 | TESGPVW SEQ ID NO:259 | NFGPE SEQ ID NO:5 | STYRWQSGNV SEQ ID NO:247 | |
| EAMC2P33D12 | NESGPVW SEQ ID NO:260 | NFGPE SEQ ID NO:5 | STYRWQSGNV SEQ ID NO:247 | |
| EAMC2P33A6 | TDLGPVS SEQ ID NO:261 | NFGPE SEQ ID NO:5 | STYRWQSGNV SEQ ID NO:247 | |
| EAMC2P33A7 | TDSGPVS SEQ ID NO:265 | NFGPE SEQ ID NO:5 | STYRWQSGNV SEQ ID NO:247 | |
| EAMC2P33A9 | MDSGPVS SEQ ID NO:263 | NFGPE SEQ ID NO:5 | STYRWQSGNV SEQ ID NO:247 | |
| EAMC2P33H2 | FESGPVS SEQ ID NO:264 | NFGPE SEQ ID NO:5 | SVYRWMSGNV SEQ ID NO:248 | |
| EAMC2P33E11 | TDSGPVS SEQ ID NO:265 | NFGPE SEQ ID NO:5 | SVYRWMSGNV SEQ ID NO:248 | N389Y |
| EAMC2P33H8 | TDSGPVS SEQ ID NO:265 | KFGPE SEQ ID NO:438 | SNFRWNSGNV SEQ ID NO:249 | N389Y |
| EAMC2P33H12 | TDSGPVS SEQ ID NO:265 | KFGPE SEQ ID NO:438 | SNLRWTKGHV SEQ ID NO:250 | |
| EAMC2P33A8 | TDAGPVS SEQ ID NO:266 | KFGPE SEQ ID NO:438 | SNLRWTKGHV SEQ ID NO:250 | Q438R |
| EAMC2P33C2 | TDAGPVS SEQ ID NO:266 | KFGPE SEQ ID NO:438 | SNLRWTKGHV SEQ ID NO:250 | |
| EAMC2P33G8 | TDSGPVS SEQ ID NO:265 | KFGPE SEQ ID NO:438 | SNLRWTKGHV SEQ ID NO:250 | Q438R |
| EAMC2P33A10 | TASGPVS SEQ ID NO:267 | KFGPE SEQ ID NO:438 | SNLRWTKGHV SEQ ID NO:250 | |
| EAMC2P33F8 | TQSGPVS SEQ ID NO:268 | KFGPE SEQ ID NO:438 | SNLRWTKGHV SEQ ID NO:250 | |
| EAMC2P33A11 | TESGPVS SEQ ID NO:269 | KFGPE SEQ ID NO:438 | SNLRWTKGHV SEQ ID NO:250 | |
| EAMC2P33A12 | TESGPVS SEQ ID NO:269 | KLGPE SEQ ID NO:437 | SNLRWTKGHV SEQ ID NO:250 | |
| EAMC2P33C3 | MQSGPVS SEQ ID NO:270 | KFGPE SEQ ID NO:438 | SNLRWTKGHV SEQ ID NO:250 | |
| EAMC2P33H3 | MESGPVS SEQ ID NO:271 | KFGPE SEQ ID NO:438 | SNLRWTKGHV SEQ ID NO:250 | |
| EAMC2P33D2 | I ESGPVS SEQ ID NO:272 | KFGPE SEQ ID NO:438 | SNLRWTKGHV SEQ ID NO:250 | |
| EAMC2P33D5 | LESGPVS SEQ ID NO:273 | KFGPE SEQ ID NO:438 | SNLRWTKGHV SEQ ID NO:250 | |
| EAMC2P33G12 | SESGPVS SEQ ID NO:274 | KFGPE SEQ ID NO:438 | SNLRWTKGHV SEQ ID NO:250 | |
| EAMC2P33C4 | TDSGPVT SEQ ID NO:275 | KFGPE SEQ ID NO:438 | SNLRWTKGHV SEQ ID NO:250 | |
| EAMC2P33C5 | LDSGPVT SEQ ID NO:276 | KFGPE SEQ ID NO:438 | SNLRWTKGHV SEQ ID NO:250 | |
| EAMC2P33F7 | TESGPVT SEQ ID NO:277 | KFGPE SEQ ID NO:438 | SNLRWTKGHV SEQ ID NO:250 | T437A |
| EAMC2P33F9 | TESGPVT SEQ ID NO:277 | KFGPE SEQ ID NO:438 | SNLRWTKGHV SEQ ID NO:250 | |
| EAMC2P33F11 | TESGPLC SEQ ID NO:278 | KFGPE SEQ ID NO:438 | SNLRWTKGHV SEQ ID NO:250 | Q438R |
| EAMC2P33H4 | TDSGPVS SEQ ID NO:265 | KFGPE SEQ ID NO:438 | SNLRWTKGHV SEQ ID NO:250 | N389D |
| EAM241b-H2 | IRAGEP SEQ ID NO:280 | NFGPE SEQ ID NO:5 | SHLRWTN SEQ ID NO:6 | |
| EAM241b-A1 | IEAGDP SEQ ID NO:281 | NFGPE SEQ ID NO:5 | SHLRWTN SEQ ID NO:6 | |
| EAM241b-E1 | IGEGP SEQ ID NO:282 | NFGPE SEQ ID NO:5 | SHLRWTN SEQ ID NO:6 | |
| EAM241b-G2 | EGEGP SEQ ID NO:283 | NFGPE SEQ ID NO:5 | SHLRWTN SEQ ID NO:6 | |
| EAM241b-E2 | NGGP SEQ ID NO:284 | NFGPE SEQ ID NO:5 | SHLRWTN SEQ ID NO:6 | |
| EAM241b-A2 | TDGP SEQ ID NO:285 | NFGPE SEQ ID NO:5 | SHLRWTN SEQ ID NO:6 | |
| EAM241b-G1 | TVDGGP SEQ ID NO:286 | NFGPE SEQ ID NO:5 | SHLRWTN SEQ ID NO:6 | |
| EAM241b-B1 | DEDGP SEQ ID NO:287 | NFGPE SEQ ID NO:5 | SHLRWTN SEQ ID NO:6 | |
| EAM241b-D2 | DEDGP SEQ ID NO:287 | NFGPE SEQ ID NO:5 | SHLRWTN SEQ ID NO:6 | C367W |
| EAM241b-C2 | NEDGP SEQ ID NO:288 | NFGPE SEQ ID NO:5 | SHLRWTN SEQ ID NO:6 | |
| EAM241b-B2 | TENGP SEQ ID NO:289 | NFGPE SEQ ID NO:5 | SHLRWTN SEQ ID NO:6 | |
| EGFR141-A3 | TESGP SEQ ID NO:135 | NFGPE SEQ ID NO:5 | SHLRWTN SEQ ID NO:6 | |
| EAM241b-C1 | LNDGP SEQ ID NO:290 | NFGPE SEQ ID NO:5 | SHLRWTN SEQ ID NO:6 | |
| EAM241b-H1 | LKDGP SEQ ID NO:291 | NFGPE SEQ ID NO:5 | SHLRWTN SEQ ID NO:6 | |
| EAM241b-F2 | LLEGP SEQ ID NO:292 | NFGPE SEQ ID NO:5 | SHLRWTN SEQ ID NO:6 | |
| EAM241b-F1 | LDSGP SEQ ID NO:293 | NFGPE SEQ ID NO:5 | SHLRWTN SEQ ID NO:6 | S403T |
| EAM241b-D1 | YQNGP SEQ ID NO:294 | NFGPE SEQ ID NO:5 | SHLRWTS SEQ ID NO:7 | |
| EAMC2P41B11 | TESGP SEQ ID NO:295 | KFGPE SEQ ID NO:438 | STYRWQSGNV SEQ ID NO:247 | A378V |
| EAMC2P41G8 | THEGGP SEQ ID NO:296 | NFGPE SEQ ID NO:5 | SHLRWTNGNV SEQ ID NO:453 | N435S |
| EAMC2P41E8 | THDGP SEQ ID NO:297 | NFGPE SEQ ID NO:5 | SHLRWTNGNV SEQ ID NO:453 | |
| EAMC2P41A4 | VNGP SEQ ID NO:298 | NFGPE SEQ ID NO:5 | SHLRWTNGNV SEQ ID NO:453 | |
| EAMC2P41B3 | GVLGP SEQ ID NO:299 | NFGPE SEQ ID NO:5 | SHLRWTNGNV SEQ ID NO:453 | |
| EAMC2P41F6 | GEFGP SEQ ID NO:300 | NFGPE SEQ ID NO:5 | SHLRWTNGNV SEQ ID NO:453 | |
| EAMC2P41A3 | MESGP SEQ ID NO:301 | NFGPE SEQ ID NO:5 | SHLRWTNGNV SEQ ID NO:453 | |
| EAMC2P41C5 | MENGP SEQ ID NO:302 | NFGPE SEQ ID NO:5 | SHLRWTNGNV SEQ ID NO:453 | |
| EAMC2P41C3 | MGNGP SEQ ID NO:303 | NFGPE SEQ ID NO:5 | SHLRWTNGNV SEQ ID NO:453 | |
| EAMC2P41C12 | DGNGP SEQ ID NO:304 | NFGPE SEQ ID NO:5 | SHLRWTNGNV SEQ ID NO:453 | |
| EAMC2P41D2 | IKNGP SEQ ID NO:305 | NFGPE SEQ ID NO:5 | SHLRWTSGNV SEQ ID NO:453 | |
| EAMC2P41F11 | KNGP SEQ ID NO:306 | NFGPE SEQ ID NO:5 | SHLRWTNGNV SEQ ID NO:453 | |
| EAMC2P41B1 | MNDGP SEQ ID NO:307 | NFGPE SEQ ID NO:5 | SHLRWTNGNV SEQ ID NO:453 | Y436H |
| EAMC2P41F1 | MNDGP SEQ ID NO:307 | NFGPE SEQ ID NO:5 | SHLRWTNGNV SEQ ID NO:453 | |
| EAMC2P41A2 | MTDGP SEQ ID NO:308 | NFGPE SEQ ID NO:5 | SHLRWTNGNV SEQ ID NO:453 | |
| EAMC2P41B10 | LNAGSP SEQ ID NO:309 | NFGPE SEQ ID NO:5 | SHLRWTNGNV SEQ ID NO:453 | |
| EAMC2P41C2 | LNEGP SEQ ID NO:310 | NFGPE SEQ ID NO:5 | SHLRWTNGNV SEQ ID NO:453 | |
| EAMC2P41C8 | LNDGP SEQ ID NO:311 | NFGPE SEQ ID NO:5 | SHLRWTNGNV SEQ ID NO:453 | |
| EAMC2P41E5 | YNDGP SEQ ID NO:312 | NFGPE SEQ ID NO:5 | SHLRWTNGNV SEQ ID NO:453 | |
| EAMC2P41C6 | TSDGP SEQ ID NO:313 | NFGPE SEQ ID NO:5 | SHLRWTNGNV SEQ ID NO:453 | |
| EAMC2P41A6 | TSADGP SEQ ID NO:314 | NFGPE SEQ ID NO:5 | SHLRWTNGNV SEQ ID NO:453 | |
| EAMC2P41A12 | MSDGP SEQ ID NO:315 | NFGPE SEQ ID NO:5 | SHLRWTNGNV SEQ ID NO:453 | |
| EAMC2P41G6 | KSDGP SEQ ID NO:316 | NFGPE SEQ ID NO:5 | SHLRWTNGNV SEQ ID NO:453 | |
| EAMC2P41B8 | EQDGP SEQ ID NO:317 | NFGPE SEQ ID NO:5 | SHLRWTNGNV SEQ ID NO:453 | |
| EAMC2P41D5 | QDGP SEQ ID NO:318 | NFGPE SEQ ID NO:5 | SHLRWTNGNV SEQ ID NO:453 | |
| EAMC2P41D6 | TQDGP SEQ ID NO:319 | NFGPE SEQ ID NO:5 | SHLRWTNGNV SEQ ID NO:453 | |
| EAMC2P41A10 | SKDGP SEQ ID NO:320 | NFGPE SEQ ID NO:5 | SHLRWTNGNV SEQ ID NO:453 | |
| EAMC2P41D3 | SVDGP SEQ ID NO:321 | NFGPE SEQ ID NO:5 | SHLRWTNGNV SEQ ID NO:453 | A378T, H433R |
| EAMC2P41E2 | SGDGP SEQ ID NO:322 | NFGPE SEQ ID NO:5 | SHLRWTNGNV SEQ ID NO:453 | |
| EAMC2P41B2 | SEDGP SEQ ID NO:323 | NFGPE SEQ ID NO:5 | SHLRWTNGNV SEQ ID NO:453 | |
| EAMC2P41B9 | GDDGP SEQ ID NO:324 | NFGPE SEQ ID NO:5 | SHLRWTNGNV SEQ ID NO:453 | |
| EAMC2P41D1 | VDDGP SEQ ID NO:325 | NFGPE SEQ ID NO:5 | SHLRWTNGNV SEQ ID NO:453 | |
| EAMC2P41F9 | VRDGP SEQ ID NO:326 | NFGPE SEQ ID NO:5 | SHLRWTNGNV SEQ ID NO:453 | |
| EAMC2P41D4 | GPNGP SEQ ID NO:327 | NFGPE SEQ ID NO:5 | SHLRWTNGNV SEQ ID NO:453 | |
| EAMC2P41D8 | GPDGP SEQ ID NO:328 | NFGPE SEQ ID NO:5 | SHLRWTNGNV SEQ ID NO:453 | |
| EAMC2P41E10 | EPDGP SEQ ID NO:329 | NFGPE SEQ ID NO:5 | SHLRWTNGNV SEQ ID NO:453 | |
| EAMC2P41B7 | NEEGP SEQ ID NO:330 | NFGPE SEQ ID NO:5 | SHLRWTNGNV SEQ ID NO:453 | |
| EAMC2P41B12 | NENGP SEQ ID NO:331 | NFGPE SEQ ID NO:5 | SHLRWTNGNV SEQ ID NO:453 | |
| EAMC2P41C9 | NEDGP SEQ ID NO:332 | NFGPE SEQ ID NO:5 | SHLRWTNGNV SEQ ID NO:453 | |
| EAMC2P41C7 | DEDGP SEQ ID NO:333 | NFGPE SEQ ID NO:5 | SHLRWTNGNV SEQ ID NO:453 | |
| EAMC2P41G5 | HEDGP SEQ ID NO:334 | NFGPE SEQ ID NO:5 | SHLRWTNGNV SEQ ID NO:453 | |
| EAMC2P41G3 | KEDGP SEQ ID NO:335 | NFGPE SEQ ID NO:5 | SHLRWTNGNV SEQ ID NO:453 | |
| EAMC2P41A1 | NTEGGP SEQ ID NO:336 | NFGPE SEQ ID NO:5 | SHLRWTNGNV SEQ ID NO:453 | |
| EAMC2P41F5 | NGGP SEQ ID NO:337 | NFGPE SEQ ID NO:5 | SHLRWTNGNV SEQ ID NO:453 | |
| EAMC2P41C4 | YEGGP SEQ ID NO:338 | NFGPE SEQ ID NO:5 | SHLRWTNGNV SEQ ID NO:453 | |
| EAMC2P41B5 | IEAGDP SEQ ID NO:339 | NFGPE SEQ ID NO:5 | SHLRWTNGNV SEQ ID NO:453 | |
| EAMC2P41E3 | IRAGEP SEQ ID NO:340 | NFGPE SEQ ID NO:5 | SHLRWTNGNV SEQ ID NO:453 | |
| EAMC2P41G7 | IDGGP SEQ ID NO:341 | NFGPE SEQ ID NO:5 | SHLRWTNGNV SEQ ID NO:453 | |
| EAMC2P41E6 | TDNGP SEQ ID NO:342 | NFGPE SEQ ID NO:5 | SHLRWTNGNV SEQ ID NO:453 | |
| EAMC2P41F2 | TDFGP SEQ ID NO:343 | NFGPE SEQ ID NO:5 | SHLRWTNGNV SEQ ID NO:453 | |
| EAMC2P41D11 | SDHGP SEQ ID NO:344 | NFGPE SEQ ID NO:5 | SHLRWTNGNV SEQ ID NO:453 | |
| EAMC2P41A5 | MSEGP SEQ ID NO:345 | NFGPE SEQ ID NO:5 | SHLRWTNGNV SEQ ID NO:453 | |
| EAMC2P41E1 | ASEGP SEQ ID NO:346 | NFGPE SEQ ID NO:5 | SHLRWTNGNV SEQ ID NO:453 | |
| EAMC2P41A11 | MDEGP SEQ ID NO:347 | NFGPE SEQ ID NO:5 | SHLRWTNGNV SEQ ID NO:453 | |
| EAMC2P41A7 | INEGP SEQ ID NO:348 | NFGPE SEQ ID NO:5 | SHLRWTNGNV SEQ ID NO:453 | |
| EAMC2P41F10 | ANEGP SEQ ID NO:349 | NFGPE SEQ ID NO:5 | SHLRWTNGNV SEQ ID NO:453 | |
| EAMC2P41B4 | GAEGP SEQ ID NO:350 | NFGPE SEQ ID NO:5 | SHLRWTNGNV SEQ ID NO:453 | |
| EAMC2P41A9 | VGEGP SEQ ID NO:351 | NFGPE SEQ ID NO:5 | SHLRWTNGNV SEQ ID NO:453 | |
| EAMC2P41B6 | TGEGP SEQ ID NO:352 | NFGPE SEQ ID NO:5 | SHLRWTNGNV SEQ ID NO:453 | |
| EAMC2P41A8 | DEEGP SEQ ID NO:353 | NFGPE SEQ ID NO:5 | SHLRWTNGNV SEQ ID NO:453 | |
| EAMC2P41F7 | SEEGP SEQ ID NO:354 | NFGPE SEQ ID NO:5 | SHLRWTNGNV SEQ ID NO:453 | |
| EAMC2P41E4 | TEEGP SEQ ID NO:355 | NFGPE SEQ ID NO:5 | SHLRWTNGNV SEQ ID NO:453 | |
| EAMC2P41D7 | LEEGP SEQ ID NO:356 | NFGPE SEQ ID NO:5 | SHLRWTNGNV SEQ ID NO:453 | |
| EAM241c-D3 | SQDGP SEQ ID NO:357 | NFGPE SEQ ID NO:5 | SHLRWTN SEQ ID NO:6 | |
| EAM241c-E4 | TQDGP SEQ ID NO:358 | NFGPE SEQ ID NO:5 | SHLRWTN SEQ ID NO:6 | |
| EAM241c-A1 | NADGP SEQ ID NO:359 | NFGPE SEQ ID NO:5 | SHLRWTN SEQ ID NO:6 | |
| EAM241c-A4 | MYADGP SEQ ID NO:360 | NFGPE SEQ ID NO:5 | SHLRWTN SEQ ID NO:6 | |
| EAM241c-F1 | WEDGP SEQ ID NO:361 | NFGPE SEQ ID NO:5 | SHLRWTN SEQ ID NO:6 | |
| EAM241c-G1 | DEDGP SEQ ID NO:362 | NFGPE SEQ ID NO:5 | SHLRWTN SEQ ID NO:6 | |
| EAM241c-B2 | NEDGP SEQ ID NO:363 | NFGPE SEQ ID NO:5 | SHLRWTN SEQ ID NO:6 | |
| EAM241c-B4 | FSDGP SEQ ID NO:364 | NFGPE SEQ ID NO:5 | SHLRWTN SEQ ID NO:6 | |
| EAM241c-A2 | YDNGP SEQ ID NO:365 | NFGPE SEQ ID NO:5 | SHLRWTN SEQ ID NO:6 | N433D |
| EAM241c-F2 | MDNGP SEQ ID NO:366 | NFGPE SEQ ID NO:5 | SHLRWTN SEQ ID NO:6 | |
| EAM241c-C1 | SDNGP SEQ ID NO:367 | NFGPE SEQ ID NO:5 | SHLRWTN SEQ ID NO:6 | |
| EAM241c-E1 | SENGP SEQ ID NO:368 | NFGPE SEQ ID NO:5 | SHLRWTN SEQ ID NO:6 | |
| EAM241c-A3 | IGSGP SEQ ID NO:369 | NFGPE SEQ ID NO:5 | SHLRWTN SEQ ID NO:6 | |
| EAM241c-C4 | LGGP SEQ ID NO:370 | NFGPE SEQ ID NO:5 | SHLRWTN SEQ ID NO:6 | E380G |
| EAM241c-F4 | MGEGP SEQ ID NO:371 | NFGPE SEQ ID NO:5 | SHLRWTN SEQ ID NO:6 | |
| EAM241c-C2 | THEGP SEQ ID NO:372 | NFGPE SEQ ID NO:5 | SHLRWTN SEQ ID NO:6 | |
| EAM241c-H2 | NDEGP SEQ ID NO:373 | NFGPE SEQ ID NO:5 | SHLRWTN SEQ ID NO:6 | |
| EAM241c-D4 | VKMGAP SEQ ID NO:374 | NFGPE SEQ ID NO:5 | SHLRWTN SEQ ID NO:6 | |
| EAM241c-F3 | VTDGP SEQ ID NO:375 | NFGPE SEQ ID NO:5 | SHLRWTN SEQ ID NO:6 | |
| EAM241 c-H4 | VDDGP SEQ ID NO:376 | NFGPE SEQ ID NO:5 | SHLRWTS SEQ ID NO:7 | |
| EAM241c-B1 | TESGP SEQ ID NO:135 | KFGPE SEQ ID NO:438 | STYRWQS SEQ ID NO:223 | A378V, N389Y |
| EAMC2P42B7 | TESGP SEQ ID NO:135 | NFGPE SEQ ID NO:5 | SWKRWKT SEQ ID NO:454 | |
| EAMC2P42C8 | TESGP SEQ ID NO:135 | NFGPE SEQ ID NO:5 | SSKRWAT SEQ ID NO:455 | |
| EAMC2P42G6 | TESGP SEQ ID NO:135 | NFGPE SEQ ID NO:5 | SQKRWMT SEQ ID NO:456 | |
| EAMC2P42F3 | TESGP SEQ ID NO:135 | NFGPE SEQ ID NO:5 | SQQRWMT SEQ ID NO:457 | |
| EAMC2P42G1 | TESGP SEQ ID NO:135 | NFGPE SEQ ID NO:5 | SQQRWMK SEQ ID NO:458 | |
| EAMC2P42E11 | TESGP SEQ ID NO:135 | NFGPE SEQ ID NO:5 | SQRRWEA SEQ ID NO:459 | Q438R |
| EAMC2P42F6 | TESGP SEQ ID NO:135 | NFGPE SEQ ID NO:5 | SQRRWEN SEQ ID NO:460 | |
| EAMC2P42B8 | TESGP SEQ ID NO:135 | NFGPE SEQ ID NO:5 | SQRRWMA SEQ ID NO:461 | |
| EAMC2P42A5 | TESGP SEQ ID NO:135 | NFGPE SEQ ID NO:5 | SERRWMT SEQ ID NO:462 | N389S |
| EAMC2P42D5 | TESGP SEQ ID NO:135 | NFGPE SEQ ID NO:5 | SERRWMS SEQ ID NO:463 | |
| EAMC2P42D10 | TESGP SEQ ID NO:135 | NFGPE SEQ ID NO:5 | STRRWMV SEQ ID NO:464 | |
| EAMC2P42A9 | TESGP SEQ ID NO:135 | NFGPE SEQ ID NO:5 | SRRRWSM SEQ ID NO:465 | |
| EAMC2P42D9 | TESGP SEQ ID NO:135 | NFGPE SEQ ID NO:5 | SARRWSM SEQ ID NO:466 | P395S |
| EAMC2P42C12 | TESGP SEQ ID NO:135 | NFGPE SEQ ID NO:5 | SARRWSV SEQ ID NO:467 | |
| EAMC2P42A12 | TESGP SEQ ID NO:135 | NFGPE SEQ ID NO:5 | SRRRWDS SEQ ID NO:468 | |
| EAMC2P42B4 | TESGP SEQ ID NO:135 | NFGPE SEQ ID NO:5 | SARRWDS SEQ ID NO:469 | |
| EAMC2P42C6 | TESGP SEQ ID NO:135 | NFGPE SEQ ID NO:5 | SPRRWNS SEQ ID NO:470 | |
| EAMC2P42F1 | TESGP SEQ ID NO:135 | NFGPE SEQ ID NO:5 | SNRRWNS SEQ ID NO:471 | |
| EAMC2P42B11 | TESGP SEQ ID NO:135 | NFGPE SEQ ID NO:5 | SQRRWNS SEQ ID NO:472 | |
| EAMC2P42F8 | TESGP SEQ ID NO:135 | NFGPE SEQ ID NO:5 | STRRWAS SEQ ID NO:473 | |
| EAMC2P42G5 | TESGP SEQ ID NO:135 | NFGPE SEQ ID NO:5 | SNRRWAS SEQ ID NO:474 | |
| EAMC2P42C7 | TESGP SEQ ID NO:135 | NFGPE SEQ ID NO:5 | SNRRWIN SEQ ID NO:475 | |
| EAMC2P42C9 | TESGP SEQ ID NO:135 | NFGPE SEQ ID NO:5 | SN RRW IA SEQ ID NO:476 | |
| EAMC2P42F7 | TESGP SEQ ID NO:135 | NFGPE SEQ ID NO:5 | SYRRWIS SEQ ID NO:477 | |
| EAMC2P42A1 | TESGP SEQ ID NO:135 | NFGPE SEQ ID NO:5 | SEMRWDA SEQ ID NO:478 | |
| EAMC2P42B9 | TESGP SEQ ID NO:135 | NFGPE SEQ ID NO:5 | SEARWKG SEQ ID NO:479 | |
| EAMC2P42A3 | TESGP SEQ ID NO:135 | NFGPE SEQ ID NO:5 | SPARWGA SEQ ID NO:480 | |
| EAMC2P42C1 | TESGP SEQ ID NO:135 | NFGPE SEQ ID NO:5 | SRARWGA SEQ ID NO:481 | |
| EAMC2P42C10 | TESGP SEQ ID NO:135 | NFGPE SEQ ID NO:5 | SSARWMA SEQ ID NO:482 | |
| EAMC2P42D7 | TESGP SEQ ID NO:135 | NFGPE SEQ ID NO:5 | SVNRWTA SEQ ID NO:483 | |
| EAMC2P42D2 | TESGP SEQ ID NO:135 | NFGPE SEQ ID NO:5 | SSRRWLH SEQ ID NO:484 | |
| EAMC2P42D6 | TESGP SEQ ID NO:135 | NFGPE SEQ ID NO:5 | SSRRWLG SEQ ID NO:485 | D356G |
| EAMC2P42F9 | TESGP SEQ ID NO:135 | NFGPE SEQ ID NO:5 | SSRRWDR SEQ ID NO:486 | |
| EAMC2P42D4 | TESGP SEQ ID NO:135 | NFGPE SEQ ID NO:5 | SSHRWAA SEQ ID NO:487 | |
| EAMC2P42F11 | TESGP SEQ ID NO:135 | NFGPE SEQ ID NO:5 | SSRRWAL SEQ ID NO:488 | |
| EAMC2P42E4 | TESGP SEQ ID NO:135 | NFGPE SEQ ID NO:5 | SSMRWMN SEQ ID NO:489 | |
| EAMC2P42G4 | TESGP SEQ ID NO:135 | NFGPE SEQ ID NO:5 | SSYRWHN SEQ ID NO:490 | |
| EAMC2P42A2 | TESGP SEQ ID NO:135 | NFGPE SEQ ID NO:5 | SHHRWFS SEQ ID NO:491 | |
| EAMC2P42D3 | TESGP SEQ ID NO:135 | NFGPE SEQ ID NO:5 | SSHRWNS SEQ ID NO:492 | |
| EAMC2P42C2 | TESGP SEQ ID NO:135 | NFGPE SEQ ID NO:5 | SSWRWTS SEQ ID NO:493 | |
| EAMC2P42F4 | TESGP SEQ ID NO:135 | NFGPE SEQ ID NO:5 | SSQRWVS SEQ ID NO:494 | |
| EAMC2P42A10 | TESGP SEQ ID NO:135 | NFGPE SEQ ID NO:5 | STWRWQS SEQ ID NO:495 | |
| EAMC2P42C11 | TESGP SEQ ID NO:135 | NFGPE SEQ ID NO:5 | STKRWLS SEQ ID NO:496 | |
| EAMC2P42B12 | TESGP SEQ ID NO:135 | NFGPE SEQ ID NO:5 | SRMRWQS SEQ ID NO:497 | |
| EAMC2P42B1 | TESGP SEQ ID NO:135 | NFGPE SEQ ID NO:5 | SQYRWLS SEQ ID NO:498 | |
| EAMC2P42D8 | TESGP SEQ ID NO:135 | NFGPE SEQ ID NO:5 | SNYRWLS SEQ ID NO:499 | |
| EAMC2P42E5 | TESGP SEQ ID NO:135 | NFGPE SEQ ID NO:5 | SHYRWSS SEQ ID NO:500 | |
| EAMC2P42E9 | TESGP SEQ ID NO:135 | NFGPE SEQ ID NO:5 | AHYRWRS SEQ ID NO:501 | |
| EAMC2P42D11 | TESGP SEQ ID NO:135 | NFGPE SEQ ID NO:5 | TLYRWNS SEQ ID NO:502 | |
| EAMC2P42D1 | TESGP SEQ ID NO:135 | NFGPE SEQ ID NO:5 | SWARWQS SEQ ID NO:503 | V422A |
| EAMC2P42E10 | TESGP SEQ ID NO:135 | NFGPE SEQ ID NO:5 | SQYRWMS SEQ ID NO:504 | V422A |
| EAMC2P42A4 | TESGP SEQ ID NO:135 | NFGPE SEQ ID NO:5 | SALRWQR SEQ ID NO:505 | |
| EAMC2P42E2 | TESGP SEQ ID NO:135 | NFGPE SEQ ID NO:5 | SQYRWQR SEQ ID NO:506 | |
| EAMC2P42B5 | TESGP SEQ ID NO:135 | NFGPE SEQ ID NO:5 | SSARWQR SEQ ID NO:507 | |
| EAMC2P42B2 | TESGP SEQ ID NO:135 | NFGPE SEQ ID NO:5 | SQARWRN SEQ ID NO:508 | |
| EAMC2P42E8 | TESGP SEQ ID NO:135 | NFGPE SEQ ID NO:5 | SQYRWRT SEQ ID NO:509 | |
| EAMC2P42E1 | TESGP SEQ ID NO:135 | NFGPE SEQ ID NO:5 | SNARWRS SEQ ID NO:510 | |
| EAMC2P42B6 | TESGP SEQ ID NO:135 | NFGPE SEQ ID NO:5 | SYWRWRR SEQ ID NO:511 | |
| EAMC2P42C5 | TESGP SEQ ID NO:135 | NFGPE SEQ ID NO:5 | SRLRWRH SEQ ID NO:512 | |
| EAMC2P42A6 | TESGP SEQ ID NO:135 | NFGPE SEQ ID NO:5 | SAKRWEH SEQ ID NO:513 | |
| EAMC2P42D12 | TESGP SEQ ID NO:135 | NFGPE SEQ ID NO:5 | SAKRWEY SEQ ID NO:514 | |
| EAMC2P42G2 | TESGP SEQ ID NO:135 | NFGPE SEQ ID NO:5 | AAHRWEA SEQ ID NO:515 | |
| EAMC2P42A8 | TESGP SEQ ID NO:135 | NFGPE SEQ ID NO:5 | SAYRWES SEQ ID NO:516 | |
| EAMC2P42A7 | TESGP SEQ ID NO:135 | NFGPE SEQ ID NO:5 | SHMRWKK SEQ ID NO:517 | |
| EAMC2P42B3 | TESGP SEQ ID NO:135 | NFGPE SEQ ID NO:5 | SHHRWKK SEQ ID NO:518 | |
| EAMC2P42E12 | TESGP SEQ ID NO:135 | NFGPE SEQ ID NO:5 | SYYRWKK SEQ ID NO:519 | |
| EAMC2P42G3 | TESGP SEQ ID NO:135 | NFGPE SEQ ID NO:5 | SKMRWEK SEQ ID NO:520 | |
| EAMC2P42C3 | TESGP SEQ ID NO:135 | NFGPE SEQ ID NO:5 | SAWRWNK SEQ ID NO:521 | |
| EAMC2P42C4 | TESGP SEQ ID NO:135 | NFGPE SEQ ID NO:5 | SHWRWHK SEQ ID NO:522 | |
| EAMC2P42F12 | TESGP SEQ ID NO:135 | NFGPE SEQ ID NO:5 | SAQRWLK SEQ ID NO:523 | |
| EAMC2P42A11 | TESGP SEQ ID NO:135 | NFGPE SEQ ID NO:5 | SNLRWGK SEQ ID NO:524 | |
| EAMC2P42F5 | TESGP SEQ ID NO:135 | NFGPE SEQ ID NO:5 | SQMRWKT SEQ ID NO:525 | |
| EAMC2P42F10 | TESGP SEQ ID NO:135 | NFGPE SEQ ID NO:5 | SQLRWKS SEQ ID NO:526 | |
| EAMC2P42E3 | TESGP SEQ ID NO:135 | NFGPE SEQ ID NO:5 | SAFRWKH SEQ ID NO:527 | |
| EAMC2P42E6 | TESGP SEQ ID NO:135 | NFGPE SEQ ID NO:5 | SWFRWKS SEQ ID NO:528 | |
| EAMC2P42B10 | TESGP SEQ ID NO:135 | NFGPE SEQ ID NO:5 | SSWRWKS SEQ ID NO:529 | |
| EAMC2P42E7 | TESGP SEQ ID NO:135 | NFGPE SEQ ID NO:5 | SNYRWKH SEQ ID NO:530 | K370R |
| EAM242b-B2 | TESGP SEQ ID NO:135 | NFGPE SEQ ID NO:5 | SNRRWGA SEQ ID NO:531 | |
| EAM242b-H4 | TESGP SEQ ID NO:135 | NFGPE SEQ ID NO:5 | SQRRWGV SEQ ID NO:532 | |
| EAM242b-C3 | TESGP SEQ ID NO:135 | NFGPE SEQ ID NO:5 | STRRWMQ SEQ ID NO:533 | |
| EAM242b-H3 | TESGP SEQ ID NO:135 | NFGPE SEQ ID NO:5 | STRRWNT SEQ ID NO:534 | |
| EAM242b-C1 | TESGP SEQ ID NO:135 | NFGPE SEQ ID NO:5 | SWARWHS SEQ ID NO:535 | |
| EAM242b-D1 | TESGP SEQ ID NO:135 | NFGPE SEQ ID NO:5 | SWARWQN SEQ ID NO:536 | |
| EAM242b-F2 | TESGP SEQ ID NO:135 | NLGPE SEQ ID NO:440 | SSARWFH SEQ ID NO:537 | F385L |
| EAM242b-F4 | TESGP SEQ ID NO:135 | NFGPE SEQ ID NO:5 | SQARWFH SEQ ID NO:538 | |
| EAM242b-E1 | TESGP SEQ ID NO:135 | NFGPE SEQ ID NO:5 | STFRWQR SEQ ID NO:539 | |
| EAM242b-E2 | TESGP SEQ ID NO:135 | NFGPE SEQ ID NO:5 | SRMRWQR SEQ ID NO:540 | |
| EAM242b-D3 | TESGP SEQ ID NO:135 | NFGPE SEQ ID NO:5 | SWLRWKR SEQ ID NO:541 | |
| EAM242b-H2 | TESGP SEQ ID NO:135 | NFGPE SEQ ID NO:5 | SRYRWLN SEQ ID NO:542 | |
| EAM242b-A3 | TESGP SEQ ID NO:135 | NFGPE SEQ ID NO:5 | SSMRWMN SEQ ID NO:543 | |
| EAM242b-D2 | TESGP SEQ ID NO:135 | NFGPE SEQ ID NO:5 | SSMRWGL SEQ ID NO:544 | N434D |
| EAM242b-B4 | TESGP SEQ ID NO:135 | NFGPE SEQ ID NO:5 | SSMRWES SEQ ID NO:545 | |
| EAM242b-G3 | TESGP SEQ ID NO:135 | NFGPE SEQ ID NO:5 | SSWRWMS SEQ ID NO:546 | |
| EAM242b-H1 | TESGP SEQ ID NO:135 | NFGPE SEQ ID NO:5 | SHWRWMA SEQ ID NO:547 | |
| EAM242b-F1 | TESGP SEQ ID NO:135 | NFGPE SEQ ID NO:5 | SSFRWTA SEQ ID NO:548 | |
| EAM242b-C2 | TESGP SEQ ID NO:135 | NFGPE SEQ ID NO:5 | SSNRWKQ SEQ ID NO:549 | |
| EAM242b-D4 | TESGP SEQ ID NO:135 | NFGPE SEQ ID NO:5 | SSSRWSD SEQ ID NO:550 | |
| EAM242b-A2 | TESGP SEQ ID NO:135 | NFGPE SEQ ID NO:5 | SAQRWST SEQ ID NO:551 | |
| EAM242b-G1 | TESGP SEQ ID NO:135 | NFGPE SEQ ID NO:5 | SAQRWAT SEQ ID NO:552 | |
| EAM242b-E3 | TESGP SEQ ID NO:135 | NFGPE SEQ ID NO:5 | SAGRWEK SEQ ID NO:553 | |
| EAM242b-F3 | TESGP SEQ ID NO:135 | NFGPE SEQ ID NO:5 | SAHRWAK SEQ ID NO:554 | |
| EAM242b-B3 | TESGP SEQ ID NO:135 | NFGPE SEQ ID NO:5 | SNRRWAS SEQ ID NO:555 | |
| EAM242b-E4 | TESGP SEQ ID NO:135 | NFGPE SEQ ID NO:5 | SNKRWKS SEQ ID NO:557 | |
| EAM242b-G2 | TESGP SEQ ID NO:135 | NFGPE SEQ ID NO:5 | SARRWYS SEQ ID NO:558 | |
| EAM242b-A4 | TESGP SEQ ID NO:135 | NFGPE SEQ ID NO:5 | SFQRWQS SEQ ID NO:559 | |
| EAM242b-A1 | TESGP SEQ ID NO:135 | NFGPE SEQ ID NO:5 | ASRRWET SEQ ID NO:560 | S383G |
| EAM242b-B1 | TESGP SEQ ID NO:135 | NFGPE SEQ ID NO:5 | SKYRWET SEQ ID NO:561 | |
| EAM243-H4 | TESGPVS SEQ ID NO:383 | KFGPE SEQ ID NO:438 | STYRWQSGDV SEQ ID NO:565 | A378V |
| EAM243-A4 | TDSGPVT SEQ ID NO:379 | KFGPE SEQ ID NO:438 | STYRWQSGNV SEQ ID NO:247 | A378V, N389Y |
| EAM243-B1 | KDDGPVT SEQ ID NO:377 | KFGPE SEQ ID NO:438 | STYRWQSGNV SEQ ID NO:247 | A378V, N389Y |
| EAM243-H2 | TDSGPVS SEQ ID NO:265 | KFGPE SEQ ID NO:438 | STYRWQSGNV SEQ ID NO:247 | A378V, N389Y |
| EAM243-G1 | TESGPVT SEQ ID NO:277 | KFGPE SEQ ID NO:438 | STYRWQSGNV SEQ ID NO:247 | A378V, N389Y |
| EAM243-E4 | TESGPVS SEQ ID NO:383 | KFGPE SEQ ID NO:438 | STYRWQSGNV SEQ ID NO:247 | A378V, N389Y |
| EAM243-F3 | TEAGPVS SEQ ID NO:381 | KFGPE SEQ ID NO:438 | STYRWQSGNV SEQ ID NO:247 | A378V, N389Y |
| EAM243-C3 | SEDGPVT SEQ ID NO:387 | NFGPE SEQ ID NO:5 | STYRWQSGNV SEQ ID NO:247 | A378V, N389Y |
| EAM243-F4 | TEDGPVT SEQ ID NO:446 | NFGPE SEQ ID NO:5 | STYRWQSGNV SEQ ID NO:247 | A378V, N389Y |
| EAM243-C2 | TENGPVS SEQ ID NO:396 | NFGPE SEQ ID NO:5 | STYRWQSGNV SEQ ID NO:247 | A378V, N389Y |
| EAM243-E3 | TESGPVT SEQ ID NO:277 | KFGPE SEQ ID NO:438 | SNLRWTKGHV SEQ ID NO:250 | A378V, N389Y |
| EAM243-A3 | TDSGPVT SEQ ID NO:379 | KFGPE SEQ ID NO:438 | SNLRWTKGHV SEQ ID NO:250 | A378V, N389Y, K409R |
| EAM243-H3 | TDDGPVT SEQ ID NO:556 | KFGPE SEQ ID NO:438 | SNLRWTKGHV SEQ ID NO:250 | A378V |
| EAMC2P51B2 | TESGP SEQ ID NO:135 | KFGPE SEQ ID NO:438 | STYRWQS SEQ ID NO:223 | N389Y, A378V |
| EAMC2P51G2 | TESGP SEQ ID NO:135 | KFGPE SEQ ID NO:438 | STYRWQS SEQ ID NO:223 | N389Y, A378V, T350A |
| EAMC2P51A2 | TESGP SEQ ID NO:135 | KFGPE SEQ ID NO:438 | STYRWQS SEQ ID NO:223 | N389Y, A378V, R344Q |
| EAM252-A3 | TESGP SEQ ID NO:135 | NFGPE SEQ ID NO:5 | STYRWQSGNV SEQ ID NO:247 | S364T, A378V, N389Y, H433R |
| EAM252-C4 | TESGP SEQ ID NO:135 | NFGPE SEQ ID NO:5 | STYRWQSGNV SEQ ID NO:247 | S364T, A378V, N389Y, |
| EAM252-B1 | MESGP SEQ ID NO:301 | KFGPE SEQ ID NO:438 | SNLRWTKGHV SEQ ID NO:250 | |
| EAM252-D4 (EAM151-5) | TESGP SEQ ID NO:135 | KFGPE SEQ ID NO:438 | SNLRWTKGHV SEQ ID NO:250 | A378V |
| EAM252-G1 | TESGP SEQ ID NO:135 | NFGPE SEQ ID NO:5 | SNRRWNSGNV SEQ ID NO:566 | |
| EAM252b-A1 | TESGP SEQ ID NO:135 | KFGPE SEQ ID NO:438 | STYRWQS SEQ ID NO:223 | A378V, N389Y |
| EAM253-H6 | KDDGPVT SEQ ID NO:377 | KFGPE SEQ ID NO:438 | STYRWQSGNV SEQ ID NO:247 | A378V, N389Y |
| EAM253-B4 | TDEGPVT SEQ ID NO:378 | KFGPE SEQ ID NO:438 | STYRWQSGNV SEQ ID NO:247 | A378V, N389Y |
| EAM253-C5 | TDSGPVT SEQ ID NO:379 | KFGPE SEQ ID NO:438 | STYRWQSGNV SEQ ID NO:247 | A378V, N389Y |
| EAM253-G5 | TDSGPVT SEQ ID NO:379 | KFGPE SEQ ID NO:438 | STYRWQSGNV SEQ ID NO:247 | A378V, N389Y, Q438R |
| EAM253-H3 | TDSGPVS SEQ ID NO:265 | KFGPE SEQ ID NO:438 | STYRWQSGDV SEQ ID NO:565 | A378V, N389Y, |
| EAM253-A3 | TESGPVT SEQ ID NO:277 | KFGPE SEQ ID NO:438 | STYRWQSGNV SEQ ID NO:247 | A378V, N389Y |
| EAM253-B5 | IESGPVT SEQ ID NO:382 | KFGPE SEQ ID NO:438 | STYRWQSGNV SEQ ID NO:247 | A378V, N389Y |
| EAM253-B2 | TESGPVS SEQ ID NO:383 | KFGPE SEQ ID NO:438 | STYRWQSGNV SEQ ID NO:247 | A378V, N389Y, G446S |
| EAM253-E2 | TESGPVS SEQ ID NO:383 | KFGPE SEQ ID NO:438 | STYRWQSGNV SEQ ID NO:247 | A378V, N389Y |
| EAM253-A2 | TESGPVS SEQ ID NO:383 | KFGPE SEQ ID NO:438 | STYRWQSGDV SEQ ID NO:565 | A378V |
| EAM131D6 | WFLTADDS SEQ ID NO:384 | NGQPE SEQ ID NO:2 | QDDRWESGNV SEQ ID NO:562 | |
| EAM131G6 | WFLTADDS SEQ ID NO:384 | NGRPE SEQ ID NO:439 | QDDRWESGNV SEQ ID NO:562 | |
| EAM121E6 | TESGPVS SEQ ID NO:383 | NFGPE SEQ ID NO:5 | SWMRWHRGNV SEQ ID NO:563 | V378A |
| EAM 131E5 | RDSGPVS SEQ ID NO:385 | NFGPE SEQ ID NO:5 | SWMRWHRGNV SEQ ID NO:564 | |
| EAM121F5 | TDSGPVS SEQ ID NO:265 | NFGPE SEQ ID NO:5 | STMRWFSGNV SEQ ID NO:567 | |
| EAM131A1 | TDSGPVT SEQ ID NO:386 | NFGPE SEQ ID NO:5 | STLRWQSGNV SEQ ID NO:568 | V378A, H433R |
| EAM121C3 | TDSGPVS SEQ ID NO:265 | NFGPE SEQ ID NO:5 | SVYRWQSGNV SEQ ID NO:569 | |
| EAM131C3 | TDSGPVS SEQ ID NO:265 | NFGPE SEQ ID NO:5 | SVYRWESGNV SEQ ID NO:570 | |
| EAM131H6 | TDSGPVS SEQ ID NO:265 | NFGPE SEQ ID NO:5 | SHYRWKSGNV SEQ ID NO:571 | |
| EAM131F6 | TESGPVS SEQ ID NO:383 | NFGPE SEQ ID NO:5 | SWARWFSGNV SEQ ID NO:572 | |
| EAM131F2 | TESGPVT SEQ ID NO:277 | NFGPE SEQ ID NO:5 | SVLRWRTGNV SEQ ID NO:573 | |
| EAM121D1 | TESGPVS SEQ ID NO:383 | NFGPE SEQ ID NO:5 | SVLRWRTGNV SEQ ID NO:573 | |
| EAM121D3 | TESGPVS SEQ ID NO:383 | NFGPE SEQ ID NO:5 | SVLRWQTGNV SEQ ID NO:574 | V378A |
| EAM121D5 | TESGPVS SEQ ID NO:383 | NFGPE SEQ ID NO:5 | SVLRWQSGNV SEQ ID NO:575 | V378A |
| EAM121A4 | I ESGPVS SEQ ID NO:388 | NFGPE SEQ ID NO:5 | SALRWESGNV SEQ ID NO:576 | V378A |
| EAM131A4 | TDSGPVS SEQ ID NO:265 | NFGPE SEQ ID NO:5 | SALRWESGNV SEQ ID NO:576 | V378A H433R |
| EAM131B1 | TDSGPVS SEQ ID NO:265 | NFGPE SEQ ID NO:5 | SARRWETGNV SEQ ID NO: 577 | V378A |
| EAM131C2 | MESGPVS SEQ ID NO:389 | NFGPE SEQ ID NO:5 | SARRWETGNV SEQ ID NO:577 | V378A |
| EAM131D2 | TESGPVS SEQ ID NO:383 | NFGPE SEQ ID NO:5 | SSRRWQTGNV SEQ ID NO:578 | V378A |
| EAM131H3 | TESGPVS SEQ ID NO:383 | NFGPE SEQ ID NO:5 | SALRWMTGNV SEQ ID NO:579 | V378A |
| EAM131F1 | TESGPVS SEQ ID NO:383 | NFGPE SEQ ID NO:5 | STYRWMSGNV SEQ ID NO:580 | R355D, V378A |
| EAM131F3 | TESGPVS SEQ ID NO:383 | NFGPE SEQ ID NO:5 | STMRWMSGNV SEQ ID NO:581 | V378A |
| EAM131B5 | TESGPVS SEQ ID NO:383 | NFGPE SEQ ID NO:5 | STYRWQSGNV SEQ ID NO:247 | V378A |
| EAM131C5 | TESGPVS SEQ ID NO:383 | NFGPE SEQ ID NO:5 | SAYRWRSGNV SEQ ID NO:582 | V378A |
| EAM131D5 | TESGPVS SEQ ID NO:383 | NFGPE SEQ ID NO:5 | SAYRWQSGNV SEQ ID NO:583 | V378A |
| EAM131A3 | TESGPVS SEQ ID NO:383 | NFGPE SEQ ID NO:5 | SYWRWHSGNV SEQ ID NO:584 | V378A |
| EAM131E2 | TESGPVS SEQ ID NO:383 | NFGPE SEQ ID NO:5 | SYYRWHSGNV SEQ ID NO:585 | V378A |
| EAM131G4 | TDSGPVS SEQ ID NO:265 | NLGPE SEQ ID NO:440 | SYYRWQAGNV SEQ ID NO:586 | V378A |
| EAM131E6 | TESGPVT SEQ ID NO:277 | NFGPE SEQ ID NO:5 | SWYRWHSGNV SEQ ID NO:587 | V378A |
| EAM131H1 | TNSGPVS SEQ ID NO:390 | NFGPE SEQ ID NO:5 | SWYRWHSGNV SEQ ID NO:587 | V378A |
| EAM131G3 | TESGPVS SEQ ID NO:383 | NFGPE SEQ ID NO:5 | SWYRWHAGNV SEQ ID NO:588 | V378A |
| EAM131H5 | TESGPVG SEQ ID NO:391 | NFGPE SEQ ID NO:5 | SNYRWHSGNV SEQ ID NO:589 | V378A |
| EAM131A5 | TESGPVS SEQ ID NO:383 | NFGPE SEQ ID NO:5 | SSRRWMSGDV SEQ ID NO:590 | V378A |
| EAM131B6 | TESGPVS SEQ ID NO:383 | NFGPE SEQ ID NO:5 | SNRRWMSGNV SEQ ID NO:591 | V378A |
| EAM131G5 | TESGPVS SEQ ID NO:383 | NFGPE SEQ ID NO:5 | SSLRWMSGNV SEQ ID NO:592 | V378A |
| EAM131E1 | TESGPVS SEQ ID NO:383 | NFGPE SEQ ID NO:5 | SHWRWMKGNV SEQ ID NO:593 | V378A |
| EAM131D4 | TESGPVS SEQ ID NO:383 | NFGPE SEQ ID NO:5 | SALRWAQGSV SEQ ID NO:594 | V378A |
| EAM121E2 | TESGPVS SEQ ID NO:383 | NFGPE SEQ ID NO:5 | STLRWISGYP SEQ ID NO:595 | |
| EAM131C1 | TESGPVS SEQ ID NO:383 | NFGPE SEQ ID NO:5 | SSLRWTSGNV SEQ ID NO:596 | |
| EAM131E4 | TESGPVS SEQ ID NO:383 | NFGPE SEQ ID NO:5 | STLRWTSGNV SEQ ID NO:597 | |
| EAM121A2 | SDSGPIW SEQ ID NO:392 | NFGPE SEQ ID NO:5 | SHLRWTNGNV SEQ ID NO:598 | |
| EAM121C1 | TDSGPVG SEQ ID NO:393 | NFGPE SEQ ID NO:5 | SHLRWTNGNV SEQ ID NO:598 | |
| EAM121H5 | TDSGPIW SEQ ID NO:394 | NFGPE SEQ ID NO:5 | SHLRWTNGNV SEQ ID NO:598 | |
| EAM121H3 | MDPGPVS SEQ ID NO:395 | NFGPE SEQ ID NO:5 | SHLRWTNGNV SEQ ID NO:598 | |
| EAM121B3 | TESGPVS SEQ ID NO:383 | NFGPE SEQ ID NO:5 | SDLRWKNGNV SEQ ID NO:599 | |
| EAM121A5 | TESGPVS SEQ ID NO:383 | NFGPE SEQ ID NO:5 | SQLRWTNDNA SEQ ID NO:600 | |
| EAM121H4 | TESGPVS SEQ ID NO:383 | NFGPE SEQ ID NO:5 | SYLRWTHGNA SEQ ID NO:601 | |
| EAM121G4 | TESGPVS SEQ ID NO:383 | NFGPE SEQ ID NO:5 | SNLRWRTGKD SEQ ID NO:602 | |
| EAM121D4 | TESGPVS SEQ ID NO:383 | NFGPE SEQ ID NO:5 | SQLRWATDKV SEQ ID NO:603 | |
| EAM121G5 | TESGPVS SEQ ID NO:383 | NFGPE SEQ ID NO:5 | SQLRWAHGRV SEQ ID NO:604 | |
| EAM121D6 | TESGPVS SEQ ID NO:383 | NFGPE SEQ ID NO:5 | SQLRWAKGKA SEQ ID NO:605 | |
| EAM121A6 | TESGPVS SEQ ID NO:383 | NFGPE SEQ ID NO:5 | SQLRWTSGKV SEQ ID NO:606 | |
| EAM121C6 | TESGPVS SEQ ID NO:383 | NFGPE SEQ ID NO:5 | SQRRWTSGKV SEQ ID NO:607 | |
| EAM121H1 | TESGPVS SEQ ID NO:383 | NFGPE SEQ ID NO:5 | SSLRWTSGKV SEQ ID NO:608 | |
| EAM121E1 | TESGPVS SEQ ID NO:383 | NFGPE SEQ ID NO:5 | SQLRWTHGKV SEQ ID NO:609 | |
| EAM131B4 | TESGPVS SEQ ID NO:383 | NLGPE SEQ ID NO:440 | SHLRWTSGKV SEQ ID NO:610 | |
| EAM131C4 | TESGPVS SEQ ID NO:383 | NLGPE SEQ ID NO:440 | SQMRWTKGKV SEQ ID NO:611 | |
| EAM121B1 | TESGPVS SEQ ID NO:383 | NFGPE SEQ ID NO:5 | SQLRWTKGKV SEQ ID NO:612 | |
| EAM121F3 | TESGPVS SEQ ID NO:383 | SFGPE SEQ ID NO:441 | SQLRWTKGKV SEQ ID NO:612 | |
| EAM121B6 | TESGPVS SEQ ID NO:383 | NFGPE SEQ ID NO:5 | SQLRWTKGKM SEQ ID NO:613 | |
| EAM121A3 | TESGPVS SEQ ID NO:383 | NFGPE SEQ ID NO:5 | SNLRWSKGKV SEQ ID NO:614 | |
| EAM121B5 | TESGPVS SEQ ID NO:383 | NFGPE SEQ ID NO:5 | SNLRWTKGDV SEQ ID NO:615 | |
| EAM131H2 | TESGPVS SEQ ID NO:383 | NFGPE SEQ ID NO:5 | SNMRWTKGDV SEQ ID NO:616 | |
| EAM121C4 | TESGPVS SEQ ID NO:383 | NFGPE SEQ ID NO:5 | SSLRWTKRDV SEQ ID NO:617 | |
| EAM121F4 | TESGPVS SEQ ID NO:383 | NFGPE SEQ ID NO:5 | SQLRWTSRDV SEQ ID NO:618 | |
| EAM131B3 | TESGPVS SEQ ID NO:383 | NFGPE SEQ ID NO:5 | SHFRWTSGRV SEQ ID NO:619 | |
| EAM121E5 | TESGPVS SEQ ID NO:383 | NFGPE SEQ ID NO:5 | SNLRWTKGRV SEQ ID NO:620 | |
| EAM131H4 | TESGPVS SEQ ID NO:383 | NFGPE SEQ ID NO:5 | SSLRWTKGRV SEQ ID NO:621 | |
| EAM121E4 | TESGPVS SEQ ID NO:383 | NFGPE SEQ ID NO:5 | SNLRWTSRKV SEQ ID NO:622 | |
| EAM131F2 | TESGPVS SEQ ID NO:383 | NFGPE SEQ ID NO:5 | SNLRWTKRKV SEQ ID NO:623 | |
| EAM121G6 | TESGPVS SEQ ID NO:383 | NFGPE SEQ ID NO:5 | SNLRWTRGKV SEQ ID NO:624 | |
| EAM121H2 | TESGPVS SEQ ID NO:383 | NFGPE SEQ ID NO:5 | SNLRWKSGKV SEQ ID NO:625 | |
| EAM121C2 | TESGPVS SEQ ID NO:383 | DFGPE SEQ ID NO:442 | SNLRWTKGKV SEQ ID NO:626 | |
| EAM121A1 | TESGPVS SEQ ID NO:383 | NFGPE SEQ ID NO:5 | SNLRWTKGKV SEQ ID NO:626 | |
| EAM121 B4 | TESGPVS SEQ ID NO:383 | NFGPE SEQ ID NO:5 | SNLRWTKGKV SEQ ID NO:626 | V378A |
| EAM141p4_p5A7 | TESGPVS SEQ ID NO:383 | NFGPE SEQ ID NO:5 | SWYRWHSGNV SEQ ID NO:587 | A378V |
| EAM141p4_p5G8 | TENGPVS SEQ ID NO:396 | NFGPE SEQ ID NO:5 | SRYRWQTGNV SEQ ID NO:627 | A378V |
| EAM141p4_p5A8 | TESGPVS SEQ ID NO:383 | NFGPE SEQ ID NO:5 | SHWRWMKGNV SEQ ID NO:593 | A378V |
| EAM141p4_p5G9 | AESGPVT SEQ ID NO:397 | NFGPE SEQ ID NO:5 | SQRRWAAGNV SEQ ID NO:628 | S364T, A378V |
| EAM141p4_p5C11 | TESGPVS SEQ ID NO:383 | NLGPE SEQ ID NO:440 | SSLRWTKGGV SEQ ID NO:629 | |
| EAM141p4_p5D12 | TESGPVS SEQ ID NO:383 | NLGPE SEQ ID NO:440 | SQLRWTSGKV SEQ ID NO:606 | Q439R |
| EAM141p4_p5G5 | TESGPVS SEQ ID NO:383 | NFGPE SEQ ID NO:5 | SALRWTSGNV SEQ ID NO:630 | |
| EAM141p4_p5A11 | WFLTADDS SEQ ID NO:398 | NGRPE SEQ ID NO:439 | QDDRWESGNV SEQ ID NO:562 | |
| EAM141p4_p5B1 | WFLTADDS SEQ ID NO:399 | NGQPE SEQ ID NO:2 | QDDRWESGNV SEQ ID NO:562 | A378V |
| EAM133E11 | WFLTADDS SEQ ID NO:399 | NGQPE SEQ ID NO:2 | QDDRWESGNV SEQ ID NO:562 | |
| EAM133B7 | TDDGPVS SEQ ID NO:400 | NFGPE SEQ ID NO:5 | SLYRWMSGNV SEQ ID NO:631 | |
| EAM133D3 | SDAGPVS SEQ ID NO:401 | NFGPE SEQ ID NO:5 | SWYRWHAGNV SEQ ID NO:588 | |
| EAM133B4 | THSGPVT SEQ ID NO:402 | DFGPE SEQ ID NO:442 | SWWRWYRGNV SEQ ID NO:632 | S364T |
| EAM133E9 | TDSGPVN SEQ ID NO:403 | NFGPE SEQ ID NO:5 | KQERWSLGNV SEQ ID NO:633 | S364N |
| EAM133G3 | TDSGPVS SEQ ID NO:265 | SFGPVSE SEQ ID NO:443 | SALRWMTGNV SEQ ID NO:579 | |
| EAM133B8 | TESGPVS SEQ ID NO:383 | NFGPE SEQ ID NO:5 | SALRWLSGNV SEQ ID NO:634 | D356Y |
| EAM133E6 | TDSGPVS SEQ ID NO:265 | NFGPE SEQ ID NO:5 | SNRRWMSGNV SEQ ID NO:591 | |
| EAM133G6 | TESGPVS SEQ ID NO:383 | NFGPE SEQ ID NO:5 | SYWRWHSGNV SEQ ID NO:584 | |
| EAM133A1 | TESGPVS SEQ ID NO:383 | NFGPE SEQ ID NO:5 | SALRWEQGNV SEQ ID NO:635 | |
| EAM133G9 | TESGPVS SEQ ID NO:383 | NFGPE SEQ ID NO:5 | SALRWESGGNV SEQ ID NO:576 | |
| EAM133A10 | TESGPVS SEQ ID NO:383 | NFGPE SEQ ID NO:5 | SYYRWLSGNV SEQ ID NO:636 | |
| EAM133D4 | TESGPVS SEQ ID NO:383 | NFGPE SEQ ID NO:5 | SVYRWQSGNV SEQ ID NO:569 | |
| EAM133A7 | TESGPVS SEQ ID NO:383 | NLGPE SEQ ID NO:440 | SSYRWAAGGNV SEQ ID NO:637 | |
| EAM133E12 | TESGPVS SEQ ID NO:383 | NFGPE SEQ ID NO:5 | SYYRWHKGNV SEQ ID NO:638 | P353T |
| EAM133A5 | TESGPVS SEQ ID NO:383 | NFGPE SEQ ID NO:5 | SSRRWESGNV SEQ ID NO:639 | |
| EAM133D9 | TESGPVS SEQ ID NO:383 | NFGPE SEQ ID NO:5 | SQRRWMSGNV SEQ ID NO:640 | |
| EAM133E3 | TESGPVS SEQ ID NO:383 | NFGPE SEQ ID NO:5 | SQRRWELGDV SEQ ID NO:641 | |
| EAM133F10 | TESGPVS SEQ ID NO:383 | NFGPE SEQ ID NO:5 | SQRRWSKGSV SEQ ID NO:642 | |
| EAM133A4 | TESGPVS SEQ ID NO:383 | NFGPE SEQ ID NO:5 | SQLRWKNGKA SEQ ID NO:643 | |
| EAM133D11 | TESGPVS SEQ ID NO:383 | NFGPE SEQ ID NO:5 | SQLRWSKGNA SEQ ID NO:644 | |
| EAM133B1 | TESGPVS SEQ ID NO:383 | NFGPE SEQ ID NO:5 | SQLRWATRKV SEQ ID NO:645 | |
| EAM133F2 | TESGPVS SEQ ID NO:383 | NFGPE SEQ ID NO:5 | SQLRWMSRKV SEQ ID NO:646 | |
| EAM133C2 | TESGPVS SEQ ID NO:383 | NFGPE SEQ ID NO:5 | SALRWSNRKV SEQ ID NO:647 | |
| EAM133A3 | TESGPVS SEQ ID NO:383 | NFGPE SEQ ID NO:5 | SSLRWTKGYV SEQ ID NO:648 | P353T |
| EAM133C3 | TESGPVS SEQ ID NO:383 | NFGPE SEQ ID NO:5 | SHLRWPKGNV SEQ ID NO:649 | P353T |
| EAM133A6 | TESGPVS SEQ ID NO:383 | NFGPE SEQ ID NO:5 | SALRWTKGNV SEQ ID NO:650 | |
| EAM133B10 | TESGPVS SEQ ID NO:383 | NFGPE SEQ ID NO:5 | SALRWTNGKV SEQ ID NO:651 | |
| EAM133B6 | TESGPVS SEQ ID NO:383 | NFGPE SEQ ID NO:5 | SKLRWTNGIV SEQ ID NO:652 | L351 Q |
| EAM133D12 | TESGPVW SEQ ID NO:262 | NFGPE SEQ ID NO:5 | SHLRWTNGNV SEQ ID NO:598 | S364W |
| EAM133B11 | TESGPVS SEQ ID NO:383 | NFGPE SEQ ID NO:5 | SSLRWTSGRI SEQ ID NO:653 | |
| EAM133C5 | TESGPVS SEQ ID NO:383 | NFGPE SEQ ID NO:5 | SNLRWTHGNG SEQ ID NO:654 | |
| EAM 133A8 | TESGPVS SEQ ID NO:383 | NFGPE SEQ ID NO:5 | SQLRWTSGKV SEQ ID NO:606 | |
| EAM133E8 | TESGPVS SEQ ID NO:383 | NFGPE SEQ ID NO:5 | SQLRWRAGKV SEQ ID NO:655 | |
| EAM133C6 | TESGPVS SEQ ID NO:383 | NFGPE SEQ ID NO:5 | SQLRWTKGSV SEQ ID NO:656 | |
| EAM133D1 | TESGPVS SEQ ID NO:383 | NFGPE SEQ ID NO:5 | SQLRWTKGGV SEQ ID NO:657 | |
| EAM133B12 | TESGPVS SEQ ID NO:383 | NFGPE SEQ ID NO:5 | SNLRWTKGDV SEQ ID NO:615 | |
| EAM133E1 | TESGPVS SEQ ID NO:383 | NFGPE SEQ ID NO:5 | SNLRWTKGKV SEQ ID NO:626 | |
| EAM133H9 | TESGPVS SEQ ID NO:383 | NFGPE SEQ ID NO:5 | SALRWTKGKV SEQ ID NO:658 | |
| EAM133B5 | TESGP SEQ ID NO:279 | NFGPE SEQ ID NO:5 | SSLRWTKGKV SEQ ID NO:659 | |
| EAM133D5 | IESGPVT SEQ ID NO:380 | NFGPE SEQ ID NO:5 | SHLRWTKGKV SEQ ID NO:660 | S364T |
| EAM133B9 | TESGPVS SEQ ID NO:383 | NFGPE SEQ ID NO:5 | SQLRWTKGKG SEQ ID NO:661 | |
| EAM151_152C12 | WFLTADDS SEQ ID NO:384 | NGQPE SEQ ID NO:2 | QDDRWESGNV SEQ ID NO:562 | |
| EAM151_152C6 | SDSGPVS SEQ ID NO:404 | NLGPE SEQ ID NO:440 | SWLRWHSGNV SEQ ID NO:662 | |
| EAM151_152A1 | TGPVS SEQ ID NO:405 | NFGPE SEQ ID NO:5 | SWYRWHSGNV SEQ ID NO:587 | A378V |
| EAM151_152A7 | IDPGPVS SEQ ID NO:406 | NFGPE SEQ ID NO:5 | SHYRWISGNV SEQ ID NO:663 | A378V |
| EAM151_152A4 | TESGPVS SEQ ID NO:383 | NFGPE SEQ ID NO:5 | STYRWQSGNV SEQ ID NO:247 | A378V, |
| EAM151_152D9 | TESGPVT SEQ ID NO:277 | NFGPE SEQ ID NO:5 | STYRWQSGNV SEQ ID NO:247 | |
| EAM151_152A5 | TESGPVS SEQ ID NO:383 | NFGPE SEQ ID NO:5 | SVYRWMSGNV SEQ ID NO:248 | N389Y, K409R, Q438R |
| EAM151_152A10 | TESGPVS SEQ ID NO:383 | NFGPE SEQ ID NO:5 | SNLRWRAGRV SEQ ID NO:664 | Q438R |
| EAM151_152C11 | TESGPVS SEQ ID NO:383 | NFGPE SEQ ID NO:5 | SSLRWRSGRV SEQ ID NO:665 | |
| EAM151_152B11 | TESGPVS SEQ ID NO:383 | NFGPE SEQ ID NO:5 | SALRWTSGRV SEQ ID NO:666 | |
| EAM151_152A12 | TESGPVS SEQ ID NO:383 | NFGPE SEQ ID NO:5 | SALRWMSRKK SEQ ID NO:667 | |
| EAM151_152D8 | TESGPVS SEQ ID NO:383 | NFGPE SEQ ID NO:5 | SALRWSSGKV SEQ ID NO:668 | |
| EAM151_152B1 | TESGPVS SEQ ID NO:383 | NFGPE SEQ ID NO:5 | SSLRWASGKV SEQ ID NO:669 | |
| EAM151_152D3 | TESGPVS SEQ ID NO:383 | NLGPE SEQ ID NO:440 | STLRWSAGKV SEQ ID NO:670 | N389D |
| EAM151_152A2 | TESGPVS SEQ ID NO:383 | NLGPE SEQ ID NO:440 | SQLRWTSGKV SEQ ID NO:606 | Q438R |
| EAM151_152A11 | TESGPVS SEQ ID NO:383 | KFGPE SEQ ID NO:438 | SQLRWTKGDV SEQ ID NO:671 | Q438R |
| EAM151_152B4 | LESGPVS SEQ ID NO:273 | NFGPE SEQ ID NO:5 | SNLRWTKGKV SEQ ID NO:626 | A378V |
| EAM151_152D2 | TESGPVS SEQ ID NO:383 | DFGPE SEQ ID NO:442 | SSLRWTKGKV SEQ ID NO:659 | Q438R |
| EAM151_152A3 | TESGPVS SEQ ID NO:383 | NLGPE SEQ ID NO:440 | SQLRWTKGGV SEQ ID NO:657 | Q438R |
| EAM151_152A9 | TESGPVS SEQ ID NO:383 | NLGPE SEQ ID NO:440 | SSLRWTKGGV SEQ ID NO:629 | Q438R |
| EAM142batch1_D10 | SDAGPVS SDAGPVS:401 | NFGPE SEQ ID NO:5 | SWYRWHAGNV SEQ ID NO:588 | |
| EAM142batch1_A4 | TESGPVS SEQ ID NO:383 | NFGPE SEQ ID NO:5 | SWWRWYKGNV SEQ ID NO:672 | |
| EAM142batch2_G7 | THSGPVT SEQ ID NO:402 | DFGPE SEQ ID NO:442 | SWWRWYRGNV SEQ ID NO:632 | |
| EAM142batch1_A6 | TESGPVS SEQ ID NO:383 | NFGPE SEQ ID NO:5 | SWHRWFKGNV SEQ ID NO:673 | A378V |
| EAM142batch1_D7 | TESGPVS SEQ ID NO:383 | NFGPE SEQ ID NO:5 | STYRWMSGNV SEQ ID NO:580 | A378V |
| EAM142batch2_F7 | TESGPVS SEQ ID NO:383 | NFGPE SEQ ID NO:5 | SHWRWMRGNV SEQ ID NO:674 | A378V |
| EAM142batch1_B3 | TESGPVS SEQ ID NO:383 | NFGPE SEQ ID NO:5 | SNRRWMSGNV SEQ ID NO:591 | |
| EAM142batch2_F3 | TDSGPVS SEQ ID NO:265 | NFGPE SEQ ID NO:5 | SNRRWMSGNV SEQ ID NO:591 | A378V |
| EAM142batch1_A10 | TESGPVS SEQ ID NO:383 | NFGPE SEQ ID NO:5 | SVYRWQSGNV SEQ ID NO:569 | |
| EAM142batch1_D11 | TESGPVS SEQ ID NO:383 | NFGPE SEQ ID NO:5 | SVYRWQSGNV SEQ ID NO:569 | P352S |
| EAM142batch1_C8 | TESGPVS SEQ ID NO:383 | NFGPE SEQ ID NO:5 | SYYRWFSGNV SEQ ID NO:675 | |
| EAM142batch2_F1 | TESGPVS SEQ ID NO:383 | NFGPE SEQ ID NO:5 | SYYRWHKGNV SEQ ID NO:638 | |
| EAM142batch1_D8 | LASGPVS SEQ ID NO:407 | KFGPE SEQ ID NO:438 | SHLRWTNGNV SEQ ID NO:598 | |
| EGFR141-A3_orig | TESGPVS SEQ ID NO:383 | NFGPE SEQ ID NO:5 | SHLRWTNGNV SEQ ID NO:598 | |
| EAM142batch1_A2 | TESGPVS SEQ ID NO:383 | NFGPE SEQ ID NO:5 | SALRWLSGNV SEQ ID NO:634 | D356Y, A378V |
| EAM142batch1_A5 | TESGPVS SEQ ID NO:383 | NFGPE SEQ ID NO:5 | SALRWESGNV SEQ ID NO:576 | |
| EAM142batch1_D12 | TESGPVS SEQ ID NO:383 | NFGPE SEQ ID NO:5 | SSLRWASGNV SEQ ID NO:676 | |
| EAM142batch1_B9 | TESGPVS SEQ ID NO:383 | NFGPE SEQ ID NO:5 | SSLRWTSGRI SEQ ID NO:653 | |
| EAM142batch1_C10 | TESGPVS SEQ ID NO:383 | NFGPE SEQ ID NO:5 | SYLRWTSGRI SEQ ID NO:677 | |
| EAM142batch1_E8 | TESGPVS SEQ ID NO:383 | NFGPE SEQ ID NO:5 | SQLRWTAGRV SEQ ID NO:678 | |
| EAM142batch2_G1 | TESGPVS SEQ ID NO:383 | NFGPE SEQ ID NO:5 | STLRWTSGDV SEQ ID NO:679 | |
| EAM142batch1_A1 | TESGPVS SEQ ID NO:383 | NFGPE SEQ ID NO:5 | SNLRWTKGDV SEQ ID NO:615 | A378V |
| EAM142batch2_F12 | TESGPVS SEQ ID NO:383 | NFGPE SEQ ID NO:5 | SNLRWTKGYV SEQ ID NO:680 | |
| EAM142batch2_G12 | AESGPVS SEQ ID NO:408 | SFGPE SEQ ID NO:441 | SNLRWTKGSV SEQ ID NO:681 | |
| EAM142batch2_H8 | TESGPVS SEQ ID NO:383 | NFGPE SEQ ID NO:5 | SNLRWTKGKV SEQ ID NO:626 | |
| EAM142batch1_E12 | TESGPVS SEQ ID NO:383 | NFGPE SEQ ID NO:5 | SSLRWTKGSV SEQ ID NO:682 | |
| EAM142batch2_F2 | TESGPVS SEQ ID NO:383 | NFGPE SEQ ID NO:5 | SQLRWTKGSV SEQ ID NO:656 | |
| EAM142batch1_A9 | TESGPVS SEQ ID NO:383 | NFGPE SEQ ID NO:5 | SSLRWSKGGV SEQ ID NO:683 | |
| EAM142batch1_D4 | TESGPVS SEQ ID NO:383 | NFGPD SEQ ID NO:444 | SSLRWPKGGV SEQ ID NO:684 | 1377F, K392N, H429L, H433L |
| EAM142batch2_G2 | TESGPVS SEQ ID NO:383 | NFGPE SEQ ID NO:5 | SQRRWELGDV SEQ ID NO:641 | |
| EAM142batch2_H3 | TESGPVS SEQ ID NO:383 | NFGPE SEQ ID NO:5 | SQRRWSKGSV SEQ ID NO:642 | |
| EAM142batch1_B11 | TESGPVS SEQ ID NO:383 | NFGPE SEQ ID NO:5 | SQLRWDSGAW SEQ ID NO:685 | |
| EAM142batch1_A8 | TESGPVS SEQ ID NO:383 | NFGPE SEQ ID NO:5 | SQLRWASGKV SEQ ID NO:686 | |
| EAM142batch1_A12 | TESGPVS SEQ ID NO:383 | NLGPE SEQ ID NO:440 | SQLRWSSGKV SEQ ID NO:687 | Q438L |
| EAM142batch1_A7 | TESGPVS SEQ ID NO:383 | NFGPE SEQ ID NO:5 | SQLRWSSGKV SEQ ID NO:687 | |
| EGFR C1P4.1 G12 | LTKNQ SEQ ID NO:409 | NGQPE SEQ ID NO:2 | DKSRWQQ SEQ ID NO:688 | |
| EGFR C1P4.1 B11 | YEGEH SEQ ID NO:410 | NGQPE SEQ ID NO:2 | DKSRWQQ SEQ ID NO:688 | |
| EGFR C1 P4.1 B2 | SWGWN SEQ ID NO:411 | NGQPE SEQ ID NO:2 | DKSRWQQ SEQ ID NO:688 | |
| EGFR C1P4.1 F10 | LSCNN SEQ ID NO:412 | NGQPE SEQ ID NO:2 | DKSRWQQ SEQ ID NO:688 | |
| EGFR C1P4.1 A12 | LTKNQ SEQ ID NO:409 | NGQPE SEQ ID NO:2 | PSKQGSF SEQ ID NO:689 | |
| EGFR C1 P4.1 E7 | LTKNQ SEQ ID NO:409 | NGQPE SEQ ID NO:2 | PSNAQEF SEQ ID NO:690 | |
| EGFR C1 P4.1 A2 | LTKNQ SEQ ID NO:409 | NGQPE SEQ ID NO:2 | PAYNYIH SEQ ID NO:691 | |
| EGFR C1P4.1 D2 | LTKNQ SEQ ID NO:409 | NGQPE SEQ ID NO:2 | PPAATSH SEQ ID NO:692 | |
| EGFR C1 P4.1 F4 | LTKNQ SEQ ID NO:409 | NGQPE SEQ ID NO:2 | PLALPDM SEQ ID NO:693 | |
| EGFR C1 P4.1 B6 | LTKNQ SEQ ID NO:409 | NGQPE SEQ ID NO:2 | PNDFHSV SEQ ID NO:694 | |
| EGFR C1P4.1 F2 | LTKNQ SEQ ID NO:409 | NGQPE SEQ ID NO:2 | YLDHGHP SEQ ID NO:695 | |
| EGFR C1P4.1 C4 | LTKNQ SEQ ID NO:409 | NGQPE SEQ ID NO:2 | STDKVWA SEQ ID NO:696 | |
| EGFR C1 P4.1 F6 | LTKNQ SEQ ID NO:409 | NGQPE SEQ ID NO:2 | GYGKVGQ SEQ ID NO:697 | |
| EGFR C1P4.1 F9 | LTKNQ SEQ ID NO:409 | NGQPE SEQ ID NO:2 | DNVKEYK SEQ ID NO:698 | |
| EGFR C1P4.1 D11 | LTKNQ SEQ ID NO:409 | NGQPE SEQ ID NO:2 | SNNQDTT SEQ ID NO:699 | |
| EGFR C1 P4.1 H2 | LTKNQ SEQ ID NO:409 | NGQPE SEQ ID NO:2 | SSPYMNG SEQ ID NO:700 | |
| EGFR C1 P4.1 E11 | LTKNQ SEQ ID NO:409 | NGQPE SEQ ID NO:2 | VGYSLRQ SEQ ID NO:701 | |
| EGFR C1 P4.1 F3 | LTKNQ SEQ ID NO:409 | NGQPE SEQ ID NO:2 | PKMYSMA SEQ ID NO:702 | |
| EGFR C1P4.1 G6 | LTKNQ SEQ ID NO:409 | NGQPE SEQ ID NO:2 | DSGRWLG SEQ ID NO:703 | |
| EGFR C1P4.1 A7 | KKKKK SEQ ID NO:413 | NASFE SEQ ID NO:445 | DRERWRR SEQ ID NO:704 | |
| EGFR C1P4.1 E8 | KKKKK SEQ ID NO:413 | NASFE SEQ ID NO:445 | DRERWRR SEQ ID NO:704 | |
| EGFR C1 P4.1 A9 | LTKNQ SEQ ID NO:409 | NGQPE SEQ ID NO:2 | IQDRWQQ SEQ ID NO:705 | |
| EGFR C1P4.1 B5 | YESKS SEQ ID NO:410 | NGQPE SEQ ID NO:2 | SWMEWSQ SEQ ID NO:706 | |
| EGFR C1P4.1 C1 | RFSNH SEQ ID N0:411 | NGQPE SEQ ID NO:2 | MQDQWWQ SEQ ID NO:707 | |
| EGFR C1P4.1 A10 | VQQGP SEQ ID NO:412 | TYGPE SEQ ID NO:447 | SEMRWKR SEQ ID NO:708 | |
| EGFR C1 P4.1 A3 | TESGP SEQ ID NO:413 | NFGPE SEQ ID NO:5 | SHLRWTN SEQ ID NO:709 | |
| EGFR C1 P4.1 A5 | WLMTN SEQ ID NO:414 | NFGPE SEQ ID NO:5 | DRIRWPA SEQ ID NO:710 | |
| EGFR C1P4.1 E3 | QRGRM SEQ ID NO:415 | KGGRE SEQ ID NO:448 | SRARWRH SEQ ID NO:711 | |
| EGFR C1P4.1 F7 | YDSDY SEQ ID N0:416 | PVPAH SEQ ID NO:449 | ASQRWRN SEQ ID NO:712 | |
| EGFR C1 P4.1 B7 | PSENY SEQ ID NO:417 | NGQPE SEQ ID NO:2 | NEHRWTM SEQ ID NO:713 | |
| EGFR C1P4.1 B9 | IDNL SEQ ID NO:418 | NMGPE SEQ ID NO:450 | VENRWTM SEQ ID NO:714 | |
| EGFR C1P4.1 B4 | AAEWM SEQ ID NO:419 | NGQPE SEQ ID NO:2 | QHNRWES SEQ ID NO:715 | |
| EGFR C1 P4.1 C6 | EHNGP SEQ ID NO:420 | NGQPE SEQ ID NO:2 | SNRRWMS SEQ ID NO:716 | |
| EGFR C1P4.1 F12 | FSEGP SEQ ID NO:421 | NGQPE SEQ ID NO:2 | SNMRWTS SEQ ID NO:717 | |
| EGFR C1P4.1 D4 | VNHKI SEQ ID NO:422 | NGQPE SEQ ID NO:2 | KYDRWTA SEQ ID NO:718 | |
| EGFR C1P4.1 F1 | SYAED SEQ ID NO:423 | NGQPE SEQ ID NO:2 | AEDRWMN SEQ ID NO:719 | |
| EGFR C1P4.1 A1 | ESDHG SEQ ID NO:424 | NGQPE SEQ ID NO:2 | VKSRWQA SEQ ID NO:720 | |
| EGFR C1 P4.1 C9 | GLGLW SEQ ID NO:425 | NGQPE SEQ ID NO:2 | EKSRWLT SEQ ID NO:721 | |
| EGFR C1 P4.1 C3 | ERTE SEQ ID NO:426 | NGQPE SEQ ID NO:2 | MKDRWHG SEQ ID NO:722 | |
| EGFR C1P4.1 A11 | YNRGA SEQ ID NO:427 | NGQPE SEQ ID NO:2 | PKNMWWN SEQ ID NO:723 | |
| EGFR C1P4.1 C10 | GRRGR SEQ ID NO:428 | NGQPE SEQ ID NO:2 | DSQRWVQ SEQ ID NO:724 | |
| EGFR C1P4.1 G9 | GRRGR SEQ ID NO:428 | NGQPE SEQ ID NO:2 | PWKRKSK SEQ ID NO:725 | |
| EGFR C1P4.1 B1 | EVHDV SEQ ID NO:429 | NGQPE SEQ ID NO:2 | PWKRKSK SEQ ID NO:725 | |
| EGFR C1 P4.1 G7 | EEEYV SEQ ID NO:430 | NGQPE SEQ ID NO:2 | PHARWVP SEQ ID NO:726 | |
| EGFR C1P4.1 C12 | WLKGV SEQ ID NO:431 | NGQPE SEQ ID NO:2 | PSWRWYY SEQ ID NO:727 | |
| EGFR C1P4.1 D5 | VGGMG SEQ ID NO:432 | NGQPE SEQ ID NO:2 | PLPLPPP SEQ ID NO:728 | |
| EGFR C1P4.2 B11 | SFVCC SEQ ID NO:433 | NGQPE SEQ ID NO:2 | DKSRWQQ SEQ ID NO:688 | |
| EGFR C1 P4.2 G7 | CFSLS SEQ ID NO:434 | NGQPE SEQ ID NO:2 | DKSRWQQ SEQ ID NO:688 | |
| EGFR C1 P4.2 A1 | SNNTS SEQ ID NO:435 | NYTTI SEQ ID NO:451 | SSQRWLE SEQ ID NO:729 | |
| EGFR C1 P4.2 E4 | SSESD SEQ ID NO:436 | KNWQA SEQ ID NO:452 | DRERWRR SEQ ID NO:704 | |
| EGFR C1P4.2 A11 | TESGP SEQ ID NO:135 | NFGPE SEQ ID NO:5 | SHLRWTN SEQ ID NO:6 | |
| EGFR C1P4.2 A12 | VQQGP SEQ ID NO:412 | TYGPE SEQ ID NO:447 | SEMRWNG SEQ ID NO:730 | |
| EGFR C1 P4.2 C10 | VQQGP SEQ ID NO:412 | TYGPE SEQ ID NO:447 | SEMRWKR SEQ ID NO:708 | |
| EGFR C1 P4.2 B3 | EHNGP SEQ ID NO:420 | NGQPE SEQ ID NO:2 | SNRRWMS SEQ ID NO:716 | |
| EGFR C1 P4.2 E10 | FSEGP SEQ ID NO:421 | NGQPE SEQ ID NO:2 | SNMRWTS SEQ ID NO:717 | |
| EGFR C1 P4.2 E8 | THVGS SEQ ID NO:437 | NGQPE SEQ ID NO:2 | DDYRWHI SEQ ID NO:731 | |
| EGFR C1 P4.2 F2 | HRGRG SEQ ID NO:438 | NGQPE SEQ ID NO:2 | PENRWHR SEQ ID NO:732 | |
| EGFR C1 P4.2 G12 | VHAAT SEQ ID NO:439 | NGQPE SEQ ID NO:2 | SYNRWNR SEQ ID NO:733 | |
| EGFR C1 P4.2 B1 | LNHSF SEQ ID NO:440 | NGQPE SEQ ID NO:2 | LGNRWKS SEQ ID NO:734 | |
| EGFR C1 P4.2 C12 | YRNFD SEQ ID NO:441 | NGQPE SEQ ID NO:2 | PHDDWWS SEQ ID NO:735 | |
| EGFR C1 P4.2 D9 | TTNLF SEQ ID NO:442 | NGQPE SEQ ID NO:2 | PSVRWGG SEQ ID NO:736 | |
| EGFR C1 P4.2 D10 | GRRGE SEQ ID NO:443 | NGQPE SEQ ID NO:2 | PWKRKSK SEQ ID NO:737 | |
| EGFR C1 P4.2 D2 | WLKGV SEQ ID NO:431 | NGQPE SEQ ID NO:2 | PSWRWYY SEQ ID NO:738 | |
| EGFR C1 P4.2 A6 | LTKNQ SEQ ID NO:1 | NGQPE SEQ ID NO:2 | PHSWWLV SEQ ID NO:739 | |
| EGFRC1P4.2A10 | LTKNQ SEQ ID NO:1 | NGQPE SEQ ID NO:2 | PFEAFQQ SEQ ID NO:740 | |
| EGFR C1 P4.2 A2 | LTKNQ SEQ ID NO:1 | NGQPE SEQ ID NO:2 | PSNAQEF SEQ ID NO:690 | |
| EGFR C1 P4.2 C1 | LTKNQ SEQ ID NO:1 | NGQPE SEQ ID NO:2 | PWIMDKH SEQ ID NO:741 | |
| EGFR C1 P4.2 G2 | LTKNQ SEQ ID NO:1 | NGQPE SEQ ID NO:2 | PPPSPPL SEQ ID NO:742 | |
| EGFR C1 P4.2 G5 | LTKNQ SEQ ID NO:1 | NGQPE SEQ ID NO:2 | PGYMVPL SEQ ID NO:743 | |
| EGFR C1P4.2B5 | LTKNQ SEQ ID NO:1 | NGQPE SEQ ID NO:2 | DRQHHND SEQ ID NO:744 | |
| EGFR C1 P4.2 D5 | LTKNQ SEQ ID NO:1 | NGQPE SEQ ID NO:2 | DAPQPVE SEQ ID NO:745 | |
| EGFR C1 P4.2 C9 | LTKNQ SEQ ID NO:1 | NGQPE SEQ ID NO:2 | STDKVWA SEQ ID NO:696 | |
| EGFR C1 P4.2 D7 | LTKNQ SEQ ID NO:1 | NGQPE SEQ ID NO:2 | YEVTEEP SEQ ID NO:746 | |

### Expression and purification of EGFR specific Fcab clones in mammalian cells:

Clones selected as described above with characteristics as described above are cloned into a mammalian expression vector such as pCEP4 (Invitrogen) as a Kpnl/BamHl fragment. The resulting Fcab expression plasmids contain an open reading frame comprising the last 6 amino acids of the hinge region, the CH2 domain and the antigen binding CH3 domain. To facilitate further cloning steps, a Xhol restriction site is introduced at the CH2/CH3 domain junction. Highly purified plasmid DNA (Qiagen) is used to transiently transfect HEK293 freestyle cells with Freestyle™ MAX Reagent as recommended by the manufacturer (Invitrogen). On day 5 post transfection, cell supernatants are cleared from cell debris by centrifugation and filtration through a 0.2µM Stericup filter (Millipore). Alternatively, HEK293 freestyle cells or CHO cells are transfected with expression plasmids containing genes for antibiotics resistance such as neomycin or puromycin. The transfected cells are cultivated in the presence of the antibiotics resulting in specific survival of cell clones which stably express the antibiotics resistance gene together with the antigen specific Fc fragment. Such stable transfectants consistently secrete the protein of interest over long time periods. The antigen specific Fcabs are purified from cell supernatants by Protein A immuno-affinity chromatography. Bound Fcabs are eluted from Protein A by washing the column with glycine buffer (pH=2.9-4.0), followed by dialysis against PBS (pH=6.8). The purity of the Fcabs is determined by non-reducing SDS-PAGE analysis and potential aggregates are detected by size-exclusion HPLC using a Zorbax GF250 column and PBS as running buffer.

### Example 2: Binding affinities of EGFR specific Fcabs

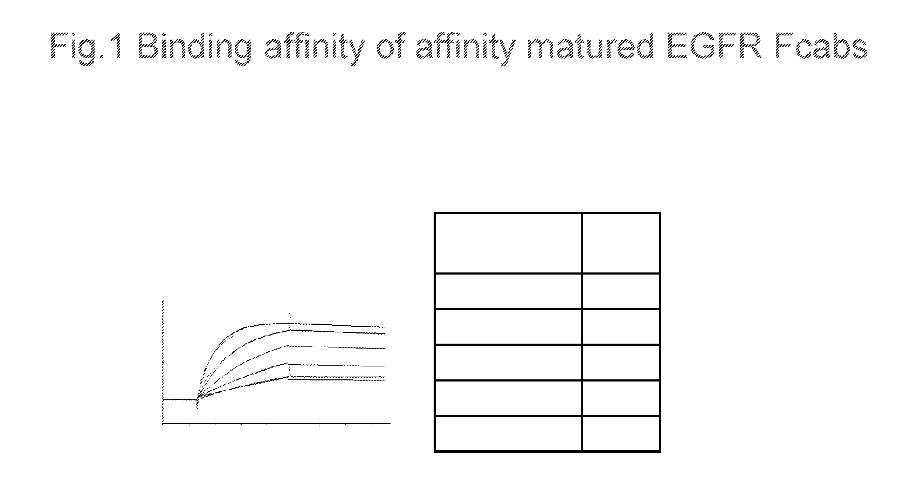

The binding affinity of human EGFR specific Fcabs is determined by surface plasmon resonance (SPR) assays in a Biacore instrument. CM-5 chips are coated with increasing concentrations of recombinant soluble EGFR protein. Afterwards, increasing concentrations of Fcabs (0.8-25µg/ml) are injected on each coated chip until binding equilibrium to EGFR is reached. Then, buffer is injected to measure the off-rate of the binding reaction. The binding affinity (K_{D}) is calculated using the BiaEval software using a 1:1 stochiometry model. These experiments indicate that EGFR specific Fcabs bind to recombinant EGFR between 0.6-15nM. (Fig. 1).

### Example 3: Engineering bi-specific antibodies (mAb²) which recognize tumor associated antigens (sTAG) RANKL or VEGF and EGFR

The monoclonal antibodies bevacizumab (AV) and denosumab (DN) recognize the cytokines VEGF and RANKL, respectively. Both antibodies are clinically approved for the treatment of certain forms of tumors. Both antibodies exert their anti-tumor effects by neutralizing the biological function of their cytokine targets by blocking the interaction of the cytokines to their respective receptor. The gene sequences encoding the VH domains of mAbs bevacizumab and denosumab are synthesized by a commercial source as Kpnl/Nhel fragments. The corresponding VL sequences are synthesized as Kpnl/Kasl fragments. The DNA fragments are ligated into two mammalian expression plasmids based on the pCEP4 vector (Invitrogen). One of the pCEP4 plasmids contains the complete heavy chain gene of the OKT3 monoclonal antibody (human IgG1 isotype) (Adair JR, Athwal DS, Bodmer MW, Bright SM, Collins AM, Pulito VL, Rao PE, Reedman R, Rothermel AL, Xu D, et al. Hum Antibodies Hybridomas. 1994;5(1-2):41-7) cloned as a Kpnl/BamHl fragment. The second pCEP4 expression vector encodes the complete light chain gene of the OKT3 antibody cloned as a Kpnl/BamHl fragment. To facilitate cloning of individual antibody domains derived from other antibodies, unique restriction enzyme cleavage sites are introduced in frame at the junctions between the OKT3 VH and CH1 domains (Nhel), between VL and CL (Kasl) and between the CH2 and CH3 domains of the heavy chain (Xhol). Therefore, replacement of the Kpnl/Nhel OKT3 VH gene segment with the VH gene fragments of the bevacizumab and denosumab antibodies regenerate complete human IgG1 heavy chains. Similarly, the OKT3 VL gene segment can be excised as a Kpnl/Kasl fragment and replaced with the VL segments of bevacizumab and denosumab resulting in regeneration of a complete light chain.

For the cloning of bevacizumab and denosumab derived mAb² expression constructs, a Xhol/BamHl fragment containing the wild type CH3 domain is replaced with the CH3 domain of EGFR specific Fcab EAM151-5 (see Table 1).

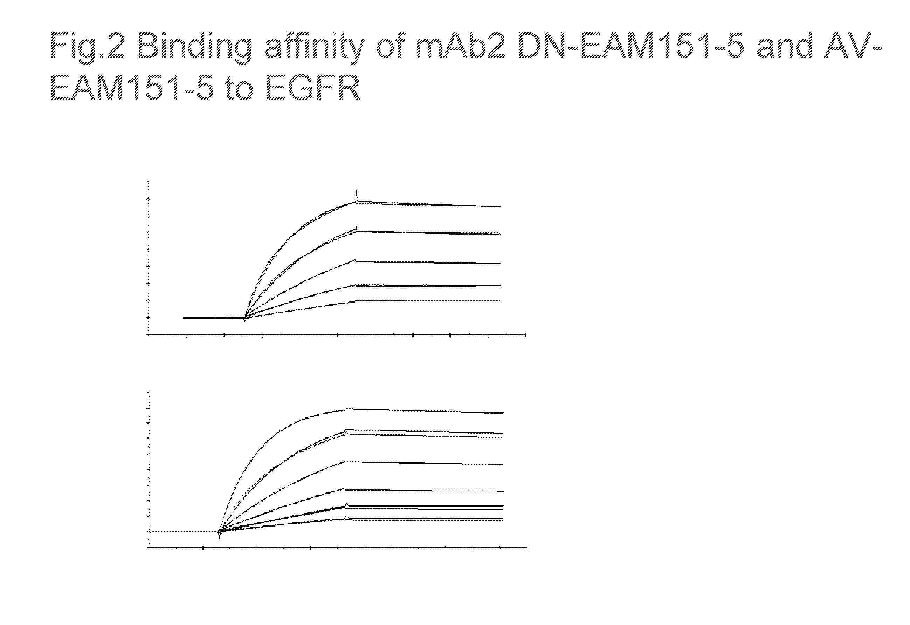

EGFR binding of the resulting bi-specific antibodies (mAb²) AV-EAM151-5 (VEGF/EGFR) and DN-EAM151-5 (RANKL/EGFR) is indistinguishable to Fcab EAM151-5 (Fig. 2).

### Example 4: Competition of EGFR Fcabs with EGF for receptor binding

The functionality of EGFR Fcabs and mAb² containing EGFR binding sites are tested in a competition assay in which binding of epidermal growth factor (EGF) to its receptor is measured in the presence of the antibodies. Increasing concentrations of antibodies are incubated with EGFR-positive MDA-MB468 cells for 30 minutes on ice. Then, a constant amount of 6.45nM biotinylated EGF is added and the mixture is incubated for another 60 minutes on ice. Receptor bound biotinylated EGF is measured by flow cytometry after addition of streptavidin coupled to the fluorescent dye phycoerythrin. The data demonstrate that EGFR specific Fcabs compete with EGF for binding to the EGFR with potencies in the single digit nM range. This functionality is not compromised when the mAb² AV-EAM151-5 and DN-EAM151-5 are used as competitors (Fig. 3).

### Example 5: EGFR specific Fcabs inhibit EGFR signal transduction

In order to assess if binding of EGFR specific Fcabs will result in abrogation of signalling through the EGFR, SKBR3 cells are stimulated with 0.4nM EGF for 10 minutes and the resulting tyrosine phosphorylation of the MAP kinase erk1/2 is measured by immuno-blotting with an antibody specific for the phosphorylated form of erk1/2. Increasing concentrations of EGFR Fcab EAM151-5 lead to gradual loss of phospho-erk1/2 while Fcab wild type has no effect. Cetuximab, used as positive control, also inhibits EGF induced erk1/2 activation. To test, if the EGFR specific Fcabs would act as signalling agonists similar to EGF, cells are stimulated with Fcabs or cetuximab in the absence of EGF. Fcab EAM151-5 does not induce phosphorylation of erk1/2 indicating that EGFR Fcabs do not trigger signal transduction via EGFR (Fig. 4). Similar results are obtained when EAM151-5 containing bevacizumab or denosomab bi-specific antibodies are tested.

### Example 6: Effector functionality of EGFR Fcabs and mAb²

To determine, if EGFR specific Fcabs are able to kill EGFR-positive target cells by antibody dependent cellular cytotoxicity (ADCC), SKBR3 cells are incubated with increasing concentrations of Fcabs for 15 minutes. Freshly prepared human natural killer (NK) cells are added at a cell ratio of NK:SKBR3=5:1 and the cell mixture is incubated for 4 hours at 37°C. Dying SKBR3 cells are enumerated by flow cytometry after addition of 7-amino-actinomycin D. The data shown in fig.5 demonstrate that EGFR specific Fcabs are able to mediate NK-cell dependent death of SKBR3 target cells with potencies in the single digit nM range with different NK cell donors. Similar experiments are carried out with the bi-specific antibodies AV-EAM151-5 and DN-EAM151-5. While bevacizumab antibodies do not promote ADCC activities, bevacizumab-based mAb² AV-EAM151-5 clearly kills SKBR3 targets by this effector mechanism. Similar data are obtained with the denosumab-EAM151-5 mAb² (DN-EAM151-5). The ADCC activity of DN-EAM151-5 is significantly reduced in the presence of excess RANKL protein (Fig. 6). Similar effects are abserved with AV-EAM151-5 (not shown). This phenomenon is most likely due to internalization of the mAb² into cells (see below).

### Example 7: Internalization of denosumab- and bevacizumab-containing EAM151-5 mAb²

One possibility to explain the loss of ADCC potency of the mAb² proteins in the presence of the sTAG RANKL or VEGF is that the binding of the soluble target to the cell bound mAb² may induce EGFR crosslinking followed by internalisation of the mAb² /sTAG complex. To test for this possibility, EGFR-positive A431 cells are loaded with DN-EAM151-5 or AV-EAM151-5 on ice for 90 minutes. Excess mAb² is washed away and the cells are incubated further in the absence or presence of RANKL or VEGF labelled with the fluorescent dye AlexaFluor488. Incubation is carried out for 90 minutes on ice (non-internalising conditions) or at 37°C (internalising conditions). Then, cell surface bound cytokine/ mAb² complexes are removed by a short treatment with acetic acid and cells are analyzed by flow cytometry. The results show that a significant percentage of the fluorescent RANKL or VEGF signal is resistant to acid treatment at 37°C compared to cells incubated on ice indicating that the cytokines are internalized as consequence of EGFR cross-linking by the cytokine bound mAb² (Fig. 7). These data are corroborated by confocal microscopy experiments. Incubation of DN-EAM151-5 loaded A431 cells for 3 hours with fluorescently labelled RANKL on ice reveals predominant fluorescence at the cell surface. In contrast, incubation at 37°C results in a punctate, speckled fluorescence indicative of accumulation of RANKL in intracellular organelles. The same cells are treated with LysotrackerRed which accumulates preferentially in endosomes giving a red fluorescence signal. Merging the green and the red florescence signals reveals significant overlap indicating localization of internalised RANKL to the endosomal compartment.

### Example 8: Tumor cell killing induced by cross-linking of cell bound mAb² with sTAG

EGFR-positive MDA-MB468 tumor cells are incubated for 4 hours with 10µg/ml Fcab EAM151-5 or mAb² AV-EAM151-5 in the presence of increasing concentrations of human VEGF. Then, dead cells are enumerated by flow cytometry after addition of 7-amino-actinomycin D. The results shown in Fig. 8 demonstrate that addition of VEGF leads to cell killing in a dose dependent fashion. This effect is specific since only cells treated with mAb² AV-EAM151-5 are killed but not cells incubated with EAM151-5 or a control antibody. Similar data are seen when another EGFR-positive cell line, A431, is cultured with mAb² DN-EAM151-5 or Fcab EAM151-5 in the presence of RANKL. Again, specific cell killing is only observed in cultures treated with DN-EAM151-5 and RANKL. It is speculated that cross-linking of cell bound mAb² with sTAGs RANKL or VEGF leads to internalisation of the sTAG/ mAb² complex eventually initiating a cell death promoting signal.

## Claims

1. An internalising modular antibody based on one or more immunoglobulin domains, which comprises at least one binding structure located within the N-terminal portion of a domain, and comprises at least one binding structure acting as a binding site located within the non-CDR region of a domain, wherein
a) a first binding structure has a specificity to bind an extra-cellular portion of a receptor on a tumor cell, and
b) a second binding structure has a specificity to bind a soluble tumor associated antigen (sTAG).

2. The modular antibody of claim 1, wherein a binding structure is derived from CDR loops of an antibody, or from a receptor polypeptide.

3. The modular antibody according to claim 1 or 2, wherein the first binding structure a) is located within the Fc, preferably within the CH3 and/or CH2 domain.

4. The modular antibody according to any of claims 1 to 3, wherein the receptor is selected from the group consisting of EGFR, HER2, HER3, HER4, Insulin growth factor receptor I, c-met, a receptor having a Lewis Y or Thomsen-Friedenreich glycosylation binding structure, DR4, DR5, DcR1, DcR2, IL-17 receptor, IL-23 receptor, IL-12 receptor and Interferon-alpha receptor.

5. The modular antibody according to any of claims 1 to 4, wherein the receptor is an erbB receptor.

6. The modular antibody according to any of claims 1 to 5, which has a binding affinity to bind the tumor cell and/or the sTAG with a KD of less than 100 nM.

7. The modular antibody according to any of claims 1 to 5, which has direct cytotoxic activity as measured in a standard DNA fragmentation assay.

8. The modular antibody according to any of claims 1 to 7, which has a cytotoxic activity as measured in a standard ADCC or CDC assay.

9. The modular antibody according to any of claims 1 to 8, wherein the sTAG is selected from the group consisting of VEGF, RANKL, EGF, APRIL, TWEAK, TNFSF3, CD27L, 4-1 BBL, LIGHT, VEGI, CD30L, AITRL, TNF-alpha, OX40L, FasL, EDA, CD40L, Blys, IFN-alpha, I L-1-beta, IL-6, IL-13, IL-17A, IL-17E, IL-12, IL-23, GM-CSF, PIGF, lymphotoxin and TRAIL.

10. The modular antibody according to any of claims 1 to 9, which binds to an EGFR epitope specifically recognised by the EAM151-5 Fcab, preferably wherein said first binding structure a) binds to such epitope.

11. The modular antibody according to any of claims 1 to 10, wherein said binding structure comprises any of the EF loop sequences, and optionally a further binding loop structure comprising any of the AB and/or CD loop sequences, as listed in Table 1.

12. The modular antibody according to claim 11, wherein the binding structure is located within a CH3 domain.

13. An Fc fragment of an immunoglobulin, which binds to an EGFR epitope and comprises a binding loop structure comprising any of the EF loop sequences, and optionally a further binding loop structure comprising any of the AB and/or CD loop sequences, as listed in Table 1.

14. An Fc fragment according to claim 13, wherein said EGFR epitope is specifically recognised by the EAM151-5 Fcab.

15. The Fc fragment according to claim 14, which is derived from EAM151, EAM141, EAM142 clones, and functional variants thereof.

16. Modular antibody comprising an Fc fragment according to any of claims 13 to 15.

17. The modular antibody of any of claims 1 to 16 for use in treating a malignant neoplasm or autoimmune disorder.
